(19) Europäisches Patentamt
European Patent Office
Office européen des brevets

(11) EP 3 827 682 A1

(12) EUROPEAN PATENT APPLICATION
published in accordance with Art. 153(4) EPC

(43) Date of publication:
02.06.2021 Bulletin 2021/22

(51) Int Cl.:
A41G 3/00 (2006.01)

(21) Application number: 19840720.7

(86) International application number:
PCT/JP2019/029077

(22) Date of filing: 24.07.2019

(87) International publication number:
WO 2020/022395 (30.01.2020 Gazette 2020/05)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
KH MA MD TN

(30) Priority: 25.07.2018 JP 2018139563

(71) Applicants:
• Spiber Inc.
Tsuruoka-shi, Yamagata 997-0052 (JP)

• Kojima Industries Corporation
Toyota-shi, Aichi 471-8588 (JP)

(72) Inventors:
• MATSUO, Masato
Tsuruoka-shi, Yamagata 997-0052 (JP)
• ISHII, Hideto
Tsuruoka-shi, Yamagata 997-0052 (JP)
• ABE, Yunosuke
Tsuruoka-shi, Yamagata 997-0052 (JP)

(74) Representative: Handley, Matthew Edward
Venner Shipley LLP
200 Aldersgate
London EC1A 4HD (GB)

(54) ARTIFICIAL HAIR FIBER, METHOD FOR MANUFACTURING SAME, AND ARTIFICIAL HAIR

(57) The present invention pertains to an artificial hair fiber that includes an artificial fibroin fiber containing a modified fibroin and that expands when being in a wet state and shrinks when being dried after the wet state.

## Fig.7

EP 3 827 682 A1

## Description

## Technical Field

The present invention relates to artificial hair fiber, a method for manufacturing the same, and artificial hair.

## Background Art

In addition to human hair, artificial hair made of synthetic fibers or the like is used as a material for wigs, hair attachments, or the like (for example, Patent Literature 1).

## Citation List

## Patent Literature

[Patent Literature 1] Japanese Unexamined Patent Publication No. 2007-297737

## Summary of Invention

## Technical Problem

Human hair is preferably used in terms of texture and the like. However, since the hair quality (a thickness, a hardness, and the like) of human hair varies greatly according to race, gender, individual differences, and the like, it is difficult to stably obtain uniform quality hair. In addition, in recent years, as the number of human hair sellers has decreased, it has become difficult to obtain a large amount of human hair.

On the other hand, artificial hair fibers made of synthetic fibers such as polyester fibers and acrylic fibers and having a desired length can be stably obtained in a large amount with uniform quality. However, synthetic fibers have a significantly different texture as compared with human hair, and also have a large variation in characteristics. For example, human hair has a property in which it elongates to a predetermined length when it absorbs water and then returns to its original length when it dries, whereas synthetic fibers do not have such a property. Therefore, when artificial hair fibers made of synthetic fibers are used, it is inevitable that a feeling of strangeness compared with human hair will occur.

An object of the present invention is to provide an artificial hair fibers capable of being stably supplied and with which occurrence of a feeling of strangeness in comparison with human hair is curbed.

## Solution to Problem

In the process of various studies by the inventors, it has been found that an artificial fibroin fiber including a modified fibroin expands and contracts in the same manner as human hair when it is wet and dried. Then, as a result of repeated diligent research based on such findings, the present invention has been completed.

The present invention relates to, for example, the following inventions.

[1] There is provided an artificial hair fiber including an artificial fibroin fiber containing a modified fibroin, wherein the artificial hair fiber expands when being in a wet state and contracts when being dried from the wet state.

[2] In the artificial hair fiber described in [1], a restoration rate defined by the following Formula (1) may be 95% or more.

$$\text{Restoration rate} = (\text{length of artificial fibroin fiber when dried from wet state}/\text{length of artificial fibroin fiber before wet state}) \times 100 \ (\%) \dots (1)$$

[3] In the artificial hair fiber described in [1] or [2], the artificial fibroin fiber may be a fiber having a contraction history in which the fiber is irreversibly contracted by contact with water after spinning, and a contraction rate A defined by the following Formula (2) may be 2% or more.

$$\text{Contraction rate A} = \{1 - (\text{length of fiber irreversibly contracted by contact with water after spinning/length of fiber after spinning and before contact with water})\} \times 100 \ (\%)...(2)$$

[4] In the artificial hair fiber described in any one of [1] to [3], the artificial fibroin fiber may be a fiber having a contraction history in which the fiber is irreversibly contracted by contact with water after spinning and then further contracted by drying, and a contraction rate B defined by the following Formula (3) may be more than 7%.

$$\text{Contraction rate B} = \{1 - (\text{length of fiber irreversibly contracted by contact with water after spinning and then further contracted by drying/length of fiber after spinning and before contact with water})\} \times 100 \ (\%)...(3)$$

[5] In the artificial hair fiber described in any one of [1] to [4], the modified fibroin may be a modified spider silk fibroin.
[6] In the artificial hair fiber described in any one of [1] to [5], a recess which extends in a fiber axis direction may be provided in a surface.
[7] In the artificial hair fiber described in any one of [1] to [6], an expansion rate defined by the following Formula (4) may be 17% or less.

$$\text{Expansion rate} = \{(\text{length of artificial fibroin fiber in wet state/length of artificial fibroin fiber before wet state}) - 1\} \times 100 \ (\%)...(4)$$

[8] In the artificial hair fiber described in any one of [1] to [7], a contraction rate C defined by the following Formula (5) may be 17% or less.

$$\text{Contraction rate C} = \{1 - (\text{length of artificial fibroin fiber when dried from wet state/length of artificial fibroin fiber in wet state}) \times 100 \ (\%)...(5)$$

[9] In the artificial hair fiber described in any one of [1] to [8], a heat contraction rate defined by the following Formula (6) may be 4% or less.

$$\text{Heat contraction rate} = \{1 - (\text{length of artificial fibroin fiber when heated to } 160°C/\text{length of artificial fibroin fiber before heating})\} \times 100 \ (\%)...(6)$$

[10] In the artificial hair fiber described in any one of [1] to [9], the artificial hair fiber may contain substantially no residual stress generated by drawing in a spinning process.
[11] There is provided a method for manufacturing an artificial hair fiber, the method including a contraction step in which a raw material fiber after spinning and before contact with water is brought into contact with water to be irreversibly contracted, then dried and further contracted, wherein the raw material fiber contains a modified fibroin.
[12] In the method described in [11], the raw material fiber may be a fiber having a contraction rate A of 2% or more defined by the following Formula (2).

$$\text{Contraction rate A} = \{1\text{-(length of fiber irreversibly contracted by contact with water after spinning/length of fiber after spinning and before contact with water)}\} \times 100 \ (\%)...(2)$$

[13] In the method described in [11] or [12], the raw material fiber may be a fiber having a contraction rate B of more than 7% defined by the following Formula (3).

$$\text{Contraction rate B} = \{1\text{-(length of fiber irreversibly contracted by contact with water after spinning and then further contracted by drying/length of fiber after spinning and before contact with water)}\} \times 100 \ (\%)...(3)$$

[14] In the method described in any one of [11] to [13], in the contraction step, substantially all residual stress in the raw material fiber generated by drawing in a spinning process may be released.

[15] In the method described in any one of [11] to [14], the contraction step may be performed without relaxing the raw material fiber.

[16] In the method described in any one of [11] to [15], the raw material fiber may be formed by introducing a spinning dope solution containing the modified fibroin and a solvent into a coagulating solution, removing the solvent from the spinning dope solution and coagulating the spinning dope solution.

[17] In the method described in any one of [11] to [16], the modified fibroin may be a modified spider silk fibroin.

[18] There is provided an artificial hair including the artificial hair fiber described in any one of [1] to [10].

**Advantageous Effects of Invention**

According to the present invention, it is possible to provide an artificial hair fiber which can be stably supplied and can curb occurrence of a feeling of strangeness with human hair.

**Brief Description of Drawings**

FIG. 1 is a schematic diagram showing an example of a domain sequence of a modified fibroin.

FIG. 2 is a diagram showing a distribution of values of z/w (%) in a naturally occurring fibroin.

FIG. 3 is a diagram showing a distribution of values of x/y (%) in the naturally occurring fibroin.

FIG. 4 is a schematic diagram showing an example of the domain sequence of the modified fibroin.

FIG. 5 is a schematic diagram showing an example of the domain sequence of the modified fibroin.

FIG. 6 is an explanatory diagram schematically showing an example of a spinning apparatus for producing raw material fibers.

FIG. 7 is a diagram showing an example of a change in a length of the raw material fiber (fiber including the modified fibroin) due to contact with water.

FIG. 8 is an explanatory diagram schematically showing an example of a manufacturing apparatus for manufacturing artificial hair fibers (artificial fibroin fibers).

FIG. 9 is an explanatory diagram schematically showing an example of the manufacturing apparatus for manufacturing artificial hair fibers (artificial fibroin fibers).

FIG. 10 is a scanning electron microscope (SEM) photograph of a surface structure (a skin layer) and an internal structure (a cutting surface) of artificial hair fibers (the artificial fibroin fibers) according to one embodiment.

FIG. 11 is a diagram showing results of measuring a heat contraction rate of the artificial hair fibers (the artificial fibroin fibers) according to one embodiment.

**Description of Embodiments**

Hereinafter, embodiments for carrying out the present invention will be described in detail. However, the present invention is not limited to the following embodiments.

An artificial hair fiber according to the embodiment is made of an artificial fibroin fiber containing a modified fibroin.

<Modified Fibroin>

The modified fibroin according to the present embodiment is a protein containing a domain sequence represented by

> Formula 1:             $[(A)_n$ motif-REP$]_m$

or

> Formula 2:             $[(A)_n$ motif-REP$]_m$-$(A)_n$

motif. In the modified fibroin, an amino acid sequence (an N-terminal sequence and a C-terminal sequence) may be further added to either or both of the N-terminal side and the C-terminal side of the domain sequence. The N-terminal sequence and the C-terminal sequence are typically, but not limited to, regions that do not have a repetition of amino acid motifs characteristic of a fibroin, and that consist of amino acids of about 100 residues.

The "modified fibroin" in the present specification means an artificially produced fibroin (an artificial fibroin). The modified fibroin may be a fibroin in which the domain sequence is different from an amino acid sequence of a naturally occurring fibroin or the same as the amino acid sequence of a naturally occurring fibroin. The "naturally occurring fibroin" referred to in the present specification also is a protein containing a domain sequence represented by

> Formula 1:             $[(A)_n$ motif-REP$]_m$

or

> Formula 2:             $[(A)_n$ motif-REP$]_m$-$(A)_n$

motif.

The "modified fibroin" may be a modified fibroin having an amino acid sequence of a naturally occurring fibroin as it is, may be a modified fibroin whose amino acid sequence has been modified based on the amino acid sequence of a naturally occurring fibroin (for example, a modified fibroin whose amino acid sequence has been modified by altering a cloned gene sequence of a naturally occurring fibroin), or may be a modified fibroin that is artificially designed and synthesized independently of a naturally occurring fibroin (for example, a modified fibroin having a desired amino acid sequence by chemically synthesizing a nucleic acid encoding a designed amino acid sequence).

The "domain sequence" in the present specification is an amino acid sequence that produces a crystalline region (typically corresponding to an $(A)_n$ motif of an amino acid sequence) and an amorphous region (typically corresponding to REP of an amino acid sequence) unique to a fibroin, and refers to an amino acid sequence represented by

> Formula 1:             $[(A)_n$ motif-REP$]_m$

or

> Formula 2:             $[(A)_n$ motif-REP$]_m$-$(A)_n$

motif. Here, the $(A)_n$ motif represents an amino acid sequence mainly containing alanine residues, and the number of amino acid residues in the $(A)_n$ motif is 2 to 27. The number of amino acid residues in the $(A)_n$ motif may be an integer of 2 to 20, 4 to 27, 4 to 20, 8 to 20, 10 to 20, 4 to 16, 8 to 16, or 10 to 16. Further, the proportion of the number of alanine residues with respect to the total number of amino acid residues in the $(A)_n$ motif may be 40% or more, and may be 60% or more, 70% or more, 80% or more, 83% or more, 85% or more, 86% or more, 90% or more, 95% or more, or 100% (meaning that the $(A)_n$ motif is composed of only alanine residues). Among a plurality of $(A)_n$ motifs present in the domain sequence, at least seven of the $(A)_n$ motifs may be composed only of alanine residues. REP represents an amino acid sequence composed of 2 to 200 amino acid residues. REP may be an amino acid sequence composed of 10 to 200 amino acid residues, and may be an amino acid sequence composed of 10 to 40, 10 to 60, 10 to 80, 10 to 100, 10 to 120, 10 to 140, 10 to 160, or 10 to 180 amino acid residues. m represents an integer of 2 to 300, and may be an integer of 8 to 300, 10 to 300, 20 to 300, 40 to 300, 60 to 300, 80 to 300, 100 to 300, 10 to 200, 20 to 200, 20 to 180, 20 to 160, 20 to 140, or 20 to 120. A plurality of (A)$_n$ motifs may have the same amino acid sequence or amino acid sequences different from each other. A plurality of REPs may have the same amino acid sequence or amino acid sequences different from each other.

The modified fibroin according to the present embodiment may be obtained, for example, by subjecting a cloned gene sequence of a naturally occurring fibroin to a modification of an amino acid sequence corresponding to, for example, substitution, deletion, insertion, and/or addition of one or a plurality of amino acid residues. The substitution, deletion, insertion, and/or addition of an amino acid residue may be performed by methods well known to those skilled in the art, such as site-directed mutagenesis. Specifically, the modification may be performed in accordance with the method described in literature such as Nucleic Acid Res. 10, 6487 (1982) and Methods in Enzymology, 100, 448 (1983).

A naturally occurring fibroin is a protein containing a domain sequence represented by

Formula 1: [(A)$_n$ motif-REP]$_m$

or

Formula 2: [(A)$_n$ motif-REP]$_m$-(A)$_n$

motif, and specific examples thereof include a fibroin produced by insects or spiders.

Examples of the fibroin produced by insects include silk proteins produced by silkworms such as Bombyx mori, Bombyx mandarina, Antheraea yamamai, Anteraea pernyi, Eriogyna pyretorum, Pilosamia Cynthia ricini, Samia cynthia, Caligura japonica, Antheraea mylitta, and Antheraea assama; and hornet silk proteins discharged by larvae of Vespa simillima xanthoptera.

More specific examples of the fibroin produced by insects include a silkworm fibroin L chain (GenBank Accession No. M76430 (nucleotide sequence), and AAA27840.1 (amino acid sequence)).

Examples of the fibroin produced by spiders include spider silk proteins produced by spiders belonging to the genus Araneus such as Araneus ventricosus, Araneus diadematus, Araneus pinguis, Araneus pentagrammicus and Araneus nojimai, spiders belonging to the genus Neoscona such as Neoscona scylla, Neoscona nautica, Neoscona adianta and Neoscona scylloides, spiders belonging to the genus Pronus such as Pronous minutes, spiders belonging to the genus Cyrtarachne such as Cyrtarachne bufo and Cyrtarachne inaequalis, spiders belonging to the genus Gasteracantha such as Gasteracantha kuhli and Gasteracantha mammosa, spiders belonging to the genus Ordgarius such as Ordgarius hobsoni and Ordgarius sexspinosus, spiders belonging to the genus Argiope such as Argiope amoena, Argiope minuta and Argiope bruennich, spiders belonging to the genus Arachnura such as Arachnura logio, spiders belonging to the genus Acusilas such as Acusilas coccineus, spiders belonging to the genus Cytophora such as Cytophora moluccensis, Cytophora exanthematica and Cytophora unicolor, spiders belonging to the genus Poltys such as Poltys illepidus, spiders belonging to the genus Cyclosa such as Cyclosa octotuberculata, Cyclosa sedeculata, Cyclosa vallata and Cyclosa atrata, and spiders belonging to the genus Chorizopes such as Chorizopes nipponicus; and spider silk proteins produced by spiders belonging to the genus Tetragnatha such as Tetragnatha praedonia, Tetragnatha maxillosa, Tetragnatha extensa and Tetragnatha squamata, spiders belonging to the genus Leucauge such as Leucauge magnifica, Leucauge blanda and Leucauge subblanda, spiders belonging to the genus Nephila such as Nephila clavata and Nephila pilipes, spiders belonging to the genus Menosira such as Menosira ornata, spiders belonging to the genus Dyschiriognatha such as Dyschiriognatha tenera, spiders belonging to the genus Latrodectus such as Latrodectus mactans, Latrodectus hasseltii, Latrodectus geometricus and Latrodectus tredecimguttatus, and spiders belonging to the family Tetragnathidae such as spiders belonging to the genus Euprosthenops. Examples of the spider silk proteins include traction yarn proteins such as MaSp (MaSp1 and MaSp2) and ADF (ADF3 and ADF4), and MiSp (MiSp1 and MiSp2).

More specific examples of the spider silk protein produced by spiders include fibroin-3 (adf-3) [derived from Araneus diadematus] (GenBank Accession No. AAC47010 (amino acid sequence), U47855 (nucleotide sequence)), fibroin-4 (adf-4) [derived from Araneus diadematus] (GenBank Accession No. AAC47011 (amino acid sequence), U47856 (nucleotide sequence)), dragline silk protein spidroin 1 [derived from Nephila clavipes] (GenBank Accession No. AAC04504 (amino acid sequence), U37520 (nucleotide sequence)), major ampullate spidroin 1 [derived from Latrodectus hesperus] (GenBank Accession No. ABR68856 (amino acid sequence), EF595246 (nucleotide sequence)), dragline silk protein spidroin 2 [derived from Nephila clavata] (GenBank Accession No. AAL32472 (amino acid sequence), AF441245 (nucleotide sequence)), major ampullate spidroin 1 [derived from Euprosthenops australis] (GenBank Accession No. CAJ00428 (amino acid sequence), AJ973155 (nucleotide sequence)) and major ampullate spidroin 2 [Euprosthenops australis] (GenBank Accession No. CAM32249.1 (amino acid sequence), AM490169 (nucleotide sequence)), minor ampullate silk protein 1 [Nephila clavipes] (GenBank Accession No. AAC14589.1 (amino acid sequence), minor ampullate silk protein 2 [Nephila clavipes] (GenBank Accession No. AAC14591.1 (amino acid sequence)), and minor ampullate spidroin-like protein [Nephilengys cruentata] (GenBank Accession No. ABR37278.1 (amino acid sequence)).

More specific examples of the naturally occurring fibroin further include a fibroin whose sequence information is registered in NCBI GenBank. For example, sequences thereof may be confirmed by extracting sequences that has a keyword of spidroin, ampullate, fibroin, "silk and polypeptide", or "silk and protein" in DEFINITION, among sequences containing INV as DIVISION in sequence information registered in NCBI GenBank; sequences that have a specific character string of a product from CDS; or sequences that have a specific character string described for TISSUE TYPE from SOURCE.

The modified fibroin according to the present embodiment may be a modified silk fibroin (a modified fibroin obtained by modifying an amino acid sequence of a silk protein produced by silkworm), or may be a modified spider silk fibroin (a modified fibroin obtained by modifying an amino acid sequence of a spider silk protein produced by spiders). As the modified fibroin, a modified spider silk fibroin is preferable. The modified spider silk fibroin is also excellent in heat retention, hygroscopic exothermicity, and/or flame retardancy.

Specific examples of the modified fibroin include a modified fibroin derived from a large spinal canal thread protein produced in the major ampullate gland of a spider (first modified fibroin), a modified fibroin having a domain sequence with a reduced content of glycine residues (second modified fibroin), a modified fibroin having a domain sequence with a reduced content of $(A)_n$ motifs (third modified fibroin), a modified fibroin with a reduced content of glycine residues and $(A)_n$ motifs (fourth modified fibroin), a modified fibroin having a domain sequence containing a region having a locally high hydropathy index (fifth modified fibroin), and a modified fibroin having a domain sequence with a reduced content of glutamine residues (sixth modified fibroin).

Examples of the first modified fibroin include a protein containing a domain sequence represented by

Formula 1: $[(A)_n \text{ motif-REP}]_m$.

In the first modified fibroin, the number of amino acid residues of the $(A)_n$ motif is preferably an integer of 3 to 20, more preferably an integer of 4 to 20, still more preferably an integer of 8 to 20, even still more preferably an integer of 10 to 20, further still preferably an integer of 4 to 16, particularly preferably an integer of 8 to 16, and most preferably an integer of 10 to 16. In the first modified fibroin, the number of amino acid residues constituting REP in Formula 1 is preferably 10 to 200 residues, more preferably 10 to 150 residues, still more preferably 20 to 100 residues, and even still more preferably 20 to 75 residues. In the first modified fibroin, the total number of the glycine residues, the serine residues, and the alanine residues contained in the amino acid sequence represented by

Formula 1: $[(A)_n \text{ motif-REP}]_m$

is preferably 40% or more, more preferably 60% or more, and still more preferably 70% or more with respect to the total number of amino acid residues.

The first modified fibroin may be a polypeptide that contains a unit of an amino acid sequence represented by

Formula 1: $[(A)_n \text{ motif-REP}]_m$,

and has the amino acid sequence set forth in any of SEQ ID NOs: 1 to 3 or an amino acid sequence having 90% or more homology with the amino acid sequence set forth in any of SEQ ID NOs: 1 to 3 as the C-terminal sequence.

The amino acid sequence set forth in SEQ ID NO: 1 is the same as the amino acid sequence consisting of 50 amino acid residues at the C-terminal of the amino acid sequence of ADF3 (GI: 1263287, NCBI), the amino acid sequence set forth in SEQ ID NO: 2 is the same as the amino acid sequence obtained by removing 20 residues from the C-terminal of the amino acid sequence set forth in SEQ ID NO: 1, and the amino acid sequence set forth in SEQ ID NO: 3 is the same as the amino acid sequence obtained by removing 29 residues from the C-terminal of the amino acid sequence set forth in SEQ ID NO: 1.

More specific examples of the first modified fibroin include a modified fibroin containing (1-i) the amino acid sequence set forth in SEQ ID NO: 4 (recombinant spider silk protein ADF3KaiLargeNRSH1), or (1-ii) an amino acid sequence having 90% or more sequence identity with the amino acid sequence set forth in SEQ ID NO: 4. The sequence identity is preferably 95% or more.

The amino acid sequence shown in SEQ ID NO 4 is obtained by performing mutation on the amino acid sequence of ADF3 in which an amino acid sequence (SEQ ID NO 5) consisting of a start codon, His10 tag, and HRV3C protease (Human rhinovirus 3C protease) recognition site is added to the N-terminal, such that the 1st to 13th repeat regions are approximately doubled, and the translation terminates at the 1154th amino acid residue. The amino acid sequence of the C-terminal of the amino acid sequence shown in SEQ ID NO 4 is the same as the amino acid sequence shown in SEQ ID NO 3.

The modified fibroin (1-i) may be a modified fibroin consisting of the amino acid sequence set forth in SEQ ID NO: 4.

The second modified fibroin has a domain sequence that has an amino acid sequence with a reduced content of glycine residues, as compared with a naturally occurring fibroin. It can be said that the second modified fibroin is a modified fibroin having an amino acid sequence that corresponds to an amino acid sequence in which, at least one or a plurality of glycine residues in REP are substituted with other amino acid residues, as compared with a naturally occurring fibroin.

The second modified fibroin may have a domain sequence that has an amino acid sequence corresponding to an amino acid sequence in which, in at least one motif sequence selected from GGX and GPGXX (where G represents a glycine residue, P represents a proline residue, and X represents an amino acid residue other than glycine) in REP, at least one glycine residue in one or a plurality of motif sequences is substituted with another amino acid residue, as compared with a naturally occurring fibroin.

In the second modified fibroin, the proportion of the above-described motif sequences in which a glycine residue is substituted with another amino acid residue may be 10% or more with respect to the all motif sequences.

The second modified fibroin may be a modified fibroin that contains a domain sequence represented by

Formula 1: $[(A)_n$ motif-REP$]_m$,

that has an amino acid sequence in which z/w is 30% or more, 40% or more, 50% or more, or 50.9% or more, where z is the total number of amino acid residues of an amino acid sequence consisting of XGX (where X represents an amino acid residue other than glycine) contained in all REPs in the domain sequence excluding the sequence between the $(A)_n$ motif positioned closest to the C-terminal side and the C-terminal of the domain sequence; and where w is the total number of amino acid residues in the domain sequence excluding the sequence between the $(A)_n$ motif positioned closest to the C-terminal side and the C-terminal of the domain sequence. The number of alanine residues with respect to a total number of amino acid residues in the $(A)_n$ motif may be 83% or more, but is preferably 86% or more, more preferably 90% or more, still more preferably 95% or more, and even still more preferably 100% (which means that the $(A)_n$ motif consists only of alanine residues).

The second modified fibroin is preferably a modified fibroin in which one glycine residue of the GGX motif is substituted with another amino acid residue to increase the content ratio of the amino acid sequence consisting of XGX. In the second modified fibroin, the content ratio of the amino acid sequence consisting of GGX in the domain sequence is preferably 30% or less, more preferably 20% or less, still more preferably 10% or less, even still more preferably 6 % or less, further still more preferably 4% or less, and particularly preferably 2% or less. The content ratio of the amino acid sequence consisting of GGX in the domain sequence can be calculated by the same method as the method for calculating a content ratio (z/w) of an amino acid sequence consisting of XGX below.

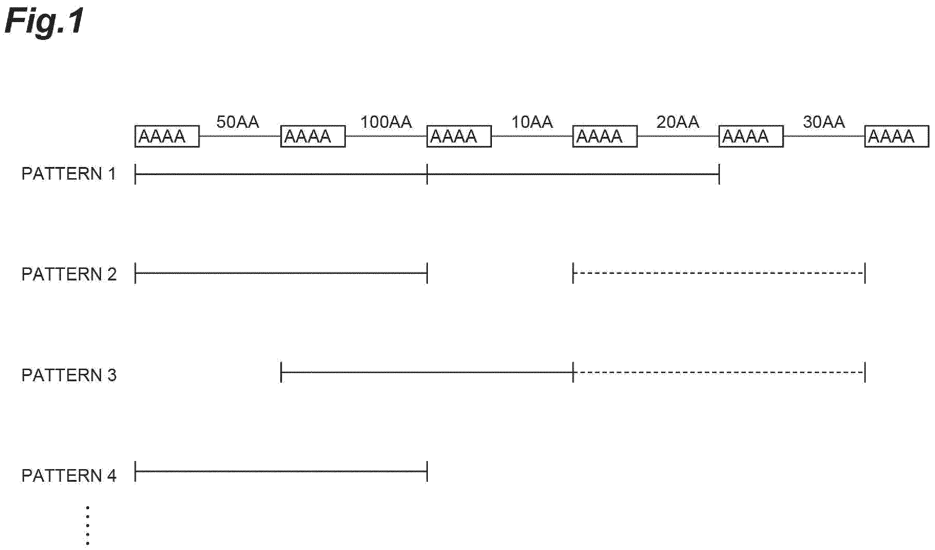

The method for calculating z/w will be described in more detail. First, in a fibroin (a modified fibroin or a naturally occurring fibroin) containing a domain sequence represented by

Formula 1: $[(A)_n$ motif-REP$]_m$,

an amino acid sequence consisting of XGX is extracted from all REPs contained in the domain sequence excluding the sequence between the $(A)_n$ motif positioned closest to the C-terminal side and the C-terminal of the domain sequence. The total number of amino acid residues constituting XGX is z. For example, in a case where 50 amino acid sequences consisting of XGX (without an overlap) are extracted, z is $50 \times 3 = 150$. Further, for example, in a case where there is X contained in two XGXs (X at the center), as in the case of an amino acid sequence consisting of XGXGX, the calculation is performed by deducting the overlap (in the case of XGXGX, 5 amino acid residues). w is the total number of amino acid residues contained in the domain sequence excluding the sequence between the $(A)_n$ motif positioned closest to the C-terminal side and the C-terminal of the domain sequence. For example, in the case of the domain sequence illustrated in Fig. 1, w is $4 + 50 + 4 + 100 + 4 + 10 + 4 + 20 + 4 + 30 = 230$ (the $(A)_n$ motif positioned closest to the C-terminal side is excluded.). Next, z/w (%) can be calculated by dividing z by w.

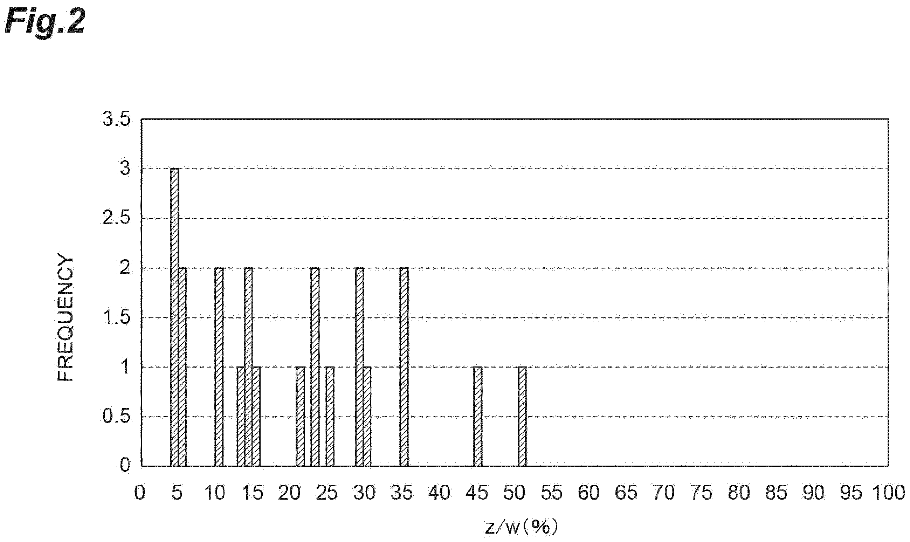

Here, z/w in a naturally occurring fibroin will be described. First, fibroins with amino acid sequence information registered in NCBI GenBank was checked by the exemplified method as described above, and 663 types of fibroins (of which 415 types were fibroins derived from spiders) were extracted. Among all the extracted fibroins, z/w was calculated from an amino acid sequence of a naturally occurring fibroin that contains a domain sequence represented by

Formula 1: $[(A)_n$ motif-REP$]_m$,

and that has a content ratio of the amino acid sequence consisting of GGX in the fibroin of 6% or less, using the above-mentioned calculation method. The results are shown in Fig. 2. In Fig. 2, the horizontal axis represents z/w (%), and the vertical axis represents frequency. As apparent from Fig. 2, z/w in all the naturally occurring fibroin is less than 50.9% (highest value: 50.86%).

In the second modified fibroin, z/w is preferably 50.9% or more, more preferably 56.1% or more, still more preferably 58.7% or more, even still more preferably 70% or more, and further still preferably 80% or more. The upper limit of z/w is not particularly limited, but may be, for example, 95% or less.

The second modified fibroin may be obtained, for example, by modifying a cloned gene sequence of a naturally occurring fibroin such that at least a part of the nucleotide sequence encoding a glycine residue is substituted to encode another amino acid residue. In this case, one glycine residue in the GGX motif and the GPGXX motif may be selected as the glycine residue to be modified, and also, substitution may be performed so that z/w becomes 50.9% or more. In addition, the second modified fibroin may also be obtained, for example, by designing an amino acid sequence that satisfies the above embodiment based on the amino acid sequence of a naturally occurring fibroin, and chemically synthesizing a nucleic acid encoding the designed amino acid sequence. In any case, in addition to the modification that corresponds to substitution of the glycine residue in REP in the amino acid sequence of a naturally occurring fibroin with another amino acid residue, a modification of the amino acid sequence that corresponds to substitution, deletion, insertion, and/or addition of one or a plurality of amino acid residues may further be performed.

The above another amino acid residue is not particularly limited as long as it is an amino acid residue other than the glycine residue, but is preferably a hydrophobic amino acid residue such as a valine (V) residue, a leucine (L) residue, a isoleucine (I) residue, a methionine (M) residue, a proline (P) residue, a phenylalanine (F) residue, and a tryptophan (W) residue, or a hydrophilic amino acid residues such a glutamine (Q) residue, an asparagine (N) residue, a serine (S) residue, a lysine (K) residue, and a glutamic acid (E) residue, more preferably the valine (V) residue, the leucine (L) residue, the isoleucine (I) residue, the phenylalanine (F) residue, and the glutamine (Q) residue, and still more preferably the glutamine (Q) residue.

More specific examples of the second modified fibroin include a modified fibroin containing (2-i) the amino acid sequence set forth in SEQ ID NO: 6 (Met-PRT380), SEQ ID NO: 7 (Met-PRT410), SEQ ID NO: 8 (Met-PRT525), or SEQ ID NO: 9 (Met-PRT799), or (2-ii) an amino acid sequence having 90% or more sequence identity with the amino acid sequence set forth in SEQ ID NO: 6, SEQ ID NO: 7, SEQ ID NO: 8, or SEQ ID NO: 9.

The modified fibroin (2-i) will be described. The amino acid sequence set forth in SEQ ID NO: 6 is an amino acid sequence in which all GGXs in REP of the amino acid sequence set forth in SEQ ID NO: 10 (Met-PRT313) corresponding to a naturally occurring fibroin are substituted with GQXs. The amino acid sequence set forth in SEQ ID NO: 7 is an amino acid sequence in which every third $(A)_n$ motif from the N-terminal side to the C-terminal side is deleted from the amino acid sequence set forth in SEQ ID NO: 6, and further, one $[(A)_n$ motif-REP] is inserted before the C-terminal sequence. The amino acid sequence set forth in SEQ ID NO: 8 is an amino acid sequence in which two alanine residues are inserted at the C-terminal side of each $(A)_n$ motif of the amino acid sequence set forth in SEQ ID NO: 7, and further some of the glutamine (Q) residues are substituted with a serine (S) residue, and some of the amino acids on the C-terminal side are deleted so that the molecular weight is almost the same as that of SEQ ID NO 7. The amino acid sequence set forth in SEQ ID NO: 9 is an amino acid sequence in which a predetermined hinge sequence and a His tag sequence are added to the C-terminal of a sequence having four-time repetition of a region of 20 domain sequences present in the amino acid sequence set forth in SEQ ID NO: 7 (however, with several amino acid residues on the C-terminal side of the region are substituted).

The value of z/w in the amino acid sequence set forth SEQ ID NO: 10 (corresponding to a naturally occurring fibroin) is 46.8%. The values of z/w in the amino acid sequence set forth in SEQ ID NO: 6, the amino acid sequence set forth in SEQ ID NO: 7, the amino acid sequence set forth in SEQ ID NO: 8, and the amino acid sequence set forth in SEQ ID NO: 9 are 58.7%, 70.1%, 66.1%, and 70.0%, respectively. In addition, the values of x/y at the Giza ratio (which will be described later) of 1:1.8 to 11.3 in the amino acid sequences set forth in SEQ ID NO: 10, SEQ ID NO: 6, SEQ ID NO: 7, SEQ ID NO: 8, and SEQ ID NO: 9 are 15.0%, 15.0%, 93.4%, 92.7%, and 89.8%, respectively.

The modified fibroin (2-i) may be a modified fibroin consisting of the amino acid sequence set forth in SEQ ID NO: 6, SEQ ID NO: 7, SEQ ID NO: 8, or SEQ ID NO: 9.

The modified fibroin (2-ii) is a modified fibroin containing an amino acid sequence having 90% or more sequence identity with the amino acid sequence set forth in SEQ ID NO: 6, SEQ ID NO: 7, SEQ ID NO: 8, or SEQ ID NO: 9. The modified fibroin (2-ii) also is a protein containing a domain sequence represented by Formula 1: $[(A)_n$ motif-REP$]_m$. The sequence identity is preferably 95% or more.

The modified fibroin (2-ii) preferably has 90% or more sequence identity with the amino acid sequence set forth in SEQ ID NO: 6, SEQ ID NO: 7, SEQ ID NO: 8, or SEQ ID NO: 9, and that z/w is 50.9% or more, where z is the total number of amino acid residues in the amino acid sequence consisting of XGX (where X represents an amino acid residue other than glycine) contained in REP; and where w is the total number of amino acid residues in REP in the domain sequence.

The second modified fibroin may contain a tag sequence at either or both of the N-terminal and C-terminal. This makes it possible to isolate, immobilize, detect, and visualize the modified fibroin.

The tag sequence may be, for example, an affinity tag utilizing specific affinity (binding property, affinity) with other molecules. Specific examples of the affinity tag include a histidine tag (His tag). The His tag is a short peptide in which about 4 to 10 histidine residues are arranged and has a property of specifically binding to a metal ion such as nickel, and thus, it can be used for isolation of a modified fibroin by a chelating metal chromatography. Specific examples of the tag sequence include an amino acid sequence set forth in SEQ ID NO: 11 (amino acid sequence containing a His tag sequence and a hinge sequence).

In addition, a tag sequence such as a glutathione-S-transferase (GST) that specifically binds to glutathione and a maltose binding protein (MBP) that specifically binds to maltose may also be used.

Furthermore, an "epitope tag" utilizing an antigen-antibody reaction may also be used. By adding a peptide (an epitope) showing antigenicity as a tag sequence, an antibody against the epitope can be bound. Examples of the epitope tag include an HA (peptide sequence of hemagglutinin of an influenza virus) tag, a myc tag, and a FLAG tag. The modified fibroin can be easily purified with high specificity by utilizing the epitope tag.

It is also possible to further use a tag sequence which can be cleaved with a specific protease. By treating a protein adsorbed via the tag sequence with a protease, it is also possible to recover a modified fibroin cleaved from the tag sequence.

More specific examples of the modified fibroin containing the tag sequence include a modified fibroin containing (2-iii) the amino acid sequence set forth in SEQ ID NO: 12 (PRT380), SEQ ID NO: 13 (PRT410), SEQ ID NO: 14 (PRT525), or SEQ ID NO: 15 (PRT799), or (2-iv) an amino acid sequence having 90% or more sequence identity with the amino acid sequence set forth in SEQ ID NO: 12, SEQ ID NO: 13, SEQ ID NO: 14, or SEQ ID NO: 15.

The amino acid sequences set forth in SEQ ID NO: 16 (PRT313), SEQ ID NO: 12, SEQ ID NO: 13, SEQ ID NO: 14, and SEQ ID NO: 15 are amino acid sequences in which the amino acid sequence (containing a His tag sequence and a hinge sequence) set forth in SEQ ID NO: 11 is added to the N-terminal of the amino acid sequences set forth in SEQ ID NO: 10, SEQ ID NO: 6, SEQ ID NO: 7, SEQ ID NO: 8, and SEQ ID NO: 9, respectively.

The modified fibroin (2-iii) may be a modified fibroin consisting of the amino acid sequence set forth in SEQ ID NO: 12, SEQ ID NO: 13, SEQ ID NO: 14, or SEQ ID NO: 15.

The modified fibroin (2-iv) is a modified fibroin containing an amino acid sequence having 90% or more sequence identity with the amino acid sequence set forth in SEQ ID NO: 12, SEQ ID NO: 13, SEQ ID NO: 14, or SEQ ID NO: 15. The modified fibroin (2-iv) also is a protein containing a domain sequence represented by Formula 1: $[(A)_n \text{ motif-REP}]_m$. The sequence identity is preferably 95% or more.

It is preferable that the modified fibroin (2-iv) has 90% or more sequence identity with the amino acid sequence set forth in SEQ ID NO: 12, SEQ ID NO: 13, SEQ ID NO: 14, or SEQ ID NO: 15, and that z/w is 50.9% or more, where z is the total number of amino acid residues of the amino acid sequence consisting of XGX (where X represents an amino acid residue other than glycine) contained in REP; and where w is the total number of amino acid residues in REP in the domain sequence.

The second modified fibroin may contain a secretory signal for releasing the protein produced in a recombinant protein production system to the outside of the host. The sequence of the secretory signal may be set as appropriate, depending on the type of the host.

The third modified fibroin has a domain sequence that has an amino acid sequence with a reduced content of $(A)_n$ motifs, as compared with a naturally occurring fibroin. It can be said that the domain sequence of the third modified fibroin is a domain sequence having an amino acid sequence that corresponds to an amino acid sequence in which at least one or a plurality of the $(A)_n$ motifs are deleted, as compared with a naturally occurring fibroin.

The third modified fibroin may be a modified fibroin having an amino acid sequence that corresponds to an amino acid sequence in which 10% to 40% of the $(A)_n$ motifs are deleted from a naturally occurring fibroin.

The domain sequence of the third modified fibroin may be a domain sequence having an amino acid sequence that corresponds to an amino acid sequence in which at least one $(A)_n$ motif for every one to three $(A)_n$ motifs from the N-terminal side to the C-terminal side is deleted, as compared with a naturally occurring fibroin.

The domain sequence of the third modified fibroin may be a domain sequence having an amino acid sequence that corresponds to an amino acid sequence in which at least deletion of two consecutive $(A)_n$ motifs and deletion of one $(A)_n$ motif are repeated in this order from the N-terminal side to the C-terminal side, as compared with a naturally occurring fibroin.

The domain sequence of the third modified fibroin may be a domain sequence having an amino acid sequence that corresponds to an amino acid sequence in which, at least, every third $(A)_n$ motif from the N-terminal side to the C-terminal side is deleted.

The third modified fibroin may be a modified fibroin that contains a domain sequence represented by

Formula 1: $[(A)_n \text{ motif-REP}]_m$,

and that has an amino acid sequence in which, when the numbers of amino acid residues of REP of two adjacent $[(A)_n \text{ motif-REP}]$ units are sequentially compared from the N-terminal side to the C-terminal side, x/y is 20% or more, 30% or more, 40% or more, or 50% or more, where x is a maximum value of a total value obtained by adding the numbers of amino acid residues of two adjacent [(A)$_n$ motif-REP] units wherein when the number of amino acid residues of one REP having less amino acid residues is taken as 1, the ratio of the number of amino acids residues of the other REP satisfies 1.8 to 11.3; and where y is a total number of amino acid residues of the domain sequence. The number of alanine residues with respect to a total number of amino acid residues in the (A)$_n$ motif may be 83% or more, but is preferably 86% or more, more preferably 90% or more, still more preferably 95% or more, and even still more preferably 100% (which means that the (A)$_n$ motif consists only of alanine residues).

A method for calculating x/y will be described in more detail with reference to Fig. 1. Fig. 1 illustrates a domain sequence obtained by removing an N-terminal sequence and a C-terminal sequence from a modified fibroin. The domain sequence has a sequence of, from the N-terminal side (left side), (A)$_n$ motif-the first REP (50 amino acid residues)-(A)$_n$ motif-the second REP (100 amino acid residues)-(A)$_n$ motif-the third REP (10 amino acid residues)-(A)$_n$ motif-the fourth REP (20 amino acid residues)-(A)$_n$ motif-the fifth REP (30 amino acid residues)-(A)$_n$ motif.

The two adjacent [(A)$_n$ motif-REP] units are sequentially selected from the N-terminal side to the C-terminal side so that there is no overlap. There may be [(A)$_n$ motif-REP] unit that is not selected. Fig. 1 illustrates Pattern 1 (a comparison of the first REP and the second REP, and a comparison of the third REP and the fourth REP), Pattern 2 (a comparison of the first REP and the second REP, and a comparison of the fourth REP and the fifth REP), Pattern 3 (a comparison of the second REP and the third REP, and a comparison of the fourth REP and the fifth REP), and Pattern 4 (a comparison of the first REP and the second REP). There are selection methods other than these.

Next, for each pattern, the number of amino acid residues of each REP in the selected adjacent two [(A)$_n$ motif-REP] units is compared. The comparison is performed by determining the ratio, relative to one REP having less amino acid residues taken as 1, of the number of amino acid residues of the other REP. For example, in a case of comparing the first REP (50 amino acid residues) and the second REP (100 amino acid residues), the ratio of the number of amino acid residues of the second REP is 100/50 = 2, where the first REP having less amino acid residues is taken as 1. Similarly, in a case of comparing the fourth REP (20 amino acid residues) and the fifth REP (30 amino acid residues), the ratio of the number of amino acid residues of the fifth REP is 30/20 = 1.5, where the fourth REP having less amino acid residues is taken as 1.

In Fig. 1, a set of [(A)$_n$ motif-REP] units in which, where one REP having less amino acid residues is taken as 1, the ratio of the number of amino acid residues of the other REP is 1.8 to 11.3, is indicated by a solid line. In the present specification, this ratio is referred to as a Giza ratio. A set of [(A)$_n$ motif-REP] units in which, where a REP having less amino acid residues is taken as 1, the ratio of the number of amino acid residues of the other REP is less than 1.8 or more than 11.3, is indicated by a dotted line.

In each pattern, the numbers of all amino acid residues (not only REP but also the number of amino acid residues in the (A)$_n$ motif) of the two adjacent [(A)$_n$ motif-REP] units indicated by a solid line are added. Then, the total values obtained by addition are compared, and the total value of the pattern having the highest total value (a maximum value of the total value) is defined as x. In the example illustrated in Fig. 1, the total value of Pattern 1 is the maximum.

Next, x/y (%) can be calculated by dividing x by the total number y of the amino acid residues of the domain sequence.

In the third modified fibroin, x/y is preferably 50% or more, more preferably 60% or more, still more preferably 65% or more, even still more preferably 70% or more, further still preferably 75% or more, and particularly preferably 80% or more. The upper limit of x/y is not particularly limited, but may be, for example, 100% or less. In a case where the Giza ratio is 1:1.9 to 11.3, x/y is preferably 89.6% or more, in a case where the Giza ratio is 1:1.8 to 3.4, x/y is more preferably 77.1% or more, in a case where the Giza ratio is 1:1.9 to 8.4, x/y is still more preferably 75.9% or more, and in a case where the Giza ratio is 1:1.9 to 4.1, x/y is even still more preferably 64.2% or more.

In a case where the third modified fibroin is a modified fibroin in which at least seven of a plurality of (A)$_n$ motifs present in the domain sequence are composed only of alanine residues, x/y is preferably 46.4% or more, more preferably 50% or more, still more preferably 55% or more, even still more preferably 60% or more, further still preferably 70% or more, and particularly preferably 80% or more. The upper limit of x/y is not particularly limited and it only needs to be 100% or less.

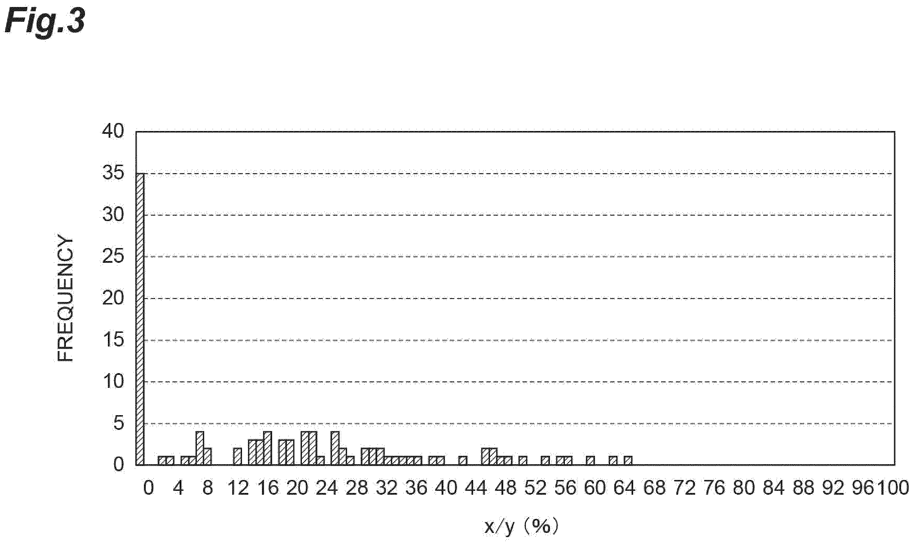

Here, x/y in a naturally occurring fibroin will be described. First, fibroins with amino acid sequence information registered in NCBI GenBank was checked by the exemplified method as described above, and 663 types of fibroins (of which 415 types were fibroins derived from spiders) were extracted. Among all the extracted fibroins, x/y was calculated from an amino acid sequence of a naturally occurring fibroin constituted with a domain sequence represented by

Formula 1: [(A)$_n$ motif-REP]$_m$,

using the above-mentioned calculation method. The results in a case where the Giza ratio is 1:1.9 to 4.1 are shown in Fig. 3.

In Fig. 3, the horizontal axis represents x/y (%) and the vertical axis represents a frequency. As apparent from Fig. 3, x/y in all the naturally occurring fibroin is less than 64.2% (highest value: 64.14%).

The third modified fibroin may be obtained, for example, by deleting one or a plurality sequences encoding $(A)_n$ motif from a cloned gene sequence of a naturally occurring fibroin so that x/y is 64.2% or more. In addition, the third modified fibroin may also be obtained, for example, by designing an amino acid sequence that corresponds to an amino acid sequence in which one or a plurality $(A)_n$ motifs are deleted from an amino acid sequence of a naturally occurring fibroin so that x/y becomes 64.2% or more, and chemically synthesizing a nucleic acid encoding the designed amino acid sequence. In any case, in addition to the modification that corresponds to deletion of the $(A)_n$ motif from an amino acid sequence of a naturally occurring fibroin, a modification of the amino acid sequence that corresponds to substitution, deletion, insertion, and/or addition of one or a plurality of amino acid residues may further be performed.

More specific examples of the third modified fibroin include a modified fibroin containing an amino acid sequence having (3-i) the amino acid sequence set forth in SEQ ID NO: 17 (Met-PRT399), SEQ ID NO: 7 (Met-PRT410), SEQ ID NO: 8 (Met-PRT525), or SEQ ID NO: 9 (Met-PRT799), or (3-ii) an amino acid sequence having 90% or more sequence identity with the amino acid sequence set forth in SEQ ID NO: 17, SEQ ID NO: 7, SEQ ID NO: 8, or SEQ ID NO: 9.

The modified fibroin (3-i) will be described. The amino acid sequence set forth in SEQ ID NO: 17 is an amino acid sequence in which every third $(A)_n$ motif from the N-terminal side to the C-terminal side is deleted from the amino acid sequence set forth in SEQ ID NO: 10 (Met-PRT313) corresponding to a naturally occurring fibroin, and further, one $[(A)_n$ motif-REP] is inserted before the C-terminal sequence. The amino acid sequences set forth in SEQ ID NO: 7, SEQ ID NO: 8, or SEQ ID NO: 9 are as described for the second modified fibroin.

The value of x/y at a Giza ratio of 1:1.8 to 11.3 in the amino acid sequence set forth in SEQ ID NO: 10 (corresponding to a naturally occurring fibroin) is 15.0%. Both the values of x/y in the amino acid sequence set forth in SEQ ID NO: 17 and the amino acid sequence set forth in SEQ ID NO: 7 are 93.4%. The value of x/y in the amino acid sequence set forth in SEQ ID NO: 8 is 92.7%. The value of x/y in the amino acid sequence set forth in SEQ ID NO: 9 is 89.8%. The values of z/w in the amino acid sequences set forth in SEQ ID NO: 10, SEQ ID NO: 17, SEQ ID NO: 7, SEQ ID NO: 8, and SEQ ID NO: 9 are 46.8%, 56.2%, 70.1%, 66.1%, and 70.0%, respectively.

The modified fibroin (3-i) may be a modified fibroin consisting of the amino acid sequence set forth in SEQ ID NO: 17, SEQ ID NO: 7, SEQ ID NO: 8, or SEQ ID NO: 9.

The modified fibroin (3-ii) is a modified fibroin containing an amino acid sequence having 90% or more sequence identity with the amino acid sequence set forth in SEQ ID NO: 17, SEQ ID NO: 7, SEQ ID NO: 8, or SEQ ID NO: 9. The modified fibroin (3-ii) also is a protein containing a domain sequence represented by

Formula 1: $[(A)_n$ motif-REP$]_m$.

The sequence identity is preferably 95% or more.

It is preferable that the modified fibroin (3-ii) has 90% or more sequence identity with the amino acid sequence set forth in SEQ ID NO: 17, SEQ ID NO: 7, SEQ ID NO: 8, or SEQ ID NO: 9, and that, when the numbers of amino acid residues of REP of two adjacent $[(A)_n$ motif-REP] units are sequentially compared from the N-terminal side to the C-terminal side, x/y is 64.2% or more, where x is a maximum value of a total value obtained by adding the numbers of amino acid residues of two adjacent $[(A)_n$ motif-REP] units wherein when the number of amino acid residues of one REP having less amino acid residues is taken as 1, the ratio of the number of amino acids residues of the other REP satisfies 1.8 to 11.3 (the Giza ratio is 1:1.8 to 11.3); and where y is a total number of amino acid residues of the domain sequence.

The third modified fibroin may contain a tag sequence described above at either or both of the N-terminal and C-terminal.

A more specific example of the modified fibroin containing a tag sequence may be a modified fibroin containing (3-iii) the amino acid sequence set forth in SEQ ID NO: 18 (PRT399), SEQ ID NO: 13 (PRT410), SEQ ID NO: 14 (PRT525), or SEQ ID NO: 15 (PRT799), or (3-iv) an amino acid sequence having 90% or more sequence identity with the amino acid sequence set forth in SEQ ID NO: 18, SEQ ID NO: 13, SEQ ID NO: 14, or SEQ ID NO: 15.

The amino acid sequences set forth in SEQ ID NO: 18, SEQ ID NO: 13, SEQ ID NO: 14, and SEQ ID NO: 15 are amino acid sequences in which the amino acid sequence (containing a His tag sequence and a hinge sequence) set forth in SEQ ID NO: 11 is added to the N-terminal of the amino acid sequences set forth in SEQ ID NO: 17, SEQ ID NO: 7, SEQ ID NO: 8, and SEQ ID NO: 9, respectively.

The modified fibroin (3-iii) may be a modified fibroin consisting of the amino acid sequence set forth in SEQ ID NO: 18, SEQ ID NO: 13, SEQ ID NO: 14, or SEQ ID NO: 15.

The modified fibroin (3-iv) is a modified fibroin containing an amino acid sequence having 90% or more sequence identity with the amino acid sequence set forth in SEQ ID NO: 18, SEQ ID NO: 13, SEQ ID NO: 14, or SEQ ID NO: 15. The modified fibroin (3-iv) also is a protein containing a domain sequence represented by

Formula 1: $[(A)_n$ motif-REP$]_m$.

The sequence identity is preferably 95% or more.

It is preferable that the modified fibroin (3-iv) has 90% or more sequence identity with the amino acid sequence set forth in SEQ ID NO: 18, SEQ ID NO: 13, SEQ ID NO: 14 or SEQ ID NO: 15, and that, when the numbers of amino acid residues of REP of two adjacent [(A)$_n$ motif-REP] units are sequentially compared from the N-terminal side to the C-terminal side, x/y is 64.2% or more, where x is a maximum value of a total value obtained by adding the numbers of amino acid residues of two adjacent [(A)$_n$ motif-REP] units wherein when the number of amino acid residues of one REP having less amino acid residues is taken as 1, the ratio of the number of amino acids residues of the other REP satisfies 1.8 to 11.3; and where y is a total number of amino acid residues of the domain sequence.

The third modified fibroin may contain a secretory signal for releasing the protein produced in a recombinant protein production system to the outside of the host. The sequence of the secretory signal may be set as appropriate, depending on the type of the host.

The fourth modified fibroin has a domain sequence that has an amino acid sequence with a reduced content of glycine residues, in addition to the reduced content of (A)$_n$ motifs, as compared with a naturally occurring fibroin. It can be said that the domain sequence of the fourth modified fibroin is a domain sequence further having an amino acid sequence that corresponds to an amino acid sequence in which at least one or a plurality of glycine residues in REP are substituted with other amino acid residues, in addition to the amino acid sequence in which at least one or a plurality of the (A)$_n$ motifs are deleted, as compared with a naturally occurring fibroin. That is, the fourth modified fibroin is a modified fibroin having both the characteristics of the second modified fibroin and the characteristics of the third modified fibroin described above. Specific embodiments and the like are as described for the second modified fibroin and the third modified fibroin.

More specific examples of the fourth modified fibroin include a modified fibroin containing (4-i) the amino acid sequence set forth in SEQ ID NO: 7 (Met-PRT410), SEQ ID NO: 8 (Met-PRT525), SEQ ID NO: 9 (Met-PRT799), SEQ ID NO: 13 (PRT410), SEQ ID NO: 14 (PRT525), or SEQ ID NO: 15 (PRT799), or (4-ii) an amino acid sequence having 90% or more sequence identity with the amino acid sequence set forth in SEQ ID NO: 7, SEQ ID NO: 8, SEQ ID NO: 9, SEQ ID NO: 13, SEQ ID NO: 14, or SEQ ID NO: 15. Specific embodiments of the modified fibroin containing the amino acid sequence set forth in SEQ ID NO: 7, SEQ ID NO: 8, SEQ ID NO: 9, SEQ ID NO: 13, SEQ ID NO: 14, or SEQ ID NO: 15 are as described above.

The fifth modified fibroin may have a domain sequence that has an amino acid sequence containing a region having a locally high hydropathy index, which corresponds to an amino acid sequence in which one or a plurality of amino acid residues in REP are substituted with an amino acid residue having a high hydropathy index and/or one or a plurality of amino acid residues having a high hydropathy index is inserted into REP, as compared with a naturally occurring fibroin.

It is preferable that the region having a locally high hydropathy index is composed of 2 to 4 consecutive amino acid residues.

It is more preferable that the amino acid residues having a high hydropathy index are selected from isoleucine (I), valine (V), leucine (L), phenylalanine (F), cysteine (C), methionine (M), and alanine (A).

The fifth modified fibroin may further have a modification of an amino acid sequence that corresponds to substitution, deletion, insertion, and/or addition of one or a plurality of amino acid residues, as compared with a naturally occurring fibroin, in addition to the modification that corresponds to substitution of one or a plurality of amino acid residues in REP with amino acid residues having a high hydropathy index and/or insertion of one or a plurality of amino acid residues having a high hydropathy index into REP, as compared with a naturally occurring fibroin.

The fifth modified fibroin may be obtained, for example, from a cloned gene sequence of a naturally occurring fibroin, by substituting one or a plurality of hydrophilic amino acid residues in REP (for example, amino acid residues having a negative hydropathy index) with a hydrophobic amino acid residue (for example, amino acid residues having a positive hydropathy index), and/or inserting one or a plurality of hydrophobic amino acid residues into REP. In addition, the fifth modified fibroin may also be obtained, for example, by designing an amino acid sequence that corresponds to an amino acid sequence in which one or a plurality of hydrophilic amino acid residues in REP are substituted with hydrophobic amino acid residues and/or one or a plurality of hydrophobic amino acid residues are inserted into REP, from an amino acid sequence of a naturally occurring fibroin, and chemically synthesizing a nucleic acid encoding the designed amino acid sequence. In any case, in addition to the modification that corresponds to substitution of one or a plurality of hydrophilic amino acid residues in REP with hydrophobic amino acid residues and/or insertion of one or a plurality of hydrophobic amino acid residues into REP in an amino acid sequence of a naturally occurring fibroin, a modification of an amino acid sequence that corresponds to substitution, deletion, insertion, and/or addition of one or a plurality of amino acid residues may further be performed.

The fifth modified fibroin may be a modified fibroin that contains a domain sequence represented by

Formula 1:        [(A)$_n$ motif-REP]$_m$,

and that has an amino acid sequence in which p/q is 6.2% or more, where p is the total number of amino acid residues contained in a region where an average value of hydropathy indices of the four consecutive amino acid residues is 2.6 or more in all REPs contained in the domain sequence excluding the sequence between the $(A)_n$ motif positioned closest to the C-terminal side and the C-terminal of the domain sequence; and where q is the total number of amino acid residues contained in the domain sequence excluding the sequence between the $(A)_n$ motif positioned closest to the C-terminal side and the C-terminal of the domain sequence.

With regard to the hydropathy index of an amino acid residue, known index from (Hydropathy index: Kyte J, & Doolittle R (1982) "A simple method for displaying the hydropathic character of a protein", J. Mol. Biol., 157, pp. 105-132) may be used. Specifically, the hydropathy index (hereinafter also referred to as "HI") of each amino acid is as shown in Table 1 below.

[Table 1]

| Amino acid | HI | Amino acid | HI |
|---|---|---|---|
| Isoleucine (Ile) | 4.5 | Tryptophan (Trp) | -0.9 |
| Valine (Val) | 4.2 | Tyrosine (Tyr) | -1.3 |
| Leucine (Leu) | 3.8 | Proline (Pro) | -1.6 |
| Phenylalanine (Phe) | 2.8 | Histidine (His) | -3.2 |
| Cysteine (Cys) | 2.5 | Asparagine (Asn) | -3.5 |
| Methionine (Met) | 1.9 | Aspartic acid (Asp) | -3.5 |
| Alanine (Ala) | 1.8 | Glutamine (Gln) | -3.5 |
| Glycine (Gly) | -0.4 | Glutamic acid (Glu) | -3.5 |
| Threonine (Thr) | -0.7 | Lysine (Lys) | -3.9 |
| Serine (Ser) | -0.8 | Arginine (Arg) | -4.5 |

A method for calculating p/q will be described in more detail. For calculation, a sequence (hereinafter also referred to as a "Sequence A") obtained by removing, from domain sequence represented by

Formula 1: $[(A)_n \text{ motif-REP}]_m$,

the sequence between the $(A)_n$ motif positioned closest to the C-terminal side and the C-terminal of the domain sequence is used. First, in all REPs contained in Sequence A, average values of hydropathy indices of the four consecutive amino acid residues are calculated. The average value of the hydropathy indices is obtained by dividing the sum of HI of each of the amino acid residues contained in the four consecutive amino acid residues by 4 (the number of amino acid residues). The average value of the hydropathy indices is obtained for all four consecutive amino acid residues (each of the amino acid residues is used for calculating the average value 1 to 4 times). Next, a region where the average value of the hydropathy indices of the four consecutive amino acid residues is 2.6 or more is identified. Even if a certain amino acid residues correspond to a plurality of the "four consecutive amino acid residues having an average value of the hydropathy indices of 2.6 or more", the amino acid residue is counted as one amino acid residue in the region. The total number of amino acid residues contained in the region is p. Further, the total number of amino acid residues contained in Sequence A is q.

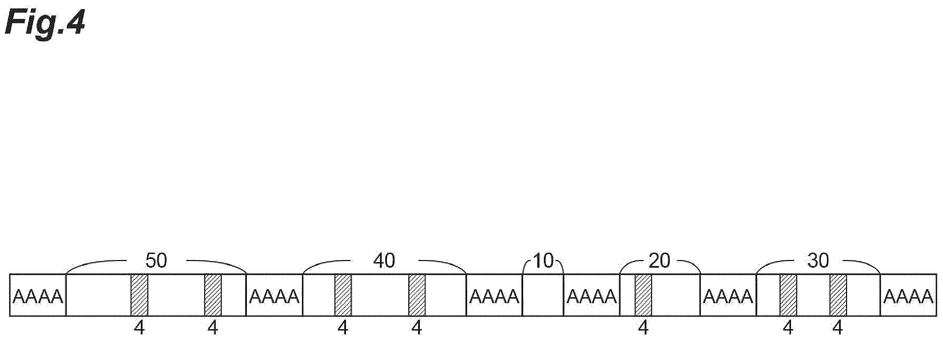

For example, in a case where 20 "four consecutive amino acid residues having an average value of the hydropathy indices of 2.6 or more " are extracted (without an overlap), there are 20 of the four consecutive amino acid residues (without an overlap) in the region in which the average value of the hydropathy indices of the four consecutive amino acid residues is 2.6 or more, and thus p is $20 \times 4 = 80$. In addition, for example, in a case where two "four consecutive amino acid residues having an average value of the hydropathy indices of 2.6 or more" overlap by only one amino acid residue, seven amino acid residues are contained in the regionin which the average value of the hydropathy indices of the four consecutive amino acid residues is 2.6 or more ($p = 2 \times 4 - 1 = 7$; "-1" is deduction for overlap). For example, in a case of the domain sequence shown in Fig. 4, since there are seven "four consecutive amino acid residues having an average value of the hydropathy indices of 2.6 or more" without an overlap, p is $7 \times 4 = 28$. Further, for example, in a case of the domain sequence illustrated in Fig. 4, q is $4 + 50 + 4 + 40 + 4 + 10 + 4 + 20 + 4 + 30 = 170$ (the $(A)_n$ motif present at the end of the C-terminal side is not included). Next, p/q (%) can be calculated by dividing p by q. In the case of Fig. 4, p/q (%) is 28/170 = 16.47%.

In the fifth modified fibroin, p/q is preferably 6.2% or more, more preferably 7% or more, still more preferably 10% or more, even still more preferably 20% or more, and further still preferably 30% or more. The upper limit of p/q is not particularly limited, but may be, for example, 45% or less.

The fifth modified fibroin may be obtained, for example, by modifying an amino acid sequence of a cloned a naturally occurring fibroin to an amino acid sequence containing a region having a locally high hydropathy index, by substituting one or a plurality of hydrophilic amino acid residues in REP (for example, amino acid residues having a negative hydropathy index) with hydrophobic amino acid residues (for example, amino acid residues having a positive hydropathy index), and/or inserting one or a plurality of hydrophobic amino acid residues into REP, so as to satisfy the above condition of p/q. In addition, the modified fibroin may also be obtained, for example, by designing an amino acid sequence satisfying the above-described condition of p/q based on the amino acid sequence of a naturally occurring fibroin, and chemically synthesizing a nucleic acid encoding the designed amino acid sequence. In any case, in addition to the modification that corresponds substitution of one or a plurality of amino acid residues in REP with amino acid residues having a high hydropathy index and/or insertion of one or a plurality of amino acid residues having a high hydropathy index, as compared with a naturally occurring fibroin, a modification that corresponds to substitution, deletion, insertion, and/or addition of one or a plurality of amino acid residues may further be performed.

The amino acid residue having a high hydropathy index is not particularly limited, but is preferably isoleucine (I), valine (V), leucine (L), phenylalanine (F), cysteine (C), methionine (M), and alanine (A), and more preferably valine (V), leucine (L), and isoleucine (I).

More specific examples of the fifth modified fibroin contains a modified fibroin containing an amino acid sequence having (5-i) the amino acid sequence set forth in SEQ ID NO: 19 (Met-PRT720), SEQ ID NO: 20 (Met-PRT665), or SEQ ID NO: 21 (Met-PRT666), or (5-ii) an amino acid sequence having 90% or more sequence identity with the amino acid sequence set forth in SEQ ID NO: 19, SEQ ID NO: 20, or SEQ ID NO: 21.

The modified fibroin (5-i) will be described. The amino acid sequence set forth in SEQ ID NO: 19 is an amino acid sequence in which two amino acid sequences, each consisting of three amino acid residues (VLI), are inserted for every other REP excluding the terminal domain sequence on the C-terminal side; further, some of the glutamine (Q) residues are substituted with serine (S) residues; and some of the amino acids on the C-terminal side are deleted, with respect to the amino acid sequence set forth in SEQ ID NO: 7 (Met-PRT410). The amino acid sequence set forth in SEQ ID NO: 20 is an amino acid sequence in which one amino acid sequence consisting of three amino acid residues (VLI) is inserted for every other REP with respect to the amino acid sequence set forth in SEQ ID NO: 8 (Met-PRT525). The amino acid sequence set forth in SEQ ID NO: 21 is an amino acid sequence in which two amino acid sequences each consisting of three amino acid residues (VLI) are inserted for every other REP with respect to the amino acid sequence set forth in SEQ ID NO: 8.

The modified fibroin (5-i) may be a modified fibroin consisting of the amino acid sequence set forth in SEQ ID NO: 19, SEQ ID NO: 20, or SEQ ID NO: 21.

The modified fibroin (5-ii) is a modified fibroin containing an amino acid sequence having 90% or more sequence identity with the amino acid sequence set forth in SEQ ID NO: 19, SEQ ID NO: 20, or SEQ ID NO: 21. The modified fibroin (5-ii) also is a protein containing a domain sequence represented by

$$\text{Formula 1:} \qquad [(A)_n \text{ motif-REP}]_m.$$

The sequence identity is preferably 95% or more.

It is preferable that the modified fibroin (5-ii) has 90% or more sequence identity with the amino acid sequence set forth in SEQ ID NO: 19, SEQ ID NO: 20, or SEQ ID NO: 21, and that p/q is 6.2% or more, where p is the total number of amino acid residues contained in a region where an average value of hydropathy indices of the four consecutive amino acid residues is 2.6 or more in all REPs contained in the domain sequence excluding the sequence between the $(A)_n$ motif positioned closest to the C-terminal side and the C-terminal of the domain sequence; and where q is the total number of amino acid residues contained in the domain sequence excluding the sequence between the $(A)_n$ motif positioned closest to the C-terminal side and the C-terminal of the domain sequence.

The fifth modified fibroin may contain a tag sequence at either or both of the N-terminal and C-terminal.

More specific examples of the modified fibroin containing a tag sequence include a modified fibroin containing (5-iii) the amino acid sequence set forth in SEQ ID NO: 22 (PRT720), SEQ ID NO: 23 (PRT665), or SEQ ID NO: 24 (PRT666), or (5-iv) an amino acid sequence having 90% or more sequence identity with the amino acid sequence set forth in SEQ ID NO: 22, SEQ ID NO: 23, or SEQ ID NO: 24.

The amino acid sequences set forth in SEQ ID NO: 22, SEQ ID NO: 23, and SEQ ID NO: 24 are amino acid sequences in which the amino acid sequence (containing a His tag sequence and a hinge sequence) set forth in SEQ ID NO: 11 is added to the N-terminal of the amino acid sequences set forth in SEQ ID NO: 19, SEQ ID NO: 20, and SEQ ID NO: 21, respectively.

The modified fibroin (5-iii) may be a modified fibroin consisting of the amino acid sequence set forth in SEQ ID NO: 22, SEQ ID NO: 23, or SEQ ID NO: 24.

The modified fibroin (5-iv) is a modified fibroin containing an amino acid sequence having 90% or more sequence identity with the amino acid sequence set forth in SEQ ID NO: 22, SEQ ID NO: 23, or SEQ ID NO: 24. The modified fibroin (5-iv) also is a protein containing a domain sequence represented by

Formula 1: $[(A)_n$ motif-REP$]_m$.

The sequence identity is preferably 95% or more.

It is preferable that the modified fibroin (5-iv) has 90% or more sequence identity with the amino acid sequence set forth in SEQ ID NO: 22, SEQ ID NO: 23, or SEQ ID NO: 24, and that p/q is 6.2% or more, where p is the total number of amino acid residues contained in a region where an average value of hydropathy indices of the four consecutive amino acid residues is 2.6 or more in all REPs contained in the domain sequence excluding the sequence between the $(A)_n$ motif positioned closest to the C-terminal side and the C-terminal of the domain sequence; and where q is the total number of amino acid residues contained in the domain sequence excluding the sequence between the $(A)_n$ motif positioned closest to the C-terminal side and the C-terminal of the domain sequence.

The fifth modified fibroin may contain a secretory signal for releasing the protein produced in a recombinant protein production system to the outside of the host. The sequence of the secretory signal may be set as appropriate, depending on the type of the host.

The sixth modified fibroin has an amino acid sequence with a reduced content of glutamine residues, as compared with a naturally occurring fibroin.

It is preferable that the sixth modified fibroin contains at least one motif selected from a GGX motif and a GPGXX motif in the amino acid sequence of REP.

In a case where the sixth modified fibroin contains a GPGXX motif in REP, a GPGXX motif content rate is usually 1% or more, may be 5% or more, and is preferably 10% or more. The upper limit of the GPGXX motif content rate is not particularly limited, and it may be 50% or less, and may be 30% or less.

In the present specification, the "GPGXX motif content rate" is a value calculated by the following method.

In a fibroin (a modified fibroin or a naturally occurring fibroin) containing a domain sequence represented by

Formula 1: $[(A)_n$ motif-REP$]_m$

or

Formula 2: $[(A)_n$ motif-REP$]_m$-$(A)_n$

motif, the GPGXX motif content rate is calculated as s/t, where s is the number obtained by tripling the total number of the GPGXX motifs (namely, corresponds to the total number of G and P in the GPGXX motifs) contained in all REPs contained in the domain sequence excluding the sequence between the $(A)_n$ motif positioned closest to the C-terminal side and the C-terminal of the domain sequence; and where t is the total number of amino acid residues in all REPs obtained by excluding the sequence between the $(A)_n$ motif positioned closest to the C-terminal side and the C-terminal of the domain sequence from the domain sequence and further excluding the $(A)_n$ motifs.

For the calculation of the GPGXX motif content rate, a "domain sequence excluding the sequence between the $(A)_n$ motif positioned closest to the C-terminal side and the C-terminal of the domain sequence" is used, because "the sequence between the $(A)_n$ motif positioned closest to the C-terminal side and the C-terminal of the domain sequence (sequence corresponding to REP) may contain a sequence having a low correlation with the sequence characteristic of a fibroin, which influences the calculation result of the GPGXX motif content rate when m is small (that is, when the domain sequence is short), and thus, this effect is to be eliminated. In a case where a "GPGXX motif" is located at the C-terminal of REP, it is treated as "GPGXX motif' even if "XX" is, for example, "AA".

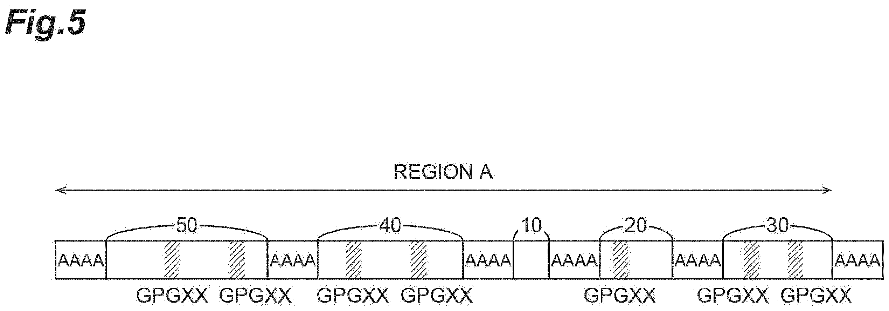

Fig. 5 is a schematic diagram illustrating a domain sequence of a modified fibroin. A method for calculating the GPGXX motif content rate will be specifically described with reference to Fig. 5. First, in a domain sequence of the modified fibroin (which is an $[(A)_n$ motif-REP$]_m$-$(A)_n$ motif] type) illustrated in Fig. 5, the number of GPGXX motifs for calculation of s is 7 and s is $7 \times 3 = 21$, because all REPs are contained in the "domain sequence excluding the sequence between the $(A)_n$ motif positioned closest to the C-terminal side and the C-terminal of the domain sequence" (indicated as "REGION A" in Fig. 5). Similarly, since all REPs are contained in the "domain sequence excluding the sequence between the $(A)_n$ motif positioned closest to the C-terminal side and the C-terminal of the domain sequence" (indicated as "REGION A" in Fig. 5), the total number, t, of amino acid residues in all REPs obtained by further excluding the $(A)_n$ motifs from the "domain sequence excluding the sequence between the $(A)_n$ motif positioned closest to the C-terminal side and the C-terminal of the domain sequence" is 50 + 40 + 10 + 20 + 30 = 150. Next, s/t (%) can be calculated by dividing s by t, and thus, in a case of the modified fibroin of Fig. 5, s/t (%) is 21/150 = 14.0%.

In the sixth modified fibroin, the glutamine residue content rate is preferably 9% or less, more preferably 7%

16

or less, still more preferably 4% or less, and particularly preferably 0%.

In the present specification, the "glutamine residue content rate" is a value calculated by the following method.

In a fibroin (a modified fibroin or a naturally occurring fibroin) containing a domain sequence represented by

$$\text{Formula 1:} \qquad [(A)_n \text{ motif-REP}]_m$$

or

$$\text{Formula 2:} \qquad [(A)_n \text{ motif-REP}]_m\text{-}(A)_n$$

motif, the glutamine residue content rate is calculated as u/t, where u is the total number of glutamine residues contained in all REPs contained in the domain excluding the sequence between the $(A)_n$ motif positioned closest to the C-terminal side and the C-terminal of the domain sequence (sequence corresponding to the "REGION A" in Fig. 5); and where t is the total number of amino acid residues in all REPs obtained by excluding the sequence between the $(A)_n$ motif positioned closest to the C-terminal side and the C-terminal of the domain sequence from the domain sequence and further excluding the $(A)_n$ motifs. The reason as to why the "domain sequence excluding the sequence between the $(A)_n$ motif positioned closest to the C-terminal side and the C-terminal of the domain sequence" is used for the calculation of the glutamine residue content rate is the same as the reason described above.

The domain sequence of the sixth modified fibroin may be a domain sequence having an amino acid sequence that corresponds to an amino acid sequence in which one or a plurality of glutamine residues in REP are deleted or substituted with other amino acid residues, as compared with a naturally occurring fibroin.

The "other amino acid residues" may be amino acid residues other than the glutamine residue, but are preferably amino acid residues having a higher hydropathy index than that of the glutamine residue. The hydropathy indices of the amino acid residues are as shown in Table 1.

As shown in Table 1, examples of the amino acid residues having a higher hydropathy index than a glutamine residue include an amino acid residue selected from isoleucine (I), valine (V), leucine (L), phenylalanine (F), cysteine (C), methionine (M), alanine (A), glycine (G), threonine (T), serine (S), tryptophan (W), tyrosine (Y), proline (P), and histidine (H). Among these, an amino acid residue selected from isoleucine (I), valine (V), leucine (L), phenylalanine (F), cysteine (C), methionine (M), and alanine (A) is more preferable, and an amino acid residue selected from isoleucine (I), valine (V), leucine (L), and phenylalanine (F) is still more preferable.

In the sixth modified fibroin, the degree of hydrophobicity of REP is preferably -0.8 or more, more preferably -0.7 or more, still more preferably 0 or more, even still more preferably 0.3 or more, and particularly preferably 0.4 or more. The upper limit of the degree of hydrophobicity of REP is not particularly limited, and it may be 1.0 or less, and may be 0.7 or less.

In the present specification, the "degree of hydrophobicity of REP" is a value calculated by the following method.

In a fibroin (a modified fibroin or a naturally occurring fibroin) containing a domain sequence represented by

$$\text{Formula 1:} \qquad [(A)_n \text{ motif-REP}]m$$

or

$$\text{Formula 2:} \qquad [(A)_n \text{ motif-REP}]_m\text{-}(A)_n$$

motif, the degree of hydrophobicity of REP is calculated as v/t, where v is the total sum of the hydropathy index of each amino acid residue contained in all REPs contained in the domain a sequence excluding the sequence between the $(A)_n$ motif positioned closest to the C-terminal side and the C-terminal of the domain sequence (sequence corresponding to a "REGION A" in Fig. 5); and where t is the total number of amino acid residues in all REPs obtained by excluding the sequence between the $(A)_n$ motif positioned closest to the C-terminal side and the C-terminal of the domain sequence from the domain sequence and further excluding the $(A)_n$ motifs. The reason as to why the "domain sequence excluding the sequence between the $(A)_n$ motif positioned closest to the C-terminal side and the C-terminal of the domain sequence" is used for the calculation of the degree of hydrophobicity of REP is the same as the reason described above.

The domain sequence of the sixth modified fibroin may further have a modification of an amino acid sequence that corresponds to substitution, deletion, insertion, and/or addition of one or a plurality of amino acid residues, in addition to the modification that corresponds to deletion of one or a plurality of glutamine residues in REP and/or substitution of one or a plurality of glutamine residues in REP with other amino acid residues, as compared with a naturally occurring fibroin.

The sixth modified fibroin may be obtained, for example, from a cloned gene sequence of a naturally occurring fibroin, by deleting one or a plurality of glutamine residues in REP and/or substituting one or a plurality of glutamine residues in REP with other amino acid residues. In addition, the sixth modified fibroin may also be obtained by designing an amino acid sequence that corresponds to an amino acid sequence in which one or a plurality of glutamine residues in REP are deleted, and/or one or a plurality of glutamine residues in REP are substituted with other amino acid residues, based on the amino acid sequence of a naturally occurring fibroin, and chemically synthesizing a nucleic acid encoding the designed amino acid sequence.

More specific examples of the sixth modified fibroin include (6-i) a modified fibroin containing the amino acid sequence set forth in SEQ ID NO: 25 (Met-PRT888), SEQ ID NO: 26 (Met-PRT965), SEQ ID NO: 27 (Met-PRT889), SEQ ID NO: 28 (Met-PRT916), SEQ ID NO: 29 (Met-PRT918), SEQ ID NO: 30 (Met-PRT699), SEQ ID NO: 31 (Met-PRT698), SEQ ID NO: 32 (Met-PRT966), SEQ ID NO: 41 (Met-PRT917), or SEQ ID NO: 42 (Met-PRT1028), or (6-ii) a modified fibroin containing an amino acid sequence having 90% or more sequence identity with the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, or SEQ ID NO: 42.

The modified fibroin (6-i) will be described. The amino acid sequence set forth in SEQ ID NO: 25 is obtained by substituting all QQs in an amino acid sequence (Met-PRT410) set forth in SEQ ID NO: 7 with VLs. The amino acid sequence set forth in SEQ ID NO: 26 is obtained by substituting all QQs in the amino acid sequence set forth in SEQ ID NO: 7 with TSs, and substituting the remaining Qs with As. The amino acid sequence set forth in SEQ ID NO: 27 is obtained by substituting all QQs in the amino acid sequence set forth in SEQ ID NO: 7 with VLs, and substituting the remaining Qs with Is. The amino acid sequence set forth in SEQ ID NO: 28 is obtained by substituting all QQs in the amino acid sequence set forth in SEQ ID NO: 7 with VIs, and substituting the remaining Qs with Ls. The amino acid sequence set forth in SEQ ID NO: 29 is obtained by substituting all QQs in the amino acid sequence set forth in SEQ ID NO: 7 with VFs, and substituting the remaining Qs with Is.

The amino acid sequence set forth in SEQ ID NO: 30 is obtained by substituting all QQs in the amino acid sequence (Met-PRT525) set forth in SEQ ID NO: 8 with VLs. The amino acid sequence set forth in SEQ ID NO: 31 is obtained by substituting all QQs in the amino acid sequence set forth in SEQ ID NO: 8 with VLs, and substituting the remaining Qs with Is.

The amino acid sequence set forth in SEQ ID NO: 32 is a sequence obtained by substituting all QQs in a sequence obtained by repeating twice a region of 20 domain sequences present in the amino acid sequence (Met-PRT410) set forth in SEQ ID NO: 7 with VFs, and substituting the remaining Qs with Is.

The amino acid sequence (Met-PRT917) set forth in SEQ ID NO: 41 is an amino acid sequence in which all QQs are substituted with LIs and the remaining Qs are substituted with Vs in the amino acid sequence set forth in SEQ ID NO: 7. The amino acid sequence (Met-PRT1028) set forth in SEQ ID NO: 42 is an amino acid sequence in which all QQs are substituted with IFs and the remaining Qs are substituted with Ts in the amino acid sequence set forth in SEQ ID NO: 7.

The glutamine residue content rate of any of the amino acid sequences set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, and SEQ ID NO: 42 is 9% or less (Table 2).

[Table 2]

| Modified fibroin | Glutamine residue content rate | GPGXX motif content rate | Degree of hydrophobicity of REP |
|---|---|---|---|
| Met-PRT410 (SEQ ID NO: 7) | 17.7% | 27.9% | -1.52 |
| Met-PRT888 (SEQ ID NO: 25) | 6.3% | 27.9% | -0.07 |
| Met-PRT965 (SEQ ID NO: 26) | 0.0% | 27.9% | -0.65 |
| Met-PRT889 (SEQ ID NO: 27) | 0.0% | 27.9% | 0.35 |
| Met-PRT916 (SEQ ID NO: 28) | 0.0% | 27.9% | 0.47 |
| Met-PRT918 (SEQ ID NO: 29) | 0.0% | 27.9% | 0.45 |
| Met-PRT699 (SEQ ID NO: 30) | 3.6% | 26.4% | -0.78 |

(continued)

| Modified fibroin | Glutamine residue content rate | GPGXX motif content rate | Degree of hydrophobicity of REP |
|---|---|---|---|
| Met-PRT698 (SEQ ID NO: 31) | 0.0% | 26.4% | -0.03 |
| Met-PRT966 (SEQ ID NO: 32) | 0.0% | 28.0% | 0.35 |
| Met-PRT917 (SEQ ID NO: 41) | 0.0% | 27.9% | 0.46 |
| Met-PRT1028 (SEQ ID NO: 42) | 0.0% | 28.1% | 0.05 |

The modified fibroin (6-i) may be a modified fibroin consisting of the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, or SEQ ID NO: 42.

The modified fibroin (6-ii) may be a modified fibroin containing an amino acid sequence having 90% or more sequence identity with the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, or SEQ ID NO: 42. The modified fibroin (6-ii) also is a protein containing a domain sequence represented by

Formula 1: $[(A)_n \text{ motif-REP}]_m$

or

Formula 2: $[(A)_n \text{ motif-REP}]_m\text{-}(A)_n$

motif. The sequence identity is preferably 95% or more.

The modified fibroin (6-ii) preferably has a glutamine residue content rate of 9% or less. In addition, the modified fibroin (6-ii) preferably has a GPGXX motif content rate of 10% or more.

The sixth modified fibroin may contain a tag sequence at either or both of the N-terminal and C-terminal. This makes it possible to isolate, immobilize, detect, and visualize the modified fibroin.

More specific examples of the modified fibroin containing a tag sequence include a modified fibroin containing (6-iii) the amino acid sequence set forth in SEQ ID NO: 33 (PRT888), SEQ ID NO: 34 (PRT965), SEQ ID NO: 35 (PRT889), SEQ ID NO: 36 (PRT916), SEQ ID NO: 37. (PRT918), SEQ ID NO: 38 (PRT699), SEQ ID NO: 39 (PRT698), SEQ ID NO: 40 (PRT966), SEQ ID NO: 43 (PRT917), or SEQ ID NO: 44 (PRT1028), or a modified fibroin containing (6-iv) an amino acid sequence having 90% or more sequence identity with the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39 or SEQ ID NO: 40, SEQ ID NO: 43, or SEQ ID NO: 44.

The amino acid sequences set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, and SEQ ID NO: 44 are amino acid sequences in which the amino acid sequence (containing a His tag sequence and a hinge sequence) set forth in SEQ ID NO: 11 is added to the N-terminal of the amino acid sequences set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, and SEQ ID NO: 42, respectively. Since only the tag sequence is added to the N-terminal, the glutamine residue content rate is not changed, and any of the amino acid sequences set forth in SEQ ID NO:33, SEQ ID NO:34, SEQ ID NO:35, SEQ ID NO:36, SEQ ID NO:37, SEQ ID NO:38, SEQ ID NO:39, SEQ ID NO: 40, SEQ ID NO: 43, and SEQ ID NO: 44 has a glutamine residue content rate of 9% or less (Table 3).

[Table 3]

| Modified fibroin | Glutamine residue content rate | GPGXX motif content rate | Degree of hydrophobicity of REP |
|---|---|---|---|
| PRT888 (SEQ ID NO: 33) | 6.3% | 27.9% | -0.07 |

(continued)

| Modified fibroin | Glutamine residue content rate | GPGXX motif content rate | Degree of hydrophobicity of REP |
|---|---|---|---|
| PRT965 (SEQ ID NO: 34) | 0.0% | 27.9% | -0.65 |
| PRT889 (SEQ ID NO: 35) | 0.0% | 27.9% | 0.35 |
| PRT916 (SEQ ID NO: 36) | 0.0% | 27.9% | 0.47 |
| PRT918 (SEQ ID NO: 37) | 0.0% | 27.9% | 0.45 |
| PRT699 (SEQ ID NO: 38) | 3.6% | 26.4% | -0.78 |
| PRT698 (SEQ ID NO: 39) | 0.0% | 26.4% | -0.03 |
| PRT966 (SEQ ID NO: 40) | 0.0% | 28.0% | 0.35 |
| PRT917 (SEQ ID NO: 43) | 0.0% | 27.9% | 0.46 |
| PRT1028 (SEQ ID NO: 44) | 0.0% | 28.1% | 0.05 |

The modified fibroin (6-iii) may be a modified fibroin consisting of the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, or SEQ ID NO: 44.

The modified fibroin (6-iv) is a modified fibroin containing an amino acid sequence having 90% or more sequence identity with the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, or SEQ ID NO: 44. The modified fibroin (6-iv) also is a protein containing a domain sequence represented by

$$\text{Formula 1:} \qquad [(A)_n \text{ motif-REP}]_m$$

or

$$\text{Formula 2:} \qquad [(A)_n \text{ motif-REP}]_m\text{-}(A)_n$$

motif. The sequence identity is preferably 95% or more.

The modified fibroin (6-iv) preferably has a glutamine residue content rate of 9% or less. In addition, the modified fibroin (6-iv) preferably has a GPGXX motif content rate of 10% or more.

The sixth modified fibroin may contain a secretory signal for releasing the protein produced in a recombinant protein production system to the outside of the host. The sequence of the secretory signal may be set as appropriate, depending on the type of the host.

The modified fibroin may be a modified fibroin simultaneously pertaining at least two or more characteristics among the characteristics of the first modified fibroin, the second modified fibroin, the third modified fibroin, the fourth modified fibroin, the fifth modified fibroin, and the sixth modified fibroin.

(Method for Manufacturing Modified Fibroin)

The modified fibroin (hereinafter also simply referred to as a "protein") according to any of the embodiments may also be produced, for example, by expressing a nucleic acid by a host transformed with an expression vector having the nucleic acid sequence encoding the protein and one or a plurality of regulatory sequences operably linked to the nucleic acid sequence.

A method for producing a nucleic acid encoding a modified fibroin is not particularly limited. For example, the nucleic acid may be produced by a method in which a gene encoding a natural fibroin is cloned by amplification with a polymerase chain reaction (PCR) or the like, and subjected to a modification by a genetic engineering technique, or a method of chemically synthesizing a nucleic acid. The method for chemically synthesizing a nucleic acid is not particularly limited, and for example, a gene may be chemically synthesized by a method in which oligonucleotides are automatically synthesized by AKTA oligopilot plus 10/100 (GE Healthcare Japan Corporation) or the like, and are linked by PCR or the like, based on the amino acid sequence information of a protein obtained from the NCBI web database or the like. At this time, in order to facilitate purification and/or confirmation of the modified fibroin, a nucleic acid encoding a modified fibroin consisting of an amino acid sequence obtained by adding an amino acid sequence consisting of a start codon and a His10 tag to the N-terminal of the above-described amino acid sequence may be synthesized.

The regulatory sequence is a sequence (for example, a promoter, an enhancer, a ribosome binding sequence, or a transcription termination sequence) that controls the expression of a modified fibroin in a host, and may be selected as appropriate, depending on the type of the host. As a promoter, an inducible promoter that functions in a host cell and is capable of inducing the expression of a modified fibroin may be used. The inducible promoter is a promoter that can control transcription by the presence of an inducer (expression inducer), the absence of a repressor molecule, or physical factors such as an increase or decrease in a temperature, an osmotic pressure, or a pH value.

The type of the expression vector such as a plasmid vector, a viral vector, a cosmid vector, a fosmid vector, or an artificial chromosome vector may be selected as appropriate, depending on the type of the host. As the expression vector, an expression vector that can autonomously replicate in a host cell or can be incorporated into a chromosome of a host and which contains a promoter at a position capable of transcribing the nucleic acid that encodes a modified fibroin is suitably used.

Both prokaryotes and eukaryotes such as yeast, filamentous fungi, insect cells, animal cells, and plant cells may be suitably used as a host.

Preferred examples of the prokaryotic host cells include bacteria belonging to the genus Escherichia, the genus Brevibacillus, the genus Serratia, the genus Bacillus, the genus Microbacterium, the genus Brevibacterium, the genus Corynebacterium, and the genus Pseudomonas. Examples of microorganisms belonging to the genus Escherichia include Escherichia coli. Examples of microorganisms belonging to the genus Brevibacillus include Brevibacillus agri. Examples of microorganisms belonging to the genus Serratia include Serratia liquefaciens. Examples of microorganisms belonging to the genus Bacillus include Bacillus subtilis. Examples of microorganisms belonging to the genus Microbacterium include Microbacterium ammoniaphilum. Examples of microorganisms belonging to the genus Brevibacterium include Brevibacterium divaricatum. Examples of microorganisms belonging to the genus Corynebacterium include Corynebacterium ammoniagenes. Examples of microorganisms belonging to the genus Pseudomonas include Pseudomonas putida.

In a case where a prokaryote is used as a host, examples of a vector into which a nucleic acid encoding a modified fibroin is introduced include pBTrp2 (manufactured by Boehringer Mannheim), pGEX (manufactured by Pharmacia), pUC18, pBluescriptII, pSupex, pET22b, pCold, pUB110, and pNCO2 (Japanese Unexamined Patent Publication No. 2002-238569).

Examples of the eukaryotic hosts include yeast and filamentous fungi (mold and the like). Examples of the yeasts include yeasts belonging to the genus Saccharomyces, the genus Pichia, and the genus Schizosaccharomyces. Examples of the filamentous fungi include filamentous fungi belonging to the genus Aspergillus, the genus Penicillium, and the genus Trichoderma.

In a case where the eukaryote is used as a host, examples of the vector into which a nucleic acid encoding a modified fibroin is introduced include YEp13 (ATCC37115) and YEp24 (ATCC37051). As a method for introducing an expression vector into the host cell, any of methods of introducing DNA into the host cell may be used. Examples thereof include a method using calcium ions [Proc. Natl. Acad. Sci. USA, 69, 2110 (1972)], electroporation method, spheroplast method, protoplast method, lithium acetate method, and competent method.

As for the method for expressing a nucleic acid using a host transformed with an expression vector, secretory production, fusion protein expression, or the like, in addition to the direct expression, may be performed in accordance with the method described in Molecular Cloning, 2nd edition, and the like.

The modified fibroin may be produced, for example, by culturing a host transformed with the expression vector in a culture medium, producing and accumulating the protein in the culture medium, and then collecting the modified fibroin from the culture medium. The method for culturing a host in a culture medium may be performed in accordance with a method commonly used for culturing a host.

In a case where the host is a prokaryote such as Escherichia coli or a eukaryote such as yeast, any of a natural medium and a synthetic medium may be used as a culture medium of the host as long as the medium contains a carbon source, a nitrogen source, inorganic salts, and the like which may be utilized by the host and the medium may be used for efficiently culturing the host.

The carbon source may be any of carbon sources which can be assimilated by a transformed microorganism, and for example, carbohydrates such as glucose, fructose, sucrose, and molasses, starch, and starch hydrolysates containing these, organic acids such as acetic acid and propionic acid, and alcohols such as ethanol and propanol may be used. As the nitrogen source, for example, ammonium salts of inorganic or organic acids such as ammonia, ammonium chloride, ammonium sulfate, ammonium acetate, and ammonium phosphate, other nitrogen-containing compounds, peptone, meat extract, yeast extract, corn steep liquor, casein hydrolysate, soybean cake and soybean cake hydrolysate, and various fermented microbial cells and digested products thereof may be used. As the inorganic salts, for example, potassium dihydrogen phosphate, dipotassium phosphate, magnesium phosphate, magnesium sulfate, sodium chloride, ferrous sulfate, manganese sulfate, copper sulfate, and calcium carbonate may be used.

Culture of a prokaryote such as Escherichia coli or a eukaryote such as a yeast may be performed under aerobic conditions such as shaking culture or deep aeration stirring culture. The culture temperature is, for example, 15°C to 40°C. The culture time is usually 16 hours to 7 days. It is preferable to maintain the pH of the culture medium during the culture at 3.0 to 9.0. The pH of the culture medium may be adjusted using an inorganic acid, an organic acid, an alkali solution, urea, calcium carbonate, ammonia, or the like.

In addition, antibiotics such as ampicillin and tetracycline may be added to the culture medium as necessary during the culture. In a case of culturing a microorganism transformed with an expression vector using an inducible promoter as the promoter, an inducer may be added to the medium as necessary. For example, in a case of culturing a microorganism transformed with an expression vector using a lac promoter, isopropyl-β-D-thiogalactopyranoside or the like may be added to the medium, and in a case of culturing a microorganism transformed with an expression vector using a trp promoter, indole acrylic acid or the like may be added to the medium.

Isolation and purification of the expressed modified fibroin may be performed by a commonly used method. For example, in a case where the modified fibroin is expressed in a dissolved state in cells, the host cells are recovered by centrifugation after the completion of the culture, suspended in an aqueous buffer solution, and then disrupted using an ultrasonicator, a French press, a Manton-Gaulin homogenizer, a Dyno-Mill, or the like to obtain a cell-free extract. From the supernatant obtained by centrifuging the cell-free extract, a purified preparation may be obtained by a method commonly used for protein isolation and purification, that is, a solvent extraction method, a salting-out method using ammonium sulfate or the like, a desalting method, a precipitation method using an organic solvent, an anion exchange chromatography method using a resin such as diethylaminoethyl (DEAE)-Sepharose or DIAION HPA-75 (manufactured by Mitsubishi Kasei Kogyo Kabushiki Kaisha), an cation exchange chromatography method using a resin such as S-Sepharose FF (manufactured by Pharmacia Corporation), a hydrophobic chromatography method using a resin such as butyl sepharose or phenyl sepharose, a gel filtration method using a molecular sieve, an affinity chromatography method, a chromatofocusing method, or an electrophoresis method such as isoelectric focusing or the like, using the above-mentioned methods singly or in combination thereof.

In addition, in a case where the modified fibroin is expressed to form an insoluble body in the cell, similarly, the host cells are recovered, disrupted, and centrifuged to recover the insoluble body of the modified fibroin as a precipitated fraction. The recovered insoluble body of the modified fibroin may be solubilized with a protein denaturing agent. After this operation, a purified preparation of the modified fibroin may be obtained by the same isolation and purification method as described above. In a case where the protein is secreted extracellularly, the modified fibroin may be recovered from the culture supernatant. That is, a culture supernatant is obtained by treating the culture by a technique such as centrifugation, and a purified preparation may be obtained from the culture supernatant by using the same isolation and purification method as described above.

(Raw material fiber)

A raw material fiber according to the embodiment is obtained by spinning the above-described modified fibroin, and includes the above-described modified fibroin as a main component. The raw material fiber according to the embodiment is a fiber after spinning and before contact with water.

(Method of manufacturing raw material fiber)

The raw material fiber according to the embodiment can be manufactured by a known spinning method. That is, for example, when a raw material fiber including a modified fibroin as a main component is manufactured, first, the modified fibroin manufactured according to the above-described method is added to and dissolved in a solvent such as dimethyl sulfoxide (DMSO), N,N-dimethylformamide (DMF), a formic acid, or hexafluoroisopropanol (HFIP), if necessary, together with an inorganic salt as a dissolution accelerator to prepare a dope solution (a spinning dope solution). Then, spinning by a known spinning method such as wet spinning, dry spinning, dry-wet spinning, or melt spinning is performed using the dope solution, and a desired raw material fiber can be obtained. Preferably, the spinning method is wet spinning or dry-wet spinning.

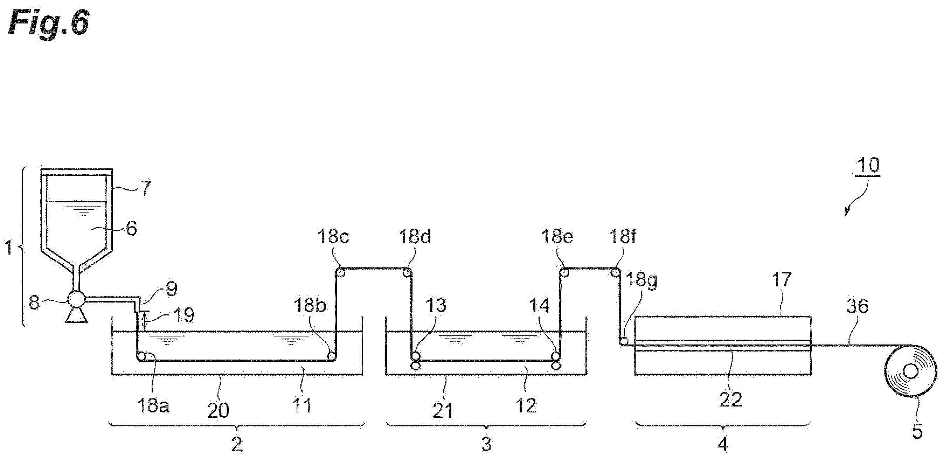

FIG. 6 is an explanatory diagram schematically showing an example of a spinning apparatus for producing a raw material fiber. A spinning apparatus 10 shown in FIG. 6 is an example of a spinning apparatus for dry-wet spinning and includes an extrusion device 1, an undrawn yarn manufacturing device 2, a wet heat drawing device 3, and a drying device 4.

A spinning method using the spinning apparatus 10 will be described. First, a dope solution 6 stored in a storage tank 7 is extruded from a tap 9 by a gear pump 8. In a laboratory scale, the dope solution may be filled into a cylinder and then may be extruded from a nozzle using a syringe pump. Next, the extruded dope solution 6 is supplied into a coagulating solution 11 of a coagulating solution tank 20 through an air gap 19, the solvent is removed, the modified fibroin is coagulated, and a fibrous coagulated body is formed. Next, the fibrous coagulated body is supplied and drawn into hot water 12 in a drawing bath 21. A drawing ratio is determined by a speed ratio between a supply nip roller 13 and a take-up nip roller 14. After that, the drawn fibrous coagulated body is supplied to the drying device 4 and dried in a yarn path 22, and a raw material fiber is obtained as a wound yarn body 5. 18a to 18g are thread guides.

The coagulating solution 11 may be any solvent which can be desolvated, and examples thereof include acetone, lower alcohols having 1 to 5 carbon atoms such as methanol, ethanol and 2-propanol, and the like. The coagulating solution 11 may appropriately contain water. A temperature of the coagulating solution 11 is preferably 0 to 30°C. When a syringe pump having a nozzle with a diameter of 0.1 to 0.6 mm is used as the tap 9, an extrusion speed is preferably 0.2 to 6.0 ml/hour, and more preferably 1.4 to 4.0 ml/hour per hole. A distance through which a coagulated protein passes through the coagulating solution 11 (substantially, a distance from the thread guide 18a to the thread guide 18b) may be a length which allows efficient desolvation, and is, for example, 200 to 500 mm. A take-up speed of the undrawn yarn may be, for example, 1 to 20 m/min, and preferably 1 to 3 m/min. A residence time in the coagulating solution 11 may be, for example, 0.01 to 3 minutes, and preferably 0.05 to 0.15 minutes. Moreover, drawing (pre-drawing) may be performed in the coagulating solution 11. The coagulating solution tank 20 may be provided in multiple stages, and the drawing may be performed in each of the stages or in a specific stage, if necessary.

For example, in addition to the pre-drawing performed in the coagulating solution tank 20 and the wet heat drawing performed in the drawing bath 21, dry heat drawing is also adopted as the drawing performed when the raw material fiber is obtained.

The wet heat drawing can be performed in warm water, in a solution in which an organic solvent or the like is added to the warm water, or in steam heating. A temperature thereof may be, for example, 50 to 90°C, and preferably 75 to 85°C. In the wet heat drawing, the undrawn yarn (or pre-drawn yarn) can be drawn, for example, 1 to 10 times, and preferably 2 to 8 times.

The dry heat drawing can be performed using an electric tubular furnace, a dry heat plate, or the like. A temperature thereof may be, for example, 140°C to 270°C, and preferably 160°C to 230°C. In the dry heat drawing, the undrawn yarn (or the pre-drawn yarn) can be drawn 0.5 to 8 times, for example, and preferably 1 to 4 times.

The wet heat drawing and the dry heat drawing may be performed individually, or may be performed in multiple stages or in combination. That is, the wet heat drawing and the dry heat drawing can be appropriately combined, such as performing the first stage drawing by the wet heat drawing and performing the second stage drawing by the dry heat drawing, or performing the first stage drawing by the wet heat drawing, performing the second stage drawing by the wet heat drawing and further performing the third stage drawing by the dry heat drawing, or the like.

The lower limit of the final drawing ratio is preferably any one of more than 1 time, 2 times or more, 3 times or more, 4 times or more, 5 times or more, 6 times or more, 7 times or more, 8 times or more, and 9 times or more with respect to the undrawn yarn (or the pre-drawn yarn), and the upper limit thereof is preferably 40 times or less, 30 times or less, 20 times or less, 15 times or less, 14 times or less, 13 times or less, 12 times or less, 11 times or less, or 10 times or less. When the raw material fiber is a fiber spun with a drawing ratio of 2 times or more, a contraction rate when the raw material fiber is brought into contact with water and is in a wet state becomes higher.

(Method of manufacturing artificial hair fiber (artificial fibroin fiber))

The artificial hair fiber (the artificial fibroin fiber) according to the embodiment can be obtained by a manufacturing method including a contracting step of contracting the raw material fiber with water. The contracting step may include, for example, a step (a contact step) in which the above-described raw material fiber (the raw material fiber after spinning and before contact with water) is brought into contact with water and irreversibly contracts. The contracting step may include a step (a drying step) of drying the fiber and further contracting the fiber after the contact step.

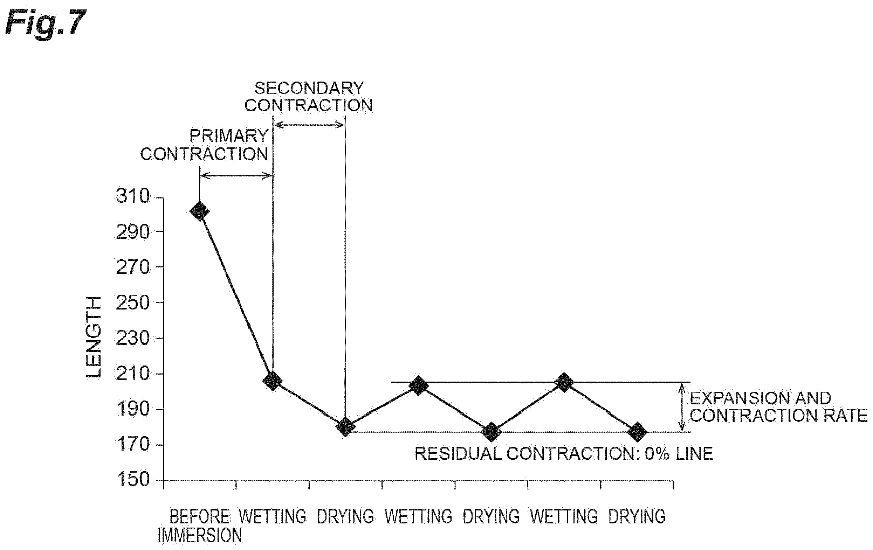

FIG. 7 is a diagram showing an example of a change in a length of the raw material fiber (the fiber containing the modified fibroin) due to contact with water. The raw material fiber (the fiber containing the modified fibroin) according to the embodiment has a property in which it contracts when it comes into contact with water (is wet) (a change in the length shown by a "primary contraction" in FIG. 7). After the primary contraction, when it is dried, it further contracts (a change in the length shown by a "secondary contraction" in FIG. 7). After the secondary contraction, when it is brought into contact with water again, it expands to the same or similar length as before the secondary contraction, and after that, when drying and wetting are repeated, the contraction and the expansion are repeated with the same width as that in the secondary contraction (a width indicated by a "expansion and contraction rate" in FIG. 7). Therefore, the artificial hair fiber according to the embodiment can be obtained by a manufacturing method which includes at least the contracting step including the contact step.

The irreversible contraction (the "primary contraction" in FIG. 7) of the raw material fiber (the fiber containing the modified fibroin) in the contact step is considered to occur, for example, for the following reasons. That is, one reason is considered to be due to a secondary structure or a tertiary structure of the raw material fiber (the fiber containing the modified fibroin), and another reason is considered to be, for example, due to residual stress being relaxed by infiltration of water between the fibers or into the fiber in the raw material fiber (the fiber containing the modified fibroin) having residual stress due to drawing or the like in the manufacturing process. Therefore, it is considered that the contraction rate of the raw material fiber (the fiber containing the modified fibroin) in the contracting step can be arbitrarily controlled, for example, according to a magnitude of the drawing ratio in the manufacturing process of the above-described raw material fiber (the fiber containing the modified fibroin).

In the contact step, the raw material fiber after spinning and before contact with water is brought into contact with water to bring the raw material fiber into the wet state. The wet state is a state in which at least a part of the raw material fiber is wet with water. Thus, the raw material fiber can be contracted without depending on an external force. This contraction is irreversible (corresponding to the "primary contraction" in FIG. 7).

A temperature of the water brought into contact with the raw material fiber in the contact step may be below a boiling point. Thus, handleability and workability of the contracting step are improved. Further, from the viewpoint of sufficiently shortening a contraction time, the lower limit of the temperature of the water is preferably 10°C or higher, more preferably 40°C or higher, and further preferably 70°C or higher. The upper limit of the temperature of the water is preferably 90°C or lower.

In the contact step, a method of bringing water into contact with the raw material fiber is not particularly limited. Examples of the method include a method of immersing the raw material fiber in water, a method of spraying the raw material fiber with water at room temperature or in a heated steam state, a method of exposing the raw material fiber in a high humidity environment filled with water vapor, and the like. In the contact step, among these methods, the method of immersing the raw material fiber in water is preferable because the contraction time can be effectively shortened and a processing facility can be simplified.

In the contact step, when the raw material fiber is brought into contact with water in a relaxed state, the raw material fiber may not only simply contract but also shrink in a wavy manner. In order to prevent occurrence of such shrinkage, the contact step may be carried out in a state in which the raw material fiber is not relaxed, for example, the raw material fiber is brought into contact with water while it is tensioned (pulled) in a fiber axis direction.

The method of manufacturing an artificial hair fiber (an artificial fibroin fiber) according to the embodiment may further include a drying step. The drying step is a step of drying the raw material fiber (or the artificial hair fiber obtained through the contact step) which have been subjected to the contact step and further contracting the raw material fiber (corresponding to the "secondary contraction" in FIG. 7). The drying may be, for example, natural drying, or forced drying using a drying facility. As the drying facility, any known contact type or non-contact type drying facility can be used. Further, although a drying temperature is not limited, for example, as long as it is lower than a temperature at which the protein contained in the raw material fiber is decomposed or the raw material fiber is thermally damaged, generally, the temperature is in a range of 20 to 150°C, and the temperature is preferably in a range of 50 to 100°C. The fiber is dried more quickly and efficiently without causing the thermal damage to the fiber or the decomposition of the protein contained in the fiber by keeping the temperature in this range. A drying time is appropriately set according to the drying temperature and the like. For example, a time during which an influence of overdrying on the quality and physical properties of the artificial hair fiber (the artificial fibroin fiber) can be eliminated as much as possible is adopted.

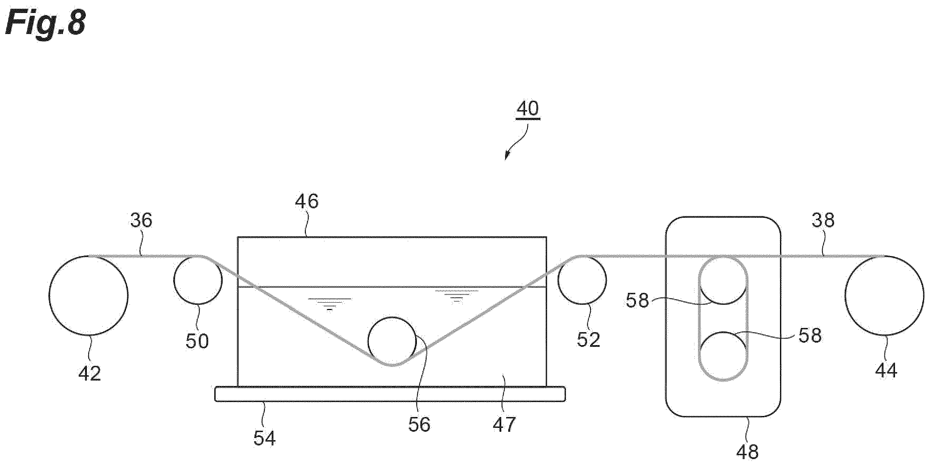

FIG. 8 is an explanatory diagram schematically showing an example of a manufacturing apparatus for manufacturing an artificial hair fiber (an artificial fibroin fiber). A manufacturing apparatus 40 shown in FIG. 8 includes a feed roller 42 which feeds out a raw material fiber, a winder 44 which winds up an artificial hair fiber 38, a water bath 46 which performs the contact step, and a dryer 48 which performs the drying step.

More specifically, the feed roller 42 is formed so that a wound material of a raw material fiber 36 can be mounted thereon, and the raw material fiber 36 can be continuously and automatically delivered from the wound material of the raw material fiber 36 by rotation of an electric motor or the like (not shown). The winder 44 can continuously and automatically wind up the artificial hair fiber 38 manufactured through the contact step and the drying step after it is delivered from the feed roller 42 by rotation of an electric motor (not shown). Here, a delivering speed of the raw material fiber 36 by the feed roller 42 and a winding speed of the artificial hair fiber 38 by the winder 44 can be controlled independently of each other.

The water bath 46 and the dryer 48 are arranged and disposed between the feed roller 42 and the winder 44 on the upstream side and the downstream side in a delivering direction of the raw material fiber 36. The manufacturing apparatus 40 shown in FIG. 8 includes relay rollers 50 and 52 which relay the raw material fiber 36 before and after the contact step which travels from the feed roller 42 toward the winder 44.

The water bath 46 includes a heater 54, and water 47 heated by the heater 54 is accommodated in the water bath 46. Further, in the water bath 46, a tension roller 56 is installed in a state in which it is immersed in the water 47. Thus, the raw material fiber 36 delivered from the feed roller 42 travels toward the winder 44 side while it is immersed in the water 47 in a state in which it is wound around the tension roller 56 in the water bath 46. An immersion time of the raw material fiber 36 in the water 47 is appropriately controlled according to a traveling speed of the raw material fiber 36.

The dryer 48 includes a pair of hot rollers 58. The pair of hot rollers 58 are formed so that the raw material fiber 36 which is separated from the water bath 46 and travels toward the winder 44 side can be wound thereon. Thus, the raw material fiber 36 immersed in the water 47 in the water bath 46 is heated by the pair of hot rollers 58 in the dryer 48, dried, and then further delivered toward the winder 44.

When the artificial hair fiber 38 is manufactured using the manufacturing apparatus 40 having such a structure, first, for example, the wound material of the raw material fiber 36 spun using the spinning apparatus 10 shown in FIG. 6 is mounted on the feed roller 42. Next, the raw material fiber 36 is continuously delivered from the feed roller 42 and is immersed in the water 47 in the water bath 46. At this time, for example, a winding speed of the winder 44 is set to be slower than the delivering speed of the feed roller 42. Thus, since the raw material fiber 36 contracts due to contact with the water 47 in a state in which it is tense between the feed roller 42 and the winder 44 not to relax, the occurrence of the shrinkage can be prevented. The raw material fiber 36 contracts irreversibly due to the contact with the water 47 (corresponding to the "primary contraction" in FIG. 7).

Next, the raw material fiber 36 after the contact with the water 47 (or the artificial hair fiber 38 manufactured through the contact with the water 47) is heated by the pair of hot rollers 58 of the dryer 48. Thus, the raw material fiber 36 after contact with water 47 (or the artificial hair fiber 38 manufactured through the contact with the water 47) can be dried and further contracted (corresponding to the "secondary contraction" in FIG. 7). At this time, the artificial hair fiber 38 can be further contracted or the length thereof can be kept unchanged by controlling a ratio between the delivering speed of the feed roller 42 and the winding speed of the winder 44. Then, the obtained artificial hair fiber 38 is wound by a winder 44, and the wound material of the artificial hair fiber 38 is obtained.

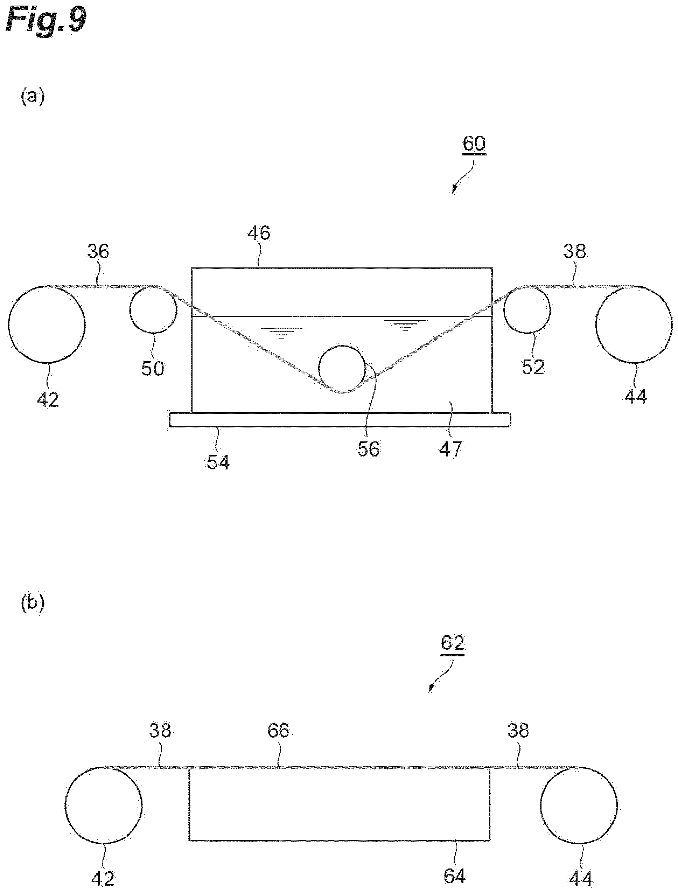

Instead of the pair of hot rollers 58, the raw material fiber 36 after the contact with the water 47 may be dried using a drying facility only including a simple heat source such as a dry heat plate 64 as shown in FIG. 9(b). Also in this case, the artificial hair fiber 38 can be further contracted, or the length thereof can be kept unchanged by adjusting a relative speed between the delivering speed of the feed roller 42 and the winding speed of the winder 44 in the same manner as when the pair of hot rollers 58 are used as the drying facility. Here, a drying means is configured of the dry heat plate 64. Moreover, the dryer 48 is not indispensable.

As described above, the target artificial hair fiber 38 can be manufactured automatically, continuously, and extremely easily using the manufacturing apparatus 40.

FIG. 9 is an explanatory diagram schematically showing another example of the manufacturing apparatus for manufacturing an artificial hair fiber (an artificial fibroin fiber). FIG. 9(a) shows a processing device provided in the manufacturing apparatus to perform the contact step, and FIG. 9(b) shows a drying device provided in the manufacturing apparatus to perform the drying step. The manufacturing apparatus shown in FIG. 9 includes a processing device 60 which performs a contact step with respect to the raw material fiber 36, and a drying device 62 which dries the raw material fiber 36 after the contact step (or the artificial hair fiber 38 manufactured through the contact step), and they have structures which are independent of each other.

More specifically, the processing device 60 shown in FIG. 9(a) has a structure in which the dryer 48 is omitted from the manufacturing apparatus 40 shown in FIG. 8, and the feed roller 42, the water bath 46, and the winder 44 are arranged and disposed in order from the upstream side to the downstream side in the traveling direction of the raw material fiber 36. Such a processing device 60 is formed to immerse the raw material fiber 36 delivered from the feed roller 42 in the water 47 in the water bath 46 and to contract the raw material fiber 36. Additionally, the processing device 60 is configured so that the obtained artificial hair fiber 38 is wound up by the winder 44.

The drying device 62 shown in FIG. 9(b) includes the feed roller 42, the winder 44, and the dry heat plate 64. The dry heat plate 64 is disposed between the feed roller 42 and the winder 44 so that a dry heat surface 66 comes into contact with the artificial hair fiber 38 and extends in the traveling direction thereof. In the drying device 62, the artificial hair fiber 38 can be further contracted or the length thereof can be kept unchanged, for example, by controlling the ratio between the delivering speed of the feed roller 42 and the winding speed of the winder 44 as described above.

The raw material fiber 36 can be contracted by the processing device 60 to obtain the artificial hair fiber 38 using the manufacturing apparatus having such a structure, and then the artificial hair fiber 38 can be dried by the drying device 62.

The feed roller 42 and the winder 44 may be omitted from the processing device 60 shown in FIG. 9(a), and the processing device may be configured only of the water bath 46. When a manufacturing apparatus having such a processing device is used, for example, the artificial hair fiber is manufactured in a so-called batch manner. Further, the drying device 62 shown in FIG. 9(b) is not indispensable.

(Artificial hair fibers (artificial fibroin fibers))

Since the artificial hair fibers (the artificial fibroin fibers) according to the embodiment are obtained by, for example, the above-described manufacturing method, they expand when they are in a wet state and contract when they dry from a wet state (corresponding to expansion and contraction after the "secondary contraction" in FIG. 7). Since the artificial hair fiber (the artificial fibroin fiber) according to the embodiment is obtained by, for example, the above-described manufacturing method, it does not substantially contain residual stress generated by drawing in the spinning process.

The artificial hair fiber according to the embodiment may have a restoration rate of 95% or more as defined by the following Formula (1).

$$\text{Formula (1): Restoration rate} = (\text{length of artificial fibroin fiber when dried from wet state/length of artificial fibroin fiber before wet state}) \times 100 \ (\%)$$

As the restoration rate defined by Formula (1) becomes higher, the behavior during wetting and drying becomes closer to that of human hair, and thus it becomes possible to curb occurrence of a feeling of strangeness with artificial hair in comparison with human hair. In the artificial hair fiber according to the embodiment, the restoration rate defined by Formula (1) is preferably 96% or more, more preferably 97% or more, further preferably 98% or more, and even more preferably 99% or more.

The artificial hair fiber according to the embodiment may have an expansion rate of 17% or less as defined by the following Formula (4). The expansion rate defined by Formula (4) is an index of expansion characteristics when the artificial hair fiber is in the wet state.

$$\text{Formula (4): Expansion rate} = \{(\text{length of artificial fibroin fiber in wet state/length of artificial fibroin fiber before wet state}) - 1\} \times 100 \ (\%)$$

The artificial hair fiber according to the embodiment is not particularly limited in the expansion rate defined by Formula (4) as long as the restoration rate defined by Formula (1) is high. Examples of the upper limit of the expansion rate defined by Formula (4) are 15% or less, 13% or less, 10% or less, or 5% or less, and examples of the lower limit thereof are more than 0%, 1% or more, 2% or more, 5% or more, 10% or more, or 13% or more. The expansion rate defined by Formula (4) in the artificial hair fiber according to the embodiment may be, for example, more than 0% and 17% or less, more than 0% and 15% or less, 2% or more and 15% or less, 5% or more and 15% or less, 5% or more and 13% or less, 5% or more and 10% or less, more than 0% and 10% or less, or more than 0% and 5% or less. However, from the viewpoint of reducing the feeling of strangeness between the artificial hair and human hair, it is preferable that the expansion rate defined by Formula (4) is small.

The artificial hair fiber according to the embodiment may have a contraction rate C of 17% or less as defined by the following Formula (5). The contraction rate C defined by Formula (5) is an index of contraction characteristics when the artificial hair fiber is dried from the wet state.

$$\text{Formula (5): Contraction rate C} = \{1 - (\text{length of artificial fibroin fiber when dried from wet state/length of artificial fibroin fiber in wet state})\} \times 100 \ (\%)$$

The artificial hair fiber according to the embodiment is not particularly limited in the contraction rate C defined by Formula (5) as long as the restoration rate defined by Formula (1) is high. Examples of the upper limit of the contraction rate C defined by Formula (5) are 15% or less, 13% or less, 10% or less, and 5% or less, and examples of the lower limit thereof are more than 0%, 1% or more, 2% or more, 5% or more, 10% or more, and 13% or more. The contraction rate C defined by Formula (5) in the artificial hair fiber according to the embodiment may be, for example, more than 0% and 17% or less, more than 0% and 15% or less, 2% or more and 15% or less, 5% or more and 15% or less, 5% or more and 13% or less, 5% or more and 10% or less, more than 0% and less than 10%, or more than 0% and less than 5%. However, from the viewpoint of reducing the feeling of strangeness between the artificial hair and the human hair, it is preferable that the contraction rate C defined by Formula (5) be small.

The artificial hair fiber according to the embodiment is a fiber having a contraction history in which it has been irreversibly contracted by contact with water after spinning, and preferably has a contraction rate A of 2% or more defined by the following Formula (2). The contraction rate A defined by Formula (2) is an index showing characteristics of the raw material fiber. When the contraction rate A defined by Formula (2) is 2% or more, the artificial hair fiber has a behavior similar to that of human hair during wetting and drying.

$$\text{Formula (2): Contraction rate A} = \{1\text{-(length of fiber irreversibly contracted by contact with water after spinning/length of fiber after spinning and before contact with water)}\} \times 100\ (\%)$$

The contraction rate A defined by Formula (2) may be 2.5% or more, 3% or more, 3.5% or more, 4% or more, 4.5% or more, 5% or more, 5.5% or more, 6% or more, 10% or more, 15% or more, 20% or more, or 25% or more. The upper limit of the contraction rate A defined by Formula (2) is not particularly limited and may be 80% or less, 60% or less, 40% or less, 20% or less, 10% or less, 7% or less, 6% or less, 5% or less, 4% or less, or 3% or less.

The artificial hair fiber according to the embodiment is a fiber having a contraction history in which it has been irreversibly contracted by contact with water after spinning and then further contracted by drying, and the contraction rate B defined by the following Formula (3) is preferably more than 7%. The contraction rate B defined by Formula (3) is an index showing characteristics of the raw material fiber. When the contraction rate B defined by Formula (3) is more than 7%, the artificial hair fiber has a behavior similar to that of human hair during wetting and drying.

$$\text{Formula (3): Contraction rate B} = \{1\text{-(length of fiber irreversibly contracted by contact with water after spinning and then further contracted by drying/length of fiber after spinning and before contact with water)}\} \times 100\ (\%)$$

The contraction rate B defined by Formula (3) may be 10% or more, 15% or more, more than 25%, 32% or more, 40% or more, 48% or more, 56% or more, 64% or more, or 72% or more. The upper limit of the contraction rate B defined by Formula (3) is not particularly limited, but it is usually 80% or less.

The artificial hair fiber according to the embodiment preferably has recesses extending in the fiber axis direction in the surface. Gloss is curbed and an exterior similar to that of human hair can be realized by providing recesses (grooves) in the surface. For example, when the raw material fiber is spun, a spinning method which is a wet spinning method, a method of slowing down a desolvation speed (for example, a method of adding a solvent of a doping solution to a coagulating solution), a method described in International Publication No. 2016/201369 (for example, a method of lengthening a residence time in a coagulation bath (60 seconds or more), and a method of changing a solvent ratio in the coagulation bath) can be adopted as a method of providing a recess in the surface of the artificial hair fiber.

The artificial hair fiber according to the embodiment may have a heat contraction rate of 4% or less as defined by the following Formula (6).

$$\text{Formula (6): Heat contraction rate} = \{1\text{-(length of artificial fibroin fiber when heated to 160°C/length of artificial fibroin fiber before heating)}\} \times 100\ (\%)$$

Since the artificial hair fiber according to the embodiment is made of the artificial fibroin fiber containing a modified fibroin, a softening point is higher than that of a synthetic fiber, and a temperature thereof is comparable to that of human hair. Therefore, a heat contraction rate at 160°C (a heat contraction rate defined by Formula (6)) is small. A hot air temperature of a hair dryer is 120 to 140°C, and an optimum temperature for using a curling iron is 170°C or less. Since the heat contraction rate defined by Formula (6) is small (for example, 4% or less), damage when a hair dryer or a curling iron is used can be curbed. The heat contraction rate defined by Formula (6) may be 3% or less, or 2.5% or less.

Since the artificial hair fiber according to the embodiment is made of the artificial fibroin fiber containing a modified fibroin, it can also be manufactured as an animal-free material (excluding animal-derived components).

The artificial hair fiber according to the embodiment is suitably used as artificial hair (for example, a wig, an extension) because it can be stably supplied and occurrence of a feeling of strangeness in comparison with human hair can be curbed therewith.

**Examples**

Hereinafter, the present invention will be described in more detail based on Examples and the like. However, the present invention is not limited to the following Examples.

[Test example 1: manufacture and evaluation of artificial hair fiber (1)]

(1) Manufacture of modified fibroin

A modified fibroin (PRT399) having the amino acid sequence set forth in SEQ ID NO: 18, a modified fibroin (PRT380) having the amino acid sequence set forth in SEQ ID NO: 12, a modified fibroin (PRT410) having the amino acid sequence set forth in SEQ ID NO: 13, and a modified fibroin (PRT799) having the amino acid sequence set forth in SEQ ID NO: 15 were designed.

Nucleic acids encoding the four designed modified fibroins were synthesized. In each of the nucleic acids, an NdeI site was added to the 5'-terminal, and an EcoRI site was added downstream of a stop codon. These four types of nucleic acids were cloned into a cloning vector (pUC118). Then, each of the nucleic acids was cut out by a restriction enzyme treatment with NdeI and EcoRI, and then was recombined into a protein expression vector pET-22b (+) to obtain an expression vector.

*Escherichia coli* BLR (DE3) was transformed with the obtained pET-22b (+) expression vector. The transformed Escherichia coli was cultured in 2 mL of an LB medium containing ampicillin for 15 hours. A culture solution was added to 100 mL of a seed culture medium (Table 4) containing ampicillin so that $OD_{600}$ was 0.005. A temperature of the culture solution was maintained at 30°C, and flask culture was carried out until the $OD_{600}$ reached 5 (about 15 hours) to obtain a seed culture solution.

[Table 4]

| Seed culture medium | |
| --- | --- |
| Reagent | Concentration(g/L) |
| Glucose | 5.0 |
| $KH_2PO_4$ | 4.0 |
| $K_2HPO_4$ | 9.3 |
| Yeast Extract | 6.0 |
| Ampicillin | 0.1 |

The seed culture solution was added to a jar fermenter to which 500 mL of a production medium (Table 5) was added so that the $OD_{600}$ was 0.05. While the temperature of the culture solution was maintained at 37°C, the culture was carried out at a constant pH of 6.9. Moreover, a dissolved oxygen concentration in the culture solution was maintained at 20% of a dissolved oxygen saturation concentration.

[Table 5]

| Production medium | |
| --- | --- |
| Reagent | Concentration(g/L) |
| Glucose | 12.0 |
| $KH_2PO_4$ | 9.0 |
| $MgSO_4 \cdot 7H_2O$ | 2.4 |

(continued)

| Production medium | |
|---|---|
| Reagent | Concentration(g/L) |
| Yeast Extract | 15 |
| $FeSO_4 \cdot 7H_2O$ | 0.04 |
| $MnSO_4 \cdot 5H_2O$ | 0.04 |
| $CaCl_2 \cdot 2H_2O$ | 0.04 |
| Adecanol (Adeka, LG-295S) | 0.1 (mL/L) |

Immediately after glucose in the production medium was completely consumed, a feed solution (glucose 455 g/1 L, Yeast Extract 120 g/1 L) was added at a rate of 1 mL/min. While the temperature of the culture solution was maintained at 37°C, the culture was carried out at a constant pH of 6.9. The culture was carried out for 20 hours while the dissolved oxygen concentration in the culture solution was maintained at 20% of the dissolved oxygen saturation concentration. Then, 1 M isopropyl-$\beta$-thiogalactopyranoside (IPTG) was added to the culture solution to a final concentration of 1 mM to induce the expression of the desired modified fibroin. When 20 hours had passed after the addition of IPTG, the culture solution was centrifuged, and cells were collected. SDS-PAGE was performed using the cells prepared from the culture solutions before and after the addition of IPTG, and the expression of the desired modified fibroin was confirmed by appearance of a band corresponding to a size of the desired modified fibroin depending on the addition of IPTG.

The cells collected 2 hours after the addition of IPTG were washed with 20 mM Tris-HCl buffer solution (pH 7.4). The washed cells were suspended in 20 mM Tris-HCl buffer solution (pH 7.4) containing about 1 mM of PMSF, and the cells were disrupted with a high-pressure homogenizer (GEA Niro Soavi Co.). The disrupted cells were centrifuged to obtain a precipitate. The obtained precipitate was washed with 20 mM Tris-HCl buffer solution (pH 7.4) until it became highly pure. The washed precipitate was suspended in 8 M guanidine buffer solution (8 M guanidine hydrochloride, 10 mM sodium dihydrogen phosphate, 20 mM NaCl, 1 mM Tris-HCl, pH 7.0) to have a concentration of 100 mg/mL, and was dissolved by stirring at 60°C for 30 minutes with a stirrer. After dissolution, dialysis was performed with water using a dialysis tube (a cellulose tube 36/32 manufactured by Sanko Junyaku Co., Ltd.). A white agglutinating protein obtained after the dialysis was recovered by centrifugation. Water was removed from the recovered aggregated protein with a lyophilizer to obtain a lyophilized powder of the desired modified fibroin.

(2) Manufacture of raw material fiber

Dimethyl sulfoxide (DMSO) in which lithium chloride was dissolved to 4.0% by mass was used as a solvent, and the lyophilized powder of the modified fibroin was added thereto to have a concentration of 18% by mass or 24% by mass (refer to Table 6) and was dissolved for 3 hours using a shaker. Then, insoluble matter and bubbles were removed to obtain a modified fibroin solution.

The obtained modified fibroin solution was used as a dope solution (a spinning dope solution), and a spun and drawn raw material fiber was manufactured by dry-wet spinning using a spinning device according to the spinning apparatus 10 shown in FIG. 6. In the spinning apparatus 10 shown in FIG. 6, the used spinning device includes a second undrawn yarn manufacturing device (a second bath) between the undrawn yarn manufacturing device 2 (a first bath) and the wet heat drawing device 3 (a third bath). The conditions for dry-wet spinning were as follows.

Extrusion nozzle diameter: 0.2 mm
Solutions and temperatures in the first to third baths: refer to Table 6
Total drawing ratio: refer to Table 6
Drying temperature: 60°C

[Table 6]

| | Dope solution | | First bath | | Second bath | | Third bath | | Total drawing ratio (times) |
|---|---|---|---|---|---|---|---|---|---|
| | Modified fibroin | Concentration (mass%) | Solution | Tem. (°C) | Solution | Tem. (°C) | Solution | Tem. (°C) | |
| Manufacturing example 1 | PRT799 | 24 | | -5 | | 16 | | | 1 |
| Manufacturing example 2 | | | | | | | | | 2 |
| Manufacturing example 3 | | | | | | | | | 3 |
| Manufacturing example | | | | | | | | | 4 |
| Manufacturing example 5 | | 18 | | | | | | | 1 |
| Manufacturing example 6 | | | | | | | | | 2 |
| Manufacturing example 7 | | | | | | | | | 3 |
| Manufacturing example 8 | | | | | | | | | 4 |

EP 3 827 682 A1

(continued)

| | Dope solution | | First bath | | Second bath | | Third bath | | Total drawing ratio (times) |
|---|---|---|---|---|---|---|---|---|---|
| | Modified fibroin | Concentration (mass%) | Solution | Tem. (°C) | Solution | Tem. (°C) | Solution | Tem. (°C) | |
| Manufacturing example 9 | PRT410 | 24 | 100% methanol | -11 | 100% methanol | 14 | Water | 17 | 1 |
| Manufacturing example 10 | | | | | | | | | 2 |
| Manufacturing example 11 | | | | | | | | | 3 |
| Manufacturing example 12 | | | | | | | | | 4 |
| Manufacturing example 13 | PRT399 | | | | | | | | 1 |
| Manufacturing example 14 | | | | | | | | | 2 |
| Manufacturing example 15 | | | | | | | | | 3 |
| Manufacturing example 16 | PRT380 | | | | | 11 | | | 1 |
| Manufacturing example 17 | | | | | | | | | 2 |
| Manufacturing example 18 | | | | | | | | | 3 |
| Manufacturing example 19 | | | | | | | | | 4 |

(3) Manufacture of artificial hair fiber and evaluation of contraction rate A and contraction rate B

Artificial fibroin fibers (artificial hair fibers) were manufactured by subjecting the raw material fibers obtained in Manufacturing examples 1 to 19 to a contact step in which they were brought into contact with water or performing a drying step in which they were dried at room temperature after the contact step had been performed.

<Evaluation of contraction rate A in contact step>

A plurality of raw material fibers having a length of 30 cm were cut out from the wound materials of the raw material fibers obtained in Manufacturing examples 1 to 19. The plurality of raw material fibers were bundled to obtain a raw material fiber bundle having a fineness of 150 denier. A 0.8 g lead sinker was mounted on each of the raw material fiber bundles, and in that state, each of the raw material fiber bundles was immersed in water at the temperatures shown in Tables 7 to 10 for 10 minutes (the contact step). Then, a length of each of the raw material fiber bundles was measured in the water. The length of the raw material fiber bundle in the water was measured with the 0.8 g lead sinker mounted on the raw material fiber bundle to eliminate shrinkage of the raw material fiber bundle. Next, the contraction rate A (%) was calculated for each of the raw material fibers according to the following Formula (2). In Formula (2), L0 indicates the length of the fiber after spinning and before contact with water, which is 30 cm here. Similarly, in Formula (2), Lw indicates the length of fiber irreversibly contracted due to contact with water after spinning, and here is the length of each of the raw material fiber bundles measured in the water.

$$\text{Formula (2): Contraction rate A} = \{1-(Lw/L0)\} \times 100 \ (\%)$$

<Evaluation of contraction rate B in drying step>

After the contact step, the raw material fiber bundle was taken out of the water. The raw material fiber bundle taken out was dried at room temperature for 2 hours (the drying step) with the 0.8 g lead sinker mounted thereon, and thus the artificial fibroin fiber (the fiber for artificial hair) was obtained. After drying, a length of each of the artificial fibroin fiber bundles was measured. Next, the contraction rate B (%) was calculated for each of the artificial fibroin fibers according to the following Formula (3). In Formula (3), L0 indicates the length of the fiber after spinning and before the contact with the water, which is 30 cm here. Similarly, in Formula (3), Lwd indicates the length of the fiber irreversibly contracted by the contact with the water after spinning and then further contracted by drying, and here is the length of each of the artificial fibroin fiber bundles measured after drying.

$$\text{Formula (3): Contraction rate B} = \{1-(Lwd/L0)\} \times 100 \ (\%)$$

The results are shown in Tables 7 to 10.

[Table 7]

| Raw material fiber/artificial fibroin fiber | | Water temperature (°C) | Contraction rate A (%) | Contraction rate B (%) |
|---|---|---|---|---|
| Manufacturing example 1 | 24 wt% PRT799 x1 | 20 | 0.0 | 7.8 |
| Manufacturing example 2 | 24 wt% PRT799 x2 | | -1.2 | 10.3 |
| Manufacturing example 3 | 24wt% PRT799x3 | | 7.2 | 21.2 |
| Manufacturing example 4 | 24wt% PRT799x4 | | 13.5 | 26.3 |
| Manufacturing example 6 | 18wt% PRT799x2 | | -2.3 | 9.5 |
| Manufacturing example 7 | 18wt% PRT799x3 | | 6.0 | 19.7 |
| Manufacturing example 8 | 18wt% PRT799x4 | | 14.3 | 27.5 |
| Manufacturing example 2 | 24wt% PRT799x2 | 40 | -5.3 | 7.2 |
| Manufacturing example 3 | 24wt% PRT799x3 | | 8.7 | 21.3 |
| Manufacturing example 4 | 24wt% PRT799x4 | | 14.5 | 26.0 |
| Manufacturing example 6 | 18wt% PRT799x2 | | -4.3 | 7.3 |
| Manufacturing example 7 | 18wt% PRT799x3 | | 6.2 | 18.3 |
| Manufacturing example 8 | 18wt% PRT799x4 | | 16.0 | 28.7 |
| Manufacturing example 3 | 24wt% PRT799x3 | 60 | 6.8 | 21.0 |
| Manufacturing example 4 | 24wt% PRT799x4 | | 15.0 | 27.5 |
| Manufacturing example 6 | 18wt% PRT799x2 | | -1.5 | 10.7 |
| Manufacturing example 7 | 18wt% PRT799x3 | | 3.3 | 18.2 |
| Manufacturing example 8 | 18wt% PRT799x4 | | 16.2 | 29.0 |

[Table 8]

| Raw material fiber/artificial fibroin fiber | | Water temperature (°C) | Contraction rate A (%) | Contraction rate B (%) |
|---|---|---|---|---|
| Manufacturing example 10 | 24wt% PRT410x2 | 20 | -2.3 | 8.7 |
| Manufacturing example 11 | 24wt% PRT410x3 | | 4.7 | 16.7 |
| Manufacturing example 12 | 24wt% PRT410x4 | | 10.3 | 22.3 |
| Manufacturing example 11 | 24wt% PRT410x3 | 40 | 4.7 | 17.5 |
| Manufacturing example 12 | 24wt% PRT410x4 | | 11.5 | 24.0 |
| Manufacturing example 11 | 24wt% PRT410x3 | 60 | 2.0 | 16.5 |
| Manufacturing example 12 | 24wt% PRT410x4 | | 10.8 | 25.0 |

[Table 9]

| Raw material fiber/artificial fibroin fiber | | Water temperature (°C) | Contraction rate A (%) | Contraction rate B (%) |
|---|---|---|---|---|
| Manufacturing example 13 | 24wt% PRT399x1 | 20 | -3.5 | 7.6 |
| Manufacturing example 14 | 24wt% PRT399x2 | | 3.7 | 12.5 |
| Manufacturing example 15 | 24wt% PRT399x3 | | 7.0 | 16.8 |
| Manufacturing example 14 | 24wt% PRT399x2 | 40 | 3.0 | 12.7 |
| Manufacturing example 15 | 24wt% PRT399x3 | | 7.3 | 16.7 |
| Manufacturing example 14 | 24wt% PRT399x2 | 60 | 3.3 | 9.3 |
| Manufacturing example 15 | 24wt% PRT399x3 | | 6.8 | 14.2 |

[Table 10]

| Raw material fiber/artificial fibroin fiber | | Water temperature (°C) | Contraction rate A (%) | Contraction rate B (%) |
|---|---|---|---|---|
| Manufacturing example 16 | 24wt% PRT380x1 | 20 | -1.1 | 9.4 |
| Manufacturing example 17 | 24wt% PRT380x2 | | 2.7 | 13.3 |
| Manufacturing example 18 | 24wt% PRT380x3 | | 7.0 | 17.7 |
| Manufacturing example 19 | 24wt% PRT380x4 | | 10.0 | 20.2 |
| Manufacturing example 17 | 24wt% PRT380x2 | 40 | 3.3 | 14.2 |
| Manufacturing example 18 | 24wt% PRT380x3 | | 7.7 | 19.0 |
| Manufacturing example 19 | 24wt% PRT380x4 | | 12.0 | 22.0 |
| Manufacturing example 17 | 24wt% PRT380x2 | 60 | 2.7 | 14.3 |
| Manufacturing example 18 | 24wt% PRT380x3 | | 8.2 | 20.3 |
| Manufacturing example 19 | 24wt% PRT380x4 | | 12.0 | 23.2 |

[Test example 2: Manufacture and evaluation of artificial hair fiber]

(1) Manufacture of modified fibroin

A modified fibroin (PRT799) having the amino acid sequence set forth in SEQ ID NO: 15, a modified fibroin (PRT918) having the amino acid sequence set forth in SEQ ID NO: 37, a modified fibroin (PRT917) having the amino acid sequence set forth in SEQ ID NO: 43, and a modified fibroin (PRT1028) having the amino acid sequence set forth in SEQ ID NO: 44 were designed.

Nucleic acids encoding the four designed modified fibroins were synthesized. In each of the nucleic acids, an NdeI site was added to the 5'-terminal, and an EcoRI site was added downstream of a stop codon. These five types of nucleic acids were cloned into a cloning vector (pUC118). Then, each of the nucleic acids was cut out by a restriction enzyme treatment with NdeI and EcoRI, and then was recombined into a protein expression vector pET-22b (+) to obtain an expression vector.

Escherichia coli BLR (DE3) was transformed with the obtained pET22b (+) expression vector. The transformed Escherichia coli was cultured in 2 mL of an LB medium containing ampicillin for 15 hours. A culture solution was added to 100 mL of a seed culture medium (Table 11) containing ampicillin so that $OD_{600}$ was 0.005. A temperature of the culture solution was maintained at 30°C, and flask culture was carried out until the $OD_{600}$ reached 5 (about 15 hours) to obtain a seed culture solution.

[Table 11]

| Seed culture medium (per 1 L at the start of culture) | |
|---|---|
| Glucose | 5 g |
| $KH_2PO_4$ | 4 g |
| $K_2HPO_4$ | 10 g |
| Yeast Extract | 6 g |

Ampicillin was added to a final concentration of 100 mg/L to obtain a seed culture medium.

The seed culture solution was added to a jar fermenter to which 500 mL of a production medium (Table 12) was added so that the $OD_{600}$ was 0.05, and transformed Escherichia coli was inoculated. While the temperature of the culture solution was maintained at 37°C, the culture was carried out at a constant pH of 6.9. Further, a dissolved oxygen concentration in the culture solution was maintained at 20% of a dissolved oxygen saturation concentration.

[Table 12]

| Production medium (per 1 L at the start of culture) | |
| --- | --- |
| Glucose | 12 g |
| $KH_2PO_4$ | 9g |
| $MgSO_4 \cdot 7H_2O$ | 2.4 g |
| Yeast Extract | 15 g |
| $FeSO_4 \cdot 7H_2O$ | 40 mg |
| $MnSO_4 \cdot 5H_2O$ | 40 mg |
| $CaCl_2 \cdot 2H_2O$ | 40 mg |
| GD-113 (antifoaming agent) | 0.1 mL |

Immediately after glucose in the production medium was completely consumed, a feed solution (glucose 455 g/1 L, Yeast Extract 120 g/1 L) was added at a rate of 1 mL/min. While the temperature of the culture solution was maintained at 37°C, the culture was carried out at a constant pH of 6.9. Further, the culture was carried out for 20 hours, while the dissolved oxygen concentration in the culture solution was maintained at 20% of the dissolved oxygen saturation concentration. Then, 1 M isopropyl-β-thiogalactopyranoside (IPTG) was added to the culture solution to a final concentration of 1 mM to induce the expression of the target protein. When 20 hours had passed after the addition of IPTG, the culture solution was centrifuged, and the cells were collected. SDS-PAGE was performed using the cells prepared from the culture solutions before and after the addition of IPTG, and the expression of the target protein was confirmed by appearance of a band of a target protein size depending on the addition of IPTG.

The cells collected 2 hours after the addition of IPTG were washed with 20 mM Tris-HCl buffer solution (pH 7.4). The washed cells were suspended in 20 mM Tris-HCl buffer solution (pH 7.4) containing about 1 mM PMSF, and the cells were disrupted with a high-pressure homogenizer (GEA Niro Soavi Co.). The disrupted cells were centrifuged to obtain a precipitate. The obtained precipitate was washed with 20 mM Tris-HCl buffer solution (pH 7.4) until it became highly pure. The washed precipitate was suspended in 8 M guanidine buffer solution (8 M guanidine hydrochloride, 10 mM sodium dihydrogen phosphate, 20 mM NaCl, 1 mM Tris-HCl, pH 7.0) to have a concentration of 100 mg/mL, and was dissolved by stirring at 60°C for 30 minutes with a stirrer. After dissolution, dialysis was performed with water using a dialysis tube (a cellulose tube 36/32 manufactured by Sanko Junyaku Co., Ltd.). A white agglutinated protein obtained after the dialysis was recovered by centrifugation. Water was removed with a lyophilizer, and a lyophilized powder was recovered.

(2) Manufacture of raw material fiber

DMSO in which lithium chloride was dissolved to 4% by mass was used as a solvent, and the lyophilized powder of the modified fibroin was added thereto to have a concentration of 24% by mass. After dissolution with an aluminum block heater at 90°C for 1 hour, insoluble matter and bubbles were removed to obtain a dope solution (a spinning dope solution).

The dope solution was filled into a reserve tank and was discharged from a monohole nozzle having a diameter of 0.3 mm into a coagulation bath containing 100% by mass of methanol (a coagulation bath at a temperature of 12°C) using a gear pump. After coagulation, washing and drawing were performed in the coagulation bath having methanol of 100% by mass. After washing and drawing, drying was performed using a dry heat plate (at a drying temperature of 80°C), and the obtained raw yarn (the raw material fiber) was wound up. The drawing ratio was 6 times.

(3) Manufacture of artificial hair fiber

Each of the raw material fibers was bundled to have a length of about 30 cm to obtain a raw material fiber bundle having a fineness of 150 denier. A 0.8 g lead sinker was mounted on each of the raw material fiber bundles, and each of the raw material fiber bundles was immersed in water at 40°C for 10 minutes to contract it (the contact step) in that state, was removed from the water and dried at room temperature for 2 hours with the 0.8 g lead sinker mounted thereon (the drying step), and thus the artificial fibroin fibers (the artificial hair fibers) of Examples 1 to 4 having different types of proteins were obtained.

(4) Evaluation of artificial hair fibers (water flexibility)

A length of each of the artificial fibroin fibers of Examples 1 to 4 obtained in (3) in a dry state (a length of each of the artificial fibroin fibers before they are brought into the wet state) was measured. Next, the 0.8 g lead sinker was mounted on each of the artificial fibroin fibers, and the raw material fiber bundle was immersed in water at 40°C for 10 minutes in that state. Then, a length of each of the artificial fibroin fibers (the length of each of the artificial fibroin fibers in the wet state) was measured in the water. The length measurement of each of the artificial fibroin fibers in the water was carried out with the 0.8 g lead sinker mounted thereon to eliminate the shrinkage of each of the artificial fibroin fibers. Then, each of the artificial fibroin fibers taken out of the water was dried at room temperature for 2 hours with the 0.8 g lead sinker mounted thereon. After drying, the length of each of the artificial fibroin fibers (the length of the artificial fibroin fibers when dried from the wet state) was measured. The restoration rate, the expansion rate and the contraction rate C of each of the artificial fibroin fibers were calculated from obtained measured values according to the following Formulas (1), (4) and (5).

$$\text{Formula (1): Restoration rate} = (\text{length of artificial fibroin fiber when dried from wet state/length of artificial fibroin fiber before wet state}) \times 100 \ (\%)$$

$$\text{Formula (4): Expansion rate} = \{(\text{length of artificial fibroin fiber in wet state/length of artificial fibroin fiber before wet state})-1\} \times 100 \ (\%)$$

$$\text{Formula (5): Contraction rate C} = \{1-(\text{length of artificial fibroin fiber when dried from wet state/length of artificial fibroin fiber in wet state})\} \times 100 \ (\%)$$

For comparison, the above-described operations were carried out on synthetic fibers (Comparative example 1: Nylon, Comparative example 2: Polyester), and the restoration rate, the expansion rate and the contraction rate C were calculated.

For the artificial fibroin fibers of Examples 1 and 2, the above-described operation was repeated, and the restoration rate, the expansion rate, and the contraction rate C in the second cycle were calculated. The results are also shown in Table 13.

[Table 13]

| | | First cycle | | | Second cycle | | |
|---|---|---|---|---|---|---|---|
| | | Expansion rate (%) | Contraction rate C (%) | Restoration rate (%) | Expansion rate (%) | Contraction rate C (%) | Restoration rate (%) |
| Example 1 | PRT799 | 15.2 | 14.1 | 99 | 16.4 | 14.1 | 100 |
| Example 2 | PRT918 | 6.6 | 6.7 | 99.5 | 6.2 | 5.8 | 100 |
| Example 3 | PRT917 | 2.3 | 4 | 98.2 | - | - | - |
| Example 4 | PRT1028 | 4.9 | 5.6 | 99 | - | - | - |
| Comparative example 1 | Nylon | 0 | 0.22 | | - | - | - |
| Comparative example 2 | Polyester | 0.43 | 0 | 0 | - | - | - |

As shown in Table 13, the artificial fibroin fibers containing the modified fibroin (Examples 1 to 4) have the same characteristics as those of human hair that they expand in the wet state and then return to their original length when dried (a restoration rate of 98.2 to 100%). In addition, these characteristics are maintained even after repeated wetting and drying (Examples 1 and 2).

(5) Evaluation of artificial hair fibers (SEM observation)

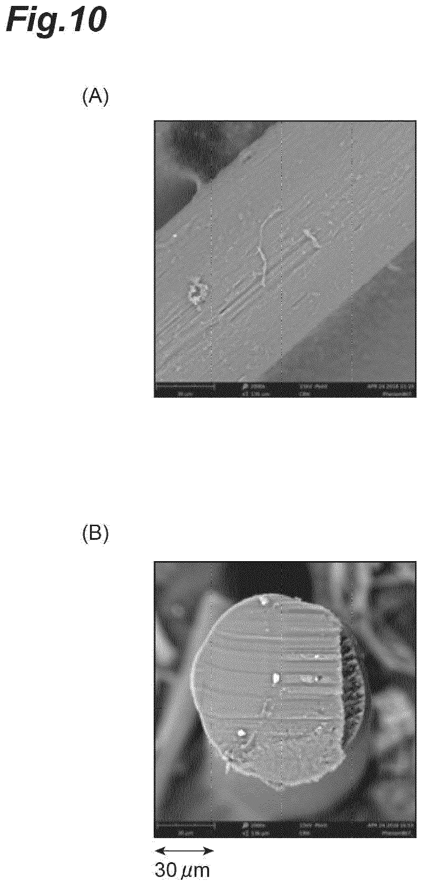

FIG. 10 is a scanning electron microscope (SEM) photograph of the artificial fibroin fiber (PRT918) of Example 2. FIG. 10(A) is an SEM photograph of a surface structure (a skin layer). FIG. 10(B) is an SEM photograph of an internal structure (a cutting surface). It can be seen that the recess is formed in the fiber axis direction.

(6) Evaluation of artificial hair fibers (heat contraction)

A heat contraction rate at 160°C was measured for the artificial fibroin fiber (PRT918) of Example 2 using a thermomechanical analyzer (model number: TMA4000SE, distributor: NETZSCH JAPAN Co., Ltd.). The measurement conditions were as follows.

Fiber length: 10 mm

Temperature rise rate: 10°C/min

Holding time: 1 min

Target temperature: 230°C

Tension load: 0.1 g

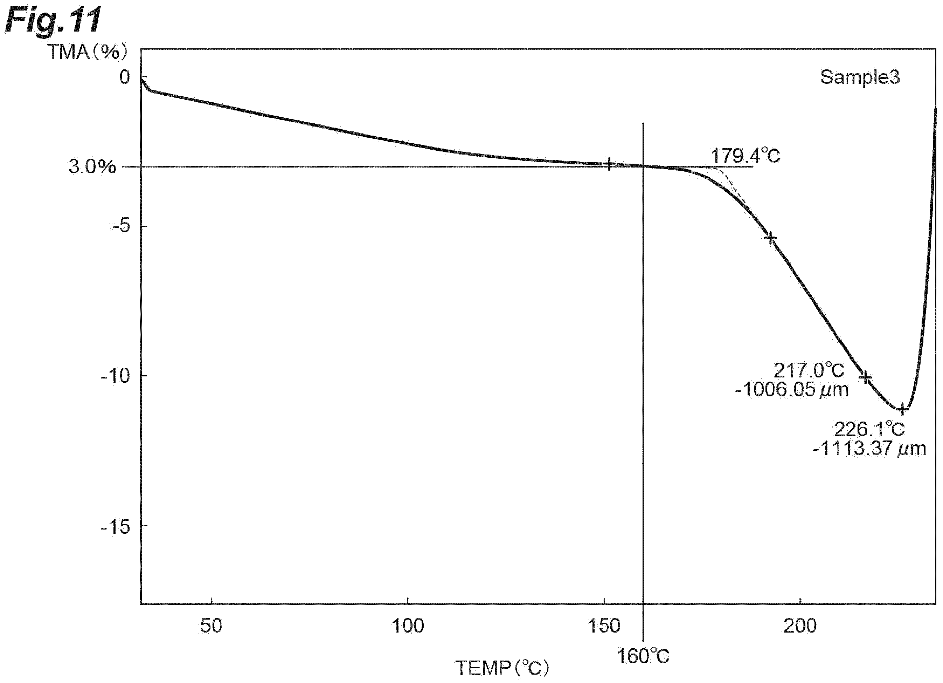

The measurement results are shown in FIG. 11. The heat contraction rate calculated according to the following Formula (6) was 3%.

$$\text{Formula (6): Heat contraction} = \{1 - (\text{length of artificial fibroin fiber when heated to } 160°\text{C/length of artificial fibroin fiber before heating})\} \times 100 \, (\%)$$

**Reference Signs List**

1 Extrusion device
2 Undrawn yarn manufacturing device
3 Wet heat drawing device
4 Drying device
6 Dope solution
10 Spinning apparatus
20 Coagulating solution tank
21 Drawing bath
36 Raw material fiber
38 Artificial hair fiber
40 Manufacturing apparatus
42 Feed roller
44 Winder
46 Water bath
48 Dryer
54 Heater
56 Tension roller

58 Hot roller
60 Processing device
62 Drying device
64 Dry heat plate

## Claims

1. An artificial hair fiber comprising:

   an artificial fibroin fiber containing a modified fibroin,
   wherein the artificial hair fiber expands when being in a wet state and contracts when being dried from the wet state.

2. The artificial hair fiber according to claim 1, wherein a restoration rate defined by the following Formula (1) is 95% or more.

$$\text{Restoration rate}=(\text{length of artificial fibroin fiber when dried from wet state/length of artificial fibroin fiber before wet state})\times100\ (\%)\ldots(1)$$

3. The artificial hair fiber according to claim 1 or 2, wherein:

the artificial fibroin fiber is a fiber having a contraction history in which the fiber is irreversibly contracted by contact with water after spinning, and
a contraction rate A defined by the following Formula (2) is 2% or more.

$$\text{Contraction rate A}=\{1-(\text{length of fiber irreversibly contracted by contact with water after spinning/length of fiber after spinning and before contact with water})\}\times100\ (\%)\ldots(2)$$

4. The artificial hair fiber according to any one of claims 1 to 3, wherein:

the artificial fibroin fiber is a fiber having a contraction history in which the fiber is irreversibly contracted by contact with water after spinning and then further contracted by drying, and
a contraction rate B defined by the following Formula (3) is more than 7%.

$$\text{Contraction rate B}=\{1-(\text{length of fiber irreversibly contracted by contact with water after spinning and then further contracted by drying/length of fiber after spinning and before contact with water})\}\times100\ (\%)\ldots(3)$$

5. The artificial hair fiber according to any one of claims 1 to 4, wherein the modified fibroin is a modified spider silk fibroin.

6. The artificial hair fiber according to any one of claims 1 to 5, wherein a recess which extends in a fiber axis direction is provided in a surface.

7. The artificial hair fiber according to any one of claims 1 to 6, wherein an expansion rate defined by the following Formula (4) is 17% or less.

$$\text{Expansion rate}=\{(\text{length of artificial fibroin fiber in wet state/length of artificial fibroin fiber before wet state})-1\}\times100\ (\%)\ldots(4)$$

8. The artificial hair fiber according to any one of claims 1 to 7, wherein a contraction rate C defined by the following Formula (5) is 17% or less.

$$\text{Contraction rate C}=\{1-(\text{length of artificial fibroin fiber when dried from wet state/length of artificial fibroin fiber in wet state})\times100\ (\%)\ldots(5)$$

9. The artificial hair fiber according to any one of claims 1 to 8, wherein a heat contraction rate defined by the following Formula (6) is 4% or less.

$$\text{Heat contraction rate} = \{1\text{-(length of artificial fibroin fiber when heated to } 160°\text{C/length of artificial fibroin fiber before heating)}\} \times 100 \ (\%)...(6)$$

10. The artificial hair fiber according to any one of claims 1 to 9, wherein the artificial hair fiber contains substantially no residual stress generated by drawing in a spinning process.

11. A method for manufacturing an artificial hair fiber, the method comprising:

a contraction step in which a raw material fiber after spinning and before contact with water is brought into contact with water to be irreversibly contracted, then dried and further contracted, wherein the raw material fiber contains a modified fibroin.

12. The method according to claim 11, wherein the raw material fiber is a fiber having a contraction rate A of 2% or more defined by the following Formula (2).

$$\text{Contraction rate A} = \{1\text{-(length of fiber irreversibly contracted by contact with water after spinning/length of fiber after spinning and before contact with water)}\} \times 100 \ (\%)...(2)$$

13. The method according to claim 11 or 12, wherein the raw material fiber is a fiber having a contraction rate B of more than 7% defined by the following Formula (3).

$$\text{Contraction rate B} = \{1\text{-(length of fiber irreversibly contracted by contact with water after spinning and then further contracted by drying/length of fiber after spinning and before contact with water)}\} \times 100 \ (\%)...(3)$$

14. The method according to any one of claims 11 to 13, wherein, in the contraction step, substantially all residual stress in the raw material fiber generated by drawing in a spinning process is released.

15. The method according to any one of claims 11 to 14, wherein the contraction step is performed without relaxing the raw material fiber.

16. The method according to any one of claims 11 to 15, wherein the raw material fiber is formed by introducing a spinning dope solution containing the modified fibroin and a solvent into a coagulating solution, removing the solvent from the spinning dope solution and coagulating the spinning dope solution.

17. The method according to any one of claims 11 to 16, wherein the modified fibroin is a modified spider silk fibroin.

18. An artificial hair comprising the artificial hair fiber according to any one of claims 1 to 10.

EP 3 827 682 A1

# Fig.1

Fig.2

EP 3 827 682 A1

*Fig.3*

Fig.4

# Fig.5

**Fig.6**

EP 3 827 682 A1

## Fig.7

Fig.8

# Fig.9

(a)

(b)

# *Fig.10*

(A)

(B)

30 μm

Fig.11

## INTERNATIONAL SEARCH REPORT

International application No.

PCT/JP2019/029077

**A. CLASSIFICATION OF SUBJECT MATTER**
Int.Cl. A41G3/00(2006.01)i

According to International Patent Classification (IPC) or to both national classification and IPC

**B. FIELDS SEARCHED**

Minimum documentation searched (classification system followed by classification symbols)
Int.Cl. A41G3/00-5/02, D01D1/00-13/02, D01F1/00-6/96, D01F9/00-9/04, D02G1/00-3/48, D02J1/00-13/00, D04H1/00-18/04

Documentation searched other than minimum documentation to the extent that such documents are included in the fields searched
Published examined utility model applications of Japan 1922–1996
Published unexamined utility model applications of Japan 1971–2019
Registered utility model specifications of Japan 1996–2019
Published registered utility model applications of Japan 1994–2019

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 6337252 B1 (SPIBER INC.) 06 June 2018, claims, fig. 1 & JP 2018-150637 A & WO 2018/164021 A1 | 1–18 |
| A | JP 5407009 B1 (SPIBER INC.) 05 February 2014, paragraph [0011] & US 2015/0141618 A1, abstract & WO 2014/002605 A1 & EP 2868782 A1 & CN 104395511 A | 1–18 |

☒ Further documents are listed in the continuation of Box C.    ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 07 October 2019 (07.10.2019) | 21 October 2019 (21.10.2019) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

International application No.

PCT/JP2019/029077

C (Continuation). DOCUMENTS CONSIDERED TO BE RELEVANT

| Category* | Citation of document, with indication, where appropriate, of the relevant passages | Relevant to claim No. |
|---|---|---|
| A | JP 2016-516141 A (N.O.M. COATINGS SIA) 02 June 2016, abstract, fig. 4 & US 2016/0348306 A1, abstract, fig. 3 & WO 2014/147459 A1 & CN 104379010 A & KR 10-2015-0132562 A & ZA 201506836 B | 1-18 |
| A | JP 45-20028 B1 (FUKAI, Shinichi) 08 July 1970, claims, fig. 2 (Family: none) | 1-18 |
| A | JP 53-3289 B2 (DAI ICHI SEIYAKU CO., LTD.) 04 February 1978, claims (Family: none) | 1-18 |
| A | JP 2009-127168 A (FUJII, Toshihiro) 11 June 2009, abstract (Family: none) | 1-18 |
| A | WO 2017/127940 A1 (DALHOUSIE UNIVERSITY) 03 August 2017, abstract, fig. 1 (Family: none) | 1-18 |

Form PCT/ISA/210 (continuation of second sheet) (January 2015)

**EP 3 827 682 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2007297737 A
- JP 2002238569 A
- WO 2016201369 A

### Non-patent literature cited in the description

- *Nucleic Acid Res.,* 1982, vol. 10, 6487
- *Methods in Enzymology,* 1983, vol. 100, 448
- **KYTE J ; DOOLITTLE R.** A simple method for displaying the hydropathic character of a protein. *J. Mol. Biol.,* 1982, vol. 157, 105-132
- *Proc. Natl. Acad. Sci. USA,* 1972, vol. 69, 2110
- Molecular Cloning

SEQUENCE LISTING

<110>  Spiber Inc.
       KOJIMA INDUSTRIES CORPORATION

<120>  A spun fiber for artificial hair and a method for producing the
       same, and an artificial hair

<130>  FP19-0704-00

<150>  JP2018-139563
<151>  2018-07-25

<160>  44

<170>  PatentIn version 3.5

<210>  1
<211>  50
<212>  PRT
<213>  Araneus diadematus

<400>  1

Ser Gly Cys Asp Val Leu Val Gln Ala Leu Leu Glu Val Val Ser Ala
1               5                   10                  15


Leu Val Ser Ile Leu Gly Ser Ser Ser Ile Gly Gln Ile Asn Tyr Gly
            20                  25                  30


Ala Ser Ala Gln Tyr Thr Gln Met Val Gly Gln Ser Val Ala Gln Ala
            35                  40                  45


Leu Ala
    50


<210>  2
<211>  30
<212>  PRT
<213>  Araneus diadematus

<400>  2

Ser Gly Cys Asp Val Leu Val Gln Ala Leu Leu Glu Val Val Ser Ala
1               5                   10                  15


Leu Val Ser Ile Leu Gly Ser Ser Ser Ile Gly Gln Ile Asn
            20                  25                  30


<210>  3
<211>  21
<212>  PRT
<213>  Araneus diadematus

<400>  3

Ser Gly Cys Asp Val Leu Val Gln Ala Leu Leu Glu Val Val Ser Ala

Leu Val Ser Ile Leu
20


<210> 4
<211> 1154
<212> PRT
<213> Artificial Sequence

<220>
<223> recombinant spider silk protein ADF3KaiLargeNRSH1

<400> 4

Met His His His His His His His His His His Ser Ser Gly Ser Ser
1                   5                   10                  15


Leu Glu Val Leu Phe Gln Gly Pro Ala Arg Ala Gly Ser Gly Gln Gln
            20                  25                  30


Gly Pro Gly Gln Gln Gly Pro Gly Gln Gln Gly Pro Gly Gln Gln Gly
            35                  40                  45


Pro Tyr Gly Pro Gly Ala Ser Ala Ala Ala Ala Ala Gly Gly Tyr
        50                  55                  60


Gly Pro Gly Ser Gly Gln Gln Gly Pro Ser Gln Gln Gly Pro Gly Gln
65                  70                  75                  80


Gln Gly Pro Gly Gly Gln Gly Pro Tyr Gly Pro Gly Ala Ser Ala Ala
                85                  90                  95


Ala Ala Ala Ala Gly Gly Tyr Gly Pro Gly Ser Gly Gln Gln Gly Pro
            100                 105                 110


Gly Gly Gln Gly Pro Tyr Gly Pro Gly Ser Ser Ala Ala Ala Ala Ala
            115                 120                 125


Ala Gly Gly Asn Gly Pro Gly Ser Gly Gln Gln Gly Ala Gly Gln Gln
            130                 135                 140


Gly Pro Gly Gln Gln Gly Pro Gly Ala Ser Ala Ala Ala Ala Ala Ala
145                 150                 155                 160


Gly Gly Tyr Gly Pro Gly Ser Gly Gln Gln Gly Pro Gly Gln Gln Gly
                165                 170                 175


Pro Gly Gly Gln Gly Pro Tyr Gly Pro Gly Ala Ser Ala Ala Ala Ala
            180                 185                 190

```
Ala Ala Gly Gly Tyr Gly Pro Gly Ser Gly Gln Gly Pro Gly Gln Gln
        195             200             205

Gly Pro Gly Gly Gln Gly Pro Tyr Gly Pro Gly Ala Ser Ala Ala Ala
        210             215             220

Ala Ala Ala Gly Gly Tyr Gly Pro Gly Ser Gly Gln Gln Gly Pro Gly
225             230             235             240

Gln Gln Gly Pro Gly Gln Gln Gly Pro Gly Gly Gln Gly Pro Tyr Gly
        245             250             255

Pro Gly Ala Ser Ala Ala Ala Ala Ala Ala Gly Gly Tyr Gly Pro Gly
        260             265             270

Tyr Gly Gln Gln Gly Pro Gly Gln Gln Gly Pro Gly Gly Gln Gly Pro
        275             280             285

Tyr Gly Pro Gly Ala Ser Ala Ala Ser Ala Ala Ser Gly Gly Tyr Gly
        290             295             300

Pro Gly Ser Gly Gln Gln Gly Pro Gly Gln Gln Gly Pro Gly Gly Gln
305             310             315             320

Gly Pro Tyr Gly Pro Gly Ala Ser Ala Ala Ala Ala Ala Ala Gly Gly
        325             330             335

Tyr Gly Pro Gly Ser Gly Gln Gln Gly Pro Gly Gln Gln Gly Pro Gly
        340             345             350

Gln Gln Gly Pro Gly Gln Gln Gly Pro Gly Gly Gln Gly Pro Tyr Gly
        355             360             365

Pro Gly Ala Ser Ala Ala Ala Ala Ala Ala Gly Gly Tyr Gly Pro Gly
        370             375             380

Ser Gly Gln Gln Gly Pro Gly Gln Gln Gly Pro Gly Gln Gln Gly Pro
385             390             395             400

Gly Gln Gln Gly Pro Gly Gln Gln Gly Pro Gly Gln Gln Gly Pro Gly
        405             410             415

Gln Gln Gly Pro Gly Gln Gln Gly Pro Gly Gln Gln Gly Pro Gly Gly
        420             425             430

Gln Gly Ala Tyr Gly Pro Gly Ala Ser Ala Ala Ala Gly Ala Ala Gly
```

435        440        445

Gly Tyr Gly Pro Gly Ser Gly Gln Gln Gly Pro Gly Gln Gln Gly Pro
    450           455           460

Gly Gln Gln Gly Pro Gly Gln Gln Gly Pro Gly Gln Gln Gly Pro Gly
465           470           475           480

Gln Gln Gly Pro Gly Gln Gln Gly Pro Gly Gln Gln Gly Pro Tyr Gly
          485           490           495

Pro Gly Ala Ser Ala Ala Ala Ala Ala Ala Gly Gly Tyr Gly Pro Gly
          500           505           510

Ser Gly Gln Gln Gly Pro Gly Gln Gln Gly Pro Gly Gln Gln Gly Pro
          515           520           525

Gly Gly Gln Gly Pro Tyr Gly Pro Gly Ala Ala Ser Ala Ala Val Ser
    530           535           540

Val Ser Arg Ala Arg Ala Gly Ser Gly Gln Gln Gly Pro Gly Gln Gln
545           550           555           560

Gly Pro Gly Gln Gln Gly Pro Gly Gln Gln Gly Pro Tyr Gly Pro Gly
          565           570           575

Ala Ser Ala Ala Ala Ala Ala Ala Gly Gly Tyr Gly Pro Gly Ser Gly
          580           585           590

Gln Gln Gly Pro Ser Gln Gln Gly Pro Gly Gln Gln Gly Pro Gly Gly
          595           600           605

Gln Gly Pro Tyr Gly Pro Gly Ala Ser Ala Ala Ala Ala Ala Ala Gly
          610           615           620

Gly Tyr Gly Pro Gly Ser Gly Gln Gln Gly Pro Gly Gly Gln Gly Pro
625           630           635           640

Tyr Gly Pro Gly Ser Ser Ala Ala Ala Ala Ala Ala Gly Gly Asn Gly
          645           650           655

Pro Gly Ser Gly Gln Gln Gly Ala Gly Gln Gln Gly Pro Gly Gln Gln
          660           665           670

Gly Pro Gly Ala Ser Ala Ala Ala Ala Ala Ala Gly Gly Tyr Gly Pro
          675           680           685

Gly Ser Gly Gln Gln Gly Pro Gly Gln Gln Gly Pro Gly Gly Gln Gly
    690               695               700

Pro Tyr Gly Pro Gly Ala Ser Ala Ala Ala Ala Ala Ala Gly Gly Tyr
705               710               715                   720

Gly Pro Gly Ser Gly Gln Gly Pro Gly Gln Gln Gly Pro Gly Gly Gln
            725               730               735

Gly Pro Tyr Gly Pro Gly Ala Ser Ala Ala Ala Ala Ala Ala Gly Gly
        740               745               750

Tyr Gly Pro Gly Ser Gly Gln Gln Gly Pro Gly Gln Gln Gly Pro Gly
        755               760               765

Gln Gln Gly Pro Gly Gly Gln Gly Pro Tyr Gly Pro Gly Ala Ser Ala
    770               775               780

Ala Ala Ala Ala Gly Gly Tyr Gly Pro Gly Tyr Gly Gln Gln Gly
785               790               795               800

Pro Gly Gln Gln Gly Pro Gly Gly Gln Gly Pro Tyr Gly Pro Gly Ala
            805               810               815

Ser Ala Ala Ser Ala Ala Ser Gly Gly Tyr Gly Pro Gly Ser Gly Gln
        820               825               830

Gln Gly Pro Gly Gln Gln Gly Pro Gly Gly Gln Gly Pro Tyr Gly Pro
        835               840               845

Gly Ala Ser Ala Ala Ala Ala Ala Ala Gly Gly Tyr Gly Pro Gly Ser
    850               855               860

Gly Gln Gln Gly Pro Gly Gln Gln Gly Pro Gly Gln Gln Gly Pro Gly
865               870               875               880

Gln Gln Gly Pro Gly Gly Gln Gly Pro Tyr Gly Pro Gly Ala Ser Ala
            885               890               895

Ala Ala Ala Ala Gly Gly Tyr Gly Pro Gly Ser Gly Gln Gln Gly
        900               905               910

Pro Gly Gln Gln Gly Pro Gly Gln Gln Gly Pro Gly Gln Gln Gly Pro
    915               920               925

Gly Gln Gln Gly Pro Gly Gln Gln Gly Pro Gly Gln Gln Gly Pro Gly
    930               935               940

```
Gln Gln Gly Pro Gly Gln Gln Gly Pro Gly Gly Gln Gly Ala Tyr Gly
945                 950             955                 960

Pro Gly Ala Ser Ala Ala Ala Gly Ala Ala Gly Gly Tyr Gly Pro Gly
                965             970                 975

Ser Gly Gln Gln Gly Pro Gly Gln Gln Gly Pro Gly Gln Gln Gly Pro
            980             985             990

Gly Gln Gln Gly Pro Gly Gln Gln  Gly Pro Gly Gln Gln  Gly Pro Gly
        995             1000                1005

Gln Gln  Gly Pro Gly Gln Gln  Gly Pro Tyr Gly Pro  Gly Ala Ser
    1010             1015             1020

Ala Ala  Ala Ala Ala Ala Gly  Gly Tyr Gly Pro Gly  Ser Gly Gln
    1025             1030             1035

Gln Gly  Pro Gly Gln Gln Gly  Pro Gly Gln Gln Gly  Pro Gly Gly
    1040             1045             1050

Gln Gly  Pro Tyr Gly Pro Gly  Ala Ala Ser Ala Ala  Val Ser Val
    1055             1060             1065

Gly Gly  Tyr Gly Pro Gln Ser  Ser Ser Val Pro Val  Ala Ser Ala
    1070             1075             1080

Val Ala  Ser Arg Leu Ser Ser  Pro Ala Ala Ser Ser  Arg Val Ser
    1085             1090             1095

Ser Ala  Val Ser Ser Leu Val  Ser Ser Gly Pro Thr  Lys His Ala
    1100             1105             1110

Ala Leu  Ser Asn Thr Ile Ser  Ser Val Val Ser Gln  Val Ser Ala
    1115             1120             1125

Ser Asn  Pro Gly Leu Ser Gly  Cys Asp Val Leu Val  Gln Ala Leu
    1130             1135             1140

Leu Glu  Val Val Ser Ala Leu  Val Ser Ile Leu
    1145             1150
```

```
<210>  5
<211>  24
<212>  PRT
<213>  Artificial Sequence

<220>
```

<223> His tag and start codon

<400> 5

Met His His His His His His His His His His Ser Ser Gly Ser Ser
1                   5                   10                  15

Leu Glu Val Leu Phe Gln Gly Pro
                20

<210> 6
<211> 597
<212> PRT
<213> Artificial Sequence

<220>
<223> Met-PRT380

<400> 6

Met Gly Pro Gly Gln Gln Gly Pro Tyr Gly Pro Gly Ala Ser Ala Ala
1                   5                   10                  15

Ala Ala Ala Gly Gln Asn Gly Pro Gly Ser Gly Gln Gln Gly Pro Gly
                20                  25                  30

Gln Ser Ala Ala Ala Ala Ala Gly Gln Tyr Gly Pro Gly Gln Gln Gly
                35                  40                  45

Pro Gly Gln Gln Gly Pro Gly Ser Ser Ala Ala Ala Ala Ala Gly Pro
        50                  55                  60

Gly Gln Tyr Gly Pro Gly Gln Gln Gly Pro Ser Ala Ser Ala Ala Ala
65                  70                  75                  80

Ala Ala Gly Pro Gly Ser Gly Gln Gln Gly Pro Gly Ala Ser Ala Ala
                85                  90                  95

Ala Ala Ala Gly Gln Tyr Gly Pro Gly Gln Gln Gly Pro Gly Gln Gln
                100                 105                 110

Gly Pro Gly Ser Ser Ala Ala Ala Ala Ala Gly Gln Tyr Gly Ser Gly
        115                 120                 125

Pro Gly Gln Gln Gly Pro Tyr Gly Ser Ala Ala Ala Ala Ala Gly Pro
        130                 135                 140

Gly Ser Gly Gln Tyr Gly Gln Gly Pro Tyr Gly Pro Gly Ala Ser Ala
145                 150                 155                 160

Ala Ala Ala Gly Pro Gly Gln Tyr Gly Pro Gly Gln Gln Gly Pro

|     | 165 |     |     |     |     | 170 |     |     |     |     | 175 |     |     |     |

Ser Ala Ser Ala Ala Ala Ala Ala Gly Ser Gly Gln Gln Gly Pro Gly
        180                 185                 190

Gln Tyr Gly Pro Tyr Ala Ser Ala Ala Ala Ala Gly Gln Tyr Gly
        195                 200                 205

Ser Gly Pro Gly Gln Gln Gly Pro Tyr Gly Pro Gly Gln Ser Ala Ala
        210                 215                 220

Ala Ala Ala Gly Ser Gly Gln Gln Gly Pro Gly Gln Gln Gly Pro Tyr
225                 230                 235                 240

Ala Ser Ala Ala Ala Ala Ala Gly Pro Gly Gln Gln Gly Pro Tyr Gly
                245                 250                 255

Pro Gly Ser Ser Ala Ala Ala Ala Ala Gly Gln Tyr Gly Tyr Gly Pro
                260                 265                 270

Gly Gln Gln Gly Pro Tyr Gly Pro Gly Ala Ser Ala Ala Ala Ala Ala
        275                 280                 285

Gly Gln Asn Gly Pro Gly Ser Gly Gln Tyr Gly Pro Gly Gln Gln Gly
        290                 295                 300

Pro Gly Gln Ser Ala Ala Ala Ala Ala Gly Pro Gly Gln Gln Gly Pro
305                 310                 315                 320

Tyr Gly Pro Gly Ala Ser Ala Ala Ala Ala Gly Gln Tyr Gly Pro
                325                 330                 335

Gly Gln Gln Gly Pro Gly Gln Tyr Gly Pro Gly Ser Ser Ala Ala Ala
        340                 345                 350

Ala Ala Gly Pro Gly Gln Gln Gly Pro Tyr Gly Pro Gly Ser Ser Ala
        355                 360                 365

Ala Ala Ala Gly Gln Tyr Gly Pro Gly Gln Gln Gly Pro Tyr Gly
        370                 375                 380

Pro Gly Gln Ser Ala Ala Ala Ala Gly Gln Tyr Gln Gln Gly Pro
385                 390                 395                 400

Gly Gln Gln Gly Pro Tyr Gly Pro Gly Ala Ser Ala Ala Ala Ala Ala
                405                 410                 415

```
Gly Pro Gly Gln Gln Gly Pro Tyr Gly Pro Gly Ala Ser Ala Ala Ala
            420                 425             430


Ala Ala Gly Pro Gly Gln Tyr Gly Pro Gly Gln Gln Gly Pro Ser Ala
            435                 440             445


Ser Ala Ala Ala Ala Ala Gly Gln Tyr Gly Ser Gly Pro Gly Gln Tyr
    450                 455             460


Gly Pro Tyr Gly Pro Gly Gln Ser Ala Ala Ala Ala Gly Pro Gly
465             470                 475             480


Ser Gly Gln Gln Gly Gln Gly Pro Tyr Gly Pro Gly Ala Ser Ala Ala
                485                 490             495


Ala Ala Ala Gly Gln Tyr Gly Pro Gly Gln Gln Gly Pro Tyr Gly Pro
            500                 505             510


Gly Gln Ser Ala Ala Ala Ala Ala Gly Pro Gly Ser Gly Gln Tyr Gly
        515                 520             525


Pro Gly Ala Ser Ala Ala Ala Ala Gly Gln Asn Gly Pro Gly Ser
        530                 535             540


Gly Gln Tyr Gly Pro Gly Gln Gln Gly Pro Gly Gln Ser Ala Ala Ala
545             550                 555             560


Ala Ala Gly Gln Tyr Gln Gln Gly Pro Gly Gln Gln Gly Pro Tyr Gly
            565                 570                 575


Pro Gly Ala Ser Ala Ala Ala Ala Ala Gly Pro Gly Ser Gly Gln Gln
            580                 585             590


Gly Pro Gly Ala Ser
            595
```

```
<210>  7
<211>  590
<212>  PRT
<213>  Artificial Sequence

<220>
<223>  Met-PRT410

<400>  7

Met Gly Pro Gly Gln Gln Gly Pro Tyr Gly Pro Gly Ala Ser Ala Ala
1               5                   10                  15


Ala Ala Ala Gly Gln Asn Gly Pro Gly Ser Gly Gln Gln Gly Pro Gly
```

|  |  |  | 20 |  |  |  | 25 |  |  |  | 30 |  |  |
|---|---|---|---|---|---|---|---|---|---|---|---|---|---|

Gln Ser Gly Gln Tyr Gly Pro Gly Gln Gln Gly Pro Gly Gln Gln Gly
       35         40        45

Pro Gly Ser Ser Ala Ala Ala Ala Ala Gly Pro Gly Gln Tyr Gly Pro
       50         55        60

Gly Gln Gln Gly Pro Ser Ala Ser Ala Ala Ala Ala Gly Pro Gly
65         70         75        80

Ser Gly Gln Gln Gly Pro Gly Ala Ser Gly Gln Tyr Gly Pro Gly Gln
         85         90        95

Gln Gly Pro Gly Gln Gln Gly Pro Gly Ser Ser Ala Ala Ala Ala Ala
       100      105      110

Gly Gln Tyr Gly Ser Gly Pro Gly Gln Gln Gly Pro Tyr Gly Ser Ala
      115      120      125

Ala Ala Ala Ala Gly Pro Gly Ser Gly Gln Tyr Gly Gln Gly Pro Tyr
      130      135      140

Gly Pro Gly Ala Ser Gly Pro Gly Gln Tyr Gly Pro Gly Gln Gln Gly
145         150      155      160

Pro Ser Ala Ser Ala Ala Ala Ala Ala Gly Ser Gly Gln Gln Gly Pro
      165      170      175

Gly Gln Tyr Gly Pro Tyr Ala Ser Ala Ala Ala Ala Ala Gly Gln Tyr
      180      185      190

Gly Ser Gly Pro Gly Gln Gln Gly Pro Tyr Gly Pro Gly Gln Ser Gly
      195      200      205

Ser Gly Gln Gln Gly Pro Gly Gln Gln Gly Pro Tyr Ala Ser Ala Ala
      210      215      220

Ala Ala Ala Gly Pro Gly Gln Gln Gly Pro Tyr Gly Pro Gly Ser Ser
225         230      235      240

Ala Ala Ala Ala Ala Gly Gln Tyr Gly Tyr Gly Pro Gly Gln Gln Gly
      245      250      255

Pro Tyr Gly Pro Gly Ala Ser Gly Gln Asn Gly Pro Gly Ser Gly Gln
      260      265      270

```
Tyr Gly Pro Gly Gln Gln Gly Pro Gly Gln Ser Ala Ala Ala Ala Ala
        275                 280                 285

Gly Pro Gly Gln Gln Gly Pro Tyr Gly Pro Gly Ala Ser Ala Ala Ala
        290                 295                 300

Ala Ala Gly Gln Tyr Gly Pro Gly Gln Gln Gly Pro Gly Gln Tyr Gly
305                 310                 315                 320

Pro Gly Ser Ser Gly Pro Gly Gln Gln Gly Pro Tyr Gly Pro Gly Ser
                325                 330                 335

Ser Ala Ala Ala Ala Ala Gly Gln Tyr Gly Pro Gly Gln Gln Gly Pro
                340                 345                 350

Tyr Gly Pro Gly Gln Ser Ala Ala Ala Ala Ala Gly Gln Tyr Gln Gln
        355                 360                 365

Gly Pro Gly Gln Gln Gly Pro Tyr Gly Pro Gly Ala Ser Gly Pro Gly
        370                 375                 380

Gln Gln Gly Pro Tyr Gly Pro Gly Ala Ser Ala Ala Ala Ala Ala Gly
385                 390                 395                 400

Pro Gly Gln Tyr Gly Pro Gly Gln Gln Gly Pro Ser Ala Ser Ala Ala
        405                 410                 415

Ala Ala Ala Gly Gln Tyr Gly Ser Gly Pro Gly Gln Tyr Gly Pro Tyr
        420                 425                 430

Gly Pro Gly Gln Ser Gly Pro Gly Ser Gly Gln Gln Gly Gln Gly Pro
        435                 440                 445

Tyr Gly Pro Gly Ala Ser Ala Ala Ala Ala Gly Gln Tyr Gly Pro
        450                 455                 460

Gly Gln Gln Gly Pro Tyr Gly Pro Gly Gln Ser Ala Ala Ala Ala Ala
465                 470                 475                 480

Gly Pro Gly Ser Gly Gln Tyr Gly Pro Gly Ala Ser Gly Gln Asn Gly
        485                 490                 495

Pro Gly Ser Gly Gln Tyr Gly Pro Gly Gln Gln Gly Pro Gly Gln Ser
        500                 505                 510

Ala Ala Ala Ala Ala Gly Gln Tyr Gln Gln Gly Pro Gly Gln Gln Gly
        515                 520                 525
```

51

Pro Tyr Gly Pro Gly Ala Ser Ala Ala Ala Ala Gly Gln Tyr Gly
    530                 535             540

Ser Gly Pro Gly Gln Gln Gly Pro Tyr Gly Pro Gly Gln Ser Gly Ser
545                 550             555                 560

Gly Gln Gln Gly Pro Gly Gln Gln Gly Pro Tyr Ala Ser Ala Ala Ala
                565             570             575

Ala Ala Gly Pro Gly Ser Gly Gln Gln Gly Pro Gly Ala Ser
            580             585             590

<210>  8
<211>  565
<212>  PRT
<213>  Artificial Sequence

<220>
<223>  Met-PRT525

<400>  8

Met Gly Pro Gly Gln Gln Gly Pro Tyr Gly Pro Gly Ala Ser Ala Ala
1               5               10              15

Ala Ala Ala Ala Ala Gly Ser Asn Gly Pro Gly Ser Gly Gln Gln Gly
            20              25              30

Pro Gly Gln Ser Gly Gln Tyr Gly Pro Gly Gln Gln Gly Pro Gly Gln
        35              40              45

Gln Gly Pro Gly Ser Ser Ala Ala Ala Ala Ala Ala Gly Pro Gly
    50              55              60

Gln Tyr Gly Pro Gly Gln Gln Gly Pro Ser Ala Ser Ala Ala Ala Ala
65              70              75              80

Ala Ala Ala Gly Pro Gly Ser Gly Gln Gln Gly Pro Gly Ala Ser Gly
            85              90              95

Gln Tyr Gly Pro Gly Gln Gln Gly Pro Gly Gln Gln Gly Pro Gly Ser
        100             105             110

Ser Ala Ala Ala Ala Ala Ala Ala Gly Ser Tyr Gly Ser Gly Pro Gly
        115             120             125

Gln Gln Gly Pro Tyr Gly Ser Ala Ala Ala Ala Ala Ala Gly Pro
    130             135             140

Gly Ser Gly Gln Tyr Gly Gln Gly Pro Tyr Gly Pro Gly Ala Ser Gly
145                150                155                160

Pro Gly Gln Tyr Gly Pro Gly Gln Gln Gly Pro Ser Ala Ser Ala Ala
                    165                170                175

Ala Ala Ala Ala Ala Gly Ser Gly Gln Gln Gly Pro Gly Gln Tyr Gly
                180                185                190

Pro Tyr Ala Ser Ala Ala Ala Ala Ala Ala Ala Gly Ser Tyr Gly Ser
            195                200                205

Gly Pro Gly Gln Gln Gly Pro Tyr Gly Pro Gly Gln Ser Gly Ser Gly
            210                215                220

Gln Gln Gly Pro Gly Gln Gln Gly Pro Tyr Ala Ser Ala Ala Ala Ala
225                230                235                240

Ala Ala Ala Gly Pro Gly Gln Gln Gly Pro Tyr Gly Pro Gly Ser Ser
            245                250                255

Ala Ala Ala Ala Ala Ala Ala Gly Ser Tyr Gly Tyr Gly Pro Gly Gln
            260                265                270

Gln Gly Pro Tyr Gly Pro Gly Ala Ser Gly Gln Asn Gly Pro Gly Ser
            275                280                285

Gly Gln Tyr Gly Pro Gly Gln Gln Gly Pro Gly Pro Ser Ala Ala Ala
            290                295                300

Ala Ala Ala Ala Gly Pro Gly Gln Gln Gly Pro Tyr Gly Pro Gly Ala
305                310                315                320

Ser Ala Ala Ala Ala Ala Ala Ala Gly Ser Tyr Gly Pro Gly Gln Gln
            325                330                335

Gly Pro Gly Gln Tyr Gly Pro Gly Ser Ser Gly Pro Gly Gln Gln Gly
            340                345                350

Pro Tyr Gly Pro Gly Ser Ser Ala Ala Ala Ala Ala Ala Ala Gly Ser
            355                360                365

Tyr Gly Pro Gly Gln Gln Gly Pro Tyr Gly Pro Gly Pro Ser Ala Ala
            370                375                380

Ala Ala Ala Ala Ala Gly Ser Tyr Gln Gln Gly Pro Gly Gln Gln Gly
385                390                395                400

53

```
Pro Tyr Gly Pro Gly Ala Ser Gly Pro Gly Gln Gln Gly Pro Tyr Gly
             405                 410                 415

Pro Gly Ala Ser Ala Ala Ala Ala Ala Ala Ala Gly Pro Gly Gln Tyr
             420                 425                 430

Gly Pro Gly Gln Gln Gly Pro Ser Ala Ser Ala Ala Ala Ala Ala Ala
             435                 440                 445

Ala Gly Ser Tyr Gly Ser Gly Pro Gly Gln Tyr Gly Pro Tyr Gly Pro
    450                 455                 460

Gly Gln Ser Gly Pro Gly Ser Gly Gln Gln Gly Gln Gly Pro Tyr Gly
465                 470                 475                 480

Pro Gly Ala Ser Ala Ala Ala Ala Ala Ala Ala Gly Ser Tyr Gly Pro
             485                 490                 495

Gly Gln Gln Gly Pro Tyr Gly Pro Gly Pro Ser Ala Ala Ala Ala Ala
             500                 505                 510

Ala Ala Gly Pro Gly Ser Gly Gln Tyr Gly Pro Gly Ala Ser Gly Gln
         515                 520                 525

Asn Gly Pro Gly Ser Gly Gln Tyr Gly Pro Gly Gln Gln Gly Pro Gly
    530                 535                 540

Pro Ser Ala Ala Ala Ala Ala Ala Ala Gly Pro Gly Ser Gly Gln Gln
545                 550                 555                 560

Gly Pro Gly Ala Ser
                565
```

<210> 9
<211> 2364
<212> PRT
<213> Artificial Sequence

<220>
<223> Met-PRT799

<400> 9

```
Met Gly Pro Gly Gln Gln Gly Pro Tyr Gly Pro Gly Ala Ser Ala Ala
1               5                   10                  15

Ala Ala Ala Gly Gln Asn Gly Pro Gly Ser Gly Gln Gln Gly Pro Gly
         20                  25                  30
```

Gln Ser Gly Gln Tyr Gly Pro Gly Gln Gln Gly Pro Gly Gln Gln Gly
35                    40                    45

Pro Gly Ser Ser Ala Ala Ala Ala Ala Gly Pro Gly Gln Tyr Gly Pro
50                    55                    60

Gly Gln Gln Gly Pro Ser Ala Ser Ala Ala Ala Ala Gly Pro Gly
65                    70                    75                    80

Ser Gly Gln Gln Gly Pro Gly Ala Ser Gly Gln Tyr Gly Pro Gly Gln
85                    90                    95

Gln Gly Pro Gly Gln Gln Gly Pro Gly Ser Ser Ala Ala Ala Ala Ala
100                   105                   110

Gly Gln Tyr Gly Ser Gly Pro Gly Gln Gln Gly Pro Tyr Gly Ser Ala
115                   120                   125

Ala Ala Ala Ala Gly Pro Gly Ser Gly Gln Tyr Gly Gln Gly Pro Tyr
130                   135                   140

Gly Pro Gly Ala Ser Gly Pro Gly Gln Tyr Gly Pro Gly Gln Gln Gly
145                   150                   155                   160

Pro Ser Ala Ser Ala Ala Ala Ala Ala Gly Ser Gly Gln Gln Gly Pro
165                   170                   175

Gly Gln Tyr Gly Pro Tyr Ala Ser Ala Ala Ala Ala Gly Gln Tyr
180                   185                   190

Gly Ser Gly Pro Gly Gln Gln Gly Pro Tyr Gly Pro Gly Gln Ser Gly
195                   200                   205

Ser Gly Gln Gln Gly Pro Gly Gln Gln Gly Pro Tyr Ala Ser Ala Ala
210                   215                   220

Ala Ala Ala Gly Pro Gly Gln Gln Gly Pro Tyr Gly Pro Gly Ser Ser
225                   230                   235                   240

Ala Ala Ala Ala Ala Gly Gln Tyr Gly Tyr Gly Pro Gly Gln Gln Gly
245                   250                   255

Pro Tyr Gly Pro Gly Ala Ser Gly Gln Asn Gly Pro Gly Ser Gly Gln
260                   265                   270

Tyr Gly Pro Gly Gln Gln Gly Pro Gly Gln Ser Ala Ala Ala Ala Ala
275                   280                   285

```
Gly Pro Gly Gln Gln Gly Pro Tyr Gly Pro Gly Ala Ser Ala Ala Ala
    290                 295                 300

Ala Ala Gly Gln Tyr Gly Pro Gly Gln Gln Gly Pro Gly Gln Tyr Gly
305                 310                 315                 320

Pro Gly Ser Ser Gly Pro Gly Gln Gln Gly Pro Tyr Gly Pro Gly Ser
                325                 330                 335

Ser Ala Ala Ala Ala Ala Gly Gln Tyr Gly Pro Gly Gln Gln Gly Pro
            340                 345                 350

Tyr Gly Pro Gly Gln Ser Ala Ala Ala Ala Ala Gly Gln Tyr Gln Gln
        355                 360                 365

Gly Pro Gly Gln Gln Gly Pro Tyr Gly Pro Gly Ala Ser Gly Pro Gly
    370                 375                 380

Gln Gln Gly Pro Tyr Gly Pro Gly Ala Ser Ala Ala Ala Ala Ala Gly
385                 390                 395                 400

Pro Gly Gln Tyr Gly Pro Gly Gln Gln Gly Pro Ser Ala Ser Ala Ala
                405                 410                 415

Ala Ala Ala Gly Gln Tyr Gly Ser Gly Pro Gly Gln Tyr Gly Pro Tyr
            420                 425                 430

Gly Pro Gly Gln Ser Gly Pro Gly Ser Gly Gln Gln Gly Gln Gly Pro
        435                 440                 445

Tyr Gly Pro Gly Ala Ser Ala Ala Ala Ala Gly Gln Tyr Gly Pro
    450                 455                 460

Gly Gln Gln Gly Pro Tyr Gly Pro Gly Gln Ser Ala Ala Ala Ala Ala
465                 470                 475                 480

Gly Pro Gly Ser Gly Gln Tyr Gly Pro Gly Ala Ser Gly Gln Asn Gly
                485                 490                 495

Pro Gly Ser Gly Gln Tyr Gly Pro Gly Gln Gln Gly Pro Gly Gln Ser
            500                 505                 510

Ala Ala Ala Ala Ala Gly Gln Tyr Gln Gln Gly Pro Gly Gln Gln Gly
    515                 520                 525

Pro Tyr Gly Pro Gly Ala Ser Ala Ala Ala Ala Ala Gly Gln Tyr Gly
    530                 535                 540
```

Ser Gly Pro Gly Gln Gln Gly Pro Tyr Gly Pro Gly Gln Ser Gly Ser
545                     550                 555                 560

Gly Gln Gln Gly Pro Gly Gln Gln Gly Pro Tyr Ala Ser Ala Ala Ala
                    565                 570                 575

Ala Ala Gly Pro Gly Ser Gly Gln Gln Gly Pro Gly Ala Ser Gly Gln
                580                 585                 590

Gln Gly Pro Tyr Gly Pro Gly Ala Ser Ala Ala Ala Ala Ala Gly Gln
            595                 600                 605

Asn Gly Pro Gly Ser Gly Gln Gln Gly Pro Gly Gln Ser Gly Gln Tyr
        610                 615                 620

Gly Pro Gly Gln Gln Gly Pro Gly Gln Gln Gly Pro Gly Ser Ser Ala
625                 630                 635                 640

Ala Ala Ala Ala Gly Pro Gly Gln Tyr Gly Pro Gly Gln Gln Gly Pro
                645                 650                 655

Ser Ala Ser Ala Ala Ala Ala Ala Gly Pro Gly Ser Gly Gln Gln Gly
            660                 665                 670

Pro Gly Ala Ser Gly Gln Tyr Gly Pro Gly Gln Gln Gly Pro Gly Gln
            675                 680                 685

Gln Gly Pro Gly Ser Ser Ala Ala Ala Ala Gly Gln Tyr Gly Ser
        690                 695                 700

Gly Pro Gly Gln Gln Gly Pro Tyr Gly Ser Ala Ala Ala Ala Ala Gly
705                 710                 715                 720

Pro Gly Ser Gly Gln Tyr Gly Gln Gly Pro Tyr Gly Pro Gly Ala Ser
            725                 730                 735

Gly Pro Gly Gln Tyr Gly Pro Gly Gln Gln Gly Pro Ser Ala Ser Ala
            740                 745                 750

Ala Ala Ala Ala Gly Ser Gly Gln Gln Gly Pro Gly Gln Tyr Gly Pro
        755                 760                 765

Tyr Ala Ser Ala Ala Ala Ala Ala Gly Gln Tyr Gly Ser Gly Pro Gly
        770                 775                 780

Gln Gln Gly Pro Tyr Gly Pro Gly Gln Ser Gly Ser Gly Gln Gln Gly

57

785                     790                     795                     800


Pro Gly Gln Gln Gly Pro Tyr Ala Ser Ala Ala Ala Ala Gly Pro
            805                 810                 815


Gly Gln Gln Gly Pro Tyr Gly Pro Gly Ser Ser Ala Ala Ala Ala
            820                 825                 830


Gly Gln Tyr Gly Tyr Gly Pro Gly Gln Gln Gly Pro Tyr Gly Pro Gly
            835                 840                 845


Ala Ser Gly Gln Asn Gly Pro Gly Ser Gly Gln Tyr Gly Pro Gly Gln
    850                 855                 860


Gln Gly Pro Gly Gln Ser Ala Ala Ala Ala Gly Pro Gly Gln Gln
865                 870                 875                 880


Gly Pro Tyr Gly Pro Gly Ala Ser Ala Ala Ala Ala Gly Gln Tyr
            885                 890                 895


Gly Pro Gly Gln Gln Gly Pro Gly Gln Tyr Gly Pro Gly Ser Ser Gly
            900                 905                 910


Pro Gly Gln Gln Gly Pro Tyr Gly Pro Gly Ser Ser Ala Ala Ala Ala
    915                 920                 925


Ala Gly Gln Tyr Gly Pro Gly Gln Gln Gly Pro Tyr Gly Pro Gly Gln
    930                 935                 940


Ser Ala Ala Ala Ala Ala Gly Gln Tyr Gln Gln Gly Pro Gly Gln Gln
945                 950                 955                 960


Gly Pro Tyr Gly Pro Gly Ala Ser Gly Pro Gly Gln Gln Gly Pro Tyr
            965                 970                 975


Gly Pro Gly Ala Ser Ala Ala Ala Ala Gly Pro Gly Gln Tyr Gly
            980                 985                 990


Pro Gly Gln Gln Gly Pro Ser Ala Ser Ala Ala Ala Ala Gly Gln
    995                 1000                1005


Tyr Gly Ser Gly Pro Gly Gln Tyr Gly Pro Tyr Gly Pro Gly Gln
    1010                1015                1020


Ser Gly Pro Gly Ser Gly Gln Gln Gly Gln Gly Pro Tyr Gly Pro
    1025                1030                1035

Gly Ala Ser Ala Ala Ala Ala Ala Gly Gln Tyr Gly Pro Gly Gln
1040 1045 1050

Gln Gly Pro Tyr Gly Pro Gly Gln Ser Ala Ala Ala Ala Ala Gly
1055 1060 1065

Pro Gly Ser Gly Gln Tyr Gly Pro Gly Ala Ser Gly Gln Asn Gly
1070 1075 1080

Pro Gly Ser Gly Gln Tyr Gly Pro Gly Gln Gln Gly Pro Gly Gln
1085 1090 1095

Ser Ala Ala Ala Ala Ala Gly Gln Tyr Gln Gln Gly Pro Gly Gln
1100 1105 1110

Gln Gly Pro Tyr Gly Pro Gly Ala Ser Ala Ala Ala Ala Ala Gly
1115 1120 1125

Gln Tyr Gly Ser Gly Pro Gly Gln Gln Gly Pro Tyr Gly Pro Gly
1130 1135 1140

Gln Ser Gly Ser Gly Gln Gln Gly Pro Gly Gln Gln Gly Pro Tyr
1145 1150 1155

Ala Ser Ala Ala Ala Ala Ala Gly Pro Gly Ser Gly Gln Gln Gly
1160 1165 1170

Pro Gly Ala Ser Gly Gln Gln Gly Pro Tyr Gly Pro Gly Ala Ser
1175 1180 1185

Ala Ala Ala Ala Ala Gly Gln Asn Gly Pro Gly Ser Gly Gln Gln
1190 1195 1200

Gly Pro Gly Gln Ser Gly Gln Tyr Gly Pro Gly Gln Gln Gly Pro
1205 1210 1215

Gly Gln Gln Gly Pro Gly Ser Ser Ala Ala Ala Ala Gly Pro
1220 1225 1230

Gly Gln Tyr Gly Pro Gly Gln Gln Gly Pro Ser Ala Ser Ala Ala
1235 1240 1245

Ala Ala Ala Gly Pro Gly Ser Gly Gln Gln Gly Pro Gly Ala Ser
1250 1255 1260

Gly Gln Tyr Gly Pro Gly Gln Gln Gly Pro Gly Gln Gln Gly Pro
1265 1270 1275

```
Gly Ser  Ser Ala Ala Ala Ala  Ala Gly Gln Tyr Gly  Ser Gly Pro
    1280             1285              1290

Gly Gln  Gln Gly Pro Tyr Gly  Ser Ala Ala Ala Ala  Ala Gly Pro
    1295             1300              1305

Gly Ser  Gly Gln Tyr Gly Gln  Gly Pro Tyr Gly Pro  Gly Ala Ser
    1310             1315              1320

Gly Pro  Gly Gln Tyr Gly Pro  Gly Gln Gln Gly Pro  Ser Ala Ser
    1325             1330              1335

Ala Ala  Ala Ala Ala Gly Ser  Gly Gln Gln Gly Pro  Gly Gln Tyr
    1340             1345              1350

Gly Pro  Tyr Ala Ser Ala Ala  Ala Ala Ala Gly Gln  Tyr Gly Ser
    1355             1360              1365

Gly Pro  Gly Gln Gln Gly Pro  Tyr Gly Pro Gly Gln  Ser Gly Ser
    1370             1375              1380

Gly Gln  Gln Gly Pro Gly Gln  Gln Gly Pro Tyr Ala  Ser Ala Ala
    1385             1390              1395

Ala Ala  Ala Gly Pro Gly Gln  Gln Gly Pro Tyr Gly  Pro Gly Ser
    1400             1405              1410

Ser Ala  Ala Ala Ala Ala Gly  Gln Tyr Gly Tyr Gly  Pro Gly Gln
    1415             1420              1425

Gln Gly  Pro Tyr Gly Pro Gly  Ala Ser Gly Gln Asn  Gly Pro Gly
    1430             1435              1440

Ser Gly  Gln Tyr Gly Pro Gly  Gln Gln Gly Pro Gly  Gln Ser Ala
    1445             1450              1455

Ala Ala  Ala Ala Gly Pro Gly  Gln Gln Gly Pro Tyr  Gly Pro Gly
    1460             1465              1470

Ala Ser  Ala Ala Ala Ala Ala  Gly Gln Tyr Gly Pro  Gly Gln Gln
    1475             1480              1485

Gly Pro  Gly Gln Tyr Gly Pro  Gly Ser Ser Gly Pro  Gly Gln Gln
    1490             1495              1500

Gly Pro  Tyr Gly Pro Gly Ser  Ser Ala Ala Ala Ala  Ala Gly Gln
    1505             1510              1515
```

60

```
Tyr Gly  Pro Gly Gln Gln Gly  Pro Tyr Gly Pro Gly  Gln Ser Ala
    1520                 1525                 1530

Ala Ala  Ala Ala Gly Gln Tyr  Gln Gln Gly Pro Gly  Gln Gln Gly
    1535                 1540                 1545

Pro Tyr  Gly Pro Gly Ala Ser  Gly Pro Gly Gln Gln  Gly Pro Tyr
    1550                 1555                 1560

Gly Pro  Gly Ala Ser Ala Ala  Ala Ala Ala Gly Pro  Gly Gln Tyr
    1565                 1570                 1575

Gly Pro  Gly Gln Gln Gly Pro  Ser Ala Ser Ala Ala  Ala Ala Ala
    1580                 1585                 1590

Gly Gln  Tyr Gly Ser Gly Pro  Gly Gln Tyr Gly Pro  Tyr Gly Pro
    1595                 1600                 1605

Gly Gln  Ser Gly Pro Gly Ser  Gly Gln Gln Gly Gln  Gly Pro Tyr
    1610                 1615                 1620

Gly Pro  Gly Ala Ser Ala Ala  Ala Ala Ala Gly Gln  Tyr Gly Pro
    1625                 1630                 1635

Gly Gln  Gln Gly Pro Tyr Gly  Pro Gly Gln Ser Ala  Ala Ala Ala
    1640                 1645                 1650

Ala Gly  Pro Gly Ser Gly Gln  Tyr Gly Pro Gly Ala  Ser Gly Gln
    1655                 1660                 1665

Asn Gly  Pro Gly Ser Gly Gln  Tyr Gly Pro Gly Gln  Gln Gly Pro
    1670                 1675                 1680

Gly Gln  Ser Ala Ala Ala Ala  Ala Gly Gln Tyr Gln  Gln Gly Pro
    1685                 1690                 1695

Gly Gln  Gln Gly Pro Tyr Gly  Pro Gly Ala Ser Ala  Ala Ala Ala
    1700                 1705                 1710

Ala Gly  Gln Tyr Gly Ser Gly  Pro Gly Gln Gln Gly  Pro Tyr Gly
    1715                 1720                 1725

Pro Gly  Gln Ser Gly Ser Gly  Gln Gln Gly Pro Gly  Gln Gln Gly
    1730                 1735                 1740

Pro Tyr  Ala Ser Ala Ala Ala  Ala Ala Gly Pro Gly  Ser Gly Gln
```

```
                  1745                          1750                               1755


        Gln Gly  Pro Gly Ala Ser Gly  Gln Gln Gly Pro Tyr  Gly Pro Gly
             1760                 1765                 1770


        Ala Ser  Ala Ala Ala Ala Ala  Gly Gln Asn Gly Pro  Gly Ser Gly
             1775                 1780                 1785


        Gln Gln  Gly Pro Gly Gln Ser  Gly Gln Tyr Gly Pro  Gly Gln Gln
             1790                 1795                 1800


        Gly Pro  Gly Gln Gln Gly Pro  Gly Ser Ser Ala Ala  Ala Ala Ala
             1805                 1810                 1815


        Gly Pro  Gly Gln Tyr Gly Pro  Gly Gln Gln Gly Pro  Ser Ala Ser
             1820                 1825                 1830


        Ala Ala  Ala Ala Ala Gly Pro  Gly Ser Gly Gln Gln  Gly Pro Gly
             1835                 1840                 1845


        Ala Ser  Gly Gln Tyr Gly Pro  Gly Gln Gln Gly Pro  Gly Gln Gln
             1850                 1855                 1860


        Gly Pro  Gly Ser Ser Ala Ala  Ala Ala Ala Gly Gln  Tyr Gly Ser
             1865                 1870                 1875


        Gly Pro  Gly Gln Gln Gly Pro  Tyr Gly Ser Ala Ala  Ala Ala Ala
             1880                 1885                 1890


        Gly Pro  Gly Ser Gly Gln Tyr  Gly Gln Gly Pro Tyr  Gly Pro Gly
             1895                 1900                 1905


        Ala Ser  Gly Pro Gly Gln Tyr  Gly Pro Gly Gln Gln  Gly Pro Ser
             1910                 1915                 1920


        Ala Ser  Ala Ala Ala Ala Ala  Gly Ser Gly Gln Gln  Gly Pro Gly
             1925                 1930                 1935


        Gln Tyr  Gly Pro Tyr Ala Ser  Ala Ala Ala Ala Ala  Gly Gln Tyr
             1940                 1945                 1950


        Gly Ser  Gly Pro Gly Gln Gln  Gly Pro Tyr Gly Pro  Gly Gln Ser
             1955                 1960                 1965


        Gly Ser  Gly Gln Gln Gly Pro  Gly Gln Gln Gly Pro  Tyr Ala Ser
             1970                 1975                 1980
```

```
Ala Ala  Ala Ala Ala Gly Pro  Gly Gln Gln Gly Pro  Tyr Gly Pro
    1985             1990                 1995

Gly Ser  Ser Ala Ala Ala Ala  Ala Gly Gln Tyr Gly  Tyr Gly Pro
    2000             2005                 2010

Gly Gln  Gln Gly Pro Tyr Gly  Pro Gly Ala Ser Gly  Gln Asn Gly
    2015             2020                 2025

Pro Gly  Ser Gly Gln Tyr Gly  Pro Gly Gln Gln Gly  Pro Gly Gln
    2030             2035                 2040

Ser Ala  Ala Ala Ala Ala Gly  Pro Gly Gln Gln Gly  Pro Tyr Gly
    2045             2050                 2055

Pro Gly  Ala Ser Ala Ala Ala  Ala Ala Gly Gln Tyr  Gly Pro Gly
    2060             2065                 2070

Gln Gln  Gly Pro Gly Gln Tyr  Gly Pro Gly Ser Ser  Gly Pro Gly
    2075             2080                 2085

Gln Gln  Gly Pro Tyr Gly Pro  Gly Ser Ser Ala Ala  Ala Ala Ala
    2090             2095                 2100

Gly Gln  Tyr Gly Pro Gly Gln  Gln Gly Pro Tyr Gly  Pro Gly Gln
    2105             2110                 2115

Ser Ala  Ala Ala Ala Ala Gly  Gln Tyr Gln Gln Gly  Pro Gly Gln
    2120             2125                 2130

Gln Gly  Pro Tyr Gly Pro Gly  Ala Ser Gly Pro Gly  Gln Gln Gly
    2135             2140                 2145

Pro Tyr  Gly Pro Gly Ala Ser  Ala Ala Ala Ala Ala  Gly Pro Gly
    2150             2155                 2160

Gln Tyr  Gly Pro Gly Gln Gln  Gly Pro Ser Ala Ser  Ala Ala Ala
    2165             2170                 2175

Ala Ala  Gly Gln Tyr Gly Ser  Gly Pro Gly Gln Tyr  Gly Pro Tyr
    2180             2185                 2190

Gly Pro  Gly Gln Ser Gly Pro  Gly Ser Gly Gln Gln  Gly Gln Gly
    2195             2200                 2205

Pro Tyr  Gly Pro Gly Ala Ser  Ala Ala Ala Ala Ala  Gly Gln Tyr
    2210             2215                 2220
```

```
Gly Pro  Gly Gln Gln Gly Pro  Tyr Gly Pro Gly Gln  Ser Ala Ala
    2225             2230              2235

Ala Ala  Ala Gly Pro Gly Ser  Gly Gln Tyr Gly Pro  Gly Ala Ser
    2240             2245              2250

Gly Gln  Asn Gly Pro Gly Ser  Gly Gln Tyr Gly Pro  Gly Gln Gln
    2255             2260              2265

Gly Pro  Gly Gln Ser Ala Ala  Ala Ala Ala Gly Gln  Tyr Gln Gln
    2270             2275              2280

Gly Pro  Gly Gln Gln Gly Pro  Tyr Gly Pro Gly Ala  Ser Ala Ala
    2285             2290              2295

Ala Ala  Ala Gly Gln Tyr Gly  Ser Gly Pro Gly Gln  Gln Gly Pro
    2300             2305              2310

Tyr Gly  Pro Gly Gln Ser Gly  Ser Gly Gln Gln Gly  Pro Gly Gln
    2315             2320              2325

Gln Gly  Pro Tyr Ala Ser Ala  Ala Ala Ala Ala Gly  Pro Gly Ser
    2330             2335              2340

Gly Gln  Gln Gly Ser Ser Val  Asp Lys Leu Ala Ala  Ala Leu Glu
    2345             2350              2355

His His  His His His His
    2360
```

```
<210>  10
<211>  597
<212>  PRT
<213>  Artificial Sequence

<220>
<223>  Met-PRT313

<400>  10

Met Gly Pro Gly Gly Gln Gly Pro Tyr Gly Pro Gly Ala Ser Ala Ala
1               5              10               15

Ala Ala Ala Gly Gly Asn Gly Pro Gly Ser Gly Gln Gln Gly Pro Gly
            20              25              30

Gly Ser Ala Ala Ala Ala Ala Gly Gly Tyr Gly Pro Gly Gly Gln Gly
        35              40              45
```

Pro Gly Gln Gln Gly Pro Gly Ser Ser Ala Ala Ala Ala Ala Gly Pro
    50            55            60

Gly Gly Tyr Gly Pro Gly Gly Gln Gly Pro Ser Ala Ser Ala Ala Ala
65            70            75            80

Ala Ala Gly Pro Gly Ser Gly Gln Gln Gly Pro Gly Ala Ser Ala Ala
        85            90            95

Ala Ala Ala Gly Gly Tyr Gly Pro Gly Gly Gln Gly Pro Gly Gln Gln
      100          105        110

Gly Pro Gly Ser Ser Ala Ala Ala Ala Ala Gly Gly Tyr Gly Ser Gly
    115          120        125

Pro Gly Gln Gln Gly Pro Tyr Gly Ser Ala Ala Ala Ala Ala Gly Pro
    130          135        140

Gly Ser Gly Gly Tyr Gly Gln Gly Pro Tyr Gly Pro Gly Ala Ser Ala
145          150        155        160

Ala Ala Ala Ala Gly Pro Gly Gly Tyr Gly Pro Gly Gly Gln Gly Pro
      165          170        175

Ser Ala Ser Ala Ala Ala Ala Ala Gly Ser Gly Gln Gln Gly Pro Gly
      180          185        190

Gly Tyr Gly Pro Tyr Ala Ser Ala Ala Ala Ala Ala Gly Gly Tyr Gly
    195          200        205

Ser Gly Pro Gly Gln Gln Gly Pro Tyr Gly Pro Gly Gly Ser Ala Ala
    210          215        220

Ala Ala Ala Gly Ser Gly Gln Gln Gly Pro Gly Gln Gln Gly Pro Tyr
225          230        235        240

Ala Ser Ala Ala Ala Ala Ala Gly Pro Gly Gly Gln Gly Pro Tyr Gly
325          245        250        255

Pro Gly Ser Ser Ala Ala Ala Ala Ala Gly Gly Tyr Gly Tyr Gly Pro
      260          265        270

Gly Gly Gln Gly Pro Tyr Gly Pro Gly Ala Ser Ala Ala Ala Ala Ala
    275          280        285

Gly Gly Asn Gly Pro Gly Ser Gly Gly Tyr Gly Pro Gly Gln Gln Gly
    290          295        300

```
Pro Gly Gly Ser Ala Ala Ala Ala Ala Gly Pro Gly Gly Gln Gly Pro
305             310             315             320

Tyr Gly Pro Gly Ala Ser Ala Ala Ala Ala Ala Gly Gly Tyr Gly Pro
            325             330             335

Gly Gly Gln Gly Pro Gly Gly Tyr Gly Pro Gly Ser Ser Ala Ala Ala
            340             345             350

Ala Ala Gly Pro Gly Gly Gln Gly Pro Tyr Gly Pro Gly Ser Ser Ala
    355             360             365

Ala Ala Ala Gly Gly Tyr Gly Pro Gly Gln Gln Gly Pro Tyr Gly
    370             375             380

Pro Gly Gly Ser Ala Ala Ala Ala Ala Gly Gly Tyr Gln Gln Gly Pro
385             390             395             400

Gly Gly Gln Gly Pro Tyr Gly Pro Gly Ala Ser Ala Ala Ala Ala Ala
            405             410             415

Gly Pro Gly Gly Gln Gly Pro Tyr Gly Pro Gly Ala Ser Ala Ala Ala
            420             425             430

Ala Ala Gly Pro Gly Gly Tyr Gly Pro Gly Gly Gln Gly Pro Ser Ala
    435             440             445

Ser Ala Ala Ala Ala Ala Gly Gly Tyr Gly Ser Gly Pro Gly Gly Tyr
    450             455             460

Gly Pro Tyr Gly Pro Gly Gly Ser Ala Ala Ala Ala Ala Gly Pro Gly
465             470             475             480

Ser Gly Gln Gln Gly Gln Gly Pro Tyr Gly Pro Gly Ala Ser Ala Ala
            485             490             495

Ala Ala Ala Gly Gly Tyr Gly Pro Gly Gln Gln Gly Pro Tyr Gly Pro
    500             505             510

Gly Gly Ser Ala Ala Ala Ala Ala Gly Pro Gly Ser Gly Gly Tyr Gly
    515             520             525

Pro Gly Ala Ser Ala Ala Ala Ala Gly Gly Asn Gly Pro Gly Ser
530             535             540

Gly Gly Tyr Gly Pro Gly Gln Gln Gly Pro Gly Gly Ser Ala Ala Ala
545             550             555             560
```

66

```
Ala Ala Gly Gly Tyr Gln Gln Gly Pro Gly Gly Gln Gly Pro Tyr Gly
            565                 570                 575

Pro Gly Ala Ser Ala Ala Ala Ala Ala Gly Pro Gly Ser Gly Gln Gln
            580                 585                 590

Gly Pro Gly Ala Ser
            595


<210>  11
<211>  12
<212>  PRT
<213>  Artificial Sequence

<220>
<223>  HisTag

<400>  11

Met His His His His His His Ser Ser Gly Ser Ser
1                   5                   10


<210>  12
<211>  608
<212>  PRT
<213>  Artificial Sequence

<220>
<223>  PRT380

<400>  12

Met His His His His His His Ser Ser Gly Ser Ser Gly Pro Gly Gln
1                   5                   10                  15

Gln Gly Pro Tyr Gly Pro Gly Ala Ser Ala Ala Ala Ala Ala Gly Gln
            20                  25                  30

Asn Gly Pro Gly Ser Gly Gln Gln Gly Pro Gly Gln Ser Ala Ala Ala
            35                  40                  45

Ala Ala Gly Gln Tyr Gly Pro Gly Gln Gln Gly Pro Gly Gln Gln Gly
        50                  55                  60

Pro Gly Ser Ser Ala Ala Ala Ala Ala Gly Pro Gly Gln Tyr Gly Pro
65                  70                  75                  80

Gly Gln Gln Gly Pro Ser Ala Ser Ala Ala Ala Ala Gly Pro Gly
            85                  90                  95

Ser Gly Gln Gln Gly Pro Gly Ala Ser Ala Ala Ala Ala Ala Gly Gln
```

67

100        105        110

Tyr Gly Pro Gly Gln Gln Gly Pro Gly Gln Gln Gly Pro Gly Ser Ser
115        120        125

Ala Ala Ala Ala Ala Gly Gln Tyr Gly Ser Gly Pro Gly Gln Gln Gly
130        135        140

Pro Tyr Gly Ser Ala Ala Ala Ala Ala Gly Pro Gly Ser Gly Gln Tyr
145        150        155        160

Gly Gln Gly Pro Tyr Gly Pro Gly Ala Ser Ala Ala Ala Ala Ala Gly
165        170        175

Pro Gly Gln Tyr Gly Pro Gly Gln Gln Gly Pro Ser Ala Ser Ala Ala
180        185        190

Ala Ala Ala Gly Ser Gly Gln Gln Gly Pro Gly Gln Tyr Gly Pro Tyr
195        200        205

Ala Ser Ala Ala Ala Ala Ala Gly Gln Tyr Gly Ser Gly Pro Gly Gln
210        215        220

Gln Gly Pro Tyr Gly Pro Gly Gln Ser Ala Ala Ala Ala Ala Gly Ser
225        230        235        240

Gly Gln Gln Gly Pro Gly Gln Gln Gly Pro Tyr Ala Ser Ala Ala Ala
245        250        255

Ala Ala Gly Pro Gly Gln Gln Gly Pro Tyr Gly Pro Gly Ser Ser Ala
260        265        270

Ala Ala Ala Ala Gly Gln Tyr Gly Tyr Gly Pro Gly Gln Gln Gly Pro
275        280        285

Tyr Gly Pro Gly Ala Ser Ala Ala Ala Ala Gly Gln Asn Gly Pro
290        295        300

Gly Ser Gly Gln Tyr Gly Pro Gly Gln Gln Gly Pro Gly Gln Ser Ala
305        310        315        320

Ala Ala Ala Ala Gly Pro Gly Gln Gln Gly Pro Tyr Gly Pro Gly Ala
325        330        335

Ser Ala Ala Ala Ala Ala Gly Gln Tyr Gly Pro Gly Gln Gln Gly Pro
340        345        350

Gly Gln Tyr Gly Pro Gly Ser Ser Ala Ala Ala Ala Ala Gly Pro Gly
355                     360             365

Gln Gln Gly Pro Tyr Gly Pro Gly Ser Ser Ala Ala Ala Ala Ala Gly
370                 375             380

Gln Tyr Gly Pro Gly Gln Gln Gly Pro Tyr Gly Pro Gly Gln Ser Ala
385                 390             395                 400

Ala Ala Ala Ala Gly Gln Tyr Gln Gln Gly Pro Gly Gln Gln Gly Pro
405                 410                 415

Tyr Gly Pro Gly Ala Ser Ala Ala Ala Ala Gly Pro Gly Gln Gln
420             425             430

Gly Pro Tyr Gly Pro Gly Ala Ser Ala Ala Ala Ala Gly Pro Gly
435             440             445

Gln Tyr Gly Pro Gly Gln Gln Gly Pro Ser Ala Ser Ala Ala Ala Ala
450             455             460

Ala Gly Gln Tyr Gly Ser Gly Pro Gly Gln Tyr Gly Pro Tyr Gly Pro
465             470             475             480

Gly Gln Ser Ala Ala Ala Ala Ala Gly Pro Gly Ser Gly Gln Gln Gly
485             490             495

Gln Gly Pro Tyr Gly Pro Gly Ala Ser Ala Ala Ala Ala Gly Gln
500             505             510

Tyr Gly Pro Gly Gln Gln Gly Pro Tyr Gly Pro Gly Gln Ser Ala Ala
515             520             525

Ala Ala Ala Gly Pro Gly Ser Gly Gln Tyr Gly Pro Gly Ala Ser Ala
530             535             540

Ala Ala Ala Gly Gln Asn Gly Pro Gly Ser Gly Gln Tyr Gly Pro
545             550             555             560

Gly Gln Gln Gly Pro Gly Gln Ser Ala Ala Ala Ala Gly Gln Tyr
565             570             575

Gln Gln Gly Pro Gly Gln Gln Gly Pro Tyr Gly Pro Gly Ala Ser Ala
580             585             590

Ala Ala Ala Gly Pro Gly Ser Gly Gln Gln Gly Pro Gly Ala Ser
595             600             605

69

<210> 13
<211> 601
<212> PRT
<213> Artificial Sequence

<220>
<223> PRT410

<400> 13

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Gly Gln
            20                  25                  30

Asn Gly Pro Gly Ser Gly Gln Gln Gly Pro Gly Gln Ser Gly Gln Tyr
            35                  40                  45

Gly Pro Gly Gln Gln Gly Pro Gly Gln Gln Gly Pro Gly Ser Ser Ala
        50                  55                  60

Ala Ala Ala Ala Gly Pro Gly Gln Tyr Gly Pro Gly Gln Gln Gly Pro
65                  70                  75                  80

Ser Ala Ser Ala Ala Ala Ala Ala Gly Pro Gly Ser Gly Gln Gln Gly
            85                  90                  95

Pro Gly Ala Ser Gly Gln Tyr Gly Pro Gly Gln Gln Gly Pro Gly Gln
            100                 105                 110

Gln Gly Pro Gly Ser Ser Ala Ala Ala Ala Gly Gln Tyr Gly Ser
            115                 120                 125

Gly Pro Gly Gln Gln Gly Pro Tyr Gly Ser Ala Ala Ala Ala Ala Gly
        130                 135                 140

Pro Gly Ser Gly Gln Tyr Gly Gln Gly Pro Tyr Gly Pro Gly Ala Ser
145                 150                 155                 160

Gly Pro Gly Gln Tyr Gly Pro Gly Gln Gln Gly Pro Ser Ala Ser Ala
            165                 170                 175

Ala Ala Ala Ala Gly Ser Gly Gln Gln Gly Pro Gly Gln Tyr Gly Pro
            180                 185                 190

Tyr Ala Ser Ala Ala Ala Ala Ala Gly Gln Tyr Gly Ser Gly Pro Gly
        195                 200                 205

Gln Gln Gly Pro Tyr Gly Pro Gly Gln Ser Gly Ser Gly Gln Gln Gly
    210                 215             220

Pro Gly Gln Gln Gly Pro Tyr Ala Ser Ala Ala Ala Ala Ala Gly Pro
225                 230             235                 240

Gly Gln Gln Gly Pro Tyr Gly Pro Gly Ser Ser Ala Ala Ala Ala Ala
            245             250                 255

Gly Gln Tyr Gly Tyr Gly Pro Gly Gln Gln Gly Pro Tyr Gly Pro Gly
        260             265                 270

Ala Ser Gly Gln Asn Gly Pro Gly Ser Gly Gln Tyr Gly Pro Gly Gln
        275             280                 285

Gln Gly Pro Gly Gln Ser Ala Ala Ala Ala Gly Pro Gly Gln Gln
    290             295                 300

Gly Pro Tyr Gly Pro Gly Ala Ser Ala Ala Ala Ala Ala Gly Gln Tyr
305             310                 315                 320

Gly Pro Gly Gln Gln Gly Pro Gly Gln Tyr Gly Pro Gly Ser Ser Gly
            325             330                 335

Pro Gly Gln Gln Gly Pro Tyr Gly Pro Gly Ser Ser Ala Ala Ala Ala
        340             345             350

Ala Gly Gln Tyr Gly Pro Gly Gln Gln Gly Pro Tyr Gly Pro Gly Gln
        355             360             365

Ser Ala Ala Ala Ala Ala Gly Gln Tyr Gln Gln Gly Pro Gly Gln Gln
    370             375                 380

Gly Pro Tyr Gly Pro Gly Ala Ser Gly Pro Gly Gln Gln Gly Pro Tyr
385             390             395                 400

Gly Pro Gly Ala Ser Ala Ala Ala Ala Gly Pro Gly Gln Tyr Gly
            405             410                 415

Pro Gly Gln Gln Gly Pro Ser Ala Ser Ala Ala Ala Ala Gly Gln
        420             425             430

Tyr Gly Ser Gly Pro Gly Gln Tyr Gly Pro Tyr Gly Pro Gly Gln Ser
    435             440             445

Gly Pro Gly Ser Gly Gln Gln Gly Gln Gly Pro Tyr Gly Pro Gly Ala
    450             455             460

71

```
Ser Ala Ala Ala Ala Ala Gly Gln Tyr Gly Pro Gly Gln Gln Gly Pro
465                 470             475                 480

Tyr Gly Pro Gly Gln Ser Ala Ala Ala Ala Gly Pro Gly Ser Gly
                485             490                 495

Gln Tyr Gly Pro Gly Ala Ser Gly Gln Asn Gly Pro Gly Ser Gly Gln
            500             505                 510

Tyr Gly Pro Gly Gln Gln Gly Pro Gly Gln Ser Ala Ala Ala Ala Ala
        515             520             525

Gly Gln Tyr Gln Gln Gly Pro Gly Gln Gln Gly Pro Tyr Gly Pro Gly
    530             535             540

Ala Ser Ala Ala Ala Ala Ala Gly Gln Tyr Gly Ser Gly Pro Gly Gln
545             550             555                 560

Gln Gly Pro Tyr Gly Pro Gly Gln Ser Gly Ser Gly Gln Gln Gly Pro
            565             570                 575

Gly Gln Gln Gly Pro Tyr Ala Ser Ala Ala Ala Ala Gly Pro Gly
        580             585             590

Ser Gly Gln Gln Gly Pro Gly Ala Ser
        595             600
```

```
<210>  14
<211>  576
<212>  PRT
<213>  Artificial Sequence

<220>
<223>  PRT525

<400>  14
```

```
Met His His His His His His Ser Ser Gly Ser Ser Gly Pro Gly Gln
1               5               10              15

Gln Gly Pro Tyr Gly Pro Gly Ala Ser Ala Ala Ala Ala Ala Ala
            20              25              30

Gly Ser Asn Gly Pro Gly Ser Gly Gln Gln Gly Pro Gly Gln Ser Gly
        35              40              45

Gln Tyr Gly Pro Gly Gln Gln Gly Pro Gly Gln Gln Gly Pro Gly Ser
    50              55              60
```

72

Ser Ala Ala Ala Ala Ala Ala Ala Gly Pro Gly Gln Tyr Gly Pro Gly
65                  70                  75                  80

Gln Gln Gly Pro Ser Ala Ser Ala Ala Ala Ala Ala Ala Ala Gly Pro
                85                  90                  95

Gly Ser Gly Gln Gln Gly Pro Gly Ala Ser Gly Gln Tyr Gly Pro Gly
            100                 105                 110

Gln Gln Gly Pro Gly Gln Gln Gly Pro Gly Ser Ser Ala Ala Ala Ala
        115                 120                 125

Ala Ala Ala Gly Ser Tyr Gly Ser Gly Pro Gly Gln Gln Gly Pro Tyr
        130                 135                 140

Gly Ser Ala Ala Ala Ala Ala Ala Ala Gly Pro Gly Ser Gly Gln Tyr
145                 150                 155                 160

Gly Gln Gly Pro Tyr Gly Pro Gly Ala Ser Gly Pro Gly Gln Tyr Gly
            165                 170                 175

Pro Gly Gln Gln Gly Pro Ser Ala Ser Ala Ala Ala Ala Ala Ala Ala
        180                 185                 190

Gly Ser Gly Gln Gln Gly Pro Gly Gln Tyr Gly Pro Tyr Ala Ser Ala
        195                 200                 205

Ala Ala Ala Ala Ala Gly Ser Tyr Gly Ser Gly Pro Gly Gln Gln
        210                 215                 220

Gly Pro Tyr Gly Pro Gly Gln Ser Gly Ser Gly Gln Gln Gly Pro Gly
225                 230                 235                 240

Gln Gln Gly Pro Tyr Ala Ser Ala Ala Ala Ala Ala Ala Gly Pro
        245                 250                 255

Gly Gln Gln Gly Pro Tyr Gly Pro Gly Ser Ser Ala Ala Ala Ala Ala
        260                 265                 270

Ala Ala Gly Ser Tyr Gly Tyr Gly Pro Gly Gln Gln Gly Pro Tyr Gly
        275                 280                 285

Pro Gly Ala Ser Gly Gln Asn Gly Pro Gly Ser Gly Gln Tyr Gly Pro
        290                 295                 300

Gly Gln Gln Gly Pro Gly Pro Ser Ala Ala Ala Ala Ala Ala Ala Gly
305                 310                 315                 320

Pro Gly Gln Gln Gly Pro Tyr Gly Pro Gly Ala Ser Ala Ala Ala Ala
325 330 335

Ala Ala Ala Gly Ser Tyr Gly Pro Gly Gln Gln Gly Pro Gly Gln Tyr
340 345 350

Gly Pro Gly Ser Ser Gly Pro Gly Gln Gln Gly Pro Tyr Gly Pro Gly
355 360 365

Ser Ser Ala Ala Ala Ala Ala Ala Ala Gly Ser Tyr Gly Pro Gly Gln
370 375 380

Gln Gly Pro Tyr Gly Pro Gly Pro Ser Ala Ala Ala Ala Ala Ala Ala
385 390 395 400

Gly Ser Tyr Gln Gln Gly Pro Gly Gln Gln Gly Pro Tyr Gly Pro Gly
405 410 415

Ala Ser Gly Pro Gly Gln Gln Gly Pro Tyr Gly Pro Gly Ala Ser Ala
420 425 430

Ala Ala Ala Ala Ala Ala Gly Pro Gly Gln Tyr Gly Pro Gly Gln Gln
435 440 445

Gly Pro Ser Ala Ser Ala Ala Ala Ala Ala Ala Gly Ser Tyr Gly
450 455 460

Ser Gly Pro Gly Gln Tyr Gly Pro Tyr Gly Pro Gly Gln Ser Gly Pro
465 470 475 480

Gly Ser Gly Gln Gln Gly Gln Gly Pro Tyr Gly Pro Gly Ala Ser Ala
485 490 495

Ala Ala Ala Ala Ala Gly Ser Tyr Gly Pro Gly Gln Gln Gly Pro
500 505 510

Tyr Gly Pro Gly Pro Ser Ala Ala Ala Ala Ala Ala Gly Pro Gly
515 520 525

Ser Gly Gln Tyr Gly Pro Gly Ala Ser Gly Gln Asn Gly Pro Gly Ser
530 535 540

Gly Gln Tyr Gly Pro Gly Gln Gln Gly Pro Gly Pro Ser Ala Ala Ala
545 550 555 560

Ala Ala Ala Ala Gly Pro Gly Ser Gly Gln Gln Gly Pro Gly Ala Ser
565 570 575

```
<210>  15
<211>  2375
<212>  PRT
<213>  Artificial Sequence

<220>
<223>  PRT799

<400>  15
```

```
Met His His His His His His Ser Ser Gly Ser Ser Gly Pro Gly Gln
1               5                   10                  15


Gln Gly Pro Tyr Gly Pro Gly Ala Ser Ala Ala Ala Ala Ala Gly Gln
            20                  25                  30


Asn Gly Pro Gly Ser Gly Gln Gln Gly Pro Gly Gln Ser Gly Gln Tyr
            35                  40                  45


Gly Pro Gly Gln Gln Gly Pro Gly Gln Gln Gly Pro Gly Ser Ser Ala
        50                  55                  60


Ala Ala Ala Ala Gly Pro Gly Gln Tyr Gly Pro Gly Gln Gln Gly Pro
65                  70                  75                  80


Ser Ala Ser Ala Ala Ala Ala Ala Gly Pro Gly Ser Gly Gln Gln Gly
                85                  90                  95


Pro Gly Ala Ser Gly Gln Tyr Gly Pro Gly Gln Gln Gly Pro Gly Gln
            100                 105                 110


Gln Gly Pro Gly Ser Ser Ala Ala Ala Ala Ala Gly Gln Tyr Gly Ser
        115                 120                 125


Gly Pro Gly Gln Gln Gly Pro Tyr Gly Ser Ala Ala Ala Ala Ala Gly
    130                 135                 140


Pro Gly Ser Gly Gln Tyr Gly Gln Gly Pro Tyr Gly Pro Gly Ala Ser
145                 150                 155                 160


Gly Pro Gly Gln Tyr Gly Pro Gly Gln Gln Gly Pro Ser Ala Ser Ala
            165                 170                 175


Ala Ala Ala Ala Gly Ser Gly Gln Gln Gly Pro Gly Gln Tyr Gly Pro
        180                 185                 190


Tyr Ala Ser Ala Ala Ala Ala Ala Gly Gln Tyr Gly Ser Gly Pro Gly
        195                 200                 205
```

Gln Gln Gly Pro Tyr Gly Pro Gly Gln Ser Gly Ser Gly Gln Gln Gly
210 215 220

Pro Gly Gln Gln Gly Pro Tyr Ala Ser Ala Ala Ala Ala Ala Gly Pro
225 230 235 240

Gly Gln Gln Gly Pro Tyr Gly Pro Gly Ser Ser Ala Ala Ala Ala Ala
245 250 255

Gly Gln Tyr Gly Tyr Gly Pro Gly Gln Gln Gly Pro Tyr Gly Pro Gly
260 265 270

Ala Ser Gly Gln Asn Gly Pro Gly Ser Gly Gln Tyr Gly Pro Gly Gln
275 280 285

Gln Gly Pro Gly Gln Ser Ala Ala Ala Ala Gly Pro Gly Gln Gln
290 295 300

Gly Pro Tyr Gly Pro Gly Ala Ser Ala Ala Ala Ala Ala Gly Gln Tyr
305 310 315 320

Gly Pro Gly Gln Gln Gly Pro Gly Gln Tyr Gly Pro Gly Ser Ser Gly
325 330 335

Pro Gly Gln Gln Gly Pro Tyr Gly Pro Gly Ser Ser Ala Ala Ala Ala
340 345 350

Ala Gly Gln Tyr Gly Pro Gly Gln Gln Gly Pro Tyr Gly Pro Gly Gln
355 360 365

Ser Ala Ala Ala Ala Ala Gly Gln Tyr Gln Gln Gly Pro Gly Gln Gln
370 375 380

Gly Pro Tyr Gly Pro Gly Ala Ser Gly Pro Gly Gln Gln Gly Pro Tyr
385 390 395 400

Gly Pro Gly Ala Ser Ala Ala Ala Ala Ala Gly Pro Gly Gln Tyr Gly
405 410 415

Pro Gly Gln Gln Gly Pro Ser Ala Ser Ala Ala Ala Ala Ala Gly Gln
420 425 430

Tyr Gly Ser Gly Pro Gly Gln Tyr Gly Pro Tyr Gly Pro Gly Gln Ser
435 440 445

Gly Pro Gly Ser Gly Gln Gln Gly Gln Gly Pro Tyr Gly Pro Gly Ala
450 455 460

Ser Ala Ala Ala Ala Ala Gly Gln Tyr Gly Pro Gly Gln Gln Gly Pro
465                 470             475                 480

Tyr Gly Pro Gly Gln Ser Ala Ala Ala Ala Ala Gly Pro Gly Ser Gly
            485                 490             495

Gln Tyr Gly Pro Gly Ala Ser Gly Gln Asn Gly Pro Gly Ser Gly Gln
            500                 505             510

Tyr Gly Pro Gly Gln Gln Gly Pro Gly Gln Ser Ala Ala Ala Ala Ala
            515                 520             525

Gly Gln Tyr Gln Gln Gly Pro Gly Gln Gln Gly Pro Tyr Gly Pro Gly
    530                 535             540

Ala Ser Ala Ala Ala Ala Ala Gly Gln Tyr Gly Ser Gly Pro Gly Gln
545                 550             555                 560

Gln Gly Pro Tyr Gly Pro Gly Gln Ser Gly Ser Gly Gln Gln Gly Pro
            565                 570             575

Gly Gln Gln Gly Pro Tyr Ala Ser Ala Ala Ala Ala Ala Gly Pro Gly
            580                 585             590

Ser Gly Gln Gln Gly Pro Gly Ala Ser Gly Gln Gln Gly Pro Tyr Gly
            595                 600             605

Pro Gly Ala Ser Ala Ala Ala Ala Ala Gly Gln Asn Gly Pro Gly Ser
    610                 615                 620

Gly Gln Gln Gly Pro Gly Gln Ser Gly Gln Tyr Gly Pro Gly Gln Gln
625                 630             635                 640

Gly Pro Gly Gln Gln Gly Pro Gly Ser Ser Ala Ala Ala Ala Ala Gly
            645                 650             655

Pro Gly Gln Tyr Gly Pro Gly Gln Gln Gly Pro Ser Ala Ser Ala Ala
            660                 665             670

Ala Ala Ala Gly Pro Gly Ser Gly Gln Gln Gly Pro Gly Ala Ser Gly
    675                 680             685

Gln Tyr Gly Pro Gly Gln Gln Gly Pro Gly Gln Gln Gly Pro Gly Ser
    690                 695             700

Ser Ala Ala Ala Ala Ala Gly Gln Tyr Gly Ser Gly Pro Gly Gln Gln

77

```
         705                    710                    715                    720


         Gly Pro Tyr Gly Ser Ala Ala Ala Ala Ala Gly Pro Gly Ser Gly Gln
                         725                    730                    735


         Tyr Gly Gln Gly Pro Tyr Gly Pro Gly Ala Ser Gly Pro Gly Gln Tyr
                         740                    745                    750


         Gly Pro Gly Gln Gln Gly Pro Ser Ala Ser Ala Ala Ala Ala Ala Gly
                         755                    760                    765


         Ser Gly Gln Gln Gly Pro Gly Gln Tyr Gly Pro Tyr Ala Ser Ala Ala
                         770                    775                    780


         Ala Ala Ala Gly Gln Tyr Gly Ser Gly Pro Gly Gln Gln Gly Pro Tyr
         785                    790                    795                    800


         Gly Pro Gly Gln Ser Gly Ser Gly Gln Gln Gly Pro Gly Gln Gln Gly
                         805                    810                    815


         Pro Tyr Ala Ser Ala Ala Ala Ala Ala Gly Pro Gly Gln Gln Gly Pro
                         820                    825                    830


         Tyr Gly Pro Gly Ser Ser Ala Ala Ala Ala Gly Gln Tyr Gly Tyr
                         835                    840                    845


         Gly Pro Gly Gln Gln Gly Pro Tyr Gly Pro Gly Ala Ser Gly Gln Asn
                 850                    855                    860


         Gly Pro Gly Ser Gly Gln Tyr Gly Pro Gly Gln Gln Gly Pro Gly Gln
         865                    870                    875                    880


         Ser Ala Ala Ala Ala Ala Gly Pro Gly Gln Gln Gly Pro Tyr Gly Pro
                         885                    890                    895


         Gly Ala Ser Ala Ala Ala Ala Ala Gly Gln Tyr Gly Pro Gly Gln Gln
                 900                    905                    910


         Gly Pro Gly Gln Tyr Gly Pro Gly Ser Ser Gly Pro Gly Gln Gln Gly
                 915                    920                    925


         Pro Tyr Gly Pro Gly Ser Ser Ala Ala Ala Ala Gly Gln Tyr Gly
             930                    935                    940


         Pro Gly Gln Gln Gly Pro Tyr Gly Pro Gly Gln Ser Ala Ala Ala Ala
         945                    950                    955                    960
```

78

Ala Gly Gln Tyr Gln Gln Gly Pro Gly Gln Gln Gly Pro Tyr Gly Pro
965 970 975

Gly Ala Ser Gly Pro Gly Gln Gln Gly Pro Tyr Gly Pro Gly Ala Ser
980 985 990

Ala Ala Ala Ala Ala Gly Pro Gly Gln Tyr Gly Pro Gly Gln Gln Gly
995 1000 1005

Pro Ser Ala Ser Ala Ala Ala Ala Ala Gly Gln Tyr Gly Ser Gly
1010 1015 1020

Pro Gly Gln Tyr Gly Pro Tyr Gly Pro Gly Gln Ser Gly Pro Gly
1025 1030 1035

Ser Gly Gln Gln Gly Gln Gly Pro Tyr Gly Pro Gly Ala Ser Ala
1040 1045 1050

Ala Ala Ala Ala Gly Gln Tyr Gly Pro Gly Gln Gln Gly Pro Tyr
1055 1060 1065

Gly Pro Gly Gln Ser Ala Ala Ala Ala Ala Gly Pro Gly Ser Gly
1070 1075 1080

Gln Tyr Gly Pro Gly Ala Ser Gly Gln Asn Gly Pro Gly Ser Gly
1085 1090 1095

Gln Tyr Gly Pro Gly Gln Gln Gly Pro Gly Gln Ser Ala Ala Ala
1100 1105 1110

Ala Ala Gly Gln Tyr Gln Gln Gly Pro Gly Gln Gln Gly Pro Tyr
1115 1120 1125

Gly Pro Gly Ala Ser Ala Ala Ala Ala Ala Gly Gln Tyr Gly Ser
1130 1135 1140

Gly Pro Gly Gln Gln Gly Pro Tyr Gly Pro Gly Gln Ser Gly Ser
1145 1150 1155

Gly Gln Gln Gly Pro Gly Gln Gln Gly Pro Tyr Ala Ser Ala Ala
1160 1165 1170

Ala Ala Ala Gly Pro Gly Ser Gly Gln Gln Gly Pro Gly Ala Ser
1175 1180 1185

Gly Gln Gln Gly Pro Tyr Gly Pro Gly Ala Ser Ala Ala Ala Ala
1190 1195 1200

Ala Gly Gln Asn Gly Pro Gly Ser Gly Gln Gln Gly Pro Gly Gln
1205 1210 1215

Ser Gly Gln Tyr Gly Pro Gly Gln Gln Gly Pro Gly Gln Gln Gly
1220 1225 1230

Pro Gly Ser Ser Ala Ala Ala Ala Ala Gly Pro Gly Gln Tyr Gly
1235 1240 1245

Pro Gly Gln Gln Gly Pro Ser Ala Ser Ala Ala Ala Ala Ala Gly
1250 1255 1260

Pro Gly Ser Gly Gln Gln Gly Pro Gly Ala Ser Gly Gln Tyr Gly
1265 1270 1275

Pro Gly Gln Gln Gly Pro Gly Gln Gln Gly Pro Gly Ser Ser Ala
1280 1285 1290

Ala Ala Ala Ala Gly Gln Tyr Gly Ser Gly Pro Gly Gln Gln Gly
1295 1300 1305

Pro Tyr Gly Ser Ala Ala Ala Ala Ala Gly Pro Gly Ser Gly Gln
1310 1315 1320

Tyr Gly Gln Gly Pro Tyr Gly Pro Gly Ala Ser Gly Pro Gly Gln
1325 1330 1335

Tyr Gly Pro Gly Gln Gln Gly Pro Ser Ala Ser Ala Ala Ala Ala
1340 1345 1350

Ala Gly Ser Gly Gln Gln Gly Pro Gly Gln Tyr Gly Pro Tyr Ala
1355 1360 1365

Ser Ala Ala Ala Ala Gly Gln Tyr Gly Ser Gly Pro Gly Gln
1370 1375 1380

Gln Gly Pro Tyr Gly Pro Gly Gln Ser Gly Ser Gly Gln Gln Gly
1385 1390 1395

Pro Gly Gln Gln Gly Pro Tyr Ala Ser Ala Ala Ala Ala Ala Gly
1400 1405 1410

Pro Gly Gln Gln Gly Pro Tyr Gly Pro Gly Ser Ser Ala Ala Ala
1415 1420 1425

Ala Ala Gly Gln Tyr Gly Tyr Gly Pro Gly Gln Gln Gly Pro Tyr
1430 1435 1440

```
Gly Pro Gly Ala Ser Gly Gln  Asn Gly Pro Gly Ser  Gly Gln Tyr
    1445          1450              1455

Gly Pro Gly Gln Gln Gly Pro  Gly Gln Ser Ala Ala  Ala Ala Ala
    1460          1465              1470

Gly Pro Gly Gln Gln Gly Pro  Tyr Gly Pro Gly Ala  Ser Ala Ala
    1475          1480              1485

Ala Ala Ala Gly Gln Tyr Gly  Pro Gly Gln Gln Gly  Pro Gly Gln
    1490          1495              1500

Tyr Gly Pro Gly Ser Ser Gly  Pro Gly Gln Gln Gly  Pro Tyr Gly
    1505          1510              1515

Pro Gly Ser Ser Ala Ala Ala  Ala Ala Gly Gln Tyr  Gly Pro Gly
    1520          1525              1530

Gln Gln Gly Pro Tyr Gly Pro  Gly Gln Ser Ala Ala  Ala Ala Ala
    1535          1540              1545

Gly Gln Tyr Gln Gln Gly Pro  Gly Gln Gln Gly Pro  Tyr Gly Pro
    1550          1555              1560

Gly Ala Ser Gly Pro Gly Gln  Gln Gly Pro Tyr Gly  Pro Gly Ala
    1565          1570              1575

Ser Ala Ala Ala Ala Ala Gly  Pro Gly Gln Tyr Gly  Pro Gly Gln
    1580          1585              1590

Gln Gly Pro Ser Ala Ser Ala  Ala Ala Ala Ala Gly  Gln Tyr Gly
    1595          1600              1605

Ser Gly Pro Gly Gln Tyr Gly  Pro Tyr Gly Pro Gly  Gln Ser Gly
    1610          1615              1620

Pro Gly Ser Gly Gln Gln Gly  Gln Gly Pro Tyr Gly  Pro Gly Ala
    1625          1630              1635

Ser Ala Ala Ala Ala Ala Gly  Gln Tyr Gly Pro Gly  Gln Gln Gly
    1640          1645              1650

Pro Tyr Gly Pro Gly Gln Ser  Ala Ala Ala Ala Ala  Gly Pro Gly
    1655          1660              1665

Ser Gly Gln Tyr Gly Pro Gly  Ala Ser Gly Gln Asn  Gly Pro Gly
```

EP 3 827 682 A1

1670                    1675                    1680

Ser Gly Gln Tyr Gly Pro Gly Gln Gln Gly Pro Gly Gln Ser Ala
1685          1690              1695

Ala Ala Ala Ala Gly Gln Tyr Gln Gln Gly Pro Gly Gln Gln Gly
1700          1705              1710

Pro Tyr Gly Pro Gly Ala Ser Ala Ala Ala Ala Ala Gly Gln Tyr
1715          1720              1725

Gly Ser Gly Pro Gly Gln Gln Gly Pro Tyr Gly Pro Gly Gln Ser
1730          1735              1740

Gly Ser Gly Gln Gln Gly Pro Gly Gln Gln Gly Pro Tyr Ala Ser
1745          1750              1755

Ala Ala Ala Ala Ala Gly Pro Gly Ser Gly Gln Gln Gly Pro Gly
1760          1765              1770

Ala Ser Gly Gln Gln Gly Pro Tyr Gly Pro Gly Ala Ser Ala Ala
1775          1780              1785

Ala Ala Ala Gly Gln Asn Gly Pro Gly Ser Gly Gln Gln Gly Pro
1790          1795              1800

Gly Gln Ser Gly Gln Tyr Gly Pro Gly Gln Gln Gly Pro Gly Gln
1805          1810              1815

Gln Gly Pro Gly Ser Ser Ala Ala Ala Ala Ala Gly Pro Gly Gln
1820          1825              1830

Tyr Gly Pro Gly Gln Gln Gly Pro Ser Ala Ser Ala Ala Ala Ala
1835          1840              1845

Ala Gly Pro Gly Ser Gly Gln Gln Gly Pro Gly Ala Ser Gly Gln
1850          1855              1860

Tyr Gly Pro Gly Gln Gln Gly Pro Gly Gln Gln Gly Pro Gly Ser
1865          1870              1875

Ser Ala Ala Ala Ala Ala Gly Gln Tyr Gly Ser Gly Pro Gly Gln
1880          1885              1890

Gln Gly Pro Tyr Gly Ser Ala Ala Ala Ala Ala Gly Pro Gly Ser
1895          1900              1905

82

```
Gly Gln  Tyr Gly Gln Gly Pro  Tyr Gly Pro Gly Ala  Ser Gly Pro
    1910             1915             1920

Gly Gln  Tyr Gly Pro Gly Gln  Gln Gly Pro Ser Ala  Ser Ala Ala
    1925             1930             1935

Ala Ala  Ala Gly Ser Gly Gln  Gln Gly Pro Gly Gln  Tyr Gly Pro
    1940             1945             1950

Tyr Ala  Ser Ala Ala Ala Ala  Ala Gly Gln Tyr Gly  Ser Gly Pro
    1955             1960             1965

Gly Gln  Gln Gly Pro Tyr Gly  Pro Gly Gln Ser Gly  Ser Gly Gln
    1970             1975             1980

Gln Gly  Pro Gly Gln Gln Gly  Pro Tyr Ala Ser Ala  Ala Ala Ala
    1985             1990             1995

Ala Gly  Pro Gly Gln Gln Gly  Pro Tyr Gly Pro Gly  Ser Ser Ala
    2000             2005             2010

Ala Ala  Ala Ala Gly Gln Tyr  Gly Tyr Gly Pro Gly  Gln Gln Gly
    2015             2020             2025

Pro Tyr  Gly Pro Gly Ala Ser  Gly Gln Asn Gly Pro  Gly Ser Gly
    2030             2035             2040

Gln Tyr  Gly Pro Gly Gln Gln  Gly Pro Gly Gln Ser  Ala Ala Ala
    2045             2050             2055

Ala Ala  Gly Pro Gly Gln Gln  Gly Pro Tyr Gly Pro  Gly Ala Ser
    2060             2065             2070

Ala Ala  Ala Ala Ala Gly Gln  Tyr Gly Pro Gly Gln  Gln Gly Pro
    2075             2080             2085

Gly Gln  Tyr Gly Pro Gly Ser  Ser Gly Pro Gly Gln  Gln Gly Pro
    2090             2095             2100

Tyr Gly  Pro Gly Ser Ser Ala  Ala Ala Ala Ala Gly  Gln Tyr Gly
    2105             2110             2115

Pro Gly  Gln Gln Gly Pro Tyr  Gly Pro Gly Gln Ser  Ala Ala Ala
    2120             2125             2130

Ala Ala  Gly Gln Tyr Gln Gln  Gly Pro Gly Gln Gln  Gly Pro Tyr
    2135             2140             2145
```

```
Gly Pro  Gly Ala Ser Gly Pro  Gly Gln Gln Gly Pro  Tyr Gly Pro
    2150             2155                 2160

Gly Ala  Ser Ala Ala Ala Ala  Ala Gly Pro Gly Gln  Tyr Gly Pro
    2165             2170                 2175

Gly Gln  Gln Gly Pro Ser Ala  Ser Ala Ala Ala Ala  Ala Gly Gln
    2180             2185                 2190

Tyr Gly  Ser Gly Pro Gly Gln  Tyr Gly Pro Tyr Gly  Pro Gly Gln
    2195             2200                 2205

Ser Gly  Pro Gly Ser Gly Gln  Gln Gly Gln Gly Pro  Tyr Gly Pro
    2210             2215                 2220

Gly Ala  Ser Ala Ala Ala Ala  Ala Gly Gln Tyr Gly  Pro Gly Gln
    2225             2230                 2235

Gln Gly  Pro Tyr Gly Pro Gly  Gln Ser Ala Ala Ala  Ala Ala Gly
    2240             2245                 2250

Pro Gly  Ser Gly Gln Tyr Gly  Pro Gly Ala Ser Gly  Gln Asn Gly
    2255             2260                 2265

Pro Gly  Ser Gly Gln Tyr Gly  Pro Gly Gln Gln Gly  Pro Gly Gln
    2270             2275                 2280

Ser Ala  Ala Ala Ala Ala Gly  Gln Tyr Gln Gln Gly  Pro Gly Gln
    2285             2290                 2295

Gln Gly  Pro Tyr Gly Pro Gly  Ala Ser Ala Ala Ala  Ala Ala Gly
    2300             2305                 2310

Gln Tyr  Gly Ser Gly Pro Gly  Gln Gln Gly Pro Tyr  Gly Pro Gly
    2315             2320                 2325

Gln Ser  Gly Ser Gly Gln Gln  Gly Pro Gly Gln Gln  Gly Pro Tyr
    2330             2335                 2340

Ala Ser  Ala Ala Ala Ala Ala  Gly Pro Gly Ser Gly  Gln Gln Gly
    2345             2350                 2355

Ser Ser  Val Asp Lys Leu Ala  Ala Ala Leu Glu His  His His His
    2360             2365                 2370

His His
    2375
```

84

```
<210>  16
<211>  608
<212>  PRT
<213>  Artificial Sequence

<220>
<223>  PRT313


<400>  16

Met His His His His His His Ser Ser Gly Ser Ser Gly Pro Gly Gly
1               5                   10                  15


Gln Gly Pro Tyr Gly Pro Gly Ala Ser Ala Ala Ala Ala Ala Gly Gly
            20                  25                  30


Asn Gly Pro Gly Ser Gly Gln Gln Gly Pro Gly Gly Ser Ala Ala Ala
            35                  40                  45


Ala Ala Gly Gly Tyr Gly Pro Gly Gly Gln Gly Pro Gly Gln Gln Gly
    50                  55                  60


Pro Gly Ser Ser Ala Ala Ala Ala Ala Gly Pro Gly Gly Tyr Gly Pro
65                  70                  75                  80


Gly Gly Gln Gly Pro Ser Ala Ser Ala Ala Ala Ala Ala Gly Pro Gly
                85                  90                  95


Ser Gly Gln Gln Gly Pro Gly Ala Ser Ala Ala Ala Ala Ala Gly Gly
            100                 105                 110


Tyr Gly Pro Gly Gly Gln Gly Pro Gly Gln Gln Gly Pro Gly Ser Ser
            115                 120                 125


Ala Ala Ala Ala Ala Gly Gly Tyr Gly Ser Gly Pro Gly Gln Gln Gly
    130                 135                 140


Pro Tyr Gly Ser Ala Ala Ala Ala Ala Gly Pro Gly Ser Gly Gly Tyr
145                 150                 155                 160


Gly Gln Gly Pro Tyr Gly Pro Gly Ala Ser Ala Ala Ala Ala Ala Gly
                165                 170                 175


Pro Gly Gly Tyr Gly Pro Gly Gly Gln Gly Pro Ser Ala Ser Ala Ala
            180                 185                 190


Ala Ala Ala Gly Ser Gly Gln Gln Gly Pro Gly Gly Tyr Gly Pro Tyr
            195                 200                 205
```

```
Ala Ser Ala Ala Ala Ala Ala Gly Gly Tyr Gly Ser Gly Pro Gly Gln
    210             215             220

Gln Gly Pro Tyr Gly Pro Gly Gly Ser Ala Ala Ala Ala Ala Gly Ser
225             230             235             240

Gly Gln Gln Gly Pro Gly Gln Gln Gly Pro Tyr Ala Ser Ala Ala Ala
            245             250             255

Ala Ala Gly Pro Gly Gly Gln Gly Pro Tyr Gly Pro Gly Ser Ser Ala
        260             265             270

Ala Ala Ala Ala Gly Gly Tyr Gly Tyr Gly Pro Gly Gly Gln Gly Pro
    275             280             285

Tyr Gly Pro Gly Ala Ser Ala Ala Ala Ala Gly Gly Asn Gly Pro
    290             295             300

Gly Ser Gly Gly Tyr Gly Pro Gly Gln Gln Gly Pro Gly Gly Ser Ala
305             310             315             320

Ala Ala Ala Gly Pro Gly Gly Gln Gly Pro Tyr Gly Pro Gly Ala
            325             330             335

Ser Ala Ala Ala Ala Gly Gly Tyr Gly Pro Gly Gly Gln Gly Pro
    340             345             350

Gly Gly Tyr Gly Pro Gly Ser Ser Ala Ala Ala Ala Gly Pro Gly
    355             360             365

Gly Gln Gly Pro Tyr Gly Pro Gly Ser Ser Ala Ala Ala Ala Gly
370             375             380

Gly Tyr Gly Pro Gly Gln Gln Gly Pro Tyr Gly Pro Gly Gly Ser Ala
385             390             395             400

Ala Ala Ala Gly Gly Tyr Gln Gln Gly Pro Gly Gly Gln Gly Pro
        405             410             415

Tyr Gly Pro Gly Ala Ser Ala Ala Ala Ala Gly Pro Gly Gly Gln
    420             425             430

Gly Pro Tyr Gly Pro Gly Ala Ser Ala Ala Ala Ala Gly Pro Gly
    435             440             445

Gly Tyr Gly Pro Gly Gly Gln Gly Pro Ser Ala Ser Ala Ala Ala Ala
    450             455             460
```

```
Ala Gly Gly Tyr Gly Ser Gly Pro Gly Gly Tyr Gly Pro Tyr Gly Pro
465             470             475             480


Gly Gly Ser Ala Ala Ala Ala Ala Gly Pro Gly Ser Gly Gln Gln Gly
            485             490             495


Gln Gly Pro Tyr Gly Pro Gly Ala Ser Ala Ala Ala Ala Ala Gly Gly
        500             505             510


Tyr Gly Pro Gly Gln Gln Gly Pro Tyr Gly Pro Gly Gly Ser Ala Ala
        515             520             525


Ala Ala Ala Gly Pro Gly Ser Gly Gly Tyr Gly Pro Gly Ala Ser Ala
    530             535             540


Ala Ala Ala Gly Gly Asn Gly Pro Gly Ser Gly Gly Tyr Gly Pro
545             550             555             560


Gly Gln Gln Gly Pro Gly Gly Ser Ala Ala Ala Ala Ala Gly Gly Tyr
            565             570             575


Gln Gln Gly Pro Gly Gly Gln Gly Pro Tyr Gly Pro Gly Ala Ser Ala
        580             585             590


Ala Ala Ala Gly Pro Gly Ser Gly Gln Gln Gly Pro Gly Ala Ser
    595             600             605
```

```
<210>  17
<211>  590
<212>  PRT
<213>  Artificial Sequence

<220>
<223>  Met-PRT399

<400>  17
```

```
Met Gly Pro Gly Gly Gln Gly Pro Tyr Gly Pro Gly Ala Ser Ala Ala
1               5               10              15


Ala Ala Ala Gly Gly Asn Gly Pro Gly Ser Gly Gln Gln Gly Pro Gly
            20              25              30


Gly Ser Gly Gly Tyr Gly Pro Gly Gly Gln Gly Pro Gly Gln Gln Gly
        35              40              45


Pro Gly Ser Ser Ala Ala Ala Ala Ala Gly Pro Gly Gly Tyr Gly Pro
    50              55              60
```

Gly Gly Gln Gly Pro Ser Ala Ser Ala Ala Ala Ala Ala Gly Pro Gly
65                  70                  75                  80

Ser Gly Gln Gln Gly Pro Gly Ala Ser Gly Gly Tyr Gly Pro Gly Gly
                    85                  90                  95

Gln Gly Pro Gly Gln Gln Gly Pro Gly Ser Ser Ala Ala Ala Ala Ala
                100                 105                 110

Gly Gly Tyr Gly Ser Gly Pro Gly Gln Gln Gly Pro Tyr Gly Ser Ala
            115                 120                 125

Ala Ala Ala Ala Gly Pro Gly Ser Gly Gly Tyr Gly Gln Gly Pro Tyr
            130                 135                 140

Gly Pro Gly Ala Ser Gly Pro Gly Gly Tyr Gly Pro Gly Gly Gln Gly
145                 150                 155                 160

Pro Ser Ala Ser Ala Ala Ala Ala Ala Gly Ser Gly Gln Gln Gly Pro
                165                 170                 175

Gly Gly Tyr Gly Pro Tyr Ala Ser Ala Ala Ala Ala Gly Gly Tyr
            180                 185                 190

Gly Ser Gly Pro Gly Gln Gln Gly Pro Tyr Gly Pro Gly Gly Ser Gly
            195                 200                 205

Ser Gly Gln Gln Gly Pro Gly Gln Gln Gly Pro Tyr Ala Ser Ala Ala
    210                 215                 220

Ala Ala Ala Gly Pro Gly Gly Gln Gly Pro Tyr Gly Pro Gly Ser Ser
225                 230                 235                 240

Ala Ala Ala Ala Ala Gly Gly Tyr Gly Tyr Gly Pro Gly Gly Gln Gly
            245                 250                 255

Pro Tyr Gly Pro Gly Ala Ser Gly Gly Asn Gly Pro Gly Ser Gly Gly
        260                 265                 270

Tyr Gly Pro Gly Gln Gln Gly Pro Gly Gly Ser Ala Ala Ala Ala Ala
        275                 280                 285

Gly Pro Gly Gly Gln Gly Pro Tyr Gly Pro Gly Ala Ser Ala Ala Ala
    290                 295                 300

Ala Ala Gly Gly Tyr Gly Pro Gly Gly Gln Gly Pro Gly Gly Tyr Gly
305                 310                 315                 320

Pro Gly Ser Ser Gly Pro Gly Gly Gln Gly Pro Tyr Gly Pro Gly Ser
325 330 335

Ser Ala Ala Ala Ala Ala Gly Gly Tyr Gly Pro Gly Gln Gln Gly Pro
340 345 350

Tyr Gly Pro Gly Gly Ser Ala Ala Ala Ala Ala Gly Gly Tyr Gln Gln
355 360 365

Gly Pro Gly Gly Gln Gly Pro Tyr Gly Pro Gly Ala Ser Gly Pro Gly
370 375 380

Gly Gln Gly Pro Tyr Gly Pro Gly Ala Ser Ala Ala Ala Ala Gly
385 390 395 400

Pro Gly Gly Tyr Gly Pro Gly Gly Gln Gly Pro Ser Ala Ser Ala Ala
405 410 415

Ala Ala Ala Gly Gly Tyr Gly Ser Gly Pro Gly Gly Tyr Gly Pro Tyr
420 425 430

Gly Pro Gly Gly Ser Gly Pro Gly Ser Gly Gln Gln Gly Gln Gly Pro
435 440 445

Tyr Gly Pro Gly Ala Ser Ala Ala Ala Ala Gly Gly Tyr Gly Pro
450 455 460

Gly Gln Gln Gly Pro Tyr Gly Pro Gly Gly Ser Ala Ala Ala Ala Ala
465 470 475 480

Gly Pro Gly Ser Gly Gly Tyr Gly Pro Gly Ala Ser Gly Gly Asn Gly
485 490 495

Pro Gly Ser Gly Gly Tyr Gly Pro Gly Gln Gln Gly Pro Gly Gly Ser
500 505 510

Ala Ala Ala Ala Ala Gly Gly Tyr Gln Gln Gly Pro Gly Gly Gln Gly
515 520 525

Pro Tyr Gly Pro Gly Ala Ser Ala Ala Ala Ala Gly Gly Tyr Gly
530 535 540

Ser Gly Pro Gly Gln Gln Gly Pro Tyr Gly Pro Gly Gly Ser Gly Ser
545 550 555 560

Gly Gln Gln Gly Pro Gly Gln Gln Gly Pro Tyr Ala Ser Ala Ala Ala

EP 3 827 682 A1

565                          570                          575

Ala Ala Gly Pro Gly Ser Gly Gln Gln Gly Pro Gly Ala Ser
            580                  585                  590

<210> 18
<211> 601
<212> PRT
<213> Artificial Sequence

<220>
<223> PRT399

<400> 18

Met His His His His His His Ser Ser Gly Ser Ser Gly Pro Gly Gly
1                   5                   10                  15

Gln Gly Pro Tyr Gly Pro Gly Ala Ser Ala Ala Ala Ala Ala Gly Gly
            20                  25                  30

Asn Gly Pro Gly Ser Gly Gln Gln Gly Pro Gly Gly Ser Gly Gly Tyr
            35                  40                  45

Gly Pro Gly Gly Gln Gly Pro Gly Gln Gln Gly Pro Gly Ser Ser Ala
        50                  55                  60

Ala Ala Ala Ala Gly Pro Gly Gly Tyr Gly Pro Gly Gly Gln Gly Pro
65                  70                  75                  80

Ser Ala Ser Ala Ala Ala Ala Ala Gly Pro Gly Ser Gly Gln Gln Gly
            85                  90                  95

Pro Gly Ala Ser Gly Gly Tyr Gly Pro Gly Gly Gln Gly Pro Gly Gln
            100                 105                 110

Gln Gly Pro Gly Ser Ser Ala Ala Ala Ala Ala Gly Gly Tyr Gly Ser
            115                 120                 125

Gly Pro Gly Gln Gln Gly Pro Tyr Gly Ser Ala Ala Ala Ala Ala Gly
            130                 135                 140

Pro Gly Ser Gly Gly Tyr Gly Gln Gly Pro Tyr Gly Pro Gly Ala Ser
145                 150                 155                 160

Gly Pro Gly Gly Tyr Gly Pro Gly Gly Gln Gly Pro Ser Ala Ser Ala
            165                 170                 175

Ala Ala Ala Ala Gly Ser Gly Gln Gln Gly Pro Gly Gly Tyr Gly Pro
            180                 185                 190

90

Tyr Ala Ser Ala Ala Ala Ala Ala Gly Gly Tyr Gly Ser Gly Pro Gly
        195                 200                 205

Gln Gln Gly Pro Tyr Gly Pro Gly Gly Ser Gly Ser Gly Gln Gln Gly
        210                 215                 220

Pro Gly Gln Gln Gly Pro Tyr Ala Ser Ala Ala Ala Ala Ala Gly Pro
225                 230                 235                 240

Gly Gly Gln Gly Pro Tyr Gly Pro Gly Ser Ser Ala Ala Ala Ala Ala
                245                 250                 255

Gly Gly Tyr Gly Tyr Gly Pro Gly Gly Gln Gly Pro Tyr Gly Pro Gly
                260                 265                 270

Ala Ser Gly Gly Asn Gly Pro Gly Ser Gly Gly Tyr Gly Pro Gly Gln
        275                 280                 285

Gln Gly Pro Gly Gly Ser Ala Ala Ala Ala Gly Pro Gly Gly Gln
        290                 295                 300

Gly Pro Tyr Gly Pro Gly Ala Ser Ala Ala Ala Ala Gly Gly Tyr
305                 310                 315                 320

Gly Pro Gly Gly Gln Gly Pro Gly Gly Tyr Gly Pro Gly Ser Ser Gly
                325                 330                 335

Pro Gly Gly Gln Gly Pro Tyr Gly Pro Gly Ser Ser Ala Ala Ala Ala
        340                 345                 350

Ala Gly Gly Tyr Gly Pro Gly Gln Gln Gly Pro Tyr Gly Pro Gly Gly
        355                 360                 365

Ser Ala Ala Ala Ala Ala Gly Gly Tyr Gln Gln Gly Pro Gly Gly Gln
        370                 375                 380

Gly Pro Tyr Gly Pro Gly Ala Ser Gly Pro Gly Gly Gln Gly Pro Tyr
385                 390                 395                 400

Gly Pro Gly Ala Ser Ala Ala Ala Ala Ala Gly Pro Gly Gly Tyr Gly
                405                 410                 415

Pro Gly Gly Gln Gly Pro Ser Ala Ser Ala Ala Ala Ala Gly Gly
        420                 425                 430

Tyr Gly Ser Gly Pro Gly Gly Tyr Gly Pro Tyr Gly Pro Gly Gly Ser

```
                435                     440                     445


        Gly Pro Gly Ser Gly Gln Gln Gly Gln Gly Pro Tyr Gly Pro Gly Ala
            450                 455                 460


        Ser Ala Ala Ala Ala Ala Gly Gly Tyr Gly Pro Gly Gln Gln Gly Pro
        465                 470                 475                 480


        Tyr Gly Pro Gly Gly Ser Ala Ala Ala Ala Gly Pro Gly Ser Gly
                        485                 490                 495


        Gly Tyr Gly Pro Gly Ala Ser Gly Gly Asn Gly Pro Gly Ser Gly Gly
                    500                 505                 510


        Tyr Gly Pro Gly Gln Gln Gly Pro Gly Gly Ser Ala Ala Ala Ala Ala
                515                 520                 525


        Gly Gly Tyr Gln Gln Gly Pro Gly Gly Gln Gly Pro Tyr Gly Pro Gly
            530                 535                 540


        Ala Ser Ala Ala Ala Ala Ala Gly Gly Tyr Gly Ser Gly Pro Gly Gln
        545                 550                 555                 560


        Gln Gly Pro Tyr Gly Pro Gly Gly Ser Gly Ser Gly Gln Gln Gly Pro
                        565                 570                 575


        Gly Gln Gln Gly Pro Tyr Ala Ser Ala Ala Ala Ala Gly Pro Gly
                    580                 585                 590


        Ser Gly Gln Gln Gly Pro Gly Ala Ser
                595                 600


        <210>  19
        <211>  612
        <212>  PRT
        <213>  Artificial Sequence

        <220>
        <223>  Met-PRT720

        <400>  19

        Met Gly Pro Gly Gln Gln Gly Pro Tyr Gly Pro Gly Ala Ser Ala Ala
        1               5                   10                  15


        Ala Ala Ala Gly Gln Asn Gly Pro Gly Ser Gly Gln Gln Gly Pro Gly
                    20                  25                  30


        Gln Ser Gly Gln Tyr Gly Pro Gly Gln Gln Gly Pro Gly Gln Gln Gly
                35                  40                  45
```

Pro Gly Ser Ser Ala Ala Ala Ala Ala Gly Pro Gly Gln Tyr Val Leu
   50             55            60

Ile Gly Pro Gly Gln Gln Val Leu Ile Gly Pro Ser Ala Ser Ala Ala
65            70            75            80

Ala Ala Ala Gly Pro Gly Ser Gly Gln Gln Gly Pro Gly Ala Ser Gly
        85            90            95

Gln Tyr Gly Pro Gly Gln Gln Gly Pro Gly Gln Gln Gly Pro Gly Ser
        100          105        110

Ser Ala Ala Ala Ala Ala Gly Ser Tyr Gly Ser Val Leu Ile Gly Pro
        115          120        125

Gly Gln Gln Val Leu Ile Gly Pro Tyr Gly Ser Ala Ala Ala Ala Ala
      130          135          140

Gly Pro Gly Ser Gly Gln Tyr Gly Gln Gly Pro Tyr Gly Pro Gly Ala
145            150            155            160

Ser Gly Pro Gly Gln Tyr Gly Pro Gly Gln Gln Gly Pro Ser Ala Ser
        165          170        175

Ala Ala Ala Ala Ala Gly Ser Gly Gln Gln Val Leu Ile Gly Pro Gly
        180          185        190

Gln Tyr Val Leu Ile Gly Pro Tyr Ala Ser Ala Ala Ala Ala Ala Gly
      195          200          205

Gln Tyr Gly Ser Gly Pro Gly Gln Gln Gly Pro Tyr Gly Pro Gly Gln
      210          215          220

Ser Gly Ser Gly Gln Gln Gly Pro Gly Gln Gln Gly Pro Tyr Ala Ser
225            230          235            240

Ala Ala Ala Ala Ala Gly Pro Gly Gln Gln Val Leu Ile Gly Pro Tyr
        245          250        255

Val Leu Ile Gly Pro Gly Ser Ser Ala Ala Ala Ala Ala Gly Gln Tyr
      260          265          270

Gly Tyr Gly Pro Gly Gln Gln Gly Pro Tyr Gly Pro Gly Ala Ser Gly
      275          280          285

Gln Asn Gly Pro Gly Ser Gly Gln Tyr Gly Pro Gly Gln Gln Gly Pro

290        295        300

Gly Gln Ser Ala Ala Ala Ala Ala Gly Pro Gly Gln Gln Val Leu Ile
305           310           315           320

Gly Pro Tyr Val Leu Ile Gly Pro Gly Ala Ser Ala Ala Ala Ala Ala
          325           330           335

Gly Gln Tyr Gly Pro Gly Gln Gln Gly Pro Gly Gln Tyr Gly Pro Gly
          340           345           350

Ser Ser Gly Pro Gly Gln Gln Gly Pro Tyr Gly Pro Gly Ser Ser Ala
          355           360           365

Ala Ala Ala Ala Gly Ser Tyr Gly Pro Gly Gln Gln Val Leu Ile Gly
          370           375           380

Pro Tyr Val Leu Ile Gly Pro Gly Pro Ser Ala Ala Ala Ala Ala Gly
385           390           395           400

Gln Tyr Gln Gln Gly Pro Gly Gln Gln Gly Pro Tyr Gly Pro Gly Ala
          405           410           415

Ser Gly Pro Gly Gln Gln Gly Pro Tyr Gly Pro Gly Ala Ser Ala Ala
          420           425           430

Ala Ala Ala Gly Pro Gly Gln Tyr Val Leu Ile Gly Pro Gly Gln Gln
          435           440           445

Val Leu Ile Gly Pro Ser Ala Ser Ala Ala Ala Ala Gly Gln Tyr
          450           455           460

Gly Ser Gly Pro Gly Gln Tyr Gly Pro Tyr Gly Pro Gly Gln Ser Gly
465           470           475           480

Pro Gly Ser Gly Gln Gln Gly Gln Gly Pro Tyr Gly Pro Gly Ala Ser
          485           490           495

Ala Ala Ala Ala Ala Gly Ser Tyr Gly Pro Gly Gln Gln Val Leu Ile
          500           505           510

Gly Pro Tyr Val Leu Ile Gly Pro Gly Pro Ser Ala Ala Ala Ala Ala
          515           520           525

Gly Pro Gly Ser Gly Gln Tyr Gly Pro Gly Ala Ser Gly Gln Asn Gly
          530           535           540

```
Pro Gly Ser Gly Gln Tyr Gly Pro Gly Gln Gln Gly Pro Gly Gln Ser
545             550             555             560


Ala Ala Ala Ala Ala Gly Gln Tyr Gln Gln Val Leu Ile Gly Pro Gly
                565             570             575


Gln Gln Gly Pro Tyr Val Leu Ile Gly Pro Gly Ala Ser Ala Ala Ala
            580             585             590


Ala Ala Gly Pro Gly Ser Gly Gln Gln Val Leu Ile Gly Pro Gly Ala
        595             600             605


Ser Val Leu Ile
    610



<210>  20
<211>  592
<212>  PRT
<213>  Artificial Sequence

<220>
<223>  Met-PRT665


<400>  20

Met Gly Pro Gly Gln Gln Gly Pro Tyr Gly Pro Gly Ala Ser Ala Ala
1               5               10              15


Ala Ala Ala Ala Ala Gly Ser Asn Gly Pro Gly Ser Gly Gln Gln Gly
                20              25              30


Pro Gly Gln Ser Gly Gln Tyr Gly Pro Gly Gln Gln Gly Pro Gly Gln
            35              40              45


Gln Gly Pro Gly Ser Ser Ala Ala Ala Ala Ala Ala Gly Pro Gly
        50              55              60


Gln Tyr Val Leu Ile Gly Pro Gly Gln Gln Gly Pro Ser Ala Ser Ala
65              70              75              80


Ala Ala Ala Ala Ala Gly Pro Gly Ser Gly Gln Gln Gly Pro Gly
                85              90              95


Ala Ser Gly Gln Tyr Gly Pro Gly Gln Gln Gly Pro Gly Gln Gln Gly
            100             105             110


Pro Gly Ser Ser Ala Ala Ala Ala Ala Ala Ala Gly Ser Tyr Gly Ser
            115             120             125


Val Leu Ile Gly Pro Gly Gln Gln Gly Pro Tyr Gly Ser Ala Ala Ala
```

                    130                        135                           140

Ala Ala Ala Ala Gly Pro Gly Ser Gly Gln Tyr Gly Gln Gly Pro Tyr
145                 150                 155                 160

Gly Pro Gly Ala Ser Gly Pro Gly Gln Tyr Gly Pro Gly Gln Gln Gly
                165                 170                 175

Pro Ser Ala Ser Ala Ala Ala Ala Ala Ala Gly Ser Gly Gln Gln
            180                 185                 190

Val Leu Ile Gly Pro Gly Gln Tyr Gly Pro Tyr Ala Ser Ala Ala Ala
            195                 200                 205

Ala Ala Ala Ala Gly Ser Tyr Gly Ser Gly Pro Gly Gln Gln Gly Pro
            210                 215                 220

Tyr Gly Pro Gly Gln Ser Gly Ser Gly Gln Gln Gly Pro Gly Gln Gln
225                 230                 235                 240

Gly Pro Tyr Ala Ser Ala Ala Ala Ala Ala Ala Gly Pro Gly Gln
            245                 250                 255

Gln Val Leu Ile Gly Pro Tyr Gly Pro Gly Ser Ser Ala Ala Ala Ala
            260                 265                 270

Ala Ala Ala Gly Ser Tyr Gly Tyr Gly Pro Gly Gln Gln Gly Pro Tyr
            275                 280                 285

Gly Pro Gly Ala Ser Gly Gln Asn Gly Pro Gly Ser Gly Gln Tyr Gly
            290                 295                 300

Pro Gly Gln Gln Gly Pro Gly Pro Ser Ala Ala Ala Ala Ala Ala Ala
305                 310                 315                 320

Gly Pro Gly Gln Gln Val Leu Ile Gly Pro Tyr Gly Pro Gly Ala Ser
            325                 330                 335

Ala Ala Ala Ala Ala Ala Ala Gly Ser Tyr Gly Pro Gly Gln Gln Gly
            340                 345                 350

Pro Gly Gln Tyr Gly Pro Gly Ser Ser Gly Pro Gly Gln Gln Gly Pro
            355                 360                 365

Tyr Gly Pro Gly Ser Ser Ala Ala Ala Ala Ala Ala Ala Gly Ser Tyr
370                 375                 380

96

Gly Pro Gly Gln Gln Val Leu Ile Gly Pro Tyr Gly Pro Gly Pro Ser
385                 390                 395                 400

Ala Ala Ala Ala Ala Ala Ala Gly Ser Tyr Gln Gln Gly Pro Gly Gln
            405                 410                 415

Gln Gly Pro Tyr Gly Pro Gly Ala Ser Gly Pro Gly Gln Gln Gly Pro
        420                 425                 430

Tyr Gly Pro Gly Ala Ser Ala Ala Ala Ala Ala Ala Ala Gly Pro Gly
        435                 440                 445

Gln Tyr Val Leu Ile Gly Pro Gly Gln Gln Gly Pro Ser Ala Ser Ala
    450                 455                 460

Ala Ala Ala Ala Ala Ala Gly Ser Tyr Gly Ser Gly Pro Gly Gln Tyr
465                 470                 475                 480

Gly Pro Tyr Gly Pro Gly Gln Ser Gly Pro Gly Ser Gly Gln Gln Gly
        485                 490                 495

Gln Gly Pro Tyr Gly Pro Gly Ala Ser Ala Ala Ala Ala Ala Ala Ala
        500                 505                 510

Gly Ser Tyr Gly Pro Gly Gln Gln Val Leu Ile Gly Pro Tyr Gly Pro
    515                 520                 525

Gly Pro Ser Ala Ala Ala Ala Ala Ala Ala Gly Pro Gly Ser Gly Gln
    530                 535                 540

Tyr Gly Pro Gly Ala Ser Gly Gln Asn Gly Pro Gly Ser Gly Gln Tyr
545                 550                 555                 560

Gly Pro Gly Gln Gln Gly Pro Gly Pro Ser Ala Ala Ala Ala Ala Ala
            565                 570                 575

Ala Gly Pro Gly Ser Gly Gln Gln Gly Pro Gly Ala Ser Val Leu Ile
        580                 585                 590

<210>    21
<211>    619
<212>    PRT
<213>    Artificial Sequence

<220>
<223>    Met-PRT666

<400>    21

Met Gly Pro Gly Gln Gln Gly Pro Tyr Gly Pro Gly Ala Ser Ala Ala

```
        1                    5                    10                   15

        Ala Ala Ala Ala Ala Gly Ser Asn Gly Pro Gly Ser Gly Gln Gln Gly
                20                   25                   30

        Pro Gly Gln Ser Gly Gln Tyr Gly Pro Gly Gln Gln Gly Pro Gly Gln
                35                   40                   45

        Gln Gly Pro Gly Ser Ser Ala Ala Ala Ala Ala Ala Gly Pro Gly
                50                   55                   60

        Gln Tyr Val Leu Ile Gly Pro Gly Gln Gln Val Leu Ile Gly Pro Ser
        65                   70                   75                   80

        Ala Ser Ala Ala Ala Ala Ala Ala Gly Pro Gly Ser Gly Gln Gln
                        85                   90                   95

        Gly Pro Gly Ala Ser Gly Gln Tyr Gly Pro Gly Gln Gln Gly Pro Gly
                    100                  105                  110

        Gln Gln Gly Pro Gly Ser Ser Ala Ala Ala Ala Ala Ala Ala Gly Ser
                    115                  120                  125

        Tyr Gly Ser Val Leu Ile Gly Pro Gly Gln Gln Val Leu Ile Gly Pro
            130                  135                  140

        Tyr Gly Ser Ala Ala Ala Ala Ala Ala Gly Pro Gly Ser Gly Gln
        145                  150                  155                  160

        Tyr Gly Gln Gly Pro Tyr Gly Pro Gly Ala Ser Gly Pro Gly Gln Tyr
                    165                  170                  175

        Gly Pro Gly Gln Gln Gly Pro Ser Ala Ser Ala Ala Ala Ala Ala Ala
                    180                  185                  190

        Ala Gly Ser Gly Gln Gln Val Leu Ile Gly Pro Gly Gln Tyr Val Leu
                    195                  200                  205

        Ile Gly Pro Tyr Ala Ser Ala Ala Ala Ala Ala Ala Gly Ser Tyr
            210                  215                  220

        Gly Ser Gly Pro Gly Gln Gln Gly Pro Tyr Gly Pro Gly Gln Ser Gly
        225                  230                  235                  240

        Ser Gly Gln Gln Gly Pro Gly Gln Gln Gly Pro Tyr Ala Ser Ala Ala
                    245                  250                  255
```

```
Ala Ala Ala Ala Ala Gly Pro Gly Gln Gln Val Leu Ile Gly Pro Tyr
            260                 265                 270

Val Leu Ile Gly Pro Gly Ser Ser Ala Ala Ala Ala Ala Ala Ala Gly
            275                 280                 285

Ser Tyr Gly Tyr Gly Pro Gly Gln Gln Gly Pro Tyr Gly Pro Gly Ala
            290                 295                 300

Ser Gly Gln Asn Gly Pro Gly Ser Gly Gln Tyr Gly Pro Gly Gln Gln
305                 310                 315                 320

Gly Pro Gly Pro Ser Ala Ala Ala Ala Ala Ala Gly Pro Gly Gln
            325                 330                 335

Gln Val Leu Ile Gly Pro Tyr Val Leu Ile Gly Pro Gly Ala Ser Ala
            340                 345                 350

Ala Ala Ala Ala Ala Ala Gly Ser Tyr Gly Pro Gly Gln Gln Gly Pro
            355                 360                 365

Gly Gln Tyr Gly Pro Gly Ser Ser Gly Pro Gly Gln Gln Gly Pro Tyr
            370                 375                 380

Gly Pro Gly Ser Ser Ala Ala Ala Ala Ala Ala Gly Ser Tyr Gly
385                 390                 395                 400

Pro Gly Gln Gln Val Leu Ile Gly Pro Tyr Val Leu Ile Gly Pro Gly
                405                 410                 415

Pro Ser Ala Ala Ala Ala Ala Ala Gly Ser Tyr Gln Gln Gly Pro
            420                 425                 430

Gly Gln Gln Gly Pro Tyr Gly Pro Gly Ala Ser Gly Pro Gly Gln Gln
            435                 440                 445

Gly Pro Tyr Gly Pro Gly Ala Ser Ala Ala Ala Ala Ala Ala Gly
            450                 455                 460

Pro Gly Gln Tyr Val Leu Ile Gly Pro Gly Gln Gln Val Leu Ile Gly
465                 470                 475                 480

Pro Ser Ala Ser Ala Ala Ala Ala Ala Ala Gly Ser Tyr Gly Ser
            485                 490                 495

Gly Pro Gly Gln Tyr Gly Pro Tyr Gly Pro Gly Gln Ser Gly Pro Gly
            500                 505                 510
```

```
Ser Gly Gln Gln Gly Gln Gly Pro Tyr Gly Pro Gly Ala Ser Ala Ala
        515                 520             525

Ala Ala Ala Ala Ala Gly Ser Tyr Gly Pro Gly Gln Gln Val Leu Ile
        530                 535             540

Gly Pro Tyr Val Leu Ile Gly Pro Gly Pro Ser Ala Ala Ala Ala Ala
545                 550             555                 560

Ala Ala Gly Pro Gly Ser Gly Gln Tyr Gly Pro Gly Ala Ser Gly Gln
            565                 570             575

Asn Gly Pro Gly Ser Gly Gln Tyr Gly Pro Gly Gln Gln Gly Pro Gly
            580                 585             590

Pro Ser Ala Ala Ala Ala Ala Ala Gly Pro Gly Ser Gly Gln Gln
            595                 600             605

Val Leu Ile Gly Pro Gly Ala Ser Val Leu Ile
        610                 615


<210>  22
<211>  623
<212>  PRT
<213>  Artificial Sequence

<220>
<223>  PRT720

<400>  22

Met His His His His His His Ser Ser Gly Ser Ser Gly Pro Gly Gln
1               5               10                  15

Gln Gly Pro Tyr Gly Pro Gly Ala Ser Ala Ala Ala Ala Ala Gly Gln
            20                  25                  30

Asn Gly Pro Gly Ser Gly Gln Gln Gly Pro Gly Gln Ser Gly Gln Tyr
            35                  40                  45

Gly Pro Gly Gln Gln Gly Pro Gly Gln Gln Gly Pro Gly Ser Ser Ala
        50                  55                  60

Ala Ala Ala Gly Pro Gly Gln Tyr Val Leu Ile Gly Pro Gly Gln
65                  70                  75                  80

Gln Val Leu Ile Gly Pro Ser Ala Ser Ala Ala Ala Ala Gly Pro
                85                  90                  95
```

```
Gly Ser Gly Gln Gln Gly Pro Gly Ala Ser Gly Gln Tyr Gly Pro Gly
            100             105             110

Gln Gln Gly Pro Gly Gln Gln Gly Pro Gly Ser Ser Ala Ala Ala Ala
            115             120             125

Ala Gly Ser Tyr Gly Ser Val Leu Ile Gly Pro Gly Gln Gln Val Leu
            130             135             140

Ile Gly Pro Tyr Gly Ser Ala Ala Ala Ala Gly Pro Gly Ser Gly
145             150             155             160

Gln Tyr Gly Gln Gly Pro Tyr Gly Pro Gly Ala Ser Gly Pro Gly Gln
                165             170             175

Tyr Gly Pro Gly Gln Gln Gly Pro Ser Ala Ser Ala Ala Ala Ala Ala
            180             185             190

Gly Ser Gly Gln Gln Val Leu Ile Gly Pro Gly Gln Tyr Val Leu Ile
            195             200             205

Gly Pro Tyr Ala Ser Ala Ala Ala Ala Gly Gln Tyr Gly Ser Gly
            210             215             220

Pro Gly Gln Gln Gly Pro Tyr Gly Pro Gly Gln Ser Gly Ser Gly Gln
225             230             235             240

Gln Gly Pro Gly Gln Gln Gly Pro Tyr Ala Ser Ala Ala Ala Ala Ala
            245             250             255

Gly Pro Gly Gln Gln Val Leu Ile Gly Pro Tyr Val Leu Ile Gly Pro
            260             265             270

Gly Ser Ser Ala Ala Ala Ala Ala Gly Gln Tyr Gly Tyr Gly Pro Gly
            275             280             285

Gln Gln Gly Pro Tyr Gly Pro Gly Ala Ser Gly Gln Asn Gly Pro Gly
            290             295             300

Ser Gly Gln Tyr Gly Pro Gly Gln Gln Gly Pro Gly Gln Ser Ala Ala
305             310             315             320

Ala Ala Ala Gly Pro Gly Gln Gln Val Leu Ile Gly Pro Tyr Val Leu
            325             330             335

Ile Gly Pro Gly Ala Ser Ala Ala Ala Ala Gly Gln Tyr Gly Pro
            340             345             350
```

101

Gly Gln Gln Gly Pro Gly Gln Tyr Gly Pro Gly Ser Ser Gly Pro Gly
        355                 360                 365

Gln Gln Gly Pro Tyr Gly Pro Gly Ser Ser Ala Ala Ala Ala Ala Gly
        370                 375                 380

Ser Tyr Gly Pro Gly Gln Gln Val Leu Ile Gly Pro Tyr Val Leu Ile
385                 390                 395                 400

Gly Pro Gly Pro Ser Ala Ala Ala Ala Ala Gly Gln Tyr Gln Gln Gly
                405                 410                 415

Pro Gly Gln Gln Gly Pro Tyr Gly Pro Gly Ala Ser Gly Pro Gly Gln
            420                 425                 430

Gln Gly Pro Tyr Gly Pro Gly Ala Ser Ala Ala Ala Ala Ala Gly Pro
        435                 440                 445

Gly Gln Tyr Val Leu Ile Gly Pro Gly Gln Gln Val Leu Ile Gly Pro
    450                 455                 460

Ser Ala Ser Ala Ala Ala Ala Ala Gly Gln Tyr Gly Ser Gly Pro Gly
465                 470                 475                 480

Gln Tyr Gly Pro Tyr Gly Pro Gly Gln Ser Gly Pro Gly Ser Gly Gln
            485                 490                 495

Gln Gly Gln Gly Pro Tyr Gly Pro Gly Ala Ser Ala Ala Ala Ala Ala
            500                 505                 510

Gly Ser Tyr Gly Pro Gly Gln Gln Val Leu Ile Gly Pro Tyr Val Leu
        515                 520                 525

Ile Gly Pro Gly Pro Ser Ala Ala Ala Ala Gly Pro Gly Ser Gly
        530                 535                 540

Gln Tyr Gly Pro Gly Ala Ser Gly Gln Asn Gly Pro Gly Ser Gly Gln
545                 550                 555                 560

Tyr Gly Pro Gly Gln Gln Gly Pro Gly Gln Ser Ala Ala Ala Ala Ala
            565                 570                 575

Gly Gln Tyr Gln Gln Val Leu Ile Gly Pro Gly Gln Gln Gly Pro Tyr
        580                 585                 590

Val Leu Ile Gly Pro Gly Ala Ser Ala Ala Ala Ala Ala Gly Pro Gly
    595                 600                 605

102

```
Ser Gly Gln Gln Val Leu Ile Gly Pro Gly Ala Ser Val Leu Ile
    610             615             620
```

<210> 23
<211> 603
<212> PRT
<213> Artificial Sequence

<220>
<223> PRT665

<400> 23

```
Met His His His His His His Ser Ser Gly Ser Ser Gly Pro Gly Gln
1               5               10              15


Gln Gly Pro Tyr Gly Pro Gly Ala Ser Ala Ala Ala Ala Ala Ala Ala
            20              25              30


Gly Ser Asn Gly Pro Gly Ser Gly Gln Gln Gly Pro Gly Gln Ser Gly
        35              40              45


Gln Tyr Gly Pro Gly Gln Gln Gly Pro Gly Gln Gln Gly Pro Gly Ser
    50              55              60


Ser Ala Ala Ala Ala Ala Ala Ala Gly Pro Gly Gln Tyr Val Leu Ile
65              70              75              80


Gly Pro Gly Gln Gln Gly Pro Ser Ala Ser Ala Ala Ala Ala Ala Ala
            85              90              95


Ala Gly Pro Gly Ser Gly Gln Gln Gly Pro Gly Ala Ser Gly Gln Tyr
            100             105             110


Gly Pro Gly Gln Gln Gly Pro Gly Gln Gln Gly Pro Gly Ser Ser Ala
        115             120             125


Ala Ala Ala Ala Ala Gly Ser Tyr Gly Ser Val Leu Ile Gly Pro
    130             135             140


Gly Gln Gln Gly Pro Tyr Gly Ser Ala Ala Ala Ala Ala Ala Ala Gly
145             150             155             160


Pro Gly Ser Gly Gln Tyr Gly Gln Gly Pro Tyr Gly Pro Gly Ala Ser
            165             170             175


Gly Pro Gly Gln Tyr Gly Pro Gly Gln Gln Gly Pro Ser Ala Ser Ala
            180             185             190
```

```
Ala Ala Ala Ala Ala Ala Gly Ser Gly Gln Gln Val Leu Ile Gly Pro
        195                 200                 205

Gly Gln Tyr Gly Pro Tyr Ala Ser Ala Ala Ala Ala Ala Ala Ala Gly
        210                 215                 220

Ser Tyr Gly Ser Gly Pro Gly Gln Gln Gly Pro Tyr Gly Pro Gly Gln
225                 230                 235                 240

Ser Gly Ser Gly Gln Gln Gly Pro Gly Gln Gln Gly Pro Tyr Ala Ser
                245                 250                 255

Ala Ala Ala Ala Ala Ala Ala Gly Pro Gly Gln Gln Val Leu Ile Gly
            260                 265                 270

Pro Tyr Gly Pro Gly Ser Ser Ala Ala Ala Ala Ala Ala Gly Ser
        275                 280                 285

Tyr Gly Tyr Gly Pro Gly Gln Gln Gly Pro Tyr Gly Pro Gly Ala Ser
    290                 295                 300

Gly Gln Asn Gly Pro Gly Ser Gly Gln Tyr Gly Pro Gly Gln Gln Gly
305                 310                 315                 320

Pro Gly Pro Ser Ala Ala Ala Ala Ala Ala Gly Pro Gly Gln Gln
            325                 330                 335

Val Leu Ile Gly Pro Tyr Gly Pro Gly Ala Ser Ala Ala Ala Ala Ala
            340                 345                 350

Ala Ala Gly Ser Tyr Gly Pro Gly Gln Gln Gly Pro Gly Gln Tyr Gly
        355                 360                 365

Pro Gly Ser Ser Gly Pro Gly Gln Gln Gly Pro Tyr Gly Pro Gly Ser
        370                 375                 380

Ser Ala Ala Ala Ala Ala Ala Gly Ser Tyr Gly Pro Gly Gln Gln
385                 390                 395                 400

Val Leu Ile Gly Pro Tyr Gly Pro Gly Pro Ser Ala Ala Ala Ala Ala
            405                 410                 415

Ala Ala Gly Ser Tyr Gln Gln Gly Pro Gly Gln Gln Gly Pro Tyr Gly
        420                 425                 430

Pro Gly Ala Ser Gly Pro Gly Gln Gln Gly Pro Tyr Gly Pro Gly Ala
        435                 440                 445
```

Ser Ala Ala Ala Ala Ala Ala Ala Gly Pro Gly Gln Tyr Val Leu Ile
        450             455             460

Gly Pro Gly Gln Gln Gly Pro Ser Ala Ser Ala Ala Ala Ala Ala Ala
465             470             475             480

Ala Gly Ser Tyr Gly Ser Gly Pro Gly Gln Tyr Gly Pro Tyr Gly Pro
                485             490             495

Gly Gln Ser Gly Pro Gly Ser Gly Gln Gln Gly Gln Gly Pro Tyr Gly
            500             505             510

Pro Gly Ala Ser Ala Ala Ala Ala Ala Ala Ala Gly Ser Tyr Gly Pro
            515             520             525

Gly Gln Gln Val Leu Ile Gly Pro Tyr Gly Pro Gly Pro Ser Ala Ala
        530             535             540

Ala Ala Ala Ala Ala Gly Pro Gly Ser Gly Gln Tyr Gly Pro Gly Ala
545             550             555             560

Ser Gly Gln Asn Gly Pro Gly Ser Gly Gln Tyr Gly Pro Gly Gln Gln
            565             570             575

Gly Pro Gly Pro Ser Ala Ala Ala Ala Ala Ala Ala Gly Pro Gly Ser
            580             585             590

Gly Gln Gln Gly Pro Gly Ala Ser Val Leu Ile
        595             600

<210> 24
<211> 630
<212> PRT
<213> Artificial Sequence

<220>
<223> PRT666

<400> 24

Met His His His His His His Ser Ser Gly Ser Ser Gly Pro Gly Gln
1               5               10              15

Gln Gly Pro Tyr Gly Pro Gly Ala Ser Ala Ala Ala Ala Ala Ala Ala
            20              25              30

Gly Ser Asn Gly Pro Gly Ser Gly Gln Gln Gly Pro Gly Gln Ser Gly
            35              40              45

Gln Tyr Gly Pro Gly Gln Gln Gly Pro Gly Gln Gln Gly Pro Gly Ser
    50              55              60

Ser Ala Ala Ala Ala Ala Ala Ala Gly Pro Gly Gln Tyr Val Leu Ile
65              70              75              80

Gly Pro Gly Gln Gln Val Leu Ile Gly Pro Ser Ala Ser Ala Ala Ala
            85              90              95

Ala Ala Ala Ala Gly Pro Gly Ser Gly Gln Gln Gly Pro Gly Ala Ser
        100             105             110

Gly Gln Tyr Gly Pro Gly Gln Gln Gly Pro Gly Gln Gln Gly Pro Gly
        115             120             125

Ser Ser Ala Ala Ala Ala Ala Ala Ala Gly Ser Tyr Gly Ser Val Leu
    130             135             140

Ile Gly Pro Gly Gln Gln Val Leu Ile Gly Pro Tyr Gly Ser Ala Ala
145             150             155             160

Ala Ala Ala Ala Ala Gly Pro Gly Ser Gly Gln Tyr Gly Gln Gly Pro
            165             170             175

Tyr Gly Pro Gly Ala Ser Gly Pro Gly Gln Tyr Gly Pro Gly Gln Gln
        180             185             190

Gly Pro Ser Ala Ser Ala Ala Ala Ala Ala Ala Ala Gly Ser Gly Gln
        195             200             205

Gln Val Leu Ile Gly Pro Gly Gln Tyr Val Leu Ile Gly Pro Tyr Ala
    210             215             220

Ser Ala Ala Ala Ala Ala Ala Ala Gly Ser Tyr Gly Ser Gly Pro Gly
225             230             235             240

Gln Gln Gly Pro Tyr Gly Pro Gly Gln Ser Gly Ser Gly Gln Gln Gly
            245             250             255

Pro Gly Gln Gln Gly Pro Tyr Ala Ser Ala Ala Ala Ala Ala Ala Ala
        260             265             270

Gly Pro Gly Gln Gln Val Leu Ile Gly Pro Tyr Val Leu Ile Gly Pro
        275             280             285

Gly Ser Ser Ala Ala Ala Ala Ala Ala Ala Gly Ser Tyr Gly Tyr Gly
        290             295             300

Pro Gly Gln Gln Gly Pro Tyr Gly Pro Gly Ala Ser Gly Gln Asn Gly
305                     310                 315                 320

Pro Gly Ser Gly Gln Tyr Gly Pro Gly Gln Gln Gly Pro Gly Pro Ser
                    325                 330                 335

Ala Ala Ala Ala Ala Ala Ala Gly Pro Gly Gln Gln Val Leu Ile Gly
                340                 345                 350

Pro Tyr Val Leu Ile Gly Pro Gly Ala Ser Ala Ala Ala Ala Ala Ala
        355                 360                 365

Ala Gly Ser Tyr Gly Pro Gly Gln Gln Gly Pro Gly Gln Tyr Gly Pro
    370                 375                 380

Gly Ser Ser Gly Pro Gly Gln Gln Gly Pro Tyr Gly Pro Gly Ser Ser
385                 390                 395                 400

Ala Ala Ala Ala Ala Ala Ala Gly Ser Tyr Gly Pro Gly Gln Gln Val
                405                 410                 415

Leu Ile Gly Pro Tyr Val Leu Ile Gly Pro Gly Pro Ser Ala Ala Ala
            420                 425                 430

Ala Ala Ala Ala Gly Ser Tyr Gln Gln Gly Pro Gly Gln Gln Gly Pro
    435                 440                 445

Tyr Gly Pro Gly Ala Ser Gly Pro Gly Gln Gln Gly Pro Tyr Gly Pro
    450                 455                 460

Gly Ala Ser Ala Ala Ala Ala Ala Ala Ala Gly Pro Gly Gln Tyr Val
465                 470                 475                 480

Leu Ile Gly Pro Gly Gln Gln Val Leu Ile Gly Pro Ser Ala Ser Ala
            485                 490                 495

Ala Ala Ala Ala Ala Gly Ser Tyr Gly Ser Gly Pro Gly Gln Tyr
            500                 505                 510

Gly Pro Tyr Gly Pro Gly Gln Ser Gly Pro Gly Ser Gly Gln Gln Gly
    515                 520                 525

Gln Gly Pro Tyr Gly Pro Gly Ala Ser Ala Ala Ala Ala Ala Ala Ala
    530                 535                 540

Gly Ser Tyr Gly Pro Gly Gln Gln Val Leu Ile Gly Pro Tyr Val Leu

545     550     555     560

```
Ile Gly Pro Gly Pro Ser Ala Ala Ala Ala Ala Ala Gly Pro Gly
                565                 570                 575

Ser Gly Gln Tyr Gly Pro Gly Ala Ser Gly Gln Asn Gly Pro Gly Ser
            580                 585                 590

Gly Gln Tyr Gly Pro Gly Gln Gln Gly Pro Gly Pro Ser Ala Ala Ala
            595                 600                 605

Ala Ala Ala Ala Gly Pro Gly Ser Gly Gln Gln Val Leu Ile Gly Pro
        610                 615                 620

Gly Ala Ser Val Leu Ile
625                 630
```

<210> 25
<211> 593
<212> PRT
<213> Artificial Sequence

<220>
<223> Met-PRT888

<400> 25

```
Met Gly Ser Ser Gly Pro Gly Val Leu Gly Pro Tyr Gly Pro Gly Ala
1               5                   10                  15

Ser Ala Ala Ala Ala Ala Gly Gln Asn Gly Pro Gly Ser Gly Val Leu
            20                  25                  30

Gly Pro Gly Gln Ser Gly Gln Tyr Gly Pro Gly Val Leu Gly Pro Gly
        35                  40                  45

Val Leu Gly Pro Gly Ser Ser Ala Ala Ala Ala Ala Gly Pro Gly Gln
        50                  55                  60

Tyr Gly Pro Gly Val Leu Gly Pro Ser Ala Ser Ala Ala Ala Ala Ala
65                  70                  75                  80

Gly Pro Gly Ser Gly Val Leu Gly Pro Gly Ala Ser Gly Gln Tyr Gly
                85                  90                  95

Pro Gly Val Leu Gly Pro Gly Val Leu Gly Pro Gly Ser Ser Ala Ala
            100                 105                 110

Ala Ala Ala Gly Gln Tyr Gly Ser Gly Pro Gly Val Leu Gly Pro Tyr
        115                 120                 125
```

108

Gly Ser Ala Ala Ala Ala Ala Gly Pro Gly Ser Gly Gln Tyr Gly Gln
130             135             140

Gly Pro Tyr Gly Pro Gly Ala Ser Gly Pro Gly Gln Tyr Gly Pro Gly
145             150             155             160

Val Leu Gly Pro Ser Ala Ser Ala Ala Ala Ala Ala Gly Ser Gly Val
165             170             175

Leu Gly Pro Gly Gln Tyr Gly Pro Tyr Ala Ser Ala Ala Ala Ala Ala
180             185             190

Gly Gln Tyr Gly Ser Gly Pro Gly Val Leu Gly Pro Tyr Gly Pro Gly
195             200             205

Gln Ser Gly Ser Gly Val Leu Gly Pro Gly Val Leu Gly Pro Tyr Ala
210             215             220

Ser Ala Ala Ala Ala Ala Gly Pro Gly Val Leu Gly Pro Tyr Gly Pro
225             230             235             240

Gly Ser Ser Ala Ala Ala Ala Ala Gly Gln Tyr Gly Tyr Gly Pro Gly
245             250             255

Val Leu Gly Pro Tyr Gly Pro Gly Ala Ser Gly Gln Asn Gly Pro Gly
260             265             270

Ser Gly Gln Tyr Gly Pro Gly Val Leu Gly Pro Gly Gln Ser Ala Ala
275             280             285

Ala Ala Ala Gly Pro Gly Val Leu Gly Pro Tyr Gly Pro Gly Ala Ser
290             295             300

Ala Ala Ala Ala Ala Gly Gln Tyr Gly Pro Gly Val Leu Gly Pro Gly
305             310             315             320

Gln Tyr Gly Pro Gly Ser Ser Gly Pro Gly Val Leu Gly Pro Tyr Gly
325             330             335

Pro Gly Ser Ser Ala Ala Ala Ala Ala Gly Gln Tyr Gly Pro Gly Val
340             345             350

Leu Gly Pro Tyr Gly Pro Gly Gln Ser Ala Ala Ala Ala Ala Gly Gln
355             360             365

Tyr Val Leu Gly Pro Gly Val Leu Gly Pro Tyr Gly Pro Gly Ala Ser

370 375 380

Gly Pro Gly Val Leu Gly Pro Tyr Gly Pro Gly Ala Ser Ala Ala Ala
385 390 395 400

Ala Ala Gly Pro Gly Gln Tyr Gly Pro Gly Val Leu Gly Pro Ser Ala
405 410 415

Ser Ala Ala Ala Ala Ala Gly Gln Tyr Gly Ser Gly Pro Gly Gln Tyr
420 425 430

Gly Pro Tyr Gly Pro Gly Gln Ser Gly Pro Gly Ser Gly Val Leu Gly
435 440 445

Gln Gly Pro Tyr Gly Pro Gly Ala Ser Ala Ala Ala Ala Ala Gly Gln
450 455 460

Tyr Gly Pro Gly Val Leu Gly Pro Tyr Gly Pro Gly Gln Ser Ala Ala
465 470 475 480

Ala Ala Ala Gly Pro Gly Ser Gly Gln Tyr Gly Pro Gly Ala Ser Gly
485 490 495

Gln Asn Gly Pro Gly Ser Gly Gln Tyr Gly Pro Gly Val Leu Gly Pro
500 505 510

Gly Gln Ser Ala Ala Ala Ala Ala Gly Gln Tyr Val Leu Gly Pro Gly
515 520 525

Val Leu Gly Pro Tyr Gly Pro Gly Ala Ser Ala Ala Ala Ala Ala Gly
530 535 540

Gln Tyr Gly Ser Gly Pro Gly Val Leu Gly Pro Tyr Gly Pro Gly Gln
545 550 555 560

Ser Gly Ser Gly Val Leu Gly Pro Gly Val Leu Gly Pro Tyr Ala Ser
565 570 575

Ala Ala Ala Ala Ala Gly Pro Gly Ser Gly Val Leu Gly Pro Gly Ala
580 585 590

Ser

<210> 26
<211> 590
<212> PRT
<213> Artificial Sequence

<220>
<223>   Met-PRT965

<400>   26

Met Gly Pro Gly Thr Ser Gly Pro Tyr Gly Pro Gly Ala Ser Ala Ala
1               5               10              15

Ala Ala Ala Gly Ala Asn Gly Pro Gly Ser Gly Thr Ser Gly Pro Gly
            20              25              30

Ala Ser Gly Ala Tyr Gly Pro Gly Thr Ser Gly Pro Gly Thr Ser Gly
        35              40              45

Pro Gly Ser Ser Ala Ala Ala Ala Ala Gly Pro Gly Ala Tyr Gly Pro
        50              55              60

Gly Thr Ser Gly Pro Ser Ala Ser Ala Ala Ala Ala Gly Pro Gly
65              70              75              80

Ser Gly Thr Ser Gly Pro Gly Ala Ser Gly Ala Tyr Gly Pro Gly Thr
                85              90              95

Ser Gly Pro Gly Thr Ser Gly Pro Gly Ser Ser Ala Ala Ala Ala Ala
            100             105             110

Gly Ala Tyr Gly Ser Gly Pro Gly Thr Ser Gly Pro Tyr Gly Ser Ala
        115             120             125

Ala Ala Ala Ala Gly Pro Gly Ser Gly Ala Tyr Gly Ala Gly Pro Tyr
    130             135             140

Gly Pro Gly Ala Ser Gly Pro Gly Ala Tyr Gly Pro Gly Thr Ser Gly
145             150             155             160

Pro Ser Ala Ser Ala Ala Ala Ala Ala Gly Ser Gly Thr Ser Gly Pro
            165             170             175

Gly Ala Tyr Gly Pro Tyr Ala Ser Ala Ala Ala Ala Gly Ala Tyr
        180             185             190

Gly Ser Gly Pro Gly Thr Ser Gly Pro Tyr Gly Pro Gly Ala Ser Gly
        195             200             205

Ser Gly Thr Ser Gly Pro Gly Thr Ser Gly Pro Tyr Ala Ser Ala Ala
    210             215             220

Ala Ala Ala Gly Pro Gly Thr Ser Gly Pro Tyr Gly Pro Gly Ser Ser

```
            225                    230                    235                    240


            Ala Ala Ala Ala Ala Gly Ala Tyr Gly Tyr Gly Pro Gly Thr Ser Gly
                        245                    250                    255


            Pro Tyr Gly Pro Gly Ala Ser Gly Ala Asn Gly Pro Gly Ser Gly Ala
                        260                    265                    270


            Tyr Gly Pro Gly Thr Ser Gly Pro Gly Ala Ser Ala Ala Ala Ala Ala
                        275                    280                    285


            Gly Pro Gly Thr Ser Gly Pro Tyr Gly Pro Gly Ala Ser Ala Ala Ala
                        290                    295                    300


            Ala Ala Gly Ala Tyr Gly Pro Gly Thr Ser Gly Pro Gly Ala Tyr Gly
            305                    310                    315                    320


            Pro Gly Ser Ser Gly Pro Gly Thr Ser Gly Pro Tyr Gly Pro Gly Ser
                        325                    330                    335


            Ser Ala Ala Ala Ala Ala Gly Ala Tyr Gly Pro Gly Thr Ser Gly Pro
                        340                    345                    350


            Tyr Gly Pro Gly Ala Ser Ala Ala Ala Ala Gly Ala Tyr Thr Ser
                        355                    360                    365


            Gly Pro Gly Thr Ser Gly Pro Tyr Gly Pro Gly Ala Ser Gly Pro Gly
                        370                    375                    380


            Thr Ser Gly Pro Tyr Gly Pro Gly Ala Ser Ala Ala Ala Ala Ala Gly
            385                    390                    395                    400


            Pro Gly Ala Tyr Gly Pro Gly Thr Ser Gly Pro Ser Ala Ser Ala Ala
                        405                    410                    415


            Ala Ala Ala Gly Ala Tyr Gly Ser Gly Pro Gly Ala Tyr Gly Pro Tyr
                        420                    425                    430


            Gly Pro Gly Ala Ser Gly Pro Gly Ser Gly Thr Ser Gly Ala Gly Pro
                        435                    440                    445


            Tyr Gly Pro Gly Ala Ser Ala Ala Ala Ala Gly Ala Tyr Gly Pro
                        450                    455                    460


            Gly Thr Ser Gly Pro Tyr Gly Pro Gly Ala Ser Ala Ala Ala Ala Ala
            465                    470                    475                    480
```

```
Gly Pro Gly Ser Gly Ala Tyr Gly Pro Gly Ala Ser Gly Ala Asn Gly
            485                 490                 495

Pro Gly Ser Gly Ala Tyr Gly Pro Gly Thr Ser Gly Pro Gly Ala Ser
            500                 505                 510

Ala Ala Ala Ala Ala Gly Ala Tyr Thr Ser Gly Pro Gly Thr Ser Gly
        515                 520                 525

Pro Tyr Gly Pro Gly Ala Ser Ala Ala Ala Ala Ala Gly Ala Tyr Gly
    530                 535                 540

Ser Gly Pro Gly Thr Ser Gly Pro Tyr Gly Pro Gly Ala Ser Gly Ser
545                 550                 555                 560

Gly Thr Ser Gly Pro Gly Thr Ser Gly Pro Tyr Ala Ser Ala Ala Ala
            565                 570                 575

Ala Ala Gly Pro Gly Ser Gly Thr Ser Gly Pro Gly Ala Ser
        580                 585                 590
```

<210> 27
<211> 593
<212> PRT
<213> Artificial Sequence

<220>
<223> Met-PRT889

<400> 27

```
Met Gly Ser Ser Gly Pro Gly Val Leu Gly Pro Tyr Gly Pro Gly Ala
1               5                   10                  15

Ser Ala Ala Ala Ala Ala Gly Ile Asn Gly Pro Gly Ser Gly Val Leu
            20                  25                  30

Gly Pro Gly Ile Ser Gly Ile Tyr Gly Pro Gly Val Leu Gly Pro Gly
            35                  40                  45

Val Leu Gly Pro Gly Ser Ser Ala Ala Ala Ala Ala Gly Pro Gly Ile
        50                  55                  60

Tyr Gly Pro Gly Val Leu Gly Pro Ser Ala Ser Ala Ala Ala Ala Ala
65                  70                  75                  80

Gly Pro Gly Ser Gly Val Leu Gly Pro Gly Ala Ser Gly Ile Tyr Gly
            85                  90                  95

Pro Gly Val Leu Gly Pro Gly Val Leu Gly Pro Gly Ser Ser Ala Ala
```

```
                    100                        105                          110


        Ala Ala Ala Gly Ile Tyr Gly Ser Gly Pro Gly Val Leu Gly Pro Tyr
                115                    120                    125


        Gly Ser Ala Ala Ala Ala Ala Gly Pro Gly Ser Gly Ile Tyr Gly Ile
                130                    135                    140


        Gly Pro Tyr Gly Pro Gly Ala Ser Gly Pro Gly Ile Tyr Gly Pro Gly
        145                    150                    155                    160


        Val Leu Gly Pro Ser Ala Ser Ala Ala Ala Ala Ala Gly Ser Gly Val
                            165                    170                    175


        Leu Gly Pro Gly Ile Tyr Gly Pro Tyr Ala Ser Ala Ala Ala Ala Ala
                        180                    185                    190


        Gly Ile Tyr Gly Ser Gly Pro Gly Val Leu Gly Pro Tyr Gly Pro Gly
                195                    200                    205


        Ile Ser Gly Ser Gly Val Leu Gly Pro Gly Val Leu Gly Pro Tyr Ala
                210                    215                    220


        Ser Ala Ala Ala Ala Ala Gly Pro Gly Val Leu Gly Pro Tyr Gly Pro
        225                    230                    235                    240


        Gly Ser Ser Ala Ala Ala Ala Ala Gly Ile Tyr Gly Tyr Gly Pro Gly
                        245                    250                    255


        Val Leu Gly Pro Tyr Gly Pro Gly Ala Ser Gly Ile Asn Gly Pro Gly
                        260                    265                    270


        Ser Gly Ile Tyr Gly Pro Gly Val Leu Gly Pro Gly Ile Ser Ala Ala
                275                    280                    285


        Ala Ala Ala Gly Pro Gly Val Leu Gly Pro Tyr Gly Pro Gly Ala Ser
                290                    295                    300


        Ala Ala Ala Ala Ala Gly Ile Tyr Gly Pro Gly Val Leu Gly Pro Gly
        305                    310                    315                    320


        Ile Tyr Gly Pro Gly Ser Ser Gly Pro Gly Val Leu Gly Pro Tyr Gly
                        325                    330                    335


        Pro Gly Ser Ser Ala Ala Ala Ala Ala Gly Ile Tyr Gly Pro Gly Val
                    340                    345                    350
```

114

Leu Gly Pro Tyr Gly Pro Gly Ile Ser Ala Ala Ala Ala Ala Gly Ile
355 360 365

Tyr Val Leu Gly Pro Gly Val Leu Gly Pro Tyr Gly Pro Gly Ala Ser
370 375 380

Gly Pro Gly Val Leu Gly Pro Tyr Gly Pro Gly Ala Ser Ala Ala Ala
385 390 395 400

Ala Ala Gly Pro Gly Ile Tyr Gly Pro Gly Val Leu Gly Pro Ser Ala
405 410 415

Ser Ala Ala Ala Ala Ala Gly Ile Tyr Gly Ser Gly Pro Gly Ile Tyr
420 425 430

Gly Pro Tyr Gly Pro Gly Ile Ser Gly Pro Gly Ser Gly Val Leu Gly
435 440 445

Ile Gly Pro Tyr Gly Pro Gly Ala Ser Ala Ala Ala Ala Ala Gly Ile
450 455 460

Tyr Gly Pro Gly Val Leu Gly Pro Tyr Gly Pro Gly Ile Ser Ala Ala
465 470 475 480

Ala Ala Ala Gly Pro Gly Ser Gly Ile Tyr Gly Pro Gly Ala Ser Gly
485 490 495

Ile Asn Gly Pro Gly Ser Gly Ile Tyr Gly Pro Gly Val Leu Gly Pro
500 505 510

Gly Ile Ser Ala Ala Ala Ala Ala Gly Ile Tyr Val Leu Gly Pro Gly
515 520 525

Val Leu Gly Pro Tyr Gly Pro Gly Ala Ser Ala Ala Ala Ala Ala Gly
530 535 540

Ile Tyr Gly Ser Gly Pro Gly Val Leu Gly Pro Tyr Gly Pro Gly Ile
545 550 555 560

Ser Gly Ser Gly Val Leu Gly Pro Gly Val Leu Gly Pro Tyr Ala Ser
565 570 575

Ala Ala Ala Ala Ala Gly Pro Gly Ser Gly Val Leu Gly Pro Gly Ala
580 585 590

Ser

<210> 28
<211> 590
<212> PRT
<213> Artificial Sequence

<220>
<223> Met-PRT916

<400> 28

Met Gly Pro Gly Val Ile Gly Pro Tyr Gly Pro Gly Ala Ser Ala Ala
1               5                   10                  15

Ala Ala Ala Gly Leu Asn Gly Pro Gly Ser Gly Val Ile Gly Pro Gly
                20                  25                  30

Leu Ser Gly Leu Tyr Gly Pro Gly Val Ile Gly Pro Gly Val Ile Gly
            35                  40                  45

Pro Gly Ser Ser Ala Ala Ala Ala Ala Gly Pro Gly Leu Tyr Gly Pro
        50                  55                  60

Gly Val Ile Gly Pro Ser Ala Ser Ala Ala Ala Ala Gly Pro Gly
65                  70                  75                  80

Ser Gly Val Ile Gly Pro Gly Ala Ser Gly Leu Tyr Gly Pro Gly Val
                85                  90                  95

Ile Gly Pro Gly Val Ile Gly Pro Gly Ser Ser Ala Ala Ala Ala Ala
            100                 105                 110

Gly Leu Tyr Gly Ser Gly Pro Gly Val Ile Gly Pro Tyr Gly Ser Ala
        115                 120                 125

Ala Ala Ala Ala Gly Pro Gly Ser Gly Leu Tyr Gly Leu Gly Pro Tyr
    130                 135                 140

Gly Pro Gly Ala Ser Gly Pro Gly Leu Tyr Gly Pro Gly Val Ile Gly
145                 150                 155                 160

Pro Ser Ala Ser Ala Ala Ala Ala Ala Gly Ser Gly Val Ile Gly Pro
                165                 170                 175

Gly Leu Tyr Gly Pro Tyr Ala Ser Ala Ala Ala Ala Gly Leu Tyr
        180                 185                 190

Gly Ser Gly Pro Gly Val Ile Gly Pro Tyr Gly Pro Gly Leu Ser Gly
        195                 200                 205

Ser Gly Val Ile Gly Pro Gly Val Ile Gly Pro Tyr Ala Ser Ala Ala
210 215 220

Ala Ala Ala Gly Pro Gly Val Ile Gly Pro Tyr Gly Pro Gly Ser Ser
225 230 235 240

Ala Ala Ala Ala Ala Gly Leu Tyr Gly Tyr Gly Pro Gly Val Ile Gly
245 250 255

Pro Tyr Gly Pro Gly Ala Ser Gly Leu Asn Gly Pro Gly Ser Gly Leu
260 265 270

Tyr Gly Pro Gly Val Ile Gly Pro Gly Leu Ser Ala Ala Ala Ala Ala
275 280 285

Gly Pro Gly Val Ile Gly Pro Tyr Gly Pro Gly Ala Ser Ala Ala Ala
290 295 300

Ala Ala Gly Leu Tyr Gly Pro Gly Val Ile Gly Pro Gly Leu Tyr Gly
305 310 315 320

Pro Gly Ser Ser Gly Pro Gly Val Ile Gly Pro Tyr Gly Pro Gly Ser
325 330 335

Ser Ala Ala Ala Ala Ala Gly Leu Tyr Gly Pro Gly Val Ile Gly Pro
340 345 350

Tyr Gly Pro Gly Leu Ser Ala Ala Ala Ala Ala Gly Leu Tyr Val Ile
355 360 365

Gly Pro Gly Val Ile Gly Pro Tyr Gly Pro Gly Ala Ser Gly Pro Gly
370 375 380

Val Ile Gly Pro Tyr Gly Pro Gly Ala Ser Ala Ala Ala Ala Ala Gly
385 390 395 400

Pro Gly Leu Tyr Gly Pro Gly Val Ile Gly Pro Ser Ala Ser Ala Ala
405 410 415

Ala Ala Ala Gly Leu Tyr Gly Ser Gly Pro Gly Leu Tyr Gly Pro Tyr
420 425 430

Gly Pro Gly Leu Ser Gly Pro Gly Ser Gly Val Ile Gly Leu Gly Pro
435 440 445

Tyr Gly Pro Gly Ala Ser Ala Ala Ala Ala Ala Gly Leu Tyr Gly Pro
450 455 460

```
Gly Val Ile Gly Pro Tyr Gly Pro Gly Leu Ser Ala Ala Ala Ala Ala
465             470             475                         480


Gly Pro Gly Ser Gly Leu Tyr Gly Pro Gly Ala Ser Gly Leu Asn Gly
                485             490                     495


Pro Gly Ser Gly Leu Tyr Gly Pro Gly Val Ile Gly Pro Gly Leu Ser
            500             505             510


Ala Ala Ala Ala Ala Gly Leu Tyr Val Ile Gly Pro Gly Val Ile Gly
        515             520             525


Pro Tyr Gly Pro Gly Ala Ser Ala Ala Ala Ala Gly Leu Tyr Gly
    530             535             540


Ser Gly Pro Gly Val Ile Gly Pro Tyr Gly Pro Gly Leu Ser Gly Ser
545             550             555                         560


Gly Val Ile Gly Pro Gly Val Ile Gly Pro Tyr Ala Ser Ala Ala Ala
            565             570                     575


Ala Ala Gly Pro Gly Ser Gly Val Ile Gly Pro Gly Ala Ser
        580             585             590
```

```
<210>  29
<211>  590
<212>  PRT
<213>  Artificial Sequence

<220>
<223>  Met-PRT918

<400>  29
```

```
Met Gly Pro Gly Val Phe Gly Pro Tyr Gly Pro Gly Ala Ser Ala Ala
1               5               10              15


Ala Ala Ala Gly Ile Asn Gly Pro Gly Ser Gly Val Phe Gly Pro Gly
            20              25              30


Ile Ser Gly Ile Tyr Gly Pro Gly Val Phe Gly Pro Gly Val Phe Gly
        35              40              45


Pro Gly Ser Ser Ala Ala Ala Ala Ala Gly Pro Gly Ile Tyr Gly Pro
    50              55              60


Gly Val Phe Gly Pro Ser Ala Ser Ala Ala Ala Ala Ala Gly Pro Gly
65              70              75              80
```

```
Ser Gly Val Phe Gly Pro Gly Ala Ser Gly Ile Tyr Gly Pro Gly Val
            85                  90                  95

Phe Gly Pro Gly Val Phe Gly Pro Gly Ser Ser Ala Ala Ala Ala Ala
            100                 105                 110

Gly Ile Tyr Gly Ser Gly Pro Gly Val Phe Gly Pro Tyr Gly Ser Ala
            115                 120                 125

Ala Ala Ala Ala Gly Pro Gly Ser Gly Ile Tyr Gly Ile Gly Pro Tyr
    130                 135                 140

Gly Pro Gly Ala Ser Gly Pro Gly Ile Tyr Gly Pro Gly Val Phe Gly
145                 150                 155                 160

Pro Ser Ala Ser Ala Ala Ala Ala Ala Gly Ser Gly Val Phe Gly Pro
                165                 170                 175

Gly Ile Tyr Gly Pro Tyr Ala Ser Ala Ala Ala Ala Gly Ile Tyr
            180                 185                 190

Gly Ser Gly Pro Gly Val Phe Gly Pro Tyr Gly Pro Gly Ile Ser Gly
            195                 200                 205

Ser Gly Val Phe Gly Pro Gly Val Phe Gly Pro Tyr Ala Ser Ala Ala
    210                 215                 220

Ala Ala Ala Gly Pro Gly Val Phe Gly Pro Tyr Gly Pro Gly Ser Ser
225                 230                 235                 240

Ala Ala Ala Ala Ala Gly Ile Tyr Gly Tyr Gly Pro Gly Val Phe Gly
                245                 250                 255

Pro Tyr Gly Pro Gly Ala Ser Gly Ile Asn Gly Pro Gly Ser Gly Ile
            260                 265                 270

Tyr Gly Pro Gly Val Phe Gly Pro Gly Ile Ser Ala Ala Ala Ala Ala
            275                 280                 285

Gly Pro Gly Val Phe Gly Pro Tyr Gly Pro Gly Ala Ser Ala Ala Ala
    290                 295                 300

Ala Ala Gly Ile Tyr Gly Pro Gly Val Phe Gly Pro Gly Ile Tyr Gly
305                 310                 315                 320

Pro Gly Ser Ser Gly Pro Gly Val Phe Gly Pro Tyr Gly Pro Gly Ser
            325                 330                 335
```

119

```
Ser Ala Ala Ala Ala Ala Gly Ile Tyr Gly Pro Gly Val Phe Gly Pro
        340                 345             350

Tyr Gly Pro Gly Ile Ser Ala Ala Ala Ala Ala Gly Ile Tyr Val Phe
        355                 360             365

Gly Pro Gly Val Phe Gly Pro Tyr Gly Pro Gly Ala Ser Gly Pro Gly
    370                 375             380

Val Phe Gly Pro Tyr Gly Pro Gly Ala Ser Ala Ala Ala Ala Ala Gly
385                 390             395                 400

Pro Gly Ile Tyr Gly Pro Gly Val Phe Gly Pro Ser Ala Ser Ala Ala
            405                 410             415

Ala Ala Ala Gly Ile Tyr Gly Ser Gly Pro Gly Ile Tyr Gly Pro Tyr
        420                 425             430

Gly Pro Gly Ile Ser Gly Pro Gly Ser Gly Val Phe Gly Ile Gly Pro
    435                 440             445

Tyr Gly Pro Gly Ala Ser Ala Ala Ala Ala Gly Ile Tyr Gly Pro
    450                 455             460

Gly Val Phe Gly Pro Tyr Gly Pro Gly Ile Ser Ala Ala Ala Ala Ala
465                 470                 475                 480

Gly Pro Gly Ser Gly Ile Tyr Gly Pro Gly Ala Ser Gly Ile Asn Gly
            485                 490                 495

Pro Gly Ser Gly Ile Tyr Gly Pro Gly Val Phe Gly Pro Gly Ile Ser
        500                 505                 510

Ala Ala Ala Ala Ala Gly Ile Tyr Val Phe Gly Pro Gly Val Phe Gly
        515                 520                 525

Pro Tyr Gly Pro Gly Ala Ser Ala Ala Ala Ala Gly Ile Tyr Gly
    530                 535                 540

Ser Gly Pro Gly Val Phe Gly Pro Tyr Gly Pro Gly Ile Ser Gly Ser
545                 550                 555                 560

Gly Val Phe Gly Pro Gly Val Phe Gly Pro Tyr Ala Ser Ala Ala Ala
            565                 570                 575

Ala Ala Gly Pro Gly Ser Gly Val Phe Gly Pro Gly Ala Ser
        580                 585                 590
```

```
<210>   30
<211>   565
<212>   PRT
<213>   Artificial Sequence

<220>
<223>   Met-PRT699

<400>   30

Met Gly Pro Gly Val Leu Gly Pro Tyr Gly Pro Gly Ala Ser Ala Ala
1               5                   10                  15


Ala Ala Ala Ala Ala Gly Ser Asn Gly Pro Gly Ser Gly Val Leu Gly
                20                  25                  30


Pro Gly Gln Ser Gly Gln Tyr Gly Pro Gly Val Leu Gly Pro Gly Val
            35                  40                  45


Leu Gly Pro Gly Ser Ser Ala Ala Ala Ala Ala Ala Gly Pro Gly
        50                  55                  60


Gln Tyr Gly Pro Gly Val Leu Gly Pro Ser Ala Ser Ala Ala Ala Ala
65                  70                  75                  80


Ala Ala Ala Gly Pro Gly Ser Gly Val Leu Gly Pro Gly Ala Ser Gly
                85                  90                  95


Gln Tyr Gly Pro Gly Val Leu Gly Pro Gly Val Leu Gly Pro Gly Ser
            100                 105                 110


Ser Ala Ala Ala Ala Ala Ala Ala Gly Ser Tyr Gly Ser Gly Pro Gly
            115                 120                 125


Val Leu Gly Pro Tyr Gly Ser Ala Ala Ala Ala Ala Ala Gly Pro
        130                 135                 140


Gly Ser Gly Gln Tyr Gly Gln Gly Pro Tyr Gly Pro Gly Ala Ser Gly
145                 150                 155                 160


Pro Gly Gln Tyr Gly Pro Gly Val Leu Gly Pro Ser Ala Ser Ala Ala
            165                 170                 175


Ala Ala Ala Ala Ala Gly Ser Gly Val Leu Gly Pro Gly Gln Tyr Gly
            180                 185                 190


Pro Tyr Ala Ser Ala Ala Ala Ala Ala Ala Ala Gly Ser Tyr Gly Ser
        195                 200                 205
```

Gly Pro Gly Val Leu Gly Pro Tyr Gly Pro Gly Gln Ser Gly Ser Gly
    210                 215                 220

Val Leu Gly Pro Gly Val Leu Gly Pro Tyr Ala Ser Ala Ala Ala Ala
    225                 230                 235                 240

Ala Ala Ala Gly Pro Gly Val Leu Gly Pro Tyr Gly Pro Gly Ser Ser
            245                 250                 255

Ala Ala Ala Ala Ala Ala Ala Gly Ser Tyr Gly Tyr Gly Pro Gly Val
            260                 265                 270

Leu Gly Pro Tyr Gly Pro Gly Ala Ser Gly Gln Asn Gly Pro Gly Ser
        275                 280                 285

Gly Gln Tyr Gly Pro Gly Val Leu Gly Pro Gly Pro Ser Ala Ala Ala
    290                 295                 300

Ala Ala Ala Ala Gly Pro Gly Val Leu Gly Pro Tyr Gly Pro Gly Ala
305                 310                 315                 320

Ser Ala Ala Ala Ala Ala Ala Ala Gly Ser Tyr Gly Pro Gly Val Leu
            325                 330                 335

Gly Pro Gly Gln Tyr Gly Pro Gly Ser Ser Gly Pro Gly Val Leu Gly
        340                 345                 350

Pro Tyr Gly Pro Gly Ser Ser Ala Ala Ala Ala Ala Ala Ala Gly Ser
        355                 360                 365

Tyr Gly Pro Gly Val Leu Gly Pro Tyr Gly Pro Gly Pro Ser Ala Ala
    370                 375                 380

Ala Ala Ala Ala Ala Gly Ser Tyr Val Leu Gly Pro Gly Val Leu Gly
385                 390                 395                 400

Pro Tyr Gly Pro Gly Ala Ser Gly Pro Gly Val Leu Gly Pro Tyr Gly
        405                 410                 415

Pro Gly Ala Ser Ala Ala Ala Ala Ala Ala Ala Gly Pro Gly Gln Tyr
        420                 425                 430

Gly Pro Gly Val Leu Gly Pro Ser Ala Ser Ala Ala Ala Ala Ala Ala
        435                 440                 445

Ala Gly Ser Tyr Gly Ser Gly Pro Gly Gln Tyr Gly Pro Tyr Gly Pro
    450                 455                 460

```
Gly Gln Ser Gly Pro Gly Ser Gly Val Leu Gly Gln Gly Pro Tyr Gly
465             470             475             480

Pro Gly Ala Ser Ala Ala Ala Ala Ala Ala Ala Gly Ser Tyr Gly Pro
            485             490             495

Gly Val Leu Gly Pro Tyr Gly Pro Gly Pro Ser Ala Ala Ala Ala Ala
            500             505             510

Ala Ala Gly Pro Gly Ser Gly Gln Tyr Gly Pro Gly Ala Ser Gly Gln
            515             520             525

Asn Gly Pro Gly Ser Gly Gln Tyr Gly Pro Gly Val Leu Gly Pro Gly
    530             535             540

Pro Ser Ala Ala Ala Ala Ala Ala Ala Gly Pro Gly Ser Gly Val Leu
545             550             555             560

Gly Pro Gly Ala Ser
                565


<210>  31
<211>  565
<212>  PRT
<213>  Artificial Sequence

<220>
<223>  Met-PRT698

<400>  31

Met Gly Pro Gly Val Leu Gly Pro Tyr Gly Pro Gly Ala Ser Ala Ala
1               5               10              15

Ala Ala Ala Ala Ala Gly Ser Asn Gly Pro Gly Ser Gly Val Leu Gly
            20              25              30

Pro Gly Ile Ser Gly Ile Tyr Gly Pro Gly Val Leu Gly Pro Gly Val
            35              40              45

Leu Gly Pro Gly Ser Ser Ala Ala Ala Ala Ala Ala Gly Pro Gly
    50              55              60

Ile Tyr Gly Pro Gly Val Leu Gly Pro Ser Ala Ser Ala Ala Ala Ala
65              70              75              80

Ala Ala Ala Gly Pro Gly Ser Gly Val Leu Gly Pro Gly Ala Ser Gly
            85              90              95
```

```
Ile Tyr Gly Pro Gly Val Leu Gly Pro Gly Val Leu Gly Pro Gly Ser
            100               105               110

Ser Ala Ala Ala Ala Ala Ala Ala Gly Ser Tyr Gly Ser Gly Pro Gly
            115               120               125

Val Leu Gly Pro Tyr Gly Ser Ala Ala Ala Ala Ala Ala Ala Gly Pro
            130               135               140

Gly Ser Gly Ile Tyr Gly Ile Gly Pro Tyr Gly Pro Gly Ala Ser Gly
145               150               155               160

Pro Gly Ile Tyr Gly Pro Gly Val Leu Gly Pro Ser Ala Ser Ala Ala
            165               170               175

Ala Ala Ala Ala Ala Gly Ser Gly Val Leu Gly Pro Gly Ile Tyr Gly
            180               185               190

Pro Tyr Ala Ser Ala Ala Ala Ala Ala Ala Gly Ser Tyr Gly Ser
            195               200               205

Gly Pro Gly Val Leu Gly Pro Tyr Gly Pro Gly Ile Ser Gly Ser Gly
    210               215               220

Val Leu Gly Pro Gly Val Leu Gly Pro Tyr Ala Ser Ala Ala Ala Ala
225               230               235               240

Ala Ala Ala Gly Pro Gly Val Leu Gly Pro Tyr Gly Pro Gly Ser Ser
            245               250               255

Ala Ala Ala Ala Ala Ala Ala Gly Ser Tyr Gly Tyr Gly Pro Gly Val
            260               265               270

Leu Gly Pro Tyr Gly Pro Gly Ala Ser Gly Ile Asn Gly Pro Gly Ser
            275               280               285

Gly Ile Tyr Gly Pro Gly Val Leu Gly Pro Gly Pro Ser Ala Ala Ala
    290               295               300

Ala Ala Ala Ala Gly Pro Gly Val Leu Gly Pro Tyr Gly Pro Gly Ala
305               310               315               320

Ser Ala Ala Ala Ala Ala Ala Ala Gly Ser Tyr Gly Pro Gly Val Leu
            325               330               335

Gly Pro Gly Ile Tyr Gly Pro Gly Ser Ser Gly Pro Gly Val Leu Gly
            340               345               350
```

```
Pro Tyr Gly Pro Gly Ser Ser Ala Ala Ala Ala Ala Ala Gly Ser
    355                 360             365

Tyr Gly Pro Gly Val Leu Gly Pro Tyr Gly Pro Gly Pro Ser Ala Ala
    370             375             380

Ala Ala Ala Ala Ala Gly Ser Tyr Val Leu Gly Pro Gly Val Leu Gly
385                 390             395                 400

Pro Tyr Gly Pro Gly Ala Ser Gly Pro Gly Val Leu Gly Pro Tyr Gly
            405             410             415

Pro Gly Ala Ser Ala Ala Ala Ala Ala Ala Gly Pro Gly Ile Tyr
        420             425             430

Gly Pro Gly Val Leu Gly Pro Ser Ala Ser Ala Ala Ala Ala Ala Ala
        435             440             445

Ala Gly Ser Tyr Gly Ser Gly Pro Gly Ile Tyr Gly Pro Tyr Gly Pro
    450             455             460

Gly Ile Ser Gly Pro Gly Ser Gly Val Leu Gly Ile Gly Pro Tyr Gly
465             470             475                 480

Pro Gly Ala Ser Ala Ala Ala Ala Ala Ala Gly Ser Tyr Gly Pro
        485             490             495

Gly Val Leu Gly Pro Tyr Gly Pro Gly Pro Ser Ala Ala Ala Ala Ala
        500             505             510

Ala Ala Gly Pro Gly Ser Gly Ile Tyr Gly Pro Gly Ala Ser Gly Ile
        515             520             525

Asn Gly Pro Gly Ser Gly Ile Tyr Gly Pro Gly Val Leu Gly Pro Gly
    530             535             540

Pro Ser Ala Ala Ala Ala Ala Ala Ala Gly Pro Gly Ser Gly Val Leu
545             550             555                 560

Gly Pro Gly Ala Ser
            565
```

<210> 32
<211> 1179
<212> PRT
<213> Artificial Sequence

125

<220>
<223>   Met-PRT966

<400>   32

Met Gly Pro Gly Val Phe Gly Pro Tyr Gly Pro Gly Ala Ser Ala Ala
1               5                   10                  15

Ala Ala Ala Gly Ile Asn Gly Pro Gly Ser Gly Val Phe Gly Pro Gly
            20                  25                  30

Ile Ser Gly Ile Tyr Gly Pro Gly Val Phe Gly Pro Gly Val Phe Gly
        35                  40                  45

Pro Gly Ser Ser Ala Ala Ala Ala Ala Gly Pro Gly Ile Tyr Gly Pro
    50                  55                  60

Gly Val Phe Gly Pro Ser Ala Ser Ala Ala Ala Ala Gly Pro Gly
65                  70                  75                  80

Ser Gly Val Phe Gly Pro Gly Ala Ser Gly Ile Tyr Gly Pro Gly Val
            85                  90                  95

Phe Gly Pro Gly Val Phe Gly Pro Gly Ser Ser Ala Ala Ala Ala Ala
            100                 105                 110

Gly Ile Tyr Gly Ser Gly Pro Gly Val Phe Gly Pro Tyr Gly Ser Ala
        115                 120                 125

Ala Ala Ala Ala Gly Pro Gly Ser Gly Ile Tyr Gly Ile Gly Pro Tyr
    130                 135                 140

Gly Pro Gly Ala Ser Gly Pro Gly Ile Tyr Gly Pro Gly Val Phe Gly
145                 150                 155                 160

Pro Ser Ala Ser Ala Ala Ala Ala Ala Gly Ser Gly Val Phe Gly Pro
            165                 170                 175

Gly Ile Tyr Gly Pro Tyr Ala Ser Ala Ala Ala Ala Gly Ile Tyr
        180                 185                 190

Gly Ser Gly Pro Gly Val Phe Gly Pro Tyr Gly Pro Gly Ile Ser Gly
        195                 200                 205

Ser Gly Val Phe Gly Pro Gly Val Phe Gly Pro Tyr Ala Ser Ala Ala
        210                 215                 220

Ala Ala Ala Gly Pro Gly Val Phe Gly Pro Tyr Gly Pro Gly Ser Ser
225                 230                 235                 240

Ala Ala Ala Ala Ala Gly Ile Tyr Gly Tyr Gly Pro Gly Val Phe Gly
245 250 255

Pro Tyr Gly Pro Gly Ala Ser Gly Ile Asn Gly Pro Gly Ser Gly Ile
260 265 270

Tyr Gly Pro Gly Val Phe Gly Pro Gly Ile Ser Ala Ala Ala Ala Ala
275 280 285

Gly Pro Gly Val Phe Gly Pro Tyr Gly Pro Gly Ala Ser Ala Ala Ala
290 295 300

Ala Ala Gly Ile Tyr Gly Pro Gly Val Phe Gly Pro Gly Ile Tyr Gly
305 310 315 320

Pro Gly Ser Ser Gly Pro Gly Val Phe Gly Pro Tyr Gly Pro Gly Ser
325 330 335

Ser Ala Ala Ala Ala Ala Gly Ile Tyr Gly Pro Gly Val Phe Gly Pro
340 345 350

Tyr Gly Pro Gly Ile Ser Ala Ala Ala Ala Ala Gly Ile Tyr Val Phe
355 360 365

Gly Pro Gly Val Phe Gly Pro Tyr Gly Pro Gly Ala Ser Gly Pro Gly
370 375 380

Val Phe Gly Pro Tyr Gly Pro Gly Ala Ser Ala Ala Ala Ala Ala Gly
385 390 395 400

Pro Gly Ile Tyr Gly Pro Gly Val Phe Gly Pro Ser Ala Ser Ala Ala
405 410 415

Ala Ala Ala Gly Ile Tyr Gly Ser Gly Pro Gly Ile Tyr Gly Pro Tyr
420 425 430

Gly Pro Gly Ile Ser Gly Pro Gly Ser Gly Val Phe Gly Ile Gly Pro
435 440 445

Tyr Gly Pro Gly Ala Ser Ala Ala Ala Ala Gly Ile Tyr Gly Pro
450 455 460

Gly Val Phe Gly Pro Tyr Gly Pro Gly Ile Ser Ala Ala Ala Ala Ala
465 470 475 480

Gly Pro Gly Ser Gly Ile Tyr Gly Pro Gly Ala Ser Gly Ile Asn Gly

```
                     485                      490                      495


          Pro Gly Ser Gly Ile Tyr Gly Pro Gly Val Phe Gly Pro Gly Ile Ser
                      500                      505                      510


          Ala Ala Ala Ala Ala Gly Ile Tyr Val Phe Gly Pro Gly Val Phe Gly
                      515                      520                      525


          Pro Tyr Gly Pro Gly Ala Ser Ala Ala Ala Ala Gly Ile Tyr Gly
              530                      535                      540


          Ser Gly Pro Gly Val Phe Gly Pro Tyr Gly Pro Gly Ile Ser Gly Ser
          545                      550                      555                      560


          Gly Val Phe Gly Pro Gly Val Phe Gly Pro Tyr Ala Ser Ala Ala Ala
                      565                      570                      575


          Ala Ala Gly Pro Gly Ser Gly Val Phe Gly Pro Gly Ala Ser Gly Pro
                      580                      585                      590


          Gly Val Phe Gly Pro Tyr Gly Pro Gly Ala Ser Ala Ala Ala Ala Ala
                      595                      600                      605


          Gly Ile Asn Gly Pro Gly Ser Gly Val Phe Gly Pro Gly Ile Ser Gly
              610                      615                      620


          Ile Tyr Gly Pro Gly Val Phe Gly Pro Gly Val Phe Gly Pro Gly Ser
          625                      630                      635                      640


          Ser Ala Ala Ala Ala Ala Gly Pro Gly Ile Tyr Gly Pro Gly Val Phe
                      645                      650                      655


          Gly Pro Ser Ala Ser Ala Ala Ala Ala Gly Pro Gly Ser Gly Val
                      660                      665                      670


          Phe Gly Pro Gly Ala Ser Gly Ile Tyr Gly Pro Gly Val Phe Gly Pro
              675                      680                      685


          Gly Val Phe Gly Pro Gly Ser Ser Ala Ala Ala Ala Gly Ile Tyr
              690                      695                      700


          Gly Ser Gly Pro Gly Val Phe Gly Pro Tyr Gly Ser Ala Ala Ala Ala
          705                      710                      715                      720


          Ala Gly Pro Gly Ser Gly Ile Tyr Gly Ile Gly Pro Tyr Gly Pro Gly
                      725                      730                      735
```

Ala Ser Gly Pro Gly Ile Tyr Gly Pro Gly Val Phe Gly Pro Ser Ala
        740             745             750

Ser Ala Ala Ala Ala Ala Gly Ser Gly Val Phe Gly Pro Gly Ile Tyr
        755             760             765

Gly Pro Tyr Ala Ser Ala Ala Ala Ala Ala Gly Ile Tyr Gly Ser Gly
        770             775             780

Pro Gly Val Phe Gly Pro Tyr Gly Pro Gly Ile Ser Gly Ser Gly Val
785             790             795             800

Phe Gly Pro Gly Val Phe Gly Pro Tyr Ala Ser Ala Ala Ala Ala Ala
            805             810             815

Gly Pro Gly Val Phe Gly Pro Tyr Gly Pro Gly Ser Ser Ala Ala Ala
            820             825             830

Ala Ala Gly Ile Tyr Gly Tyr Gly Pro Gly Val Phe Gly Pro Tyr Gly
        835             840             845

Pro Gly Ala Ser Gly Ile Asn Gly Pro Gly Ser Gly Ile Tyr Gly Pro
        850             855             860

Gly Val Phe Gly Pro Gly Ile Ser Ala Ala Ala Ala Gly Pro Gly
865             870             875             880

Val Phe Gly Pro Tyr Gly Pro Gly Ala Ser Ala Ala Ala Ala Ala Gly
            885             890             895

Ile Tyr Gly Pro Gly Val Phe Gly Pro Gly Ile Tyr Gly Pro Gly Ser
        900             905             910

Ser Gly Pro Gly Val Phe Gly Pro Tyr Gly Pro Gly Ser Ser Ala Ala
        915             920             925

Ala Ala Ala Gly Ile Tyr Gly Pro Gly Val Phe Gly Pro Tyr Gly Pro
        930             935             940

Gly Ile Ser Ala Ala Ala Ala Gly Ile Tyr Val Phe Gly Pro Gly
945             950             955             960

Val Phe Gly Pro Tyr Gly Pro Gly Ala Ser Gly Pro Gly Val Phe Gly
            965             970             975

Pro Tyr Gly Pro Gly Ala Ser Ala Ala Ala Ala Ala Gly Pro Gly Ile
        980             985             990

129

Tyr Gly Pro Gly Val Phe Gly Pro  Ser Ala Ser Ala Ala  Ala Ala Ala
        995                 1000              1005

Gly Ile  Tyr Gly Ser Gly Pro  Gly Ile Tyr Gly Pro  Tyr Gly Pro
        1010              1015              1020

Gly Ile  Ser Gly Pro Gly Ser  Gly Val Phe Gly Ile  Gly Pro Tyr
        1025              1030              1035

Gly Pro  Gly Ala Ser Ala Ala  Ala Ala Ala Gly Ile  Tyr Gly Pro
        1040              1045              1050

Gly Val  Phe Gly Pro Tyr Gly  Pro Gly Ile Ser Ala  Ala Ala Ala
        1055              1060              1065

Ala Gly  Pro Gly Ser Gly Ile  Tyr Gly Pro Gly Ala  Ser Gly Ile
        1070              1075              1080

Asn Gly  Pro Gly Ser Gly Ile  Tyr Gly Pro Gly Val  Phe Gly Pro
        1085              1090              1095

Gly Ile  Ser Ala Ala Ala Ala  Ala Gly Ile Tyr Val  Phe Gly Pro
        1100              1105              1110

Gly Val  Phe Gly Pro Tyr Gly  Pro Gly Ala Ser Ala  Ala Ala Ala
        1115              1120              1125

Ala Gly  Ile Tyr Gly Ser Gly  Pro Gly Val Phe Gly  Pro Tyr Gly
        1130              1135              1140

Pro Gly  Ile Ser Gly Ser Gly  Val Phe Gly Pro Gly  Val Phe Gly
        1145              1150              1155

Pro Tyr  Ala Ser Ala Ala Ala  Ala Ala Gly Pro Gly  Ser Gly Val
        1160              1165              1170

Phe Gly  Pro Gly Ala Ser
        1175

<210> 33
<211> 601
<212> PRT
<213> Artificial Sequence

<220>
<223> PRT888

<400> 33

```
Met His His His His His His Ser Ser Gly Ser Ser Gly Pro Gly Val
1                   5                   10                  15

Leu Gly Pro Tyr Gly Pro Gly Ala Ser Ala Ala Ala Ala Ala Gly Gln
            20                  25                  30

Asn Gly Pro Gly Ser Gly Val Leu Gly Pro Gly Gln Ser Gly Gln Tyr
            35                  40                  45

Gly Pro Gly Val Leu Gly Pro Gly Val Leu Gly Pro Gly Ser Ser Ala
        50                  55                  60

Ala Ala Ala Ala Gly Pro Gly Gln Tyr Gly Pro Gly Val Leu Gly Pro
65                  70                  75                  80

Ser Ala Ser Ala Ala Ala Ala Ala Gly Pro Gly Ser Gly Val Leu Gly
                85                  90                  95

Pro Gly Ala Ser Gly Gln Tyr Gly Pro Gly Val Leu Gly Pro Gly Val
            100                 105                 110

Leu Gly Pro Gly Ser Ser Ala Ala Ala Ala Ala Gly Gln Tyr Gly Ser
            115                 120                 125

Gly Pro Gly Val Leu Gly Pro Tyr Gly Ser Ala Ala Ala Ala Ala Gly
    130                 135                 140

Pro Gly Ser Gly Gln Tyr Gly Gln Gly Pro Tyr Gly Pro Gly Ala Ser
145                 150                 155                 160

Gly Pro Gly Gln Tyr Gly Pro Gly Val Leu Gly Pro Ser Ala Ser Ala
            165                 170                 175

Ala Ala Ala Ala Gly Ser Gly Val Leu Gly Pro Gly Gln Tyr Gly Pro
            180                 185                 190

Tyr Ala Ser Ala Ala Ala Ala Ala Gly Gln Tyr Gly Ser Gly Pro Gly
            195                 200                 205

Val Leu Gly Pro Tyr Gly Pro Gly Gln Ser Gly Ser Gly Val Leu Gly
    210                 215                 220

Pro Gly Val Leu Gly Pro Tyr Ala Ser Ala Ala Ala Ala Ala Gly Pro
225                 230                 235                 240

Gly Val Leu Gly Pro Tyr Gly Pro Gly Ser Ser Ala Ala Ala Ala Ala
            245                 250                 255
```

```
Gly Gln Tyr Gly Tyr Gly Pro Gly Val Leu Gly Pro Tyr Gly Pro Gly
        260             265             270

Ala Ser Gly Gln Asn Gly Pro Gly Ser Gly Gln Tyr Gly Pro Gly Val
        275             280             285

Leu Gly Pro Gly Gln Ser Ala Ala Ala Ala Ala Gly Pro Gly Val Leu
        290             295             300

Gly Pro Tyr Gly Pro Gly Ala Ser Ala Ala Ala Ala Ala Gly Gln Tyr
305             310             315             320

Gly Pro Gly Val Leu Gly Pro Gly Gln Tyr Gly Pro Gly Ser Ser Gly
        325             330             335

Pro Gly Val Leu Gly Pro Tyr Gly Pro Gly Ser Ser Ala Ala Ala Ala
        340             345             350

Ala Gly Gln Tyr Gly Pro Gly Val Leu Gly Pro Tyr Gly Pro Gly Gln
        355             360             365

Ser Ala Ala Ala Ala Ala Gly Gln Tyr Val Leu Gly Pro Gly Val Leu
    370             375             380

Gly Pro Tyr Gly Pro Gly Ala Ser Gly Pro Gly Val Leu Gly Pro Tyr
385             390             395             400

Gly Pro Gly Ala Ser Ala Ala Ala Ala Ala Gly Pro Gly Gln Tyr Gly
            405             410             415

Pro Gly Val Leu Gly Pro Ser Ala Ser Ala Ala Ala Ala Gly Gln
        420             425             430

Tyr Gly Ser Gly Pro Gly Gln Tyr Gly Pro Tyr Gly Pro Gly Gln Ser
        435             440             445

Gly Pro Gly Ser Gly Val Leu Gly Gln Gly Pro Tyr Gly Pro Gly Ala
    450             455             460

Ser Ala Ala Ala Ala Ala Gly Gln Tyr Gly Pro Gly Val Leu Gly Pro
465             470             475             480

Tyr Gly Pro Gly Gln Ser Ala Ala Ala Ala Gly Pro Gly Ser Gly
        485             490             495

Gln Tyr Gly Pro Gly Ala Ser Gly Gln Asn Gly Pro Gly Ser Gly Gln
        500             505             510
```

```
Tyr Gly Pro Gly Val Leu Gly Pro Gly Gln Ser Ala Ala Ala Ala Ala
        515             520         525

Gly Gln Tyr Val Leu Gly Pro Gly Val Leu Gly Pro Tyr Gly Pro Gly
    530             535             540

Ala Ser Ala Ala Ala Ala Ala Gly Gln Tyr Gly Ser Gly Pro Gly Val
545             550             555             560

Leu Gly Pro Tyr Gly Pro Gly Gln Ser Gly Ser Gly Val Leu Gly Pro
            565             570             575

Gly Val Leu Gly Pro Tyr Ala Ser Ala Ala Ala Ala Ala Gly Pro Gly
            580             585             590

Ser Gly Val Leu Gly Pro Gly Ala Ser
        595             600
```

<210> 34
<211> 601
<212> PRT
<213> Artificial Sequence

<220>
<223> PRT965

<400> 34

```
Met His His His His His His Ser Ser Gly Ser Ser Gly Pro Gly Thr
1               5               10              15

Ser Gly Pro Tyr Gly Pro Gly Ala Ser Ala Ala Ala Ala Ala Gly Ala
            20              25              30

Asn Gly Pro Gly Ser Gly Thr Ser Gly Pro Gly Ala Ser Gly Ala Tyr
        35              40              45

Gly Pro Gly Thr Ser Gly Pro Gly Thr Ser Gly Pro Gly Ser Ser Ala
    50              55              60

Ala Ala Ala Ala Gly Pro Gly Ala Tyr Gly Pro Gly Thr Ser Gly Pro
65              70              75              80

Ser Ala Ser Ala Ala Ala Ala Ala Gly Pro Gly Ser Gly Thr Ser Gly
            85              90              95

Pro Gly Ala Ser Gly Ala Tyr Gly Pro Gly Thr Ser Gly Pro Gly Thr
            100             105             110
```

133

Ser Gly Pro Gly Ser Ser Ala Ala Ala Ala Ala Gly Ala Tyr Gly Ser
115 120 125

Gly Pro Gly Thr Ser Gly Pro Tyr Gly Ser Ala Ala Ala Ala Ala Gly
130 135 140

Pro Gly Ser Gly Ala Tyr Gly Ala Gly Pro Tyr Gly Pro Gly Ala Ser
145 150 155 160

Gly Pro Gly Ala Tyr Gly Pro Gly Thr Ser Gly Pro Ser Ala Ser Ala
165 170 175

Ala Ala Ala Ala Gly Ser Gly Thr Ser Gly Pro Gly Ala Tyr Gly Pro
180 185 190

Tyr Ala Ser Ala Ala Ala Ala Ala Gly Ala Tyr Gly Ser Gly Pro Gly
195 200 205

Thr Ser Gly Pro Tyr Gly Pro Gly Ala Ser Gly Ser Gly Thr Ser Gly
210 215 220

Pro Gly Thr Ser Gly Pro Tyr Ala Ser Ala Ala Ala Ala Ala Gly Pro
225 230 235 240

Gly Thr Ser Gly Pro Tyr Gly Pro Gly Ser Ser Ala Ala Ala Ala Ala
245 250 255

Gly Ala Tyr Gly Tyr Gly Pro Gly Thr Ser Gly Pro Tyr Gly Pro Gly
260 265 270

Ala Ser Gly Ala Asn Gly Pro Gly Ser Gly Ala Tyr Gly Pro Gly Thr
275 280 285

Ser Gly Pro Gly Ala Ser Ala Ala Ala Ala Gly Pro Gly Thr Ser
290 295 300

Gly Pro Tyr Gly Pro Gly Ala Ser Ala Ala Ala Ala Gly Ala Tyr
305 310 315 320

Gly Pro Gly Thr Ser Gly Pro Gly Ala Tyr Gly Pro Gly Ser Ser Gly
325 330 335

Pro Gly Thr Ser Gly Pro Tyr Gly Pro Gly Ser Ser Ala Ala Ala Ala
340 345 350

Ala Gly Ala Tyr Gly Pro Gly Thr Ser Gly Pro Tyr Gly Pro Gly Ala
355 360 365

```
Ser Ala Ala Ala Ala Ala Gly Ala Tyr Thr Ser Gly Pro Gly Thr Ser
    370             375             380

Gly Pro Tyr Gly Pro Gly Ala Ser Gly Pro Gly Thr Ser Gly Pro Tyr
    385             390             395             400

Gly Pro Gly Ala Ser Ala Ala Ala Ala Ala Gly Pro Gly Ala Tyr Gly
                405             410             415

Pro Gly Thr Ser Gly Pro Ser Ala Ser Ala Ala Ala Ala Gly Ala
            420             425             430

Tyr Gly Ser Gly Pro Gly Ala Tyr Gly Pro Tyr Gly Pro Gly Ala Ser
            435             440             445

Gly Pro Gly Ser Gly Thr Ser Gly Ala Gly Pro Tyr Gly Pro Gly Ala
    450             455             460

Ser Ala Ala Ala Ala Ala Gly Ala Tyr Gly Pro Gly Thr Ser Gly Pro
465             470             475             480

Tyr Gly Pro Gly Ala Ser Ala Ala Ala Ala Gly Pro Gly Ser Gly
            485             490             495

Ala Tyr Gly Pro Gly Ala Ser Gly Ala Asn Gly Pro Gly Ser Gly Ala
    500             505             510

Tyr Gly Pro Gly Thr Ser Gly Pro Gly Ala Ser Ala Ala Ala Ala Ala
    515             520             525

Gly Ala Tyr Thr Ser Gly Pro Gly Thr Ser Gly Pro Tyr Gly Pro Gly
    530             535             540

Ala Ser Ala Ala Ala Ala Ala Gly Ala Tyr Gly Ser Gly Pro Gly Thr
545             550             555             560

Ser Gly Pro Tyr Gly Pro Gly Ala Ser Gly Ser Gly Thr Ser Gly Pro
            565             570             575

Gly Thr Ser Gly Pro Tyr Ala Ser Ala Ala Ala Ala Gly Pro Gly
            580             585             590

Ser Gly Thr Ser Gly Pro Gly Ala Ser
    595             600
```

<210> 35

<211> 601
<212> PRT
<213> Artificial Sequence

<220>
<223> PRT889

<400> 35

Met His His His His His His Ser Ser Gly Ser Ser Gly Pro Gly Val
1               5                   10                  15

Leu Gly Pro Tyr Gly Pro Gly Ala Ser Ala Ala Ala Ala Ala Gly Ile
            20                  25                  30

Asn Gly Pro Gly Ser Gly Val Leu Gly Pro Gly Ile Ser Gly Ile Tyr
            35                  40                  45

Gly Pro Gly Val Leu Gly Pro Gly Val Leu Gly Pro Gly Ser Ser Ala
        50                  55                  60

Ala Ala Ala Ala Gly Pro Gly Ile Tyr Gly Pro Gly Val Leu Gly Pro
65                  70                  75                  80

Ser Ala Ser Ala Ala Ala Ala Ala Gly Pro Gly Ser Gly Val Leu Gly
                85                  90                  95

Pro Gly Ala Ser Gly Ile Tyr Gly Pro Gly Val Leu Gly Pro Gly Val
            100                 105                 110

Leu Gly Pro Gly Ser Ser Ala Ala Ala Ala Ala Gly Ile Tyr Gly Ser
            115                 120                 125

Gly Pro Gly Val Leu Gly Pro Tyr Gly Ser Ala Ala Ala Ala Ala Gly
        130                 135                 140

Pro Gly Ser Gly Ile Tyr Gly Ile Gly Pro Tyr Gly Pro Gly Ala Ser
145                 150                 155                 160

Gly Pro Gly Ile Tyr Gly Pro Gly Val Leu Gly Pro Ser Ala Ser Ala
            165                 170                 175

Ala Ala Ala Ala Gly Ser Gly Val Leu Gly Pro Gly Ile Tyr Gly Pro
            180                 185                 190

Tyr Ala Ser Ala Ala Ala Ala Ala Gly Ile Tyr Gly Ser Gly Pro Gly
            195                 200                 205

Val Leu Gly Pro Tyr Gly Pro Gly Ile Ser Gly Ser Gly Val Leu Gly
        210                 215                 220

136

```
Pro Gly Val Leu Gly Pro Tyr Ala Ser Ala Ala Ala Ala Ala Gly Pro
225             230             235                     240

Gly Val Leu Gly Pro Tyr Gly Pro Gly Ser Ser Ala Ala Ala Ala Ala
                245             250                     255

Gly Ile Tyr Gly Tyr Gly Pro Gly Val Leu Gly Pro Tyr Gly Pro Gly
            260             265                     270

Ala Ser Gly Ile Asn Gly Pro Gly Ser Gly Ile Tyr Gly Pro Gly Val
            275             280                     285

Leu Gly Pro Gly Ile Ser Ala Ala Ala Ala Gly Pro Gly Val Leu
    290             295                     300

Gly Pro Tyr Gly Pro Gly Ala Ser Ala Ala Ala Ala Gly Ile Tyr
305             310             315                     320

Gly Pro Gly Val Leu Gly Pro Gly Ile Tyr Gly Pro Gly Ser Ser Gly
                325             330                     335

Pro Gly Val Leu Gly Pro Tyr Gly Pro Gly Ser Ser Ala Ala Ala Ala
                340             345                     350

Ala Gly Ile Tyr Gly Pro Gly Val Leu Gly Pro Tyr Gly Pro Gly Ile
            355             360                     365

Ser Ala Ala Ala Ala Ala Gly Ile Tyr Val Leu Gly Pro Gly Val Leu
    370                 375                     380

Gly Pro Tyr Gly Pro Gly Ala Ser Gly Pro Gly Val Leu Gly Pro Tyr
385             390                 395                     400

Gly Pro Gly Ala Ser Ala Ala Ala Ala Ala Gly Pro Gly Ile Tyr Gly
                405                 410                     415

Pro Gly Val Leu Gly Pro Ser Ala Ser Ala Ala Ala Ala Ala Gly Ile
            420                 425                     430

Tyr Gly Ser Gly Pro Gly Ile Tyr Gly Pro Tyr Gly Pro Gly Ile Ser
        435                 440                     445

Gly Pro Gly Ser Gly Val Leu Gly Ile Gly Pro Tyr Gly Pro Gly Ala
    450                 455                     460

Ser Ala Ala Ala Ala Ala Gly Ile Tyr Gly Pro Gly Val Leu Gly Pro
```

```
                465                   470                   475                   480


                Tyr Gly Pro Gly Ile Ser Ala Ala Ala Ala Gly Pro Gly Ser Gly
                            485                   490                   495


                Ile Tyr Gly Pro Gly Ala Ser Gly Ile Asn Gly Pro Gly Ser Gly Ile
                        500                   505                   510


                Tyr Gly Pro Gly Val Leu Gly Pro Gly Ile Ser Ala Ala Ala Ala Ala
                        515                   520                   525


                Gly Ile Tyr Val Leu Gly Pro Gly Val Leu Gly Pro Tyr Gly Pro Gly
                        530                   535                   540


                Ala Ser Ala Ala Ala Ala Ala Gly Ile Tyr Gly Ser Gly Pro Gly Val
                545                   550                   555                   560


                Leu Gly Pro Tyr Gly Pro Gly Ile Ser Gly Ser Gly Val Leu Gly Pro
                            565                   570                   575


                Gly Val Leu Gly Pro Tyr Ala Ser Ala Ala Ala Ala Ala Gly Pro Gly
                            580                   585                   590


                Ser Gly Val Leu Gly Pro Gly Ala Ser
                            595                   600


                <210>   36
                <211>   601
                <212>   PRT
                <213>   Artificial Sequence

                <220>
                <223>   PRT916

                <400>   36

                Met His His His His His His Ser Ser Gly Ser Ser Gly Pro Gly Val
                1               5                   10                  15


                Ile Gly Pro Tyr Gly Pro Gly Ala Ser Ala Ala Ala Ala Ala Gly Leu
                            20                  25                  30


                Asn Gly Pro Gly Ser Gly Val Ile Gly Pro Gly Leu Ser Gly Leu Tyr
                            35                  40                  45


                Gly Pro Gly Val Ile Gly Pro Gly Val Ile Gly Pro Gly Ser Ser Ala
                        50                  55                  60


                Ala Ala Ala Ala Gly Pro Gly Leu Tyr Gly Pro Gly Val Ile Gly Pro
                65                  70                  75                  80
```

Ser Ala Ser Ala Ala Ala Ala Ala Gly Pro Gly Ser Gly Val Ile Gly
85 90 95

Pro Gly Ala Ser Gly Leu Tyr Gly Pro Gly Val Ile Gly Pro Gly Val
100 105 110

Ile Gly Pro Gly Ser Ser Ala Ala Ala Ala Gly Leu Tyr Gly Ser
115 120 125

Gly Pro Gly Val Ile Gly Pro Tyr Gly Ser Ala Ala Ala Ala Gly
130 135 140

Pro Gly Ser Gly Leu Tyr Gly Leu Gly Pro Tyr Gly Pro Gly Ala Ser
145 150 155 160

Gly Pro Gly Leu Tyr Gly Pro Gly Val Ile Gly Pro Ser Ala Ser Ala
165 170 175

Ala Ala Ala Ala Gly Ser Gly Val Ile Gly Pro Gly Leu Tyr Gly Pro
180 185 190

Tyr Ala Ser Ala Ala Ala Ala Ala Gly Leu Tyr Gly Ser Gly Pro Gly
195 200 205

Val Ile Gly Pro Tyr Gly Pro Gly Leu Ser Gly Ser Gly Val Ile Gly
210 215 220

Pro Gly Val Ile Gly Pro Tyr Ala Ser Ala Ala Ala Ala Ala Gly Pro
225 230 235 240

Gly Val Ile Gly Pro Tyr Gly Pro Gly Ser Ser Ala Ala Ala Ala Ala
245 250 255

Gly Leu Tyr Gly Tyr Gly Pro Gly Val Ile Gly Pro Tyr Gly Pro Gly
260 265 270

Ala Ser Gly Leu Asn Gly Pro Gly Ser Gly Leu Tyr Gly Pro Gly Val
275 280 285

Ile Gly Pro Gly Leu Ser Ala Ala Ala Ala Gly Pro Gly Val Ile
290 295 300

Gly Pro Tyr Gly Pro Gly Ala Ser Ala Ala Ala Ala Ala Gly Leu Tyr
305 310 315 320

Gly Pro Gly Val Ile Gly Pro Gly Leu Tyr Gly Pro Gly Ser Ser Gly

```
                    325                     330                         335


        Pro Gly Val Ile Gly Pro Tyr Gly Pro Gly Ser Ser Ala Ala Ala Ala
                    340             345             350


        Ala Gly Leu Tyr Gly Pro Gly Val Ile Gly Pro Tyr Gly Pro Gly Leu
                    355             360             365


        Ser Ala Ala Ala Ala Ala Gly Leu Tyr Val Ile Gly Pro Gly Val Ile
                    370             375             380


        Gly Pro Tyr Gly Pro Gly Ala Ser Gly Pro Gly Val Ile Gly Pro Tyr
            385             390             395             400


        Gly Pro Gly Ala Ser Ala Ala Ala Ala Ala Gly Pro Gly Leu Tyr Gly
                    405             410             415


        Pro Gly Val Ile Gly Pro Ser Ala Ser Ala Ala Ala Ala Ala Gly Leu
                    420             425             430


        Tyr Gly Ser Gly Pro Gly Leu Tyr Gly Pro Tyr Gly Pro Gly Leu Ser
                    435             440             445


        Gly Pro Gly Ser Gly Val Ile Gly Leu Gly Pro Tyr Gly Pro Gly Ala
                    450             455             460


        Ser Ala Ala Ala Ala Ala Gly Leu Tyr Gly Pro Gly Val Ile Gly Pro
            465             470             475             480


        Tyr Gly Pro Gly Leu Ser Ala Ala Ala Ala Gly Pro Gly Ser Gly
                    485             490             495


        Leu Tyr Gly Pro Gly Ala Ser Gly Leu Asn Gly Pro Gly Ser Gly Leu
                    500             505             510


        Tyr Gly Pro Gly Val Ile Gly Pro Gly Leu Ser Ala Ala Ala Ala Ala
                    515             520             525


        Gly Leu Tyr Val Ile Gly Pro Gly Val Ile Gly Pro Tyr Gly Pro Gly
                    530             535             540


        Ala Ser Ala Ala Ala Ala Ala Gly Leu Tyr Gly Ser Gly Pro Gly Val
            545             550             555             560


        Ile Gly Pro Tyr Gly Pro Gly Leu Ser Gly Ser Gly Val Ile Gly Pro
                    565             570             575
```

140

```
Gly Val Ile Gly Pro Tyr Ala Ser Ala Ala Ala Ala Gly Pro Gly
            580                 585                 590


Ser Gly Val Ile Gly Pro Gly Ala Ser
            595                 600


<210>   37
<211>   601
<212>   PRT
<213>   Artificial Sequence

<220>
<223>   PRT918

<400>   37

Met His His His His His His Ser Ser Gly Ser Ser Gly Pro Gly Val
1                   5                   10                  15


Phe Gly Pro Tyr Gly Pro Gly Ala Ser Ala Ala Ala Ala Ala Gly Ile
            20                  25                  30


Asn Gly Pro Gly Ser Gly Val Phe Gly Pro Gly Ile Ser Gly Ile Tyr
            35                  40                  45


Gly Pro Gly Val Phe Gly Pro Gly Val Phe Gly Pro Gly Ser Ser Ala
        50                  55                  60


Ala Ala Ala Ala Gly Pro Gly Ile Tyr Gly Pro Gly Val Phe Gly Pro
65                  70                  75                  80


Ser Ala Ser Ala Ala Ala Ala Ala Gly Pro Gly Ser Gly Val Phe Gly
                85                  90                  95


Pro Gly Ala Ser Gly Ile Tyr Gly Pro Gly Val Phe Gly Pro Gly Val
            100                 105                 110


Phe Gly Pro Gly Ser Ser Ala Ala Ala Ala Ala Gly Ile Tyr Gly Ser
            115                 120                 125


Gly Pro Gly Val Phe Gly Pro Tyr Gly Ser Ala Ala Ala Ala Ala Gly
        130                 135                 140


Pro Gly Ser Gly Ile Tyr Gly Ile Gly Pro Tyr Gly Pro Gly Ala Ser
145                 150                 155                 160


Gly Pro Gly Ile Tyr Gly Pro Gly Val Phe Gly Pro Ser Ala Ser Ala
                165                 170                 175


Ala Ala Ala Ala Gly Ser Gly Val Phe Gly Pro Gly Ile Tyr Gly Pro
```

```
                    180                      185                            190

        Tyr Ala Ser Ala Ala Ala Ala Ala Gly Ile Tyr Gly Ser Gly Pro Gly
                195                      200                      205

        Val Phe Gly Pro Tyr Gly Pro Gly Ile Ser Gly Ser Gly Val Phe Gly
                210                      215                      220

        Pro Gly Val Phe Gly Pro Tyr Ala Ser Ala Ala Ala Ala Gly Pro
        225                      230                      235                      240

        Gly Val Phe Gly Pro Tyr Gly Pro Gly Ser Ser Ala Ala Ala Ala Ala
                        245                      250                      255

        Gly Ile Tyr Gly Tyr Gly Pro Gly Val Phe Gly Pro Tyr Gly Pro Gly
                        260                      265                      270

        Ala Ser Gly Ile Asn Gly Pro Gly Ser Gly Ile Tyr Gly Pro Gly Val
                        275                      280                      285

        Phe Gly Pro Gly Ile Ser Ala Ala Ala Ala Gly Pro Gly Val Phe
                290                      295                      300

        Gly Pro Tyr Gly Pro Gly Ala Ser Ala Ala Ala Ala Ala Gly Ile Tyr
        305                      310                      315                      320

        Gly Pro Gly Val Phe Gly Pro Gly Ile Tyr Gly Pro Gly Ser Ser Gly
                        325                      330                      335

        Pro Gly Val Phe Gly Pro Tyr Gly Pro Gly Ser Ser Ala Ala Ala Ala
                        340                      345                      350

        Ala Gly Ile Tyr Gly Pro Gly Val Phe Gly Pro Tyr Gly Pro Gly Ile
                        355                      360                      365

        Ser Ala Ala Ala Ala Ala Gly Ile Tyr Val Phe Gly Pro Gly Val Phe
                370                      375                      380

        Gly Pro Tyr Gly Pro Gly Ala Ser Gly Pro Gly Val Phe Gly Pro Tyr
        385                      390                      395                      400

        Gly Pro Gly Ala Ser Ala Ala Ala Ala Ala Gly Pro Gly Ile Tyr Gly
                        405                      410                      415

        Pro Gly Val Phe Gly Pro Ser Ala Ser Ala Ala Ala Ala Ala Gly Ile
                        420                      425                      430
```

142

```
Tyr Gly Ser Gly Pro Gly Ile Tyr Gly Pro Tyr Gly Pro Gly Ile Ser
        435                 440                 445

Gly Pro Gly Ser Gly Val Phe Gly Ile Gly Pro Tyr Gly Pro Gly Ala
        450                 455                 460

Ser Ala Ala Ala Ala Ala Gly Ile Tyr Gly Pro Gly Val Phe Gly Pro
465                 470                 475                 480

Tyr Gly Pro Gly Ile Ser Ala Ala Ala Ala Gly Pro Gly Ser Gly
                485                 490                 495

Ile Tyr Gly Pro Gly Ala Ser Gly Ile Asn Gly Pro Gly Ser Gly Ile
            500                 505                 510

Tyr Gly Pro Gly Val Phe Gly Pro Gly Ile Ser Ala Ala Ala Ala Ala
        515                 520                 525

Gly Ile Tyr Val Phe Gly Pro Gly Val Phe Gly Pro Tyr Gly Pro Gly
        530                 535                 540

Ala Ser Ala Ala Ala Ala Ala Gly Ile Tyr Gly Ser Gly Pro Gly Val
545                 550                 555                 560

Phe Gly Pro Tyr Gly Pro Gly Ile Ser Gly Ser Gly Val Phe Gly Pro
            565                 570                 575

Gly Val Phe Gly Pro Tyr Ala Ser Ala Ala Ala Ala Ala Gly Pro Gly
            580                 585                 590

Ser Gly Val Phe Gly Pro Gly Ala Ser
            595                 600
```

```
<210>   38
<211>   576
<212>   PRT
<213>   Artificial Sequence

<220>
<223>   PRT699

<400>   38

Met His His His His His His Ser Ser Gly Ser Ser Gly Pro Gly Val
1               5                   10                  15

Leu Gly Pro Tyr Gly Pro Gly Ala Ser Ala Ala Ala Ala Ala Ala Ala
            20                  25                  30

Gly Ser Asn Gly Pro Gly Ser Gly Val Leu Gly Pro Gly Gln Ser Gly
```

```
                    35                      40                      45

        Gln Tyr Gly Pro Gly Val Leu Gly Pro Gly Val Leu Gly Pro Gly Ser
            50                  55                  60

        Ser Ala Ala Ala Ala Ala Ala Ala Gly Pro Gly Gln Tyr Gly Pro Gly
        65              70                  75                      80

        Val Leu Gly Pro Ser Ala Ser Ala Ala Ala Ala Ala Ala Ala Gly Pro
                        85                  90                  95

        Gly Ser Gly Val Leu Gly Pro Gly Ala Ser Gly Gln Tyr Gly Pro Gly
                    100                 105                 110

        Val Leu Gly Pro Gly Val Leu Gly Pro Gly Ser Ser Ala Ala Ala Ala
                    115                 120                 125

        Ala Ala Ala Gly Ser Tyr Gly Ser Gly Pro Gly Val Leu Gly Pro Tyr
            130                 135                 140

        Gly Ser Ala Ala Ala Ala Ala Ala Ala Gly Pro Gly Ser Gly Gln Tyr
        145             150                 155                     160

        Gly Gln Gly Pro Tyr Gly Pro Gly Ala Ser Gly Pro Gly Gln Tyr Gly
                    165                 170                 175

        Pro Gly Val Leu Gly Pro Ser Ala Ser Ala Ala Ala Ala Ala Ala Ala
                    180                 185                 190

        Gly Ser Gly Val Leu Gly Pro Gly Gln Tyr Gly Pro Tyr Ala Ser Ala
                    195                 200                 205

        Ala Ala Ala Ala Ala Ala Gly Ser Tyr Gly Ser Gly Pro Gly Val Leu
            210                 215                 220

        Gly Pro Tyr Gly Pro Gly Gln Ser Gly Ser Gly Val Leu Gly Pro Gly
        225                 230                 235                 240

        Val Leu Gly Pro Tyr Ala Ser Ala Ala Ala Ala Ala Ala Gly Pro
                    245                 250                 255

        Gly Val Leu Gly Pro Tyr Gly Pro Gly Ser Ser Ala Ala Ala Ala Ala
                    260                 265                 270

        Ala Ala Gly Ser Tyr Gly Tyr Gly Pro Gly Val Leu Gly Pro Tyr Gly
            275                 280                 285
```

144

Pro Gly Ala Ser Gly Gln Asn Gly Pro Gly Ser Gly Gln Tyr Gly Pro
290              295              300

Gly Val Leu Gly Pro Gly Pro Ser Ala Ala Ala Ala Ala Ala Ala Gly
305              310              315              320

Pro Gly Val Leu Gly Pro Tyr Gly Pro Gly Ala Ser Ala Ala Ala Ala
             325              330              335

Ala Ala Ala Gly Ser Tyr Gly Pro Gly Val Leu Gly Pro Gly Gln Tyr
         340              345              350

Gly Pro Gly Ser Ser Gly Pro Gly Val Leu Gly Pro Tyr Gly Pro Gly
         355              360              365

Ser Ser Ala Ala Ala Ala Ala Ala Ala Gly Ser Tyr Gly Pro Gly Val
370              375              380

Leu Gly Pro Tyr Gly Pro Gly Pro Ser Ala Ala Ala Ala Ala Ala Ala
385              390              395              400

Gly Ser Tyr Val Leu Gly Pro Gly Val Leu Gly Pro Tyr Gly Pro Gly
             405              410              415

Ala Ser Gly Pro Gly Val Leu Gly Pro Tyr Gly Pro Gly Ala Ser Ala
         420              425              430

Ala Ala Ala Ala Ala Ala Gly Pro Gly Gln Tyr Gly Pro Gly Val Leu
         435              440              445

Gly Pro Ser Ala Ser Ala Ala Ala Ala Ala Ala Gly Ser Tyr Gly
         450              455              460

Ser Gly Pro Gly Gln Tyr Gly Pro Tyr Gly Pro Gly Gln Ser Gly Pro
465              470              475              480

Gly Ser Gly Val Leu Gly Gln Gly Pro Tyr Gly Pro Gly Ala Ser Ala
             485              490              495

Ala Ala Ala Ala Ala Gly Ser Tyr Gly Pro Gly Val Leu Gly Pro
         500              505              510

Tyr Gly Pro Gly Pro Ser Ala Ala Ala Ala Ala Ala Gly Pro Gly
         515              520              525

Ser Gly Gln Tyr Gly Pro Gly Ala Ser Gly Gln Asn Gly Pro Gly Ser
530              535              540

Gly Gln Tyr Gly Pro Gly Val Leu Gly Pro Gly Pro Ser Ala Ala Ala
545                 550             555                 560

Ala Ala Ala Ala Gly Pro Gly Ser Gly Val Leu Gly Pro Gly Ala Ser
                565             570                 575

```
<210>  39
<211>  576
<212>  PRT
<213>  Artificial Sequence

<220>
<223>  PRT698

<400>  39
```

Met His His His His His His Ser Ser Gly Ser Ser Gly Pro Gly Val
1               5               10                  15

Leu Gly Pro Tyr Gly Pro Gly Ala Ser Ala Ala Ala Ala Ala Ala Ala
            20              25                  30

Gly Ser Asn Gly Pro Gly Ser Gly Val Leu Gly Pro Gly Ile Ser Gly
        35              40                  45

Ile Tyr Gly Pro Gly Val Leu Gly Pro Gly Val Leu Gly Pro Gly Ser
        50              55                  60

Ser Ala Ala Ala Ala Ala Ala Ala Gly Pro Gly Ile Tyr Gly Pro Gly
65                  70              75                  80

Val Leu Gly Pro Ser Ala Ser Ala Ala Ala Ala Ala Ala Ala Gly Pro
                85              90                  95

Gly Ser Gly Val Leu Gly Pro Gly Ala Ser Gly Ile Tyr Gly Pro Gly
            100             105                 110

Val Leu Gly Pro Gly Val Leu Gly Pro Gly Ser Ser Ala Ala Ala Ala
            115             120                 125

Ala Ala Ala Gly Ser Tyr Gly Ser Gly Pro Gly Val Leu Gly Pro Tyr
        130             135                 140

Gly Ser Ala Ala Ala Ala Ala Ala Ala Gly Pro Gly Ser Gly Ile Tyr
145                 150                 155                 160

Gly Ile Gly Pro Tyr Gly Pro Gly Ala Ser Gly Pro Gly Ile Tyr Gly
                165             170                 175

146

```
Pro Gly Val Leu Gly Pro Ser Ala Ser Ala Ala Ala Ala Ala Ala
        180             185             190

Gly Ser Gly Val Leu Gly Pro Gly Ile Tyr Gly Pro Tyr Ala Ser Ala
        195             200             205

Ala Ala Ala Ala Ala Ala Gly Ser Tyr Gly Ser Gly Pro Gly Val Leu
        210             215             220

Gly Pro Tyr Gly Pro Gly Ile Ser Gly Ser Gly Val Leu Gly Pro Gly
225             230             235             240

Val Leu Gly Pro Tyr Ala Ser Ala Ala Ala Ala Ala Ala Gly Pro
            245             250             255

Gly Val Leu Gly Pro Tyr Gly Pro Gly Ser Ser Ala Ala Ala Ala Ala
        260             265             270

Ala Ala Gly Ser Tyr Gly Tyr Gly Pro Gly Val Leu Gly Pro Tyr Gly
        275             280             285

Pro Gly Ala Ser Gly Ile Asn Gly Pro Gly Ser Gly Ile Tyr Gly Pro
        290             295             300

Gly Val Leu Gly Pro Gly Pro Ser Ala Ala Ala Ala Ala Ala Gly
305             310             315             320

Pro Gly Val Leu Gly Pro Tyr Gly Pro Gly Ala Ser Ala Ala Ala Ala
            325             330             335

Ala Ala Ala Gly Ser Tyr Gly Pro Gly Val Leu Gly Pro Gly Ile Tyr
        340             345             350

Gly Pro Gly Ser Ser Gly Pro Gly Val Leu Gly Pro Tyr Gly Pro Gly
        355             360             365

Ser Ser Ala Ala Ala Ala Ala Ala Ala Gly Ser Tyr Gly Pro Gly Val
370             375             380

Leu Gly Pro Tyr Gly Pro Gly Pro Ser Ala Ala Ala Ala Ala Ala Ala
385             390             395             400

Gly Ser Tyr Val Leu Gly Pro Gly Val Leu Gly Pro Tyr Gly Pro Gly
            405             410             415

Ala Ser Gly Pro Gly Val Leu Gly Pro Tyr Gly Pro Gly Ala Ser Ala
        420             425             430
```

147

```
Ala Ala Ala Ala Ala Ala Gly Pro Gly Ile Tyr Gly Pro Gly Val Leu
        435             440             445

Gly Pro Ser Ala Ser Ala Ala Ala Ala Ala Ala Gly Ser Tyr Gly
    450             455             460

Ser Gly Pro Gly Ile Tyr Gly Pro Tyr Gly Pro Gly Ile Ser Gly Pro
465             470             475             480

Gly Ser Gly Val Leu Gly Ile Gly Pro Tyr Gly Pro Gly Ala Ser Ala
            485             490             495

Ala Ala Ala Ala Ala Ala Gly Ser Tyr Gly Pro Gly Val Leu Gly Pro
        500             505             510

Tyr Gly Pro Gly Pro Ser Ala Ala Ala Ala Ala Ala Gly Pro Gly
    515             520             525

Ser Gly Ile Tyr Gly Pro Gly Ala Ser Gly Ile Asn Gly Pro Gly Ser
    530             535             540

Gly Ile Tyr Gly Pro Gly Val Leu Gly Pro Gly Pro Ser Ala Ala Ala
545             550             555             560

Ala Ala Ala Ala Gly Pro Gly Ser Gly Val Leu Gly Pro Gly Ala Ser
            565             570             575
```

```
<210>   40
<211>   1190
<212>   PRT
<213>   Artificial Sequence

<220>
<223>   PRT966

<400>   40
```

```
Met His His His His His His Ser Ser Gly Ser Ser Gly Pro Gly Val
1                   5                   10                  15

Phe Gly Pro Tyr Gly Pro Gly Ala Ser Ala Ala Ala Ala Ala Gly Ile
            20                  25                  30

Asn Gly Pro Gly Ser Gly Val Phe Gly Pro Gly Ile Ser Gly Ile Tyr
            35                  40                  45

Gly Pro Gly Val Phe Gly Pro Gly Val Phe Gly Pro Gly Ser Ser Ala
    50                  55                  60
```

```
Ala Ala Ala Ala Gly Pro Gly Ile Tyr Gly Pro Gly Val Phe Gly Pro
65          70              75              80

Ser Ala Ser Ala Ala Ala Ala Ala Gly Pro Gly Ser Gly Val Phe Gly
            85              90              95

Pro Gly Ala Ser Gly Ile Tyr Gly Pro Gly Val Phe Gly Pro Gly Val
        100             105             110

Phe Gly Pro Gly Ser Ser Ala Ala Ala Ala Gly Ile Tyr Gly Ser
        115             120             125

Gly Pro Gly Val Phe Gly Pro Tyr Gly Ser Ala Ala Ala Ala Gly
    130             135             140

Pro Gly Ser Gly Ile Tyr Gly Ile Gly Pro Tyr Gly Pro Gly Ala Ser
145             150             155             160

Gly Pro Gly Ile Tyr Gly Pro Gly Val Phe Gly Pro Ser Ala Ser Ala
        165             170             175

Ala Ala Ala Ala Gly Ser Gly Val Phe Gly Pro Gly Ile Tyr Gly Pro
        180             185             190

Tyr Ala Ser Ala Ala Ala Ala Gly Ile Tyr Gly Ser Gly Pro Gly
        195             200             205

Val Phe Gly Pro Tyr Gly Pro Gly Ile Ser Gly Ser Gly Val Phe Gly
    210             215             220

Pro Gly Val Phe Gly Pro Tyr Ala Ser Ala Ala Ala Ala Gly Pro
225             230             235             240

Gly Val Phe Gly Pro Tyr Gly Pro Gly Ser Ser Ala Ala Ala Ala
        245             250             255

Gly Ile Tyr Gly Tyr Gly Pro Gly Val Phe Gly Pro Tyr Gly Pro Gly
        260             265             270

Ala Ser Gly Ile Asn Gly Pro Gly Ser Gly Ile Tyr Gly Pro Gly Val
        275             280             285

Phe Gly Pro Gly Ile Ser Ala Ala Ala Ala Gly Pro Gly Val Phe
    290             295             300

Gly Pro Tyr Gly Pro Gly Ala Ser Ala Ala Ala Ala Gly Ile Tyr
305             310             315             320
```

Gly Pro Gly Val Phe Gly Pro Gly Ile Tyr Gly Pro Gly Ser Ser Gly
              325                 330                 335

Pro Gly Val Phe Gly Pro Tyr Gly Pro Gly Ser Ser Ala Ala Ala Ala
              340                 345                 350

Ala Gly Ile Tyr Gly Pro Gly Val Phe Gly Pro Tyr Gly Pro Gly Ile
              355                 360                 365

Ser Ala Ala Ala Ala Ala Gly Ile Tyr Val Phe Gly Pro Gly Val Phe
              370                 375                 380

Gly Pro Tyr Gly Pro Gly Ala Ser Gly Pro Gly Val Phe Gly Pro Tyr
385                 390                 395                 400

Gly Pro Gly Ala Ser Ala Ala Ala Ala Ala Gly Pro Gly Ile Tyr Gly
              405                 410                 415

Pro Gly Val Phe Gly Pro Ser Ala Ser Ala Ala Ala Ala Gly Ile
              420                 425                 430

Tyr Gly Ser Gly Pro Gly Ile Tyr Gly Pro Tyr Gly Pro Gly Ile Ser
              435                 440                 445

Gly Pro Gly Ser Gly Val Phe Gly Ile Gly Pro Tyr Gly Pro Gly Ala
              450                 455                 460

Ser Ala Ala Ala Ala Ala Gly Ile Tyr Gly Pro Gly Val Phe Gly Pro
465                 470                 475                 480

Tyr Gly Pro Gly Ile Ser Ala Ala Ala Ala Gly Pro Gly Ser Gly
              485                 490                 495

Ile Tyr Gly Pro Gly Ala Ser Gly Ile Asn Gly Pro Gly Ser Gly Ile
              500                 505                 510

Tyr Gly Pro Gly Val Phe Gly Pro Gly Ile Ser Ala Ala Ala Ala Ala
              515                 520                 525

Gly Ile Tyr Val Phe Gly Pro Gly Val Phe Gly Pro Tyr Gly Pro Gly
              530                 535                 540

Ala Ser Ala Ala Ala Ala Gly Ile Tyr Gly Ser Gly Pro Gly Val
545                 550                 555                 560

Phe Gly Pro Tyr Gly Pro Gly Ile Ser Gly Ser Gly Val Phe Gly Pro
              565                 570                 575

```
Gly Val Phe Gly Pro Tyr Ala Ser Ala Ala Ala Ala Ala Gly Pro Gly
            580                 585                 590

Ser Gly Val Phe Gly Pro Gly Ala Ser Gly Pro Gly Val Phe Gly Pro
            595                 600                 605

Tyr Gly Pro Gly Ala Ser Ala Ala Ala Ala Gly Ile Asn Gly Pro
    610                 615                 620

Gly Ser Gly Val Phe Gly Pro Gly Ile Ser Gly Ile Tyr Gly Pro Gly
625                 630                 635                 640

Val Phe Gly Pro Gly Val Phe Gly Pro Gly Ser Ser Ala Ala Ala Ala
            645                 650                 655

Ala Gly Pro Gly Ile Tyr Gly Pro Gly Val Phe Gly Pro Ser Ala Ser
            660                 665                 670

Ala Ala Ala Ala Ala Gly Pro Gly Ser Gly Val Phe Gly Pro Gly Ala
    675                 680                 685

Ser Gly Ile Tyr Gly Pro Gly Val Phe Gly Pro Gly Val Phe Gly Pro
    690                 695                 700

Gly Ser Ser Ala Ala Ala Ala Ala Gly Ile Tyr Gly Ser Gly Pro Gly
705                 710                 715                 720

Val Phe Gly Pro Tyr Gly Ser Ala Ala Ala Ala Gly Pro Gly Ser
            725                 730                 735

Gly Ile Tyr Gly Ile Gly Pro Tyr Gly Pro Gly Ala Ser Gly Pro Gly
            740                 745                 750

Ile Tyr Gly Pro Gly Val Phe Gly Pro Ser Ala Ser Ala Ala Ala Ala
    755                 760                 765

Ala Gly Ser Gly Val Phe Gly Pro Gly Ile Tyr Gly Pro Tyr Ala Ser
    770                 775                 780

Ala Ala Ala Ala Ala Gly Ile Tyr Gly Ser Gly Pro Gly Val Phe Gly
785                 790                 795                 800

Pro Tyr Gly Pro Gly Ile Ser Gly Ser Gly Val Phe Gly Pro Gly Val
            805                 810                 815

Phe Gly Pro Tyr Ala Ser Ala Ala Ala Ala Ala Gly Pro Gly Val Phe
```

820                     825                     830

Gly Pro Tyr Gly Pro Gly Ser Ser Ala Ala Ala Ala Ala Gly Ile Tyr
        835                 840                 845

Gly Tyr Gly Pro Gly Val Phe Gly Pro Tyr Gly Pro Gly Ala Ser Gly
        850                 855                 860

Ile Asn Gly Pro Gly Ser Gly Ile Tyr Gly Pro Gly Val Phe Gly Pro
865                 870                 875                 880

Gly Ile Ser Ala Ala Ala Ala Ala Gly Pro Gly Val Phe Gly Pro Tyr
            885                 890                 895

Gly Pro Gly Ala Ser Ala Ala Ala Ala Ala Gly Ile Tyr Gly Pro Gly
            900                 905                 910

Val Phe Gly Pro Gly Ile Tyr Gly Pro Gly Ser Ser Gly Pro Gly Val
        915                 920                 925

Phe Gly Pro Tyr Gly Pro Gly Ser Ser Ala Ala Ala Ala Ala Gly Ile
        930                 935                 940

Tyr Gly Pro Gly Val Phe Gly Pro Tyr Gly Pro Gly Ile Ser Ala Ala
945                 950                 955                 960

Ala Ala Ala Gly Ile Tyr Val Phe Gly Pro Gly Val Phe Gly Pro Tyr
            965                 970                 975

Gly Pro Gly Ala Ser Gly Pro Gly Val Phe Gly Pro Tyr Gly Pro Gly
            980                 985                 990

Ala Ser Ala Ala Ala Ala Ala Gly  Pro Gly Ile Tyr Gly  Pro Gly Val
        995                 1000                1005

Phe Gly  Pro Ser Ala Ser Ala  Ala Ala Ala Ala Gly  Ile Tyr Gly
        1010                1015                1020

Ser Gly  Pro Gly Ile Tyr Gly  Pro Tyr Gly Pro Gly  Ile Ser Gly
        1025                1030                1035

Pro Gly  Ser Gly Val Phe Gly  Ile Gly Pro Tyr Gly  Pro Gly Ala
        1040                1045                1050

Ser Ala  Ala Ala Ala Ala Gly  Ile Tyr Gly Pro Gly  Val Phe Gly
        1055                1060                1065

```
Pro Tyr  Gly Pro Gly Ile Ser  Ala Ala Ala Ala Ala  Gly Pro Gly
    1070             1075              1080


Ser Gly  Ile Tyr Gly Pro Gly  Ala Ser Gly Ile Asn  Gly Pro Gly
    1085             1090              1095


Ser Gly  Ile Tyr Gly Pro Gly  Val Phe Gly Pro Gly  Ile Ser Ala
    1100             1105              1110


Ala Ala  Ala Ala Gly Ile Tyr  Val Phe Gly Pro Gly  Val Phe Gly
    1115             1120              1125


Pro Tyr  Gly Pro Gly Ala Ser  Ala Ala Ala Ala Ala  Gly Ile Tyr
    1130             1135              1140


Gly Ser  Gly Pro Gly Val Phe  Gly Pro Tyr Gly Pro  Gly Ile Ser
    1145             1150              1155


Gly Ser  Gly Val Phe Gly Pro  Gly Val Phe Gly Pro  Tyr Ala Ser
    1160             1165              1170


Ala Ala  Ala Ala Ala Gly Pro  Gly Ser Gly Val Phe  Gly Pro Gly
    1175             1180              1185


Ala Ser
    1190



<210>  41
<211>  590
<212>  PRT
<213>  Artificial Sequence

<220>
<223>  Met-PRT917

<400>  41

Met Gly Pro Gly Leu Ile Gly Pro Tyr Gly Pro Gly Ala Ser Ala Ala
1                 5                 10                15


Ala Ala Ala Gly Val Asn Gly Pro Gly Ser Gly Leu Ile Gly Pro Gly
            20                25                30


Val Ser Gly Val Tyr Gly Pro Gly Leu Ile Gly Pro Gly Leu Ile Gly
            35                40                45


Pro Gly Ser Ser Ala Ala Ala Ala Ala Gly Pro Gly Val Tyr Gly Pro
    50                55                60


Gly Leu Ile Gly Pro Ser Ala Ser Ala Ala Ala Ala Ala Gly Pro Gly
```

|  | 65 | | | 70 | | | 75 | | | 80 |
|---|---|---|---|---|---|---|---|---|---|---|

Ser Gly Leu Ile Gly Pro Gly Ala Ser Gly Val Tyr Gly Pro Gly Leu
           85                 90          95

Ile Gly Pro Gly Leu Ile Gly Pro Gly Ser Ser Ala Ala Ala Ala Ala
       100           105          110

Gly Val Tyr Gly Ser Gly Pro Gly Leu Ile Gly Pro Tyr Gly Ser Ala
      115          120          125

Ala Ala Ala Ala Gly Pro Gly Ser Gly Val Tyr Gly Val Gly Pro Tyr
    130          135          140

Gly Pro Gly Ala Ser Gly Pro Gly Val Tyr Gly Pro Gly Leu Ile Gly
145          150          155          160

Pro Ser Ala Ser Ala Ala Ala Ala Gly Ser Gly Leu Ile Gly Pro
          165          170          175

Gly Val Tyr Gly Pro Tyr Ala Ser Ala Ala Ala Ala Gly Val Tyr
        180          185          190

Gly Ser Gly Pro Gly Leu Ile Gly Pro Tyr Gly Pro Gly Val Ser Gly
      195          200          205

Ser Gly Leu Ile Gly Pro Gly Leu Ile Gly Pro Tyr Ala Ser Ala Ala
    210          215          220

Ala Ala Ala Gly Pro Gly Leu Ile Gly Pro Tyr Gly Pro Gly Ser Ser
225          230          235          240

Ala Ala Ala Ala Ala Gly Val Tyr Gly Tyr Gly Pro Gly Leu Ile Gly
        245          250          255

Pro Tyr Gly Pro Gly Ala Ser Gly Val Asn Gly Pro Gly Ser Gly Val
      260          265          270

Tyr Gly Pro Gly Leu Ile Gly Pro Gly Val Ser Ala Ala Ala Ala Ala
      275          280          285

Gly Pro Gly Leu Ile Gly Pro Tyr Gly Pro Gly Ala Ser Ala Ala Ala
      290          295          300

Ala Ala Gly Val Tyr Gly Pro Gly Leu Ile Gly Pro Gly Val Tyr Gly
305          310          315          320

```
Pro Gly Ser Ser Gly Pro Gly Leu Ile Gly Pro Tyr Gly Pro Gly Ser
            325             330             335

Ser Ala Ala Ala Ala Ala Gly Val Tyr Gly Pro Gly Leu Ile Gly Pro
            340             345             350

Tyr Gly Pro Gly Val Ser Ala Ala Ala Ala Gly Val Tyr Leu Ile
            355             360             365

Gly Pro Gly Leu Ile Gly Pro Tyr Gly Pro Gly Ala Ser Gly Pro Gly
            370             375             380

Leu Ile Gly Pro Tyr Gly Pro Gly Ala Ser Ala Ala Ala Ala Gly
385             390             395             400

Pro Gly Val Tyr Gly Pro Gly Leu Ile Gly Pro Ser Ala Ser Ala Ala
            405             410             415

Ala Ala Ala Gly Val Tyr Gly Ser Gly Pro Gly Val Tyr Gly Pro Tyr
            420             425             430

Gly Pro Gly Val Ser Gly Pro Gly Ser Gly Leu Ile Gly Val Gly Pro
            435             440             445

Tyr Gly Pro Gly Ala Ser Ala Ala Ala Ala Gly Val Tyr Gly Pro
            450             455             460

Gly Leu Ile Gly Pro Tyr Gly Pro Gly Val Ser Ala Ala Ala Ala Ala
465             470             475             480

Gly Pro Gly Ser Gly Val Tyr Gly Pro Gly Ala Ser Gly Val Asn Gly
            485             490             495

Pro Gly Ser Gly Val Tyr Gly Pro Gly Leu Ile Gly Pro Gly Val Ser
            500             505             510

Ala Ala Ala Ala Ala Gly Val Tyr Leu Ile Gly Pro Gly Leu Ile Gly
            515             520             525

Pro Tyr Gly Pro Gly Ala Ser Ala Ala Ala Ala Gly Val Tyr Gly
            530             535             540

Ser Gly Pro Gly Leu Ile Gly Pro Tyr Gly Pro Gly Val Ser Gly Ser
545             550             555             560

Gly Leu Ile Gly Pro Gly Leu Ile Gly Pro Tyr Ala Ser Ala Ala Ala
            565             570             575
```

```
Ala Ala Gly Pro Gly Ser Gly Leu Ile Gly Pro Gly Ala Ser
            580                 585                 590
```

```
<210>  42
<211>  587
<212>  PRT
<213>  Artificial Sequence

<220>
<223>  Met-PRT1028

<400>  42
```

```
Met Gly Pro Gly Ile Phe Gly Pro Tyr Gly Pro Gly Ala Ser Ala Ala
1               5               10              15
```

```
Ala Ala Ala Gly Thr Gly Pro Gly Ser Gly Ile Phe Gly Pro Gly Thr
            20                  25              30
```

```
Ser Gly Thr Tyr Gly Pro Gly Ile Phe Gly Pro Gly Ile Phe Gly Pro
        35                  40              45
```

```
Gly Ser Ser Ala Ala Ala Ala Ala Gly Pro Gly Thr Tyr Gly Pro Gly
        50                  55              60
```

```
Ile Phe Gly Pro Ser Ala Ser Ala Ala Ala Ala Gly Pro Gly Ser
65                  70              75                  80
```

```
Gly Ile Phe Gly Pro Gly Ala Ser Gly Thr Tyr Gly Pro Gly Ile Phe
            85                  90              95
```

```
Gly Pro Gly Ile Phe Gly Pro Gly Ser Ser Ala Ala Ala Ala Ala Gly
            100                 105             110
```

```
Thr Tyr Gly Ser Gly Pro Gly Ile Phe Gly Pro Tyr Gly Ser Ala Ala
        115                 120             125
```

```
Ala Ala Ala Gly Pro Gly Ser Gly Thr Tyr Gly Thr Gly Pro Tyr Gly
    130                 135             140
```

```
Pro Gly Ala Ser Gly Pro Gly Thr Tyr Gly Pro Gly Ile Phe Gly Pro
145                 150             155                 160
```

```
Ser Ala Ser Ala Ala Ala Ala Ala Gly Ser Gly Ile Phe Gly Pro Gly
            165                 170             175
```

```
Thr Tyr Gly Pro Tyr Ala Ser Ala Ala Ala Ala Ala Gly Thr Tyr Gly
        180                 185             190
```

Ser Gly Pro Gly Ile Phe Gly Pro Tyr Gly Pro Gly Thr Ser Gly Ser
        195                 200             205

Gly Ile Phe Gly Pro Gly Ile Phe Gly Pro Tyr Ala Ser Ala Ala Ala
        210                 215             220

Ala Ala Gly Pro Gly Ile Phe Gly Pro Tyr Gly Pro Gly Ser Ser Ala
225             230                 235                 240

Ala Ala Ala Ala Gly Thr Tyr Gly Tyr Gly Pro Gly Ile Phe Gly Pro
            245                 250                 255

Tyr Gly Pro Gly Ala Ser Gly Thr Gly Pro Gly Ser Gly Thr Tyr Gly
        260                 265                 270

Pro Gly Ile Phe Gly Pro Gly Thr Ser Ala Ala Ala Ala Ala Gly Pro
        275                 280                 285

Gly Ile Phe Gly Pro Tyr Gly Pro Gly Ala Ser Ala Ala Ala Ala Ala
        290                 295                 300

Gly Thr Tyr Gly Pro Gly Ile Phe Gly Pro Gly Thr Tyr Gly Pro Gly
305             310                 315                 320

Ser Ser Gly Pro Gly Ile Phe Gly Pro Tyr Gly Pro Gly Ser Ser Ala
            325                 330                 335

Ala Ala Ala Ala Gly Thr Tyr Gly Pro Gly Ile Phe Gly Pro Tyr Gly
            340                 345                 350

Pro Gly Thr Ser Ala Ala Ala Ala Ala Gly Thr Tyr Ile Phe Gly Pro
        355                 360                 365

Gly Ile Phe Gly Pro Tyr Gly Pro Gly Ala Ser Gly Pro Gly Ile Phe
        370                 375                 380

Gly Pro Tyr Gly Pro Gly Ala Ser Ala Ala Ala Ala Gly Pro Gly
385                 390                 395                 400

Thr Tyr Gly Pro Gly Ile Phe Gly Pro Ser Ala Ser Ala Ala Ala Ala
            405                 410                 415

Ala Gly Thr Tyr Gly Ser Gly Pro Gly Thr Tyr Gly Pro Tyr Gly Pro
        420                 425                 430

Gly Thr Ser Gly Pro Gly Ser Gly Ile Phe Gly Thr Gly Pro Tyr Gly
        435                 440                 445

```
Pro Gly Ala Ser Ala Ala Ala Ala Ala Gly Thr Tyr Gly Pro Gly Ile
    450             455             460

Phe Gly Pro Tyr Gly Pro Gly Thr Ser Ala Ala Ala Ala Ala Gly Pro
465             470             475             480

Gly Ser Gly Thr Tyr Gly Pro Gly Ala Ser Gly Thr Gly Pro Gly Ser
            485             490             495

Gly Thr Tyr Gly Pro Gly Ile Phe Gly Pro Gly Thr Ser Ala Ala Ala
            500             505             510

Ala Ala Gly Thr Tyr Ile Phe Gly Pro Gly Ile Phe Gly Pro Tyr Gly
            515             520             525

Pro Gly Ala Ser Ala Ala Ala Ala Ala Gly Thr Tyr Gly Ser Gly Pro
    530             535             540

Gly Ile Phe Gly Pro Tyr Gly Pro Gly Thr Ser Gly Ser Gly Ile Phe
545             550             555             560

Gly Pro Gly Ile Phe Gly Pro Tyr Ala Ser Ala Ala Ala Ala Ala Gly
            565             570             575

Pro Gly Ser Gly Ile Phe Gly Pro Gly Ala Ser
            580             585
```

```
<210>  43
<211>  601
<212>  PRT
<213>  Artificial Sequence

<220>
<223>  PRT917

<400>  43
```

```
Met His His His His His His Ser Ser Gly Ser Ser Gly Pro Gly Leu
1               5               10              15

Ile Gly Pro Tyr Gly Pro Gly Ala Ser Ala Ala Ala Ala Ala Gly Val
            20              25              30

Asn Gly Pro Gly Ser Gly Leu Ile Gly Pro Gly Val Ser Gly Val Tyr
            35              40              45

Gly Pro Gly Leu Ile Gly Pro Gly Leu Ile Gly Pro Gly Ser Ser Ala
    50              55              60
```

Ala Ala Ala Ala Gly Pro Gly Val Tyr Gly Pro Gly Leu Ile Gly Pro
65              70          75              80

Ser Ala Ser Ala Ala Ala Ala Ala Gly Pro Gly Ser Gly Leu Ile Gly
                85              90              95

Pro Gly Ala Ser Gly Val Tyr Gly Pro Gly Leu Ile Gly Pro Gly Leu
        100             105             110

Ile Gly Pro Gly Ser Ser Ala Ala Ala Ala Gly Val Tyr Gly Ser
        115             120             125

Gly Pro Gly Leu Ile Gly Pro Tyr Gly Ser Ala Ala Ala Ala Ala Gly
        130             135             140

Pro Gly Ser Gly Val Tyr Gly Val Gly Pro Tyr Gly Pro Gly Ala Ser
145             150             155             160

Gly Pro Gly Val Tyr Gly Pro Gly Leu Ile Gly Pro Ser Ala Ser Ala
        165             170             175

Ala Ala Ala Ala Gly Ser Gly Leu Ile Gly Pro Gly Val Tyr Gly Pro
        180             185             190

Tyr Ala Ser Ala Ala Ala Ala Gly Val Tyr Gly Ser Gly Pro Gly
        195             200             205

Leu Ile Gly Pro Tyr Gly Pro Gly Val Ser Gly Ser Gly Leu Ile Gly
        210             215             220

Pro Gly Leu Ile Gly Pro Tyr Ala Ser Ala Ala Ala Ala Gly Pro
225             230             235             240

Gly Leu Ile Gly Pro Tyr Gly Pro Gly Ser Ser Ala Ala Ala Ala Ala
        245             250             255

Gly Val Tyr Gly Tyr Gly Pro Gly Leu Ile Gly Pro Tyr Gly Pro Gly
        260             265             270

Ala Ser Gly Val Asn Gly Pro Gly Ser Gly Val Tyr Gly Pro Gly Leu
        275             280             285

Ile Gly Pro Gly Val Ser Ala Ala Ala Ala Gly Pro Gly Leu Ile
        290             295             300

Gly Pro Tyr Gly Pro Gly Ala Ser Ala Ala Ala Ala Gly Val Tyr
305             310             315             320

```
Gly Pro Gly Leu Ile Gly Pro Gly Val Tyr Gly Pro Gly Ser Ser Gly
                325                 330                 335

Pro Gly Leu Ile Gly Pro Tyr Gly Pro Gly Ser Ser Ala Ala Ala Ala
                340                 345                 350

Ala Gly Val Tyr Gly Pro Gly Leu Ile Gly Pro Tyr Gly Pro Gly Val
                355                 360                 365

Ser Ala Ala Ala Ala Ala Gly Val Tyr Leu Ile Gly Pro Gly Leu Ile
    370                 375                 380

Gly Pro Tyr Gly Pro Gly Ala Ser Gly Pro Gly Leu Ile Gly Pro Tyr
385                 390                 395                 400

Gly Pro Gly Ala Ser Ala Ala Ala Ala Ala Gly Pro Gly Val Tyr Gly
                405                 410                 415

Pro Gly Leu Ile Gly Pro Ser Ala Ser Ala Ala Ala Ala Ala Gly Val
                420                 425                 430

Tyr Gly Ser Gly Pro Gly Val Tyr Gly Pro Tyr Gly Pro Gly Val Ser
        435                 440                 445

Gly Pro Gly Ser Gly Leu Ile Gly Val Gly Pro Tyr Gly Pro Gly Ala
        450                 455                 460

Ser Ala Ala Ala Ala Ala Gly Val Tyr Gly Pro Gly Leu Ile Gly Pro
465                 470                 475                 480

Tyr Gly Pro Gly Val Ser Ala Ala Ala Ala Ala Gly Pro Gly Ser Gly
                485                 490                 495

Val Tyr Gly Pro Gly Ala Ser Gly Val Asn Gly Pro Gly Ser Gly Val
        500                 505                 510

Tyr Gly Pro Gly Leu Ile Gly Pro Gly Val Ser Ala Ala Ala Ala Ala
        515                 520                 525

Gly Val Tyr Leu Ile Gly Pro Gly Leu Ile Gly Pro Tyr Gly Pro Gly
    530                 535                 540

Ala Ser Ala Ala Ala Ala Ala Gly Val Tyr Gly Ser Gly Pro Gly Leu
545                 550                 555                 560

Ile Gly Pro Tyr Gly Pro Gly Val Ser Gly Ser Gly Leu Ile Gly Pro
                565                 570                 575
```

```
Gly Leu Ile Gly Pro Tyr Ala Ser Ala Ala Ala Ala Ala Gly Pro Gly
        580             585             590


Ser Gly Leu Ile Gly Pro Gly Ala Ser
        595             600


<210>  44
<211>  598
<212>  PRT
<213>  Artificial Sequence

<220>
<223>  PRT1028

<400>  44

Met His His His His His His Ser Ser Gly Ser Ser Gly Pro Gly Ile
1               5               10              15


Phe Gly Pro Tyr Gly Pro Gly Ala Ser Ala Ala Ala Ala Ala Gly Thr
        20              25              30


Gly Pro Gly Ser Gly Ile Phe Gly Pro Gly Thr Ser Gly Thr Tyr Gly
        35              40              45


Pro Gly Ile Phe Gly Pro Gly Ile Phe Gly Pro Gly Ser Ser Ala Ala
        50              55              60


Ala Ala Ala Gly Pro Gly Thr Tyr Gly Pro Gly Ile Phe Gly Pro Ser
65              70              75              80


Ala Ser Ala Ala Ala Ala Ala Gly Pro Gly Ser Gly Ile Phe Gly Pro
                85              90              95


Gly Ala Ser Gly Thr Tyr Gly Pro Gly Ile Phe Gly Pro Gly Ile Phe
        100             105             110


Gly Pro Gly Ser Ser Ala Ala Ala Ala Ala Gly Thr Tyr Gly Ser Gly
        115             120             125


Pro Gly Ile Phe Gly Pro Tyr Gly Ser Ala Ala Ala Ala Gly Pro
        130             135             140


Gly Ser Gly Thr Tyr Gly Thr Gly Pro Tyr Gly Pro Gly Ala Ser Gly
145             150             155             160


Pro Gly Thr Tyr Gly Pro Gly Ile Phe Gly Pro Ser Ala Ser Ala Ala
                165             170             175
```

Ala Ala Ala Gly Ser Gly Ile Phe Gly Pro Gly Thr Tyr Gly Pro Tyr
            180                 185                 190

Ala Ser Ala Ala Ala Ala Ala Gly Thr Tyr Gly Ser Gly Pro Gly Ile
            195                 200                 205

Phe Gly Pro Tyr Gly Pro Gly Thr Ser Gly Ser Gly Ile Phe Gly Pro
            210                 215                 220

Gly Ile Phe Gly Pro Tyr Ala Ser Ala Ala Ala Ala Ala Gly Pro Gly
225                 230                 235                 240

Ile Phe Gly Pro Tyr Gly Pro Gly Ser Ser Ala Ala Ala Ala Ala Gly
            245                 250                 255

Thr Tyr Gly Tyr Gly Pro Gly Ile Phe Gly Pro Tyr Gly Pro Gly Ala
            260                 265                 270

Ser Gly Thr Gly Pro Gly Ser Gly Thr Tyr Gly Pro Gly Ile Phe Gly
            275                 280                 285

Pro Gly Thr Ser Ala Ala Ala Ala Ala Gly Pro Gly Ile Phe Gly Pro
            290                 295                 300

Tyr Gly Pro Gly Ala Ser Ala Ala Ala Ala Gly Thr Tyr Gly Pro
305                 310                 315                 320

Gly Ile Phe Gly Pro Gly Thr Tyr Gly Pro Gly Ser Ser Gly Pro Gly
            325                 330                 335

Ile Phe Gly Pro Tyr Gly Pro Gly Ser Ser Ala Ala Ala Ala Ala Gly
            340                 345                 350

Thr Tyr Gly Pro Gly Ile Phe Gly Pro Tyr Gly Pro Gly Thr Ser Ala
            355                 360                 365

Ala Ala Ala Ala Gly Thr Tyr Ile Phe Gly Pro Gly Ile Phe Gly Pro
            370                 375                 380

Tyr Gly Pro Gly Ala Ser Gly Pro Gly Ile Phe Gly Pro Tyr Gly Pro
385                 390                 395                 400

Gly Ala Ser Ala Ala Ala Ala Ala Gly Pro Gly Thr Tyr Gly Pro Gly
            405                 410                 415

Ile Phe Gly Pro Ser Ala Ser Ala Ala Ala Ala Ala Gly Thr Tyr Gly
            420                 425                 430

162

```
Ser Gly Pro Gly Thr Tyr Gly Pro Tyr Gly Pro Gly Thr Ser Gly Pro
        435                 440             445

Gly Ser Gly Ile Phe Gly Thr Gly Pro Tyr Gly Pro Gly Ala Ser Ala
    450                 455             460

Ala Ala Ala Ala Gly Thr Tyr Gly Pro Gly Ile Phe Gly Pro Tyr Gly
465             470                 475                 480

Pro Gly Thr Ser Ala Ala Ala Ala Ala Gly Pro Gly Ser Gly Thr Tyr
            485                 490             495

Gly Pro Gly Ala Ser Gly Thr Gly Pro Gly Ser Gly Thr Tyr Gly Pro
        500                 505             510

Gly Ile Phe Gly Pro Gly Thr Ser Ala Ala Ala Ala Ala Gly Thr Tyr
    515                 520                 525

Ile Phe Gly Pro Gly Ile Phe Gly Pro Tyr Gly Pro Gly Ala Ser Ala
    530                 535             540

Ala Ala Ala Ala Gly Thr Tyr Gly Ser Gly Pro Gly Ile Phe Gly Pro
545             550                 555                 560

Tyr Gly Pro Gly Thr Ser Gly Ser Gly Ile Phe Gly Pro Gly Ile Phe
            565                 570                 575

Gly Pro Tyr Ala Ser Ala Ala Ala Ala Ala Gly Pro Gly Ser Gly Ile
        580                 585             590

Phe Gly Pro Gly Ala Ser
        595
```